# EUROPEAN PATENT APPLICATION

(11) **EP 4 464 722 A1**
(43) Date of publication of application: **20.11.2024**
(21) Application number: 23737190.1
(22) Date of filing: 09.01.2023
(51) Int. Cl.: C07K 16/28, A61K 39/00

(54) **ANTIBODY AND USE THEREOF**

(30) Priority: 10.01.2022 WO PCT/CN2022/071080; 01.04.2022 CN 202210352672; 03.01.2023 CN 202310005123
(71) Applicant: Nanjing Leads Biolabs Co., Ltd., Jiangbei New Area Nanjing Jiangsu 210031 (CN)
(72) Inventor: SUN, Jianming, Nanjing, Jiangsu 210019 (CN); HUANG, Xiao, Nanjing, Jiangsu 210019 (CN); QIN, Yurong, Nanjing, Jiangsu 210019 (CN); ZHANG, Peng, Nanjing, Jiangsu 210019 (CN); FANG, Xingxing, Nanjing, Jiangsu 210019 (CN); SHANG, Hongyan, Nanjing, Jiangsu 210019 (CN); LING, Hong, Nanjing, Jiangsu 210019 (CN)
(74) Representative: Bianchetti & Minoja with Trevisan & Cuonzo IPS SRL
(86) International application number: PCT/CN2023/071314
(87) International publication number: WO 2023/131328

(57) **Abstract**

Provided in the present invention is an antibody or an antigen binding protein thereof. The antibody or the antigen binding protein thereof can specifically bind to human CD3 or human GPRC5D protein or MUC16, or specifically bind to human CD3 and a second antigen. Further provided in the present invention are a nucleic acid molecule encoding the antibody, an expression vector for expressing the antibody, a host cell and a preparation method therefor. Also provided in the present invention is a diagnosis and treatment method using the antibody of the invention.

## Description

### TECHNICAL FIELD

The present invention provides an antibody or an antigen-binding protein thereof, and an asymmetric bispecific antibody constructed based thereon. The present invention further provides a nucleic acid molecule encoding the antibody, an expression vector for expressing the antibody, a host cell, and a preparation method therefor. The present invention also provides a diagnosis or treatment method using the antibody of the present invention.

### BACKGROUND OF THE INVENTION

CD3 is a homodimeric or heterodimeric antigen expressed on T cells, which binds to the T cell receptor (TCR) complex and is required for the activation of T cells. Functional CD3 is formed by dimeric association of two of four different chains: ε, ζ, δ, and γ. The CD3 dimer arrangements include γ/ε, δ/ε, and ζ/ζ. Antibodies directed against CD3 have been shown to cluster CD3 on T cells, thereby causing the activation of T cells in a manner similar to the engagement of the TCR by peptide-loaded MHC molecules. Thus, anti-CD3 antibodies have been proposed for therapeutic purposes involving the activation of T cells. In addition, it has been proposed that bispecific antibodies capable of binding to CD3 and targeting tumor surface antigens are capable of engaging tumor cells with T cells, thereby directly activating the T cells, releasing granzymes, performs, and cytokines to kill tumors, and then achieving the therapeutic purpose of inhibiting the tumors.

G protein-coupled receptor family C group 5 member D (GPRC5D), an orphan receptor, was found to be highly expressed in plasma cells of patients with multiple myeloma and associated with the survival rate of the patients (Atamaniuk, J., et al. (2012). "Overexpression of G protein-coupled receptor 5D in the bone marrow is associated with poor prognosis in patients with multiple myeloma." Eur J Clin Invest 42(9): 953-960). Further studies show that GPRC5D is expressed on the surface of plasma cells in normal human blood cells; the expression of GPRC5D is negative on the surface of other blood cells; GPRC5D is highly expressed on the surface of CD38+ CD138+ plasma cells isolated from the patients with multiple myeloma (Kodama, T., et al. (2019). "Anti-GPRC5D/CD3 Bispecific T-Cell-Redirecting Antibody for the Treatment of Multiple Myeloma." Mol. Cancer Ther. 18(9): 1555-1564); the analysis of multiple myeloma cells of the patients shows that GPRC5D has no correlation with the expression of BCMA (Smith, E. L., et al. (2019). "GPRC5D is a target for the immunotherapy of multiple myeloma with rationally designed CAR T cells." Sci Transl Med 11(485)) (Pillarisetti, K., et al. (2020). "A T-cell-redirecting bispecific G-protein-coupled receptor class 5 member D × CD3 antibody to treat multiple myeloma." Blood 135(15): 1232-1243). Therefore, GPRC5D can be used as a tumor-specific target for multiple myeloma. In addition, GPRC5D can also be used as a tumor-specific target for other tumors.

MUC16 protein, since its discovery, has been used as a specific marker for ovarian carcinoma, and is also thought to be associated with poor prognosis for ovarian carcinoma. MUC16 is a transmembrane glycoprotein with a large molecular weight consisting of an extracellular domain, a transmembrane segment, and an intracellular portion; the extracellular domain is in two parts, the first part of which is highly glycosylated and the second part of which consists of approximately 60 tandem repeat domains, wherein each repeat domain has 156 amino acids. The N-terminus of the MUC16 protein contains 56 SEA domains, and the MUC16 protein is truncated at the first or second SEA domain in the membrane-proximal end of the extracellular domain and releases free CA125 (Das, S. and S. K. Batra (2015). "Understanding the Unique Attributes of MUC16 (CA125): Potential Implications in Targeted Therapy." Cancer Res 75(22): 4669-4674, CA125 enters the circulation by being shed from the full-length MUC16 at the cell surface, Das, S., et al. (2015). "Membrane proximal ectodomain cleavage of MUC16 occurs in the acidifying Golgi/post-Golgi compartments" Sci Rep 5: 9759). In addition to ovarian carcinoma, CA125 level is also found to be elevated in other tumors, such as breast cancer, pancreatic cancer, colorectal cancer, non-small cell lung cancer, and the like. There are studies showing that MUC16 promotes the invasion and metastasis of tumors and inhibits immune response. Therefore, based on this, MUC16 may be a potential target for treating cancers. Although therapies targeting MUC16 have been in clinical research for a long time, none of the clinical results have been as expected (Aithal, A., et al. (2018), "MUC16 as a novel target for cancer therapy", Expert Opin Ther Targets, 22(8): 675-686). The main reason is that most of the developed antibodies can bind to circulating free CA125, thereby reducing the binding amount of the antibodies to membrane MUC16, and then reducing the tumor-penetrating ability of the antibodies.

Immune checkpoint inhibitors (ICIs) have recently opened a new era in tumor immunotherapy by blocking the binding of immune checkpoint molecules (e.g., PD-1 or CTLA4) to ligands to relieve immunosuppression, thereby restoring T cell function. Until now, immune checkpoint inhibitor drugs have been approved for marketing in several indications, such as melanoma, non-small cell lung cancer, lymphoma, and the like (Pico de Coana, Y, et al. (2015). "Checkpoint blockade for cancer therapy: revitalizing a suppressed immune system." Trends Mol Med 21(8): 482-491) (Bu, X., et al. (2017). "Immune Checkpoint Blockade in Breast Cancer Therapy." Adv Exp Med Biol 1026: 383-402) (Hargadon, K. M., et al. (2018). "Immune checkpoint blockade therapy for cancer: An overview of FDA-approved immune checkpoint inhibitors." Int Immunopharmacol 62: 29-39). Nevertheless, only a small number of patients with ovarian carcinoma can benefit from the treatment with the immune checkpoint inhibitor drugs (Borella, F., et al. (2020). "Immune Checkpoint Inhibitors in Epithelial Ovarian Cancer: An Overview on Efficacy and Future Perspectives." Diagnostics (Basel) 10(3)) (Lorusso, D., et al. (2020). "Emerging role of immune checkpoint inhibitors in the treatment of ovarian cancer." Expert Opin Emerg Drugs 25(4): 445-453). The immune system primarily eliminates tumor cells in the body through T cells. However, the tumor cells escape the killing of the immune system by a variety of methods. T-cell engagers are a class of bispecific antibodies that can simultaneously bind to CD3 on T cells and tumor-associated antigens (TAAs) on tumor cells (Labrijn, A. F., et al. (2019). "Bispecific antibodies: a mechanistic review of the pipeline." Nat Rev Drug Discov 18(8): 585-608.) (Ellerman, D. (2019). "Bispecific T-cell engagers: Towards understanding variables influencing the in vitro potency and tumor selectivity and their modulation to enhance their efficacy and safety" Methods 154: 102-117). Such bispecific antibodies can simultaneously bind to T cells and tumor cells, drive the formation of immune synapses among the cells, and activate the T cells to kill the tumor cells. Blinatumomab, as the first marketed T-cell engager, has confirmed the feasibility of this theory, and more T-cell engagers have been in early development and widely used for the treatment of hematological tumors and solid tumors (Goebeler, M. E. and R. C. Bargou (2020). "T cell-engaging therapies - BiTEs and beyond." Nat Rev Clin Oncol 17(7): 418-434).

Accordingly, there is a need in the art to develop an antibody specifically binding to CD3, an antibody specifically binding to CD3 and another antigen, and a bispecific antibody constructed based thereon, for use in the treatment of cancer.

### SUMMARY OF THE INVENTION

The present invention provides an anti-CD3 antibody or an antigen-binding fragment thereof targeting human CD3, which has the following advantages:
1) binding to human CD3 and target cells expressing human CD3 with high specificity;
2) suitable for use as a genetically engineered component; and
3) low immunogenicity.

The present invention further provides an anti-MUC16 antibody targeting human MUC16, which has one or more of the following advantages:
(1) binding to MUC16 (e.g., human or monkey MUC16) and target cells expressing human MUC16 with high affinity;
(2) not binding to circulating free CA125;
(3) suitable for use as a genetically engineered component;
(4) low immunogenicity;
(5) high expression purity; and
(6) still having high affinity after humanization.

The present invention provides a novel anti-GPRC5D antibody and a bispecific antibody constructed by using same and a CD3 antibody. In some embodiments, the anti-GPRC5D antibody of the present invention has high binding affinity for human GPRC5D and is capable of recognizing both human and monkey (e.g., cynomolgus monkey) GPRC5D.

In some embodiments, the anti-GPRC5D antibody can specifically bind to GPRC5D (e.g., human GPRC5D and/or monkey (e.g., cynomolgus monkey) GPRC5D) with high affinity, and the binding activity is significantly superior to that of a control antibody GC5B596.

The present invention further provides a bispecific antibody based on CD3, such as a bispecific antibody specifically binding to CD3 and MUC16.

The anti-CD3×MUC16 bispecific antibody of the present invention has one or more of the following properties:
(1) binding to human CD3 and target cells expressing human CD3 with high specificity, while binding to human MUC16 and target cells expressing human MUC16 with high affinity;
(2) not binding to circulating free CA125, while targeting more T cells to cancer cells expressing MUC16;
(3) activating T cells and eliciting cytotoxic activity against the cancer cells expressing MUC16, thereby effectively killing the cancer cells;
(4) low immunogenicity; and
(5) an excellent anti-tumor effect.

The anti-CD3/GPRC5D bispecific antibody of the present invention has one or more of the following properties, and therefore has optimistic therapeutic prospects in GPRC5D-related indications:
(1) binding to CD3 expressed on a cell (e.g., a T cell, such as a Jurkat cell);
(2) binding to human and/or monkey (e.g., cynomolgus monkey) GPRC5D, e.g., binding to GPRC5D expressed on a cell, e.g., binding to GPRC5D with high affinity;
(3) capable of activating the CD3 signaling pathway in the presence of cells expressing GPRC5D, e.g., only capable of activating the CD3 signaling pathway in the presence of the cells expressing GPRC5D;
(4) only capable of activating the CD3 signaling pathway at a high concentration in the absence of the cells expressing GPRC5D;
(5) having less non-specific activation on the CD3 signaling pathway, for example, only activating the signaling pathway at a high concentration and being concentration-dependent in cells not expressing GPRC5D (e.g., HEK293T cells);
(6) more specifically inducing T cells to kill the cells expressing GPRC5D without non-specific activation or without visible non-specific activation, for example, only killing target cells expressing GPRC5D and not killing cells not expressing GPRC5D;
(7) having better cytokine induction specificity, for example, inducing cells to release cytokines, e.g., interferons such as IFNγ, tumor necrosis factors such as TNFα, and/or interleukins such as IL-6 only in the cells expressing GPRC5D (e.g., HEK293T-GPRC5D cells);
(8) capable of specifically binding to a human and monkey (e.g., cynomolgus monkey) CD3 protein, i.e., having species cross-activity, for example, in an *in-vitro* binding assay, such as Fortebio;
(9) retaining an optimal affinity for CD3, for example, in an *in-vitro* binding assay, such as Fortebio, with an affinity K_{D} of 1-1000 nM, e.g., binding affinity K_{D} for a monkey (e.g., cynomolgus monkey) of 1-100 nM, e.g., 10-100 nM, e.g., 50-100 nM, e.g., binding affinity K_{D} for a human of 1-1000 nM, e.g., 1-100 nM, e.g., 10-100 nM, e.g., 50-100 nM, e.g., 100-1000 nM, e.g., 200-1000 nM;
(10) having a better tumor inhibitory effect, e.g., with tumor growth inhibition of 35%, 40%, 45%, 50%, 55%, 60%, 65%, 70%, 75%, 80%, 85%, 90%, 95%, or more, and even capable of allowing a tumor to completely regress; and
(11) capable of binding to human and monkey (e.g., cynomolgus monkey) GPRC5D expressed on a cell, and/or capable of binding to human and monkey (e.g., cynomolgus monkey) CD3 expressed on a cell, and having species cross-activity towards GPRC5D and/or CD3.

### BRIEF DESCRIPTION OF THE DRAWINGS

FIG. 1 shows results of the activation of a Jurkat/NFAT-luc reporter gene system by humanized anti-CD3 antibodies Roche-CD3/017-Roche-CD3, DA023AH23L2 (FIG. 1A) and 017-2 (FIG. 1B).
FIG. 2 shows the binding of murine anti-GPRC5D antibodies to cells expressing a human GPRC5D protein (top) and a cynomolgus monkey GPRC5D protein (bottom).
FIG. 3 shows the binding of humanized anti-GPRC5D antibodies to engineered cells expressing the human GPRC5D protein.
FIG. 4 shows schematic structural diagrams of bispecific molecules.
FIG. 5 shows the binding of anti-CD3/anti-GPRC5D bispecific antibodies to Jurkat cells stably expressing a human CD3 protein.
FIG. 6 shows panels A and B: the binding of anti-CD3/anti-GPRC5D bispecific antibodies to HEK293T cells stably expressing a human GPRC5D protein; panel C: the binding of the anti-CD3/anti-GPRC5D bispecific antibody Bi-29H6-6 to HEK293T cells stably expressing a cynomolgus monkey GPRC5D protein; panels D-F: the anti-CD3/GPRC5D bispecific antibody Bi-29H6-6 not binding to cells expressing GPRC5A, GPRC5B, or GPRC5C proteins.
FIG. 7 shows that the anti-CD3/GPRC5D bispecific antibody is able to activate the CD3 signaling pathway in the presence of cells expressing human GPRC5D.
FIG. 8 shows that the anti-CD3/GPRC5D bispecific antibody is unable to activate the CD3 signaling pathway in the presence of cells not expressing human GPRC5D.
FIG. 9 show that the anti-CD3/GPRC5D bispecific antibody induces PBMC-specific killing of tumor cells expressing GPRC5D.
FIG. 10 shows the killing of unrelated target cells Raji by anti-CD3/GPRC5D bispecific antibody-mediated PBMC.
FIG. 11 shows T-cell release assays of secretion factors IFNγ (panel A), IL-6 (panel B), and TNFα (panel C) by the anti-CD3/GPRC5D bispecific antibody in an NCI-H929 system.
FIG. 12 shows T-cell release assays of secretion factors IFNγ (panel A), IL-6 (panel B), and TNFα (panel C) by the anti-CD3/GPRC5D bispecific antibody in an Raji system not expressing GPRC5D.
FIG. 13 shows panel A: an anti-tumor effect of the anti-CD3/anti-GPRC5D bispecific antibody in a human CD3E transgenic mouse MC38-huGPRC5D model; panel B: an anti-tumor effect of the anti-CD3/anti-GPRC5D bispecific antibody in an NCG mouse transplanted with human PBMC and inoculated with an NCI-H929 tumor model.
FIG. 14 shows SDS-PAGE electrophoresis results of a murine anti-MUC16 antibody 776.1 and mutants thereof (the left panel shows non-reduced SDS-PAGE electrophoresis results, and the right panel shows reduced SDS-PAGE electrophoresis results).
FIG. 15 shows flow cytometry assay results of the binding of the murine anti-human MUC16 antibody 776.1 and mutants thereof to 293T cells expressing a human Muc16 protein.
FIG. 16 shows flow cytometry assay results of the binding of the murine anti-human MUC16 antibody 776.1 and mutants thereof to 293T cells expressing a rhesus monkey MUC16 protein.
FIG. 17 shows ELISA results of the binding of a humanized anti-MUC-16 antibody Hu-L4H7 to 293T cells expressing a human MUC16 protein.
FIG. 18 shows ELISA results of the humanized anti-MUC-16 antibody Hu-L4H7 not binding to human CA125.
FIG. 19 shows immunohistochemical assay results of MUC16 and CD8 in tumor tissues.
FIG. 20 shows schematic structural diagrams of bispecific antibody molecules.
FIG. 21 shows the binding of the anti-CD3/anti-MUC16 bispecific antibodies to OVCAR3 cells.
FIG. 22 shows the binding of the anti-CD3/anti-MUC16 bispecific antibodies to 293T/rhesus monkey MUC16 cells.
FIG. 23 shows the binding of the anti-CD3/anti-MUC16 bispecific antibodies to Jurkat cells.
FIG. 24 shows that the anti-CD3/anti-MUC16 bispecific antibody activates Jurkat/NFAT-luc cells in the presence of OVCAR3 tumor cells.
FIG. 25 shows that the anti-CD3/anti-MUC16 bispecific antibodies do not activate Jurkat/NFAT-luc cells in the presence of 293T cells.
FIG. 26 shows panel A: the killing of tumor cells by T cells specifically mediated by the anti-CD3/anti-MUC16 bispecific antibodies; panel B: the activity of the anti-CD3/anti-MUC16 bispecific antibodies in specifically mediating T cells to kill OVCAR3 tumor cells after incubation for 72 h.
FIG. 27 shows the results of cytokine release induced after the activation of T cells by the anti-CD3/anti-MUC16 bispecific antibodies, in which the release of cytokines IFNγ, TNFα, and IL-6 factors in co-culture with OVCAR3 or 293T cells are separately compared.
FIG. 28A shows tumor volume increase results in each group after administration.
FIG. 28B shows relative tumor volume increase results in each group after administration.
FIG. 29 shows T cell activation, exhaustion, and apoptosis assays in TDCC experiments.
FIG. 30 shows the repeated killing of tumor cells by T cells specifically mediated by the anti-CD3/anti-MUC16 bispecific antibodies.
FIG. 31 shows T cell activation, exhaustion, and apoptosis assays in TDCC repeated killing experiments.

### DETAILED DESCRIPTION OF THE INVENTION

It should be understood that the present invention is not limited to the particular methodology, protocols, and reagents described herein, as these may vary. It should also be understood that the terminology used herein is only intended to describe specific embodiments rather than limit the scope of the present invention, which will be limited only by the appended claims.

### I. Definitions

For the purpose of explaining this specification, the following definitions will be used, and wherever appropriate, terms used in the singular form may also include the plural form, and vice versa. Unless otherwise defined, any technical and scientific term used herein has the same meaning as commonly understood by those of ordinary skill in the art to which the present invention belongs.

The term "about" used in combination with a numerical value is intended to encompass the numerical values in a range from a lower limit less than the specified numerical value by 10% to an upper limit greater than the specified numerical value by 10%.

As used herein, the term "and/or" refers to any one of the options or any two or more or all of the options.

As used herein, the term "comprise" or "include" is intended to mean that the elements, integers, or steps are included, but not to the exclusion of any other elements, integers, or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

When referring to "first" and "second" herein, it is only to distinguish two domains or two chains, and does not indicate the location of the two domains in any way.

The term "CD3" as used herein refers to an antigen expressed on a T cell as part of a multimolecular T cell receptor (TCR), i.e., a T-cell engaging antigen, T-cell surface glycoprotein CD3, which consists of a homodimer or heterodimer formed from two of the following four receptor chains: CD3-ε, CD3-δ, CD3-ζ, and CD3-γ. Human CD3-ε (hCD3ε) comprises an amino acid sequence described in UniProtKB/Swiss-Prot: P07766. Human CD3-δ (hCD3δ) comprises an amino acid sequence described in UniProtKB/Swiss-Prot: P04234. In some embodiments, the CD3 described herein refers to CD3 from a human or monkey (e.g., cynomolgus monkey).

The term "antibody binding to CD3" or "anti-CD3 antibody" as used herein includes an antibody and an antigen-binding fragment thereof that specifically recognize or bind to a single CD3 subunit (e.g., ε, δ, γ, or ζ), as well as an antibody and an antigen-binding fragment thereof that specifically recognize and bind to a dimeric complex of two CD3 subunits (e.g., γ/ε, δ/ε, and ζ/ζ CD3 dimers). The antibody and the antigen-binding fragment of the present invention may bind to soluble CD3, binding CD3, and/or CD3 expressed on the cell surface. The soluble CD3 includes native CD3 proteins and recombinant CD3 protein variants, e.g., monomeric and dimeric CD3 structures that lack a transmembrane region or otherwise do not bind to the cell membrane. In one embodiment, an antigen-binding region binding to CD3 in the anti-CD3 antibody or the antigen-binding fragment thereof (e.g., ScFv) or the bispecific antibody of the present invention may have relatively low binding activity to CD3 or cells expressing CD3 (e.g., T cells). The binding affinity of the antibody for CD3 may be detected by flow cytometry or bio-layer interferometry, such as the assays described in Example 6.5 or Example 10.2. Preferably, in the bispecific antibody molecule of the present invention, the antigen-binding region binding to CD3 has binding affinity of 1-1000 nM for human or monkey (cynomolgus monkey) CD3. In some embodiments, the antigen-binding region binding to CD3 in the anti-CD3 antibody or the antigen-binding fragment thereof or the bispecific antibody of the present invention binds to human and/or monkey (e.g., cynomolgus monkey) CD3 with relatively low binding affinity, thereby enabling the activation of human and/or monkey (e.g., cynomolgus monkey) T cells.

Effector cells include effector T cells (T lymphocytes), such as CD4+ T cells, CD8+ T cells, Th1, Th2, and regulatory T cells (Tregs). The effector cells may also include natural killer cells, macrophages, granulocytes, plasma cells, or B cells (lymphocytes).

The term "GPRC5D" refers to a tumor-associated antigen, G protein-coupled receptor family C group 5 member D (e.g., human GPRC5D protein under accession number NP_061124.1 or cynomolgus monkey GPRC5D protein under accession number XP_005570249.2). In one embodiment, the human GPRC5D protein of the present invention comprises or consists of an amino acid sequence set forth in SEQ ID NO: 57, or an amino acid sequence having at least 85%, 90%, 91%, 92%, 93%, 94%, 95%, 96%, 97%, 98%, or 99% identity. In one embodiment, the cynomolgus monkey GPRC5D protein of the present invention comprises or consists of an amino acid sequence set forth in SEQ ID NO: 58, or an amino acid sequence having at least 85%, 90%, 91%, 92%, 93%, 94%, 95%, 96%, 97%, 98%, or 99% identity. In one embodiment, an antigen-binding region binding to GPRC5D in the anti-GPRC5D antibody or the antigen-binding fragment thereof (e.g., Fab) or the bispecific antibody of the present invention has high-affinity binding activity for cells expressing human GPRC5D, e.g., has higher binding affinity than that of a control antibody (e.g., GC5B596). In one embodiment, the assay is performed by flow cytometry, such as the assay experiment described in Example 6.2. In one embodiment, the antigen-binding region binding to GPRC5D in the anti-GPRC5D antibody or the antigen-binding fragment thereof (e.g., Fab) or the bispecific antibody of the present invention has cross-reactivity to human and monkey (e.g., cynomolgus monkey) GPRC5D, i.e., it is capable of binding to human and monkey (e.g., cynomolgus monkey) GPRC5D.

The terms "whole antibody", "full-length antibody", "complete antibody", and "intact antibody" are used interchangeably herein to refer to a naturally occurring glycoprotein comprising at least two heavy (H) chains and two light (L) chains interconnected by disulfide bonds. Each heavy chain consists of a heavy chain variable region (abbreviated herein as VH) and a heavy chain constant region. The heavy chain constant region consists of 3 domains, CH1, CH2, and CH3. Each light chain consists of a light chain variable region (abbreviated herein as VL) and a light chain constant region. The light chain constant region consists of one domain CL. The VH region and the VL region can be further divided into hypervariable regions (complementarity determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3, and FR4. The constant regions are not directly involved in the binding of antibodies to antigens, but exhibit a variety of effector functions. In some embodiments, the antibody heavy chain constant region HC of the present invention is a heavy chain constant region of IgG1, IgG2, IgG3 or IgG4, preferably a heavy chain constant region of IgG1. In some embodiments, the heavy chain constant region comprises an LALA mutation. In some embodiments, the heavy chain constant region comprises a D265A mutation and a P329A mutation. In some embodiments, the heavy chain constant region comprises an LALA mutation, a D265A mutation, and a P329A mutation. In some embodiments, the heavy chain constant region of the bispecific antibody molecule of the present invention comprises a "knob-in-hole mutation". In some preferred embodiments, the antibody heavy chain constant region HC of the present invention
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 45 or 48;
(ii) comprises or consists of an amino acid sequence selected from SEQ ID NO: 45 or 48; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared to an amino acid sequence selected from SEQ ID NO: 45 or 48.

In some embodiments, the antibody light chain constant region LC of the present invention is a Lambda or Kappa light chain constant region. In some embodiments, the antibody light chain constant region LC of the present invention
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 47 or 50;
(ii) comprises or consists of an amino acid sequence selected from SEQ ID NO: 47 or 50; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared to an amino acid sequence selected from SEQ ID NO: 47 or 50.

The term "antibody fragment" includes a portion of an intact antibody. In a preferred embodiment, the antibody fragment is an antigen-binding fragment.

The term "antigen-binding fragment" is a portion or segment of an intact antibody or a complete antibody that has fewer amino acid residues than the intact antibody or the complete antibody, which can bind to an antigen or compete with an intact antibody (i.e., an intact antibody from which the antigen-binding fragment is derived) for binding to an antigen. The antigen-binding fragment may be prepared by recombinant DNA techniques, or by enzymatic or chemical cleavage of an intact antibody. The antigen-binding fragments include, but are not limited to, Fab, Fab', F(ab')2, Fv, single-chain Fv, diabodies, and single-domain antibodies (sdAbs). The Fab fragment is a monovalent fragment consisting of VL, VH, CL, and CH1 domains and can be obtained, for example, by papain digestion of a complete antibody. In addition, the F(ab')2, a dimer of the Fab', is a bivalent antibody fragment produced by pepsin digestion of a portion below disulfide bonds in a hinge region of a complete antibody. The F(ab')2 can be reduced by disrupting the disulfide bonds in the hinge region under neutral conditions, and the F(ab')2 dimer is thus converted into Fab' monomers. The Fab' monomer is substantially a Fab fragment with a hinge region (for more detailed descriptions of other antibody fragments, see Fundamental Immunology, W. E. Paul (ed.), Raven Press, N.Y. (1993)). The Fv fragment consists of the VL and VH domains of a single arm of an antibody. In addition, although the two domains VL and VH of the Fv fragment are encoded by separate genes, the domains can be linked, using recombinant methods, by a synthetic linker peptide capable of making these two domains produced as a single protein chain in which the VL and VH regions are paired to form a single-chain Fv (scFv). The antibody fragment can be obtained by a chemical method, a recombinant DNA method, or a protease digestion method.

The "Fab fragment" and "Fab" are used interchangeably herein to refer to an immunoglobulin fragment consisting of two polypeptide chains and comprising an immunoglobulin heavy chain variable domain VH, a heavy chain constant domain CH1, a light chain variable domain VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and the other constant region selected from CL and CH1, wherein the VH and VL domains are paired to form an antigen-binding site. Herein, a Fab chain comprising a heavy chain constant region CH1 is also referred to as a "Fab heavy chain"; correspondingly, a Fab chain comprising a light chain constant region CL is also referred to as a "Fab light chain".

The term "target" refers to the bound substance against which the binding molecule is directed. The target may be an antigen, or may be a ligand or a receptor.

The term "antigen" refers to a molecule that induces an immune response. Such an immune response may involve antibody production or activation of specific immune cells, or both. Those skilled will understand that any macromolecules, including essentially all proteins or peptides, can be used as antigens. In addition, an antigen may be derived from recombinant or genomic DNA. As used herein, the term "epitope" refers to a portion of an antigen that specifically interacts with an antibody molecule.

The term "target-binding region" as used herein refers to a portion of a multispecific binding molecule, e.g., a bispecific binding molecule, that binds to a particular target or an antigen. The target-binding region may be, for example, an antibody or immunoglobulin per se or an antibody fragment. Such target-binding region may or may not have a tertiary structure independent of the remainder of the bispecific antibody molecule, and may or may not bind to its target as a separate entity. The target-binding region may also be a receptor or a ligand, or a domain of a receptor capable of binding to a ligand. In the case of multispecific antibodies or bispecific antibodies, the "target-binding region" is also referred to as the "antigen-binding region". In one embodiment, the antigen-binding region used in the bispecific antibody molecule of the present invention comprises a VH/VL pair consisting of a light chain variable region (VL) and a heavy chain variable region (VH) of the antibody, and the VH/VL pair may be contained in a single polypeptide chain (e.g., scFv) or in two separate polypeptide chains (e.g., contained in a Fab heavy chain and a Fab light chain, respectively).

As used herein, the term "monospecific" antibody refers to an antibody having one or more binding sites, each of which binds to the same epitope of the same antigen. As used herein, the term "multispecific" antibody refers to an antibody having at least two antigen-binding sites, wherein at least one antigen site binds to a different antigenic epitope, e.g., a different epitope on the same antigen or a different epitope on a different antigen, relative to the remaining antigen-binding sites. For example, the present invention provides an antibody against GPRC5D, an antibody against CD3, and a bispecific antibody against GPRC5D and CD3. The present invention further provides a bispecific antibody against CD3 and MUC16.

The term "immunoglobulin molecule" refers to a protein having a structure of a naturally occurring antibody. For example, an IgG is a heterotetrameric glycoprotein of about 150,000 Daltons consisting of two light chains and two heavy chains which are disulfide-bonded. Each immunoglobulin heavy chain has a heavy chain variable region (VH), also called a heavy chain variable domain, followed by three heavy chain constant domains (CH1, CH2, and CH3), from N-terminus to C-terminus. Similarly, each immunoglobulin light chain has a light chain variable region (VL), also called a light chain variable domain, followed by a light chain constant domain (CL) from N-terminus to C-terminus. The heavy chains of an immunoglobulin can be assigned to one of five classes, α (IgA), δ (IgD), ε (IgE), γ (IgG), or µ (IgM), in which some classes can be further divided into subclasses such as γ1 (IgG1), γ2 (IgG2), γ3 (IgG3), γ4 (IgG4), α1 (IgA1), and α2 (IgA2). The light chains of an immunoglobulin can be divided into one of the two classes, κ or λ, based on the amino acid sequence of constant domains thereof. The IgG immunoglobulin consists essentially of two Fab molecules and two dimerized Fc regions (Fc dimers) linked by an immunoglobulin hinge region.

The term "variable region" or "variable domain" refers to a domain of a heavy chain or light chain of an antibody involved in the binding of the antibody to an antigen. Variable regions of heavy and light chains of native antibodies generally have similar structures, wherein each domain comprises four conserved framework regions (FRs) and three complementarity determining regions.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2 and LCDR3. In a given amino acid sequence of a light chain variable region or a heavy chain variable region, the exact amino acid sequence boundaries of the CDRs may be determined using any one or a combination of many well-known antibody CDR assignment schemes including, for example, Chothia based on the three-dimensional structure of antibodies and the topology of CDR loops (Chothia et al., (1989) Nature, 342: 877-883; Al-Lazikani et al., "Standard conformations for the canonical structures of immunoglobulins", Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

The following are the regional ranges of the CDRs defined using the kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H3 5B |
| HCDR1 (Chothia numbering system) | H31-H35 | H26-H35 | H26-H32 | H30-H35 | H26-H3 5 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

Unless otherwise stated, the term "CDR" or "CDR sequence" used herein encompasses CDR sequences determined by any one of the schemes described above. CDRs can also be determined based on having the same Kabat numbering positions as a reference CDR sequence (e.g., any of the exemplary CDRs of the present invention). Unless otherwise stated, residue positions of an antibody variable region (including heavy chain variable region residues and light chain variable region residues) used herein are numbered according to the Kabat numbering system (Kabat et al., Sequences of Proteins of Immunological Interest, 5th Ed., Public Health Service, National Institutes of Health, Bethesda, Md. (1991)).

In one embodiment, the HCDRs and LCDRs in the antibody of the present invention are determined according to the Kabat scheme.

It should be noted that the boundaries of the CDRs of the variable regions of the same antibody obtained based on different assignment schemes may differ. That is, CDR sequences of the variable regions of the same antibody defined under different assignment schemes are different. Therefore, when it comes to defining an antibody with specific CDR sequences defined in the present invention, the scope of antibody also encompasses such antibodies whose variable region sequences comprise the specific CDR sequences, but having claimed CDR boundaries different from the specific CDR boundaries defined by the present invention due to a different scheme (e.g., different assignment scheme rules or their combinations) applied.

The term "Fc domain", "Fc region", or "Fc fragment" is used herein to define a C-terminus region of an immunoglobulin heavy chain, which comprises at least a portion of a constant region. The term includes Fc regions of native sequences and variant Fc regions. A native immunoglobulin "Fc domain" comprises two or three constant domains, i.e., a CH2 domain, a CH3 domain, and an optional CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third, and the fourth constant domains (CH2 domain, CH3 domain, and CH4 domain) derived from two heavy chains of IgM and IgE antibodies. Unless otherwise stated herein, amino acid residues in the Fc region or heavy chain constant region are numbered according to the EU numbering system (also known as the EU index) as described in, for example, Edelman, G.M. et al., Proc. Natl. Acad. USA, 63, 78-85 (1969) (https://pubmed.ncbi.nlm.nih.gov/5257969/), see also http://www.imgt.org/IMGTScientificChart/Numbering/Hu_IGHGnber.html. Herein, the terms "Fc domain", "Fc region", or "Fc fragment" does not comprise a heavy chain variable region VH and a light chain variable region VL as well as a heavy chain constant region CH1 and a light chain constant region CL of an immunoglobulin, but in some cases, it can comprise a hinge region at the N-terminus of the heavy chain constant region, such as EPKSS or EPKSC. In some embodiments, the heavy chain constant region Fc suitable for use in the present invention is from an antibody heavy chain constant region, for example, a constant region of human IgG1, IgG2, IgG3, or IgG4, preferably from a constant region of IgG1. In some embodiments, the Fc region comprises a mutation that reduces binding to an Fcy receptor, e.g., an LALA mutation, a D265A mutation and/or a P329A mutation, preferably an LALA mutation, a D265A mutation and a P329A mutation. In some embodiments, the Fc comprises or consists of an amino acid sequence set forth in SEQ ID NO: 46 or SEQ ID NO: 49, or an amino acid sequence having at least 85%, 90%, 91%, 92%, 93%, 94%, 95%, 96%, 97%, 98%, or 99% identity thereto. In some embodiments of the present invention, the Fc fragments form an Fc dimer by dimerization. In some embodiments, the Fc fragments form an Fc heterodimer by heterodimerization. In the case of heterodimerization of the Fc fragments into a heterodimer, the Fc fragments may comprise mutations for heterodimerization, such as a knob-in-hole mutation.

Examples of "effector functions" of immunoglobulins include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down-regulation of cell surface receptors (such as B-cell receptors), and B-cell activation.

The term "chimeric antibody" is an antibody molecule in which: (a) a constant region or a portion thereof is modified, substituted, or exchanged such that antigen-binding sites are linked to constant regions of different or modified classes, effector functions, and/or species, or disparate molecules imparting new properties (e.g., enzymes, toxins, hormones, growth factors, and drugs) to chimeric antibodies, etc.; or (b) a constant region or a portion thereof is modified, substituted, or exchanged by variable regions with different or modified antigen-binding specificities. For example, a mouse antibody can be modified by substituting its constant region with a constant region from a human immunoglobulin. Due to the substitution with a human constant region, the chimeric antibody can retain its specificity for recognizing antigens, while having reduced immunogenicity in humans as compared to the original mouse antibody.

"Humanized antibody" is an antibody that retains the antigen-specific reactivity of a non-human antibody (such as a mouse monoclonal antibody) and has lower immunogenicity when administered to humans as a therapeutic agent. This can be achieved, for example, by retaining non-human antigen-binding sites and substituting the remainder of the antibodies with their human counterparts (i.e., the portions of the constant and variable regions not involved in binding are substituted with the corresponding parts of human antibodies).

As used herein, the term "anti", "binding", or "specific binding" means that the binding effect is selective for targets or antigens and may be distinguished from unwanted or non-specific interactions. The ability of a binding site to bind to a particular target or an antigen may be determined by flow cytometry, enzyme-linked immunosorbent assay (ELISA), or conventional binding assays known in the art, such as radioimmunoassay (RIA), bio-layer interferometry, MSD assay, or surface plasmon resonance (SPR).

"Affinity" or "binding affinity" refers to the inherent binding affinity that reflects the interaction between members of a binding pair. The affinity of molecule X for its partner Y may be generally represented by the dissociation constant (K_{D}), which is a ratio of the dissociation rate constant (K_{dis}) to the association rate constant (Kₒₙ). Affinity can be measured by common methods known in the art. One specific method for measuring affinity is the ForteBio kinetic binding assay described herein.

The "percent identity (%)" of an amino acid sequence refers to the percentage of amino acid residues in a candidate sequence that are the same as those of a specific amino acid sequence shown in this specification when aligning the candidate sequence with the specific amino acid sequence shown in this specification, with gaps introduced if necessary to achieve maximum percent sequence identity and without considering any conservative replacements as part of sequence identity. In some embodiments, the present invention considers variants of the antibody molecule of the present invention that have a considerable degree of identity to the antibody molecule and sequence thereof specifically disclosed herein. For example, the identity is at least 80%, 85%, 90%, 95%, 97%, 98%, 99% or higher. The variants may comprise conservative changes, or be conservatively modified variants.

For polypeptide sequences, "conservative changes" include replacements of, deletions of, or additions to a polypeptide sequence that do not substantially change the desired functional activity of the polypeptide sequence. For example, conservative replacements often result in the replacement of an amino acid with a chemically similar amino acid. Conservative replacement tables providing functionally similar amino acids are well known in the art. 8 groups comprising amino acids that are conservatively substituted with each other are listed as follows: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M) and valine (V); 6) phenylalanine (F), tyrosine (Y), and tryptophan (W); 7) serine (S) and threonine (T); and 8) cysteine (C) and methionine (M). In some embodiments, the term "conservative sequence change" is used to refer to an amino acid modification that does not significantly affect or change the binding characteristics for an antigen of interest of the antibody molecule or binding protein molecule of the present invention comprising the amino acid sequence. For example, conservatively modified variants retain at least 80%, 85%, 90%, 95%, 98%, 99%, or more, such as 100%-110% or more, binding affinity for an antigen of interest relative to the parent antibody or binding protein.

A "knob-in-hole" mutation or "knob-into-hole" is used herein to refer to the introduction of mutations in a first Fc polypeptide and a second Fc polypeptide, respectively, using the "knob-in-hole" technique to form a protuberance ("knob") and a complementary cavity ("hole") at the interface of the first Fc polypeptide and the interface of the second Fc polypeptide. It is known in the art that the "knob-in-hole" technique enables the engineering of the interface between different chains of an antibody molecule to promote the correct association of the chains of the antibody molecule. Generally, this technique involves introducing a "protuberance/knob" at the interface of one chain, and introducing a corresponding "cavity/hole" at the interface of the other chain to be paired with, such that the protuberance can be placed at the cavity. A preferred interface comprises the CH3 domain from the heavy chain constant domains of one chain and the CH3 domain from the heavy chain constant domains of the other chain to be paired with. The protuberance can be constructed by replacing small amino acid side chains at an interface of the CH3 domain from the heavy chain constant domains of one chain with large side chains, such as tyrosine or tryptophan. The compensating cavity of the same size as, or a similar size to, the protuberance is constructed at an interface of the CH3 domain from the heavy chain constant domains of the other chain to be paired with, by replacing large amino acid side chains with small side chains, such as alanine or threonine. Another optional interface comprises a light chain CL domain and a heavy chain CH1 domain of the Fab fragment described above, and the correct heterodimerization between the two chains of the Fab fragment is promoted by constructing a protuberance-cavity interaction.

"Single-chain variable fragment" or "scFv" is used herein to refer to a single-chain antibody fragment comprising a heavy chain variable domain VH and a light chain variable domain VL linked via a linker, wherein the VH and the VL are paired to form an antigen-binding site.

Herein, antibody constant regions or antibody constant domains, including CH1, CL, and an Fc domain as well as CH2, CH3, and optional CH4 domains that constitute the Fc domain, may be selected according to the intended function of the antibody molecule. For example, the constant region may be an IgA, IgD, IgE, IgG, or IgM region, particularly an immunoglobulin constant domain of human IgG, such as a constant domain of human IgG1, IgG2, IgG3, or IgG4, preferably a constant domain of human IgG1. As an example, a Fab fragment of the antibody may comprise CH and CL constant regions derived from IgG1. As yet another example, an Fc region of the antibody may comprise CH2 and CH3 domains derived from IgG1. The immunoglobulin constant region may have a native sequence or a variant sequence.

The term "linker" as used herein refers to any molecule that enables direct connection of different portions of a bispecific binding molecule. Examples of linkers to establish covalent linkages between different portions of a molecule include peptide linkers and non-protein polymers including, but not limited to, polyethylene glycol (PEG), polypropylene glycol, polyalkylene oxide, or copolymers of polyethylene glycol and polypropylene glycol. In some embodiments, the linker is a peptide linker (also referred to as a "linker peptide") and refers to a short amino acid sequence consisting of amino acids, such as glycine (G) and/or serine (S) and/or threonine (T) residues used alone or in combination, or a hinge region derived from an immunoglobulin, which is used for linking the amino acid sequence of a first portion of a binding molecule to a second portion of the binding molecule. For example, the peptide linker may link a first target-binding region of a binding molecule to a second target-binding region. For example, the peptide linker may also link one portion of an antibody to another portion of the antibody, for example, a light chain variable region to a heavy chain variable region. Preferably, the peptide linker has a length sufficient to link two entities in a manner that maintains their conformation relative to each other without interference with the desired activities. In one embodiment, the linker peptide has a length of 5-50 amino acids, such as 10, 15, 20, 25, or 30 amino acids. In one embodiment, the linker peptide comprises amino acid sequences (GS)n, (GGS)n, (GSGGS)n, (GGGGS)n, (GGGS)n, and (GGGGS)nG, wherein n is an integer equal to or greater than 1, for example, n is an integer of 2, 3, 4, 5, 6, 7, 8, 9, or 10. Useful linkers also include glycine-alanine polymers, alanine-serine polymers, and other flexible linkers. In some embodiments, the peptide linker is (GGGGS)n, wherein n = 1, 2, 3, or 4, such as the sequence set forth in SEQ ID NO: 12.

In yet another embodiment, the linker peptide is a hinge region or a portion of a hinge region derived from an immunoglobulin, including a native hinge region or a portion thereof, or a mutated hinge region or a portion thereof. In one embodiment, the linker peptide is, for example, a hinge region or a portion thereof (e.g., EPKSC) of an immunoglobulin (e.g., IgG, such as IgG1, IgG2, IgG3, or IgG4), or it is a mutated hinge region or a portion thereof (e.g., EPKSS). Alternatively, a computer program can be used to simulate three-dimensional structures of proteins and peptides, or a suitable flexible linker peptide is rationally designed by a phage display method.

The term "half maximal effective concentration (EC₅₀)" refers to the concentration of a drug, an antibody or a toxin that induces a response of 50% between the baseline and the maximum after a particular exposure time.

The term "host cell" refers to a cell into which an exogenous polynucleotide has been introduced, including the progeny of such cells. Host cells include "transformants" and "transformed cells", which include primary transformed cells and progeny derived therefrom. Host cells are any type of cell system that can be used to produce the antibody molecule of the present invention, including eukaryotic cells, e.g., mammalian cells, insect cells, and yeast cells; and prokaryotic cells, e.g., *E. coli* cells. Host cells include cultured cells, as well as cells within a transgenic animal, a transgenic plant, or cultured plant tissue or animal tissue.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. The term "expression vector" refers to a vector comprising a recombinant polynucleotide, which comprises an expression control sequence operably linked to a nucleotide sequence to be expressed. Expression vectors contain sufficient cis-regulatory elements for expression, and other elements for expression may be provided by a host cell or in an *in vitro* expression system. Expression vectors include all those known in the art, including cosmids, plasmids (e.g., naked or contained in liposomes), and viruses (e.g., lentiviruses, retroviruses, adenoviruses, and adeno-associated viruses) incorporated into recombinant polynucleotides.

The terms "individual" and "subject" are used interchangeably and refer to a mammal. The mammals include, but are not limited to, domesticated animals (e.g., cows, sheep, cats, dogs, and horses), primates (e.g., human and non-human primates such as monkeys), rabbits and rodents (e.g., mice and rats). In particular, individuals are humans.

The term "treatment" (or "treat" or "treating") refers to slowing, interrupting, arresting, alleviating, stopping, lowering, or reversing the onset of symptoms, complications, or biochemical indications of a disease, or alleviating symptoms, or arresting or inhibiting the further progression of the disease, symptom, or disorder.

The term "prevention" (or "prevent" or "preventing") includes the inhibition of the development or progression of symptoms of a disease or disorder, or a specific disease or disorder. In some embodiments, subjects with family history of cancer are candidates for preventive regimens. Generally, in the context of cancer, the term "prevention" (or "prevent" or "preventing") refers to the administration of a drug prior to the onset of signs or symptoms of cancer, particularly in subjects at risk of cancer.

The term "therapeutic agent" described herein encompasses any substance that is effective in preventing or treating a tumor, e.g., cancer, including a chemotherapeutic agent, a cytokine, a cytotoxic agent, an additional antibody, a small molecule drug or an immunomodulatory agent (e.g., an immunosuppressant).

The term "cytotoxic agent" used herein refers to a substance that inhibits or prevents cell functions and/or causes cell death or cell destruction.

"Chemotherapeutic agents" include chemical compounds useful in the treatment of cancers or immune system diseases.

The term "small molecule drug" refers to a low molecular weight organic compound capable of regulating biological processes. "Small molecule" is defined as a molecule with a molecular weight of less than 10 kD, usually less than 2 kD, and preferably less than 1 kD. The small molecule includes but is not limited to inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation and are less likely to induce an immune response compared with large molecules.

The term "immunomodulatory agent" used herein refers to a natural or synthetic active agent or drug that suppresses or modulates an immune response. The immune response may be a humoral response or a cellular response. The immunomodulatory agent includes an immunosuppressant. In some embodiments, the immunomodulatory agent of the present invention includes an immune checkpoint inhibitor or an immune checkpoint agonist.

The term "effective amount" refers to an amount or dosage of the antibody, fragment, composition, or combination of the present invention which generates expected effects in a patient in need of treatment or prevention after being administered to the patient in a single or multiple doses.

"Therapeutically effective amount" refers to an amount effective to achieve a desired therapeutic result at a necessary dose for a necessary period of time. The therapeutically effective amount is also such an amount that any toxic or undesired effect of the antibody, fragment thereof, composition, or combination is inferior to the therapeutically beneficial effect. The "therapeutically effective amount" preferably inhibits a measurable parameter or improves a measurable parameter by at least about 40%, and even more preferably by at least about 50%, 55%, 60%, 65%, 70%, 75%, 80%, 85%, 90%, or even 100%, relative to untreated subj ects.

"Prophylactically effective amount" refers to an amount effective to achieve a desired prophylactic result at a necessary dose for a necessary period of time. Generally, since a prophylactic dose is administered in a subject before or at an earlier stage of a disease, a prophylactically effective amount will be less than a therapeutically effective amount.

The term "tumor" refers to all neoplastic cell growth and proliferation, whether malignant or benign, and all pre-cancerous and cancerous cells and tissues. The terms "cancer", "carcinoma", and "tumor" are not mutually exclusive when referred to herein. The term "tumor" encompasses solid tumors and liquid tumors.

The term "anti-tumor effect" or "tumor inhibitory effect" refers to a biological effect that can be demonstrated by a variety of means, including but not limited to, for example, decrease in tumor volume, decrease in the number of tumor cells, decrease in tumor cell proliferation, or decrease in tumor cell viability.

The term "pharmaceutical supplementary material" refers to diluents, adjuvants (e.g., Freund's adjuvants (complete and incomplete)), excipients, carriers, stabilizers, or the like, that are administered with the active substance.

The term "pharmaceutical composition" refers to a composition that exists in a form allowing effective biological activity of the active ingredient contained therein and does not contain additional ingredients having unacceptable toxicity to a subject to which the composition is administered.

The term "pharmaceutical combination or combination product" refers to a non-fixed combination product or a fixed combination product, including but not limited to, a kit and a pharmaceutical composition. The term "non-fixed combination" means that the active ingredients (e.g., (i) the immunoconjugate of the present invention, and (ii) an additional therapeutic agent) are administered, either simultaneously or sequentially (without specific time limitation or at identical or different time intervals), to a patient as separate entities, wherein such administration provides two or more prophylactically or therapeutically effective active agents in the patient. The term "fixed combination" means that two or more active agents are administered to a patient simultaneously in the form of a single entity. The dose and/or time intervals of two or more active agents are preferably selected such that the combined use of the components can result in a therapeutic effect on the disease or disorder which is greater than that achieved by the use of either component alone. The ingredients may each take a separate formulation form and such separate formulation forms may be the same or different.

The term "combination therapy" refers to the administration of two or more therapeutic agents or modalities (e.g., radiotherapy or surgery) to treat the diseases as described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (such as tablets, capsules, powder and liquid). The powder and/or liquid can be reconstituted or diluted to a desired dose before administration. In addition, such administration also includes using each type of the therapeutic agents at approximately the same time or in a sequential manner at different times. In any case, the therapeutic regimen will provide the beneficial effect of the pharmaceutical combination in the treatment of disorders or symptoms described herein.

"Subject/patient/individual sample" refers to a collection of cells or fluids obtained from a patient or a subject. The source of tissue or cell samples can be solid tissues, e.g., from fresh, frozen and/or preserved organ or tissue samples or biopsy samples or puncture samples; blood or any blood component; body fluids such as tears, vitreous humors, cerebrospinal fluids, amniotic fluids, peritoneal fluids, or interstitial fluids; and cells from a subject at any time during pregnancy or development. In some embodiments, the tissue sample is a tumor tissue. Tissue samples may comprise compounds which are naturally not mixed with tissues, such as preservatives, anticoagulants, buffers, fixatives, nutrients, and antibiotics.

### II. Humanized CD3 antibody

In one aspect, the present invention provides a humanized CD3 antibody, which has better binding affinity for CD3 and is therefore more suitable for use in the construction of an antigen-binding region in a bispecific antibody molecule.

In some embodiments, the anti-CD3 antibody or the antigen-binding fragment thereof of the present invention binds to CD3 (e.g., human CD3 or monkey CD3, such as cynomolgus monkey CD3) with desired affinity. In some embodiments, the anti-CD3 antibody or the antigen-binding fragment thereof of the present invention is capable of binding to both human CD3 and monkey CD3 (e.g., cynomolgus monkey CD3). In some embodiments, the affinity of the antibody is determined by bio-layer interferometry or surface plasmon resonance.

In some embodiments, the anti-CD3 antibody of the present invention binds to human CD3 or monkey CD3 (e.g., cynomolgus monkey CD3) with an equilibrium dissociation constant (K_{D}) of about 1-1000 nM. In some embodiments, the anti-CD3 antibody of the present invention binds to monkey CD3 (e.g., cynomolgus monkey CD3) with a K_{D} of about 10-100 nM, or 20-100 nM, or 50-100 nM. In some embodiments, the anti-CD3 antibody of the present invention binds to human CD3 with a K_{D} of about 100-1000 nM (e.g., about 200-1000 nM, 300-1000 nM, 400-1000 nM, or 500-1000 nM).

In some embodiments, the antibody or the antigen-binding fragment thereof of the present invention binds to CD3 on the effector cell surface. In some embodiments, the antibody or the antigen-binding fragment thereof of the present invention is capable of activating effector cells. In some embodiments, the effector cell is a T cell. In some embodiments, the binding is determined by flow cytometry. In some embodiments, the activation of the CD3 antibody is determined using a reporter gene assay system (e.g., a Jurkat/NFAT-luc reporter gene system). In some embodiments, the antibody or the antigen-binding fragment thereof of the present invention is capable of activating effector cells to induce the killing of tumor cells.

In some embodiments, the anti-CD3 antibody or the antigen-binding fragment thereof of the present invention comprises 3 complementarity determining regions from a heavy chain variable region (HCDRs): HCDR1, HCDR2 and HCDR3.

In some embodiments, the anti-CD3 antibody or the antigen-binding fragment thereof of the present invention comprises 3 complementarity determining regions from a light chain variable region (LCDRs): LCDR1, LCDR2 and LCDR3.

In some embodiments, the anti-CD3 antibody or the antigen-binding fragment thereof of the present invention comprises 3 complementarity determining regions from a heavy chain variable region (HCDRs) and 3 complementarity determining regions from a light chain variable region (LCDRs).

In some aspects, the anti-CD3 antibody or the antigen-binding fragment thereof of the present invention comprises a heavy chain variable region (VH). In some aspects, the anti-CD3 antibody or the antigen-binding fragment thereof of the present invention comprises a light chain variable region (VL). In some aspects, the anti-CD3 antibody or the antigen-binding fragment thereof of the present invention comprises a heavy chain variable region (VH) and a light chain variable region (VL). In some embodiments, the heavy chain variable region comprises 3 complementarity determining regions (CDRs) from the heavy chain variable region: HCDR1, HCDR2 and HCDR3. In some embodiments, the light chain variable region comprises 3 complementarity determining regions (CDRs) from the light chain variable region: LCDR1, LCDR2 and LCDR3.

In some embodiments, the anti-CD3 antibody or the antigen-binding fragment thereof of the present invention further comprises an antibody heavy chain constant region HC. In some embodiments, the anti-CD3 antibody or the antigen-binding fragment thereof of the present invention further comprises an antibody light chain constant region LC. In some embodiments, the anti-CD3 antibody or the antigen-binding fragment thereof of the present invention further comprises a heavy chain constant region HC and a light chain constant region LC.

In some embodiments, the heavy chain variable region VH of the anti-CD3 antibody of the present invention
(i) comprises or consists of an amino acid sequence having at least 90%, 91%, 92%, 93%, 94%, 95%, 96%, 97%, 98%, or 99% identity to an amino acid sequence of SEQ ID NO: 3; or
(ii) comprises or consists of an amino acid sequence of SEQ ID NO: 3; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared to an amino acid sequence of SEQ ID NO: 3, wherein preferably, the amino acid changes do not occur in the CDRs.

In some embodiments, the light chain variable region VL of the anti-CD3 antibody of the present invention
(i) comprises or consists of an amino acid sequence having at least 90%, 91%, 92%, 93%, 94%, 95%, 96%, 97%, 98%, or 99% identity to an amino acid sequence of SEQ ID NO: 4; or
(ii) comprises or consists of an amino acid sequence of SEQ ID NO: 4; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared to an amino acid sequence of SEQ ID NO: 4, wherein preferably, the amino acid changes do not occur in the CDRs.

In some embodiments, the 3 complementarity determining regions from the heavy chain variable region (HCDRs) of the anti-CD3 antibody of the present invention, HCDR1, HCDR2 and HCDR3, are selected from
(i) three complementarity determining regions HCDR1, HCDR2 and HCDR3 contained in a VH set forth in any one of SEQ ID NO: 3; or
(ii) a sequence comprising a total of at least one and no more than 5, 4, 3, 2, or 1 amino acid change (preferably amino acid replacement, and more preferably conservative replacement) in the three HCDRs relative to any sequence of (i).

In some embodiments, the 3 complementarity determining regions from the light chain variable region (LCDRs) of the anti-CD3 antibody of the present invention, LCDR1, LCDR2 and LCDR3, are selected from
(i) three complementarity determining regions LCDR1, LCDR2 and LCDR3 contained in a VL set forth in any one of SEQ ID NO: 4; or
(ii) a sequence comprising a total of at least one and no more than 5, 4, 3, 2, or 1 amino acid change (preferably amino acid replacement, and more preferably conservative replacement) in the three LCDRs relative to any sequence of (i).

In some embodiments, in the anti-CD3 antibody of the present invention,
the HCDR1 comprises or consists of an amino acid sequence of SEQ ID NO: 5; the HCDR2 comprises or consists of an amino acid sequence of SEQ ID NO: 6; the HCDR3 comprises or consists of an amino acid sequence of SEQ ID NO: 7; the LCDR1 comprises or consists of an amino acid sequence of SEQ ID NO: 8; the LCDR2 comprises or consists of an amino acid sequence of SEQ ID NO: 9; and/or the LCDR3 comprises or consists of an amino acid sequence of SEQ ID NO: 10.

In some specific embodiments of the present invention, the anti-CD3 antibody or the antigen-binding fragment thereof of the present invention comprises a VH and a VL, wherein
the VH comprises or consists of an amino acid sequence set forth in SEQ ID NO: 3 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 4 or an amino acid sequence having at least 90%, 91%, 92%, 93%, 94%, 95%, 96%, 97%, 98%, or 99% identity thereto.

In some specific embodiments of the present invention, the anti-CD3 antibody or the antigen-binding fragment thereof comprises three complementarity determining regions HCDR1, HCDR2 and HCDR3 contained in the VH set forth in SEQ ID NO: 3, and three complementarity determining regions LCDR1, LCDR2 and LCDR3 contained in the VL set forth in SEQ ID NO: 4.

In some specific embodiments of the present invention, the anti-CD3 antibody or the antigen-binding fragment thereof of the present invention comprises: an HCDR1 set forth in SEQ ID NO: 5, an HCDR2 set forth in SEQ ID NO: 6, an HCDR3 set forth in SEQ ID NO: 7, an LCDR1 set forth in SEQ ID NO: 8, an LCDR2 set forth in SEQ ID NO: 9, and an LCDR3 set forth in SEQ ID NO: 10.

In one embodiment of the present invention, the amino acid change described herein includes amino acid replacement, insertion or deletion. In a preferred embodiment, the amino acid change described herein occurs in a region outside the CDR (e.g., in FR). More preferably, the amino acid change described herein occurs in a region outside the heavy chain variable region and/or outside the light chain variable region. Preferably, the amino acid change described herein is an amino acid replacement, preferably a conservative replacement.

In some embodiments, the anti-CD3 antibody or the antigen-binding fragment thereof of the present invention has one or more of the following properties:
(i) showing the same or similar binding affinity and/or specificity for CD3 as the antibody of the present invention;
(ii) inhibiting (e.g., competitively inhibiting) the binding of the antibody of the present invention to CD3;
(iii) binding to the same or overlapping epitope as the antibody of the present invention;
(iv) competing with the antibody of the present invention for binding to CD3; and
(v) having one or more biological properties of the antibody of the present invention.

In some embodiments, the anti-CD3 antibody of the present invention is an antibody in the form of IgG1, IgG2, IgG3, or IgG4, for example, an antibody in the form of IgG1. In some embodiments, the light chain constant region of the anti-CD3 antibody of the present invention is a kappa or lambda light chain constant region, e.g., a lambda light chain constant region.

In some embodiments, the anti-CD3 antibody is a monoclonal antibody.

In some embodiments, the anti-CD3 antibody is humanized.

In one embodiment, the anti-CD3 antibody of the present invention also encompasses an antibody fragment (e.g., an antigen-binding fragment) thereof, preferably an antibody fragment selected from: a Fab, a Fab', a Fab'-SH, an Fv, a single-chain antibody (e.g., scFv), an (Fab')₂, a single-domain antibody (e.g., VHH), a domain antibody (dAb), or a linear antibody.

In one embodiment, the anti-CD3 antibody fragment of the present invention is an scFv comprising the VH and the VL described herein as well as a linker peptide, wherein, for example, the linker peptide is (GGGGS)ₙ, wherein n = 1, 2, 3, 4, or 5, e.g., n = 3 or 4; for example, the linker peptide is a linker set forth in SEQ ID NO: 12. In some embodiments, the scFv comprises or consists of an amino acid sequence set forth in SEQ ID NO: 11 or an amino acid sequence having at least 85%, 90%, 91%, 92%, 93%, 94%, 95%, 96%, 97%, 98%, or 99% identity thereto.

In one embodiment, the anti-CD3 antibody of the present invention is a single-chain antibody comprising or consisting of an anti-CD3 antibody fragment scFv and a heavy chain constant region Fc (optionally the Fc region comprises a hinge region).

In some embodiments, the anti-CD3 single-chain antibody of the present invention comprises or consists of an amino acid sequence set forth in SEQ ID NO: 13 or an amino acid sequence having at least 85%, 90%, 91%, 92%, 93%, 94%, 95%, 96%, 97%, 98%, or 99% identity thereto.

In one aspect, the present invention provides an anti-CD3 antibody or an antigen-binding fragment thereof targeting human CD3, which has the following advantages:
1) binding to human CD3 and target cells expressing human CD3 with high specificity;
2) suitable for use as a genetically engineered component; and
3) low immunogenicity.

In one embodiment, the anti-CD3 antibody or the antigen-binding fragment thereof is humanized.

In one specific embodiment, the anti-CD3 antibody or the antigen-binding fragment thereof comprises:
1) a heavy chain variable region (VH_{CD3}) having a sequence set forth in SEQ ID NO: 3, or a heavy chain variable region having at least 90%, 91%, 92%, 93%, 94%, 95%, 96%, 97%, 98%, 99% or more sequence identity and the same CDRs as compared to SEQ ID NO: 3; and
2) a light chain variable region (VL_{CD3}) having a sequence set forth in SEQ ID NO: 4, or a light chain variable region having at least 90%, 91%, 92%, 93%, 94%, 95%, 96%, 97%, 98%, 99% or more sequence identity and the same CDRs as compared to SEQ ID NO: 4.

In another specific embodiment, the anti-CD3 antibody or the antigen-binding fragment thereof comprises:
1) a heavy chain (H) having a sequence set forth in SEQ ID NO: 1, or a heavy chain having at least 90%, 91%, 92%, 93%, 94%, 95%, 96%, 97%, 98%, 99% or more sequence identity and the same CDRs as compared to SEQ ID NO: 1; and
2) a light chain (L) having a sequence set forth in SEQ ID NO: 2, or a light chain having at least 90%, 91%, 92%, 93%, 94%, 95%, 96%, 97%, 98%, 99% or more sequence identity and the same CDRs as compared to SEQ ID NO: 2.

In one embodiment, the antigen-binding fragment of the anti-CD3 antibody is an Fv, a Fab, a Fab', a Fab'-SH, an F(ab')₂, a linear antibody, or a single-chain antibody (e.g., scFv). In one specific embodiment, the antigen-binding fragment of the anti-CD3 antibody is an scFv comprising a sequence set forth in SEQ ID NO: 11, or a sequence having at least 90%, 91%, 92%, 93%, 94%, 95%, 96%, 97%, 98%, 99% or more sequence identity and the same CDRs as compared to SEQ ID NO: 11. In another embodiment, the anti-CD3 antibody comprises an scFv and an Fc region comprising a sequence set forth in SEQ ID NO: 13, or a sequence having at least 90%, 91%, 92%, 93%, 94%, 95%, 96%, 97%, 98%, 99% or more sequence identity and the same CDRs as compared to SEQ ID NO: 13.

### III. Anti-GPRC5D antibody

In one aspect, the present invention provides a GPRC5D antibody, which has a higher binding affinity for GPRC5D, for example, the GPRC5D antibody of the present invention is more suitable for use in the construction of an antigen-binding region in a bispecific antibody molecule.

In some embodiments, the anti-GPRC5D antibody or an antigen-binding fragment thereof of the present invention binds to GPRC5D (e.g., human GPRC5D or monkey GPRC5D, such as cynomolgus monkey GPRC5D) with higher affinity, e.g., as compared to a control antibody such as GC5B596. In some embodiments, the anti-GPRC5D antibody or the antigen-binding fragment thereof of the present invention is capable of binding to both human GPRC5D and monkey GPRC5D (e.g., cynomolgus monkey GPRC5D).

In some embodiments, the anti-GPRC5D antibody or the antigen-binding fragment thereof of the present invention binds to GPRC5D expressed by a cell. In some embodiments, the affinity of the anti-GPRC5D antibody for GPRC5D expressed by a cell is determined by flow cytometry.

In some embodiments, the anti-GPRC5D antibody or the antigen-binding fragment thereof of the present invention comprises 3 complementarity determining regions from a heavy chain variable region (HCDRs): HCDR1, HCDR2 and HCDR3.

In some embodiments, the anti-GPRC5D antibody or the antigen-binding fragment thereof comprises 3 complementarity determining regions from a light chain variable region (LCDRs): LCDR1, LCDR2 and LCDR3.

In some embodiments, the anti-GPRC5D antibody or the antigen-binding fragment thereof of the present invention comprises 3 complementarity determining regions from a heavy chain variable region (HCDRs) and 3 complementarity determining regions from a light chain variable region (LCDRs).

In some aspects, the anti-GPRC5D antibody or the antigen-binding fragment thereof of the present invention comprises a heavy chain variable region (VH). In some aspects, the anti-GPRC5D antibody or the antigen-binding fragment thereof of the present invention comprises a light chain variable region (VL). In some aspects, the anti-GPRC5D antibody or the antigen-binding fragment thereof of the present invention comprises a heavy chain variable region (VH) and a light chain variable region (VL). In some embodiments, the heavy chain variable region comprises 3 complementarity determining regions (CDRs) from the heavy chain variable region: HCDR1, HCDR2 and HCDR3. In some embodiments, the light chain variable region comprises 3 complementarity determining regions (CDRs) from the light chain variable region: LCDR1, LCDR2 and LCDR3.

In some embodiments, the anti-GPRC5D antibody or the antigen-binding fragment thereof of the present invention further comprises an antibody heavy chain constant region HC. In some embodiments, the anti-GPRC5D antibody or the antigen-binding fragment thereof of the present invention further comprises an antibody light chain constant region LC. In some embodiments, the anti-GPRC5D antibody or the antigen-binding fragment thereof of the present invention further comprises a heavy chain constant region HC and a light chain constant region LC.

In some embodiments, the heavy chain variable region of the anti-GPRC5D antibody of the present invention
(i) comprises or consists of an amino acid sequence having at least 90%, 91%, 92%, 93%, 94%, 95%, 96%, 97%, 98%, or 99% identity to an amino acid sequence of SEQ ID NO: 62, 72, 82, 16, 23, 25, 27, 29, 38, or 40; or
(ii) comprises or consists of an amino acid sequence of SEQ ID NO: 62, 72, 82, 16, 23, 25, 27, 29, 38, or 40; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared to an amino acid sequence of SEQ ID NO: 62, 72, 82, 16, 23, 25, 27, 29, 38, or 40, wherein preferably, the amino acid changes do not occur in the CDRs.

In some embodiments, the light chain variable region of the anti-GPRC5D antibody of the present invention
(i) comprises or consists of an amino acid sequence having at least 90%, 91%, 92%, 93%, 94%, 95%, 96%, 97%, 98%, or 99% identity to an amino acid sequence of SEQ ID NO: 63, 73, 83, 17, 19, or 21; or
(ii) comprises or consists of an amino acid sequence of SEQ ID NO: 63, 73, 83, 17, 19, or 21; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared to an amino acid sequence of SEQ ID NO: 63, 73, 83, 17, 19, or 21, wherein preferably, the amino acid changes do not occur in the CDRs.

In some embodiments, the 3 complementarity determining regions from the heavy chain variable region (HCDRs) of the anti-GPRC5D antibody of the present invention, HCDR1, HCDR2 and HCDR3, are selected from
(i) three complementarity determining regions HCDR1, HCDR2 and HCDR3 contained in a VH set forth in any one of SEQ ID NOs: 62, 72, 82, 16, 23, 25, 27, 29, 38, and 40; or
(ii) a sequence comprising a total of at least one and no more than 5, 4, 3, 2, or 1 amino acid change (preferably amino acid replacement, and more preferably conservative replacement) in the three HCDRs relative to any sequence of (i).

For example, the CDRs are determined by the Kabat scheme.

In some embodiments, the 3 complementarity determining regions from the light chain variable region (LCDRs) of the anti-GPRC5D antibody of the present invention, LCDR1, LCDR2 and LCDR3, are selected from
(i) three complementarity determining regions LCDR1, LCDR2 and LCDR3 contained in a VL set forth in any one of SEQ ID NOs: 63, 73, 83, 17, 19, and 21; or
(ii) a sequence comprising a total of at least one and no more than 5, 4, 3, 2, or 1 amino acid change (preferably amino acid replacement, and more preferably conservative replacement) in the three LCDRs relative to any sequence of (i).

For example, the CDRs are determined by the Kabat scheme.

In some embodiments, the HCDR1 comprises or consists of an amino acid sequence of SEQ ID NO: 64, 74, or 84, or the HCDR1 comprises an amino acid sequence having one, two, or three changes (preferably amino acid replacements, and more preferably conservative replacements) compared to the amino acid sequence of SEQ ID NO: 64, 74, or 84.

In some embodiments, the HCDR2 comprises or consists of an amino acid sequence of SEQ ID NO: 65, 75, 85, 39, or 41, or the HCDR2 comprises an amino acid sequence having one, two, or three changes (preferably amino acid replacements, and more preferably conservative replacements) compared to the amino acid sequence of SEQ ID NO: 65, 75, 85, 39, or 41.

In some embodiments, the HCDR3 comprises or consists of an amino acid sequence of SEQ ID NO: 66, 76, or 86, or the HCDR3 comprises an amino acid sequence having one, two, or three changes (preferably amino acid replacements, and more preferably conservative replacements) compared to the amino acid sequence of SEQ ID NO: 66, 76, or 86.

In some embodiments, the LCDR1 comprises or consists of an amino acid sequence of SEQ ID NO: 67, 77, or 87, or the LCDR1 comprises an amino acid sequence having one, two, or three changes (preferably amino acid replacements, and more preferably conservative replacements) compared to the amino acid sequence of SEQ ID NO: 67, 77, or 87.

In some embodiments, the LCDR2 comprises or consists of an amino acid sequence of SEQ ID NO: 68 or 78, or the LCDR2 comprises an amino acid sequence having one, two, or three changes (preferably amino acid replacements, and more preferably conservative replacements) compared to the amino acid sequence of SEQ ID NO: 68 or 78.

In some embodiments, the LCDR3 comprises or consists of an amino acid sequence of SEQ ID NO: 69, 79, or 88, or the LCDR3 comprises an amino acid sequence having one, two, or three changes (preferably amino acid replacements, and more preferably conservative replacements) compared to the amino acid sequence of SEQ ID NO: 69, 79, or 88.

In some specific embodiments of the present invention, the anti-GPRC5D antibody or the antigen-binding fragment thereof of the present invention comprises a VH and a VL, wherein
(i) the VH comprises or consists of an amino acid sequence set forth in SEQ ID NO: 62 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 63 or an amino acid sequence having at least 90%, 91%, 92%, 93%, 94%, 95%, 96%, 97%, 98%, or 99% identity thereto;
(ii) the VH comprises or consists of an amino acid sequence set forth in SEQ ID NO: 72 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 73 or an amino acid sequence having at least 90%, 91%, 92%, 93%, 94%, 95%, 96%, 97%, 98%, or 99% identity thereto;
(iii) the VH comprises or consists of an amino acid sequence set forth in SEQ ID NO: 82 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 83 or an amino acid sequence having at least 90%, 91%, 92%, 93%, 94%, 95%, 96%, 97%, 98%, or 99% identity thereto; and
(iv) the VH comprises or consists of an amino acid sequence set forth in SEQ ID NO: 16, 23, 25, 27, 29, 38, or 40 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 17, 19, or 21 or an amino acid sequence having at least 90%, 91%, 92%, 93%, 94%, 95%, 96%, 97%, 98%, or 99% identity thereto.

In some specific embodiments of the present invention, the anti-GPRC5D antibody or the antigen-binding fragment thereof of the present invention comprises:
(i) an HCDR1 set forth in SEQ ID NO: 64, an HCDR2 set forth in SEQ ID NO: 65, 39, or 41, an HCDR3 set forth in SEQ ID NO: 66, an LCDR1 set forth in SEQ ID NO: 67, an LCDR2 set forth in SEQ ID NO: 68, and an LCDR3 set forth in SEQ ID NO: 69;
(ii) an HCDR1 set forth in SEQ ID NO: 74, an HCDR2 set forth in SEQ ID NO: 75, an HCDR3 set forth in SEQ ID NO: 76, an LCDR1 set forth in SEQ ID NO: 77, an LCDR2 set forth in SEQ ID NO: 78, and an LCDR3 set forth in SEQ ID NO: 79; or
(iii) an HCDR1 set forth in SEQ ID NO: 84, an HCDR2 set forth in SEQ ID NO: 85, an HCDR3 set forth in SEQ ID NO: 86, an LCDR1 set forth in SEQ ID NO: 87, an LCDR2 set forth in SEQ ID NO: 78, and an LCDR3 set forth in SEQ ID NO: 88.

In some specific embodiments of the present invention, the anti-GPRC5D antibody or the antigen-binding fragment thereof of the present invention comprises a VH and a VL, wherein the VH and the VL comprise or consist of amino acid sequences set forth below, respectively:
(i). SEQ ID NO: 62 and SEQ ID NO: 63;
(ii). SEQ ID NO: 72 and SEQ ID NO: 73;
(iii). SEQ ID NO: 82 and SEQ ID NO: 83;
(iv). SEQ ID NO: 16, 23, 25, 27, 29, 38, or 40 and SEQ ID NO: 21;
(v). SEQ ID NO: 16 or 25 and SEQ ID NO: 17; or
(vi). SEQ ID NO: 16 or 27 and SEQ ID NO: 19.

In some embodiments of the present invention, the anti-GPRC5D antibody or the antigen-binding fragment thereof of the present invention comprises a heavy chain. In some embodiments of the present invention, the anti-GPRC5D antibody or the antigen-binding fragment thereof of the present invention comprises a light chain. In some embodiments of the present invention, the anti-GPRC5D antibody or the antigen-binding fragment thereof of the present invention comprises a heavy chain and a light chain.

In some specific embodiments, the heavy chain of the anti-GPRC5D antibody comprises or consists of an amino acid sequence of SEQ ID NO: 60, 70, 80, 14, 22, 24, 26, or 28, or an amino acid sequence having at least 85%, 90%, 91%, 92%, 93%, 94%, 95%, 96%, 97%, 98%, or 99% identity thereto.

In some specific embodiments, the light chain of the anti-GPRC5D antibody comprises or consists of an amino acid sequence of SEQ ID NO: 61, 71, 81, 15, 18, or 20, or an amino acid sequence having at least 85%, 90%, 91%, 92%, 93%, 94%, 95%, 96%, 97%, 98%, or 99% identity thereto.

In some specific embodiments, the anti-GPRC5D antibody comprises a heavy chain and a light chain, wherein the heavy chain comprises an amino acid sequence set forth in SEQ ID NO: 14, 22, 24, 26, or 28, or an amino acid sequence having at least 85%, 90%, 91%, 92%, 93%, 94%, 95%, 96%, 97%, 98%, or 99% identity thereto, and the light chain comprises or consists of an amino acid sequence set forth in SEQ ID NO: 15, 18, or 20, or an amino acid sequence having at least 85%, 90%, 91%, 92%, 93%, 94%, 95%, 96%, 97%, 98%, or 99% identity thereto.

In some specific embodiments, the anti-GPRC5D antibody comprises a heavy chain and a light chain, wherein the heavy chain and the light chain comprise or consist of amino acid sequences set forth in SEQ ID NOs shown below, or amino acid sequences having at least 85%, 90%, 91%, 92%, 93%, 94%, 95%, 96%, 97%, 98%, or 99% identity thereto, respectively:
(i). SEQ ID NO: 60 and SEQ ID NO: 61;
(ii). SEQ ID NO: 70 and SEQ ID NO: 71;
(iii). SEQ ID NO: 80 and SEQ ID NO: 81;
(iv). SEQ ID NO: 14 or 24 and SEQ ID NO: 15;
(v). SEQ ID NO: 14 or 26 and SEQ ID NO: 18; or
(vi). SEQ ID NO: 14, 22, 24, 26, or 28 and SEQ ID NO: 20.

In one embodiment of the present invention, the amino acid changes of the anti-GPRC5D antibody described herein include amino acid replacements, insertions, or deletions. In a preferred embodiment, the amino acid change described herein occurs in a region outside the CDR (e.g., in FR). More preferably, the amino acid change described herein occurs in a region outside the heavy chain variable region and/or outside the light chain variable region. Preferably, the amino acid change described herein is an amino acid replacement, preferably a conservative replacement.

In one embodiment of the present invention, the amino acid changes of the anti-GPRC5D antibody described herein are changes that eliminate post-translational modifications, for example, which are present in the CDRs of the anti-GPRC5D antibody, such as the HCDR2 of the anti-GPRC5D antibody, which comprises N55S and/or G56E replacements.

In some embodiments, the anti-GPRC5D antibody or the antigen-binding fragment thereof of the present invention has one or more of the following properties:
(i) showing the same or similar binding affinity and/or specificity for GPRC5D as the antibody of the present invention;
(ii) inhibiting (e.g., competitively inhibiting) the binding of the antibody of the present invention to GPRC5D;
(iii) binding to the same or overlapping epitope as the antibody of the present invention;
(iv) competing with the antibody of the present invention for binding to GPRC5D; and
(v) having one or more biological properties of the antibody of the present invention.

In some embodiments, the anti-GPRC5D antibody of the present invention is an antibody in the form of IgG1, IgG2, IgG3, or IgG4, for example, an antibody in the form of IgG1. In some embodiments, the light chain constant region of the anti-GPRC5D antibody of the present invention is a lambda or kappa light chain constant region, e.g., a kappa light chain constant region.

In some embodiments, the anti-GPRC5D antibody is a monoclonal antibody.

In some embodiments, the anti-GPRC5D antibody is humanized.

In some embodiments, the anti-GPRC5D antibody is a chimeric antibody.

In one embodiment, the anti-GPRC5D antibody of the present invention also encompasses an antibody fragment (e.g., an antigen-binding fragment) thereof, preferably an antibody fragment selected from: a Fab, a Fab', a Fab'-SH, an Fv, a single-chain antibody (e.g., scFv), an (Fab')₂, a single-domain antibody (e.g., VHH), a domain antibody (dAb), or a linear antibody.

### IV. MUC16 antibody

The present invention provides an anti-MUC16 antibody targeting human MUC16, which has one or more of the following advantages:
(1) binding to MUC16 (e.g., human or monkey MUC16) and target cells expressing human MUC16 with high affinity;
(2) not binding to circulating free CA125;
(3) suitable for use as a genetically engineered component;
(4) low immunogenicity;
(5) high expression purity; and
(6) still having high affinity after humanization.

In one embodiment, the anti-MUC16 antibody or an antigen-binding fragment thereof is humanized.

In some embodiments, the anti-MUC16 antibody or the antigen-binding fragment thereof of the present invention comprises 3 complementarity determining regions from a heavy chain variable region (HCDRs): HCDR1, HCDR2 and HCDR3.

In some embodiments, the anti-MUC16 antibody or the antigen-binding fragment thereof of the present invention comprises 3 complementarity determining regions from a light chain variable region (LCDRs): LCDR1, LCDR2 and LCDR3.

In some embodiments, the anti-MUC16 antibody or the antigen-binding fragment thereof of the present invention comprises 3 complementarity determining regions from a heavy chain variable region (HCDRs) and 3 complementarity determining regions from a light chain variable region (LCDRs).

In some aspects, the anti-MUC16 antibody or the antigen-binding fragment thereof of the present invention comprises a heavy chain variable region (VH). In some aspects, the anti-MUC16 antibody or the antigen-binding fragment thereof of the present invention comprises a light chain variable region (VL). In some aspects, the anti-MUC16 antibody or the antigen-binding fragment thereof of the present invention comprises a heavy chain variable region (VH) and a light chain variable region (VL). In some embodiments, the heavy chain variable region comprises 3 complementarity determining regions (CDRs) from the heavy chain variable region: HCDR1, HCDR2 and HCDR3. In some embodiments, the light chain variable region comprises 3 complementarity determining regions (CDRs) from the light chain variable region: LCDR1, LCDR2 and LCDR3.

In some embodiments, the anti-MUC16 antibody or the antigen-binding fragment thereof of the present invention further comprises an antibody heavy chain constant region HC. In some embodiments, the anti-MUC16 antibody or the antigen-binding fragment thereof of the present invention further comprises an antibody light chain constant region LC. In some embodiments, the anti-MUC16 antibody or the antigen-binding fragment thereof of the present invention further comprises a heavy chain constant region HC and a light chain constant region LC.

In some embodiments, the heavy chain variable region VH of the anti-MUC16 antibody of the present invention
(i) comprises or consists of an amino acid sequence having at least 90%, 91%, 92%, 93%, 94%, 95%, 96%, 97%, 98%, or 99% identity to an amino acid sequence of SEQ ID NO: 92 or 113; or
(ii) comprises or consists of an amino acid sequence of SEQ ID NO: 92 or 113; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared to an amino acid sequence of SEQ ID NO: 92 or 113, wherein preferably, the amino acid changes do not occur in the CDRs.

In some embodiments, the light chain variable region VL of the anti-MUC16 antibody of the present invention
(i) comprises or consists of an amino acid sequence having at least 90%, 91%, 92%, 93%, 94%, 95%, 96%, 97%, 98%, or 99% identity to an amino acid sequence of SEQ ID NO: 96, 100, 102, or 114; or
(ii) comprises or consists of an amino acid sequence of SEQ ID NO: 96, 100, 102, or 114; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared to an amino acid sequence of SEQ ID NO: 96, 100, 102, or 114, wherein preferably, the amino acid changes do not occur in the CDRs.

In some embodiments, the 3 complementarity determining regions from the heavy chain variable region (HCDRs) of the anti-MUC16 antibody of the present invention, HCDR1, HCDR2 and HCDR3, are selected from
(i) three complementarity determining regions HCDR1, HCDR2 and HCDR3 contained in a VH set forth in any one of SEQ ID NOs: 92 and 113; or
(ii) a sequence comprising a total of at least one and no more than 5, 4, 3, 2, or 1 amino acid change (preferably amino acid replacement, and more preferably conservative replacement) in the three HCDRs relative to any sequence of (i).

In some embodiments, the 3 complementarity determining regions from the light chain variable region (LCDRs) of the anti-MUC16 antibody of the present invention, LCDR1, LCDR2 and LCDR3, are selected from
(i) three complementarity determining regions LCDR1, LCDR2 and LCDR3 contained in a VL set forth in any one of SEQ ID NOs: 96, 100, 102, and 114; or
(ii) a sequence comprising a total of at least one and no more than 5, 4, 3, 2, or 1 amino acid change (preferably amino acid replacement, and more preferably conservative replacement) in the three LCDRs relative to any sequence of (i).

In some embodiments, in the anti-MUC16 antibody of the present invention,
the HCDR1 comprises or consists of an amino acid sequence of SEQ ID NO: 93; the HCDR2 comprises or consists of an amino acid sequence of SEQ ID NO: 94; the HCDR3 comprises or consists of an amino acid sequence of SEQ ID NO: 95; the LCDR1 comprises or consists of an amino acid sequence of SEQ ID NO: 97, 101, or 103; the LCDR2 comprises or consists of an amino acid sequence of SEQ ID NO: 98; and/or the LCDR3 comprises or consists of an amino acid sequence of SEQ ID NO: 99.

In some specific embodiments of the present invention, the anti-MUC16 antibody or the antigen-binding fragment thereof of the present invention comprises a VH and a VL, wherein the VH comprises or consists of an amino acid sequence set forth in SEQ ID NO: 92 or 113 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 96, 100, 102, or 114 or an amino acid sequence having at least 90%, 91%, 92%, 93%, 94%, 95%, 96%, 97%, 98%, or 99% identity thereto.

In some specific embodiments of the present invention, the anti-MUC16 antibody or the antigen-binding fragment thereof comprises three complementarity determining regions HCDR1, HCDR2 and HCDR3 contained in the VH set forth in SEQ ID NO: 92 or 113, and three complementarity determining regions LCDR1, LCDR2 and LCDR3 contained in the VL set forth in SEQ ID NO: 96, 100, 102, or 114.

In some specific embodiments of the present invention, the anti-MUC16 antibody or the antigen-binding fragment thereof of the present invention comprises: an HCDR1 set forth in SEQ ID NO: 93, an HCDR2 set forth in SEQ ID NO: 94, an HCDR3 set forth in SEQ ID NO: 95, an LCDR1 set forth in SEQ ID NO: 97, 101, or 103, an LCDR2 set forth in SEQ ID NO: 98, and an LCDR3 set forth in SEQ ID NO: 99.

In one embodiment of the present invention, the amino acid change described herein includes amino acid replacement, insertion or deletion. In a preferred embodiment, the amino acid change described herein occurs in a region outside the CDR (e.g., in FR). More preferably, the amino acid change described herein occurs in a region outside the heavy chain variable region and/or outside the light chain variable region. Preferably, the amino acid change described herein is an amino acid replacement, preferably a conservative replacement.

In some embodiments, the anti-MUC16 antibody or the antigen-binding fragment thereof of the present invention has one or more of the following properties:
(i) showing the same or similar binding affinity and/or specificity for MUC16 as the antibody of the present invention;
(ii) inhibiting (e.g., competitively inhibiting) the binding of the antibody of the present invention to MUC16;
(iii) binding to the same or overlapping epitope as the antibody of the present invention;
(iv) competing with the antibody of the present invention for binding to MUC16; and
(v) having one or more biological properties of the antibody of the present invention.

In some embodiments, the anti-MUC16 antibody of the present invention is an antibody in the form of IgG1, IgG2, IgG3, or IgG4, for example, an antibody in the form of IgG1. In some embodiments, the light chain constant region of the anti-MUC16 antibody of the present invention is a kappa or lambda light chain constant region, e.g., a lambda light chain constant region.

In some embodiments, the anti-MUC16 antibody is a monoclonal antibody.

In some embodiments, the anti-MUC16 antibody is humanized.

In one embodiment, the anti-MUC16 antibody of the present invention also encompasses an antibody fragment (e.g., an antigen-binding fragment) thereof, preferably an antibody fragment selected from: a Fab, a Fab', a Fab'-SH, an Fv, a single-chain antibody (e.g., scFv), an (Fab')₂, a single-domain antibody (e.g., VHH), a domain antibody (dAb), or a linear antibody.

In one specific embodiment, the anti-MUC16 antibody or the antigen-binding fragment thereof comprises a VH and a VL, wherein:
1) the VH comprises an HCDR1 set forth in SEQ ID NO: 93, an HCDR2 set forth in SEQ ID NO: 94, and an HCDR3 set forth in SEQ ID NO: 95, and the VL comprises an LCDR1 set forth in SEQ ID NO: 97, an LCDR2 set forth in SEQ ID NO: 98, and an LCDR3 set forth in SEQ ID NO: 99; or
2) the VH comprises an HCDR1 set forth in SEQ ID NO: 93, an HCDR2 set forth in SEQ ID NO: 94, and an HCDR3 set forth in SEQ ID NO: 95, and the VL comprises an LCDR1 set forth in SEQ ID NO: 101, an LCDR2 set forth in SEQ ID NO: 98, and an LCDR3 set forth in SEQ ID NO: 99; or
3) the VH comprises an HCDR1 set forth in SEQ ID NO: 93, an HCDR2 set forth in SEQ ID NO: 94, and an HCDR3 set forth in SEQ ID NO: 95, and the VL comprises an LCDR1 set forth in SEQ ID NO: 103, an LCDR2 set forth in SEQ ID NO: 98, and an LCDR3 set forth in SEQ ID NO: 99.

In one specific embodiment, the anti-MUC16 antibody or the antigen-binding fragment thereof comprises:
1) a VH having a sequence set forth in SEQ ID NO: 92, or a VH having at least 90%, 91%, 92%, 93%, 94%, 95%, 96%, 97%, 98%, 99% or more sequence identity and the same CDRs as compared to SEQ ID NO: 92; and a VL having a sequence set forth in SEQ ID NO: 96, or a VL having at least 90%, 91%, 92%, 93%, 94%, 95%, 96%, 97%, 98%, 99% or more sequence identity and the same CDRs as compared to SEQ ID NO: 96; or
2) a VH having a sequence set forth in SEQ ID NO: 92, or a VH having at least 90%, 91%, 92%, 93%, 94%, 95%, 96%, 97%, 98%, 99% or more sequence identity and the same CDRs as compared to SEQ ID NO: 92; and a VL having a sequence set forth in SEQ ID NO: 100, or a VL having at least 90%, 91%, 92%, 93%, 94%, 95%, 96%, 97%, 98%, 99% or more sequence identity and the same CDRs as compared to SEQ ID NO: 100; or
3) a VH having a sequence set forth in SEQ ID NO: 92, or a VH having at least 90%, 91%, 92%, 93%, 94%, 95%, 96%, 97%, 98%, 99% or more sequence identity and the same CDRs as compared to SEQ ID NO: 92; and a VL having a sequence set forth in SEQ ID NO: 102, or a VL having at least 90%, 91%, 92%, 93%, 94%, 95%, 96%, 97%, 98%, 99% or more sequence identity and the same CDRs as compared to SEQ ID NO: 102; or
4) a VH having a sequence set forth in SEQ ID NO: 113, or a VH having at least 90%, 91%, 92%, 93%, 94%, 95%, 96%, 97%, 98%, 99% or more sequence identity and the same CDRs as compared to SEQ ID NO: 113; and a VL having a sequence set forth in SEQ ID NO: 114, or a VL having at least 90%, 91%, 92%, 93%, 94%, 95%, 96%, 97%, 98%, 99% or more sequence identity and the same CDRs as compared to SEQ ID NO: 114.

In another specific embodiment, the anti-MUC16 antibody or the antigen-binding fragment thereof comprises:
1) a heavy chain (H) having a sequence set forth in SEQ ID NO: 106, or a heavy chain having at least 90%, 91%, 92%, 93%, 94%, 95%, 96%, 97%, 98%, 99% or more sequence identity and the same CDRs as compared to SEQ ID NO: 106; and a light chain (L) having a sequence set forth in SEQ ID NO: 107, or a light chain having at least 90%, 91%, 92%, 93%, 94%, 95%, 96%, 97%, 98%, 99% or more sequence identity and the same CDRs as compared to SEQ ID NO: 107; or
2) a heavy chain (H) having a sequence set forth in SEQ ID NO: 106, or a heavy chain having at least 90%, 91%, 92%, 93%, 94%, 95%, 96%, 97%, 98%, 99% or more sequence identity and the same CDRs as compared to SEQ ID NO: 106; and a light chain (L) having a sequence set forth in SEQ ID NO: 108, or a light chain having at least 90%, 91%, 92%, 93%, 94%, 95%, 96%, 97%, 98%, 99% or more sequence identity and the same CDRs as compared to SEQ ID NO: 108; or
3) a heavy chain (H) having a sequence set forth in SEQ ID NO: 106, or a heavy chain having at least 90%, 91%, 92%, 93%, 94%, 95%, 96%, 97%, 98%, 99% or more sequence identity and the same CDRs as compared to SEQ ID NO: 106; and a light chain (L) having a sequence set forth in SEQ ID NO: 109, or a light chain having at least 90%, 91%, 92%, 93%, 94%, 95%, 96%, 97%, 98%, 99% or more sequence identity and the same CDRs as compared to SEQ ID NO: 109; or
4) a heavy chain (H) having a sequence set forth in SEQ ID NO: 115, or a heavy chain having at least 90%, 91%, 92%, 93%, 94%, 95%, 96%, 97%, 98%, 99% or more sequence identity and the same CDRs as compared to SEQ ID NO: 115; and a light chain (L) having a sequence set forth in SEQ ID NO: 116, or a light chain having at least 90%, 91%, 92%, 93%, 94%, 95%, 96%, 97%, 98%, 99% or more sequence identity and the same CDRs as compared to SEQ ID NO: 116.

In some embodiments, the antibody comprises amino acid changes. In one embodiment of the present invention, the amino acid change described herein includes amino acid replacement, insertion or deletion. In a preferred embodiment, the amino acid change described herein occurs in a region outside the CDR (e.g., in FR). More preferably, the amino acid change described herein occurs in a region outside the heavy chain variable region and/or outside the light chain variable region. Preferably, the amino acid change described herein is an amino acid replacement, preferably a conservative replacement.

In certain embodiments, the antibody of the present invention may be a cysteine-engineered antibody, such as "sulfo-mAb", wherein one or more residues of the antibody are replaced by cysteine residues.

### V. Bispecific antibody

In some embodiments, the anti-CD3 antibody of the present invention is a bispecific antibody or a multispecific antibody, e.g., comprising a first binding specificity for CD3 and other binding specificities for one or more molecules (e.g., tumor-associated antigens, such as GPRC5D or MUC16).

In some embodiments, the anti-GPRC5D antibody of the present invention is a bispecific antibody or a multispecific antibody, e.g., comprising a first binding specificity for GPRC5D and other binding specificities for one or more molecules (e.g., CD3).

In some embodiments, the anti-MUC16 antibody of the present invention is a bispecific antibody or a multispecific antibody, e.g., comprising a first binding specificity for MUC16 and other binding specificities for one or more molecules (e.g., CD3).

Therefore, one aspect of the present invention relates to a bispecific antibody, which comprises a first antigen-binding region and a second antigen-binding region, wherein the first antigen-binding region specifically binds to a tumor-associated antigen, and/or the second antigen-binding region specifically binds to CD3.

As used herein, "tumor-associated antigen" refers to an antigen associated with a tumor, such as a protein highly expressed in a tumor, e.g., GPRC5D or MUC16.

Therefore, one aspect of the present invention relates to a bispecific antibody, which comprises a first antigen-binding region and a second antigen-binding region, wherein the first antigen-binding region specifically binds to GPRC5D or MUC16, and/or the second antigen-binding region specifically binds to CD3.

In some embodiments, the first antigen-binding region is from the anti-GPRC5D antibody described herein, e.g., a Fab fragment of the anti-GPRC5D antibody. In some embodiments, the first antigen-binding region is from the anti-MUC16 antibody described herein, e.g., a Fab fragment of the anti-MUC16 antibody.

In some embodiments, the second antigen-binding region is from the anti-CD3 antibody, e.g., an SP34 antibody or a humanized antibody thereof, e.g., an anti-CD3 humanized antibody described herein, e.g., an scFv fragment of the anti-CD3 antibody.

The first antigen-binding region suitable for use in the bispecific antibody of the present invention can comprise or consist of the anti-GPRC5D or anti-MUC16 full-length antibody or the antigen-binding fragment thereof of the present invention as long as it is capable of specifically binding to GPRC5D or MUC16, including, but not limited to, for example, a full-length antibody, a single-chain Fv, a Fab, a Fab', an (Fab)₂, a single-domain antibody, a VHH, a heavy chain antibody, or the like specifically binding to GPRC5D.

The second antigen-binding region suitable for use in the bispecific antibody of the present invention can comprise or consist of the anti-CD3 full-length antibody or the antigen-binding fragment thereof as long as it is capable of specifically binding to CD3, including, but not limited to, for example, a full-length antibody, a single-chain Fv, a Fab, a Fab', an (Fab)₂, a single-domain antibody, a VHH, a heavy chain antibody, or the like specifically binding to CD3.

In some embodiments, the bispecific antibody of the present invention is an IgG-like bispecific antibody. The term "IgG-like bispecific antibody" described herein refers to a bispecific antibody comprising an Fc dimer. Therefore, in some embodiments, the bispecific antibody of the present invention comprises an Fc dimer.

In some embodiments, the bispecific antibody of the present invention is an IgG-like bispecific antibody comprising a Fab fragment as one antigen-binding region and an scFv fragment as another antigen-binding region. In some embodiments, the IgG-like bispecific antibody comprises a Fab fragment that specifically binds to GPRC5D or MUC16 as a first antigen-binding region, and comprises an scFv that specifically binds to CD3 as a second antigen-binding region.

### ➢ Structure of bispecific antibody of the present invention

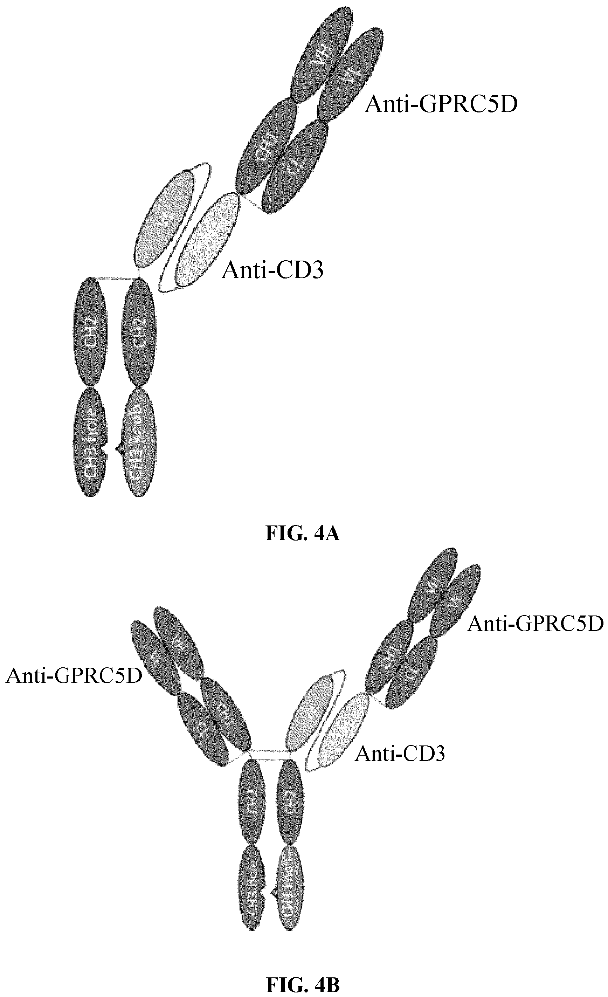

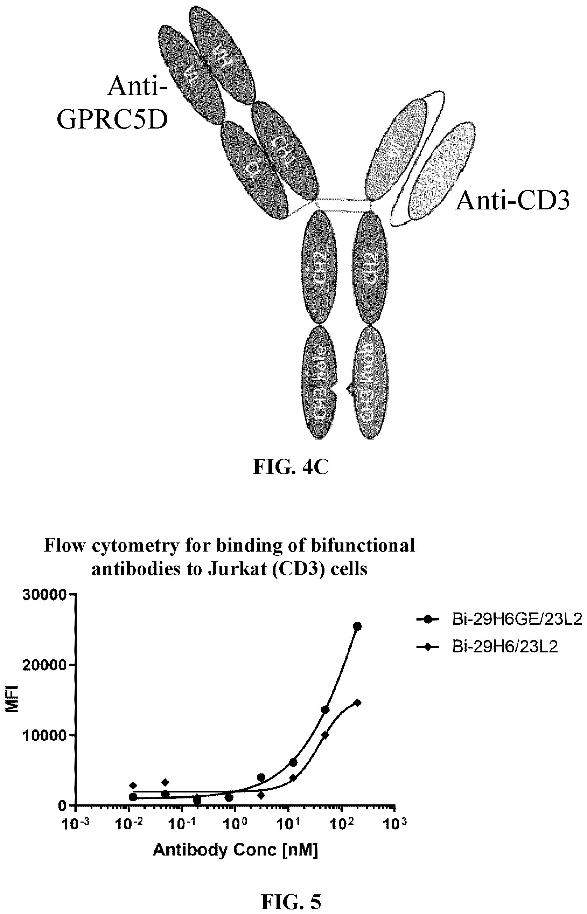

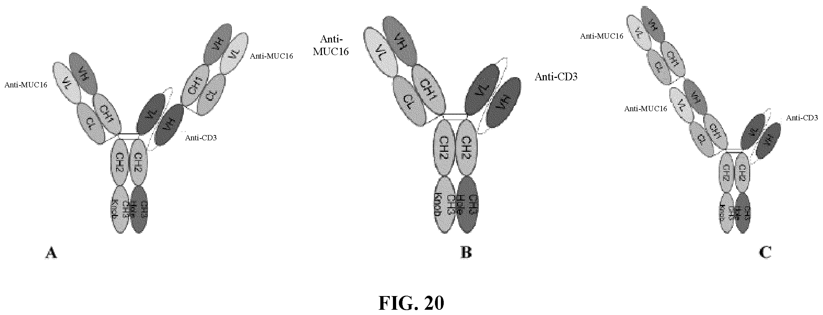

In some embodiments, the present invention provides a bispecific antibody comprising one or two Fab fragments specifically binding to a first antigen, one scFv specifically binding to a second antigen, and an Fc heterodimer, wherein
(1) the Fab fragment specifically binding to the first antigen is fused at the C-terminus of a CH1 of a Fab heavy chain to the N-terminus (e.g., the N-terminus of a VH or a VL) of the scFv fragment specifically binding to the second antigen, and the C-terminus (e.g., the C-terminus of a VL or a VH) of the scFv fragment is fused to a CH2 or a hinge region of one of Fc regions of the Fc heterodimer (e.g., an Fc region comprising a knob or a Fc region comprising a hole), for example, as shown in the structure of FIG. 4A;
(2) the first Fab fragment specifically binding to the first antigen is fused at the C-terminus of a CH1 of a Fab heavy chain to a CH2 or a hinge region of one of Fc regions of the Fc heterodimer (e.g., an Fc region comprising a hole or an Fc region comprising a knob); the second Fab fragment specifically binding to the first antigen is fused at the C-terminus of a CH1 of a Fab heavy chain to the N-terminus (e.g., the N-terminus of a VH or a VL) of the scFv fragment specifically binding to the second antigen, and the C-terminus (e.g., the C-terminus of a VL or a VH) of the scFv fragment is fused to a CH2 or a hinge region of another Fc region of the Fc heterodimer (e.g., an Fc region comprising a knob or an Fc region comprising a hole), for example, as shown in the structure of FIG. 4B or FIG. 20A;
(3) the Fab fragment specifically binding to the first antigen is fused at the C-terminus of a CH1 of a Fab heavy chain to a CH2 or a hinge region of one of Fc regions of the Fc heterodimer (e.g., an Fc region comprising a hole or an Fc region comprising a knob); the C-terminus (e.g., the C-terminus of a VL or a VH) of the scFv fragment specifically binding to the second antigen is fused to a CH2 or a hinge region of another Fc region of the Fc heterodimer (e.g., an Fc region comprising a knob or an Fc region comprising a hole), for example, as shown in the structure of FIG. 4C or FIG. 20B; or
(4) the first Fab fragment specifically binding to the first antigen is fused at the C-terminus of a CH1 of a Fab heavy chain to the N-terminus of a VH of the second Fab fragment specifically binding to the first antigen, and the second Fab fragment is fused at the C-terminus of a CH1 of a Fab heavy chain to a CH2 or a hinge region of one of Fc regions of the Fc heterodimer (e.g., an Fc region comprising a knob or an Fc region comprising a hole); the C-terminus (e.g., the C-terminus of a VL or a VH) of the scFv fragment specifically binding to the second antigen is fused to a CH2 or a hinge region of another Fc region of the Fc heterodimer (e.g., an Fc region comprising a hole or an Fc region comprising a knob), for example, as shown in the structure of FIG. 20C.

In some embodiments, the fusion includes direct fusion or fusion through a linker.

Therefore, in some embodiments, the present invention relates to a bispecific antibody which is an IgG-like bispecific antibody comprising one Fab fragment specifically binding to a first antigen, one scFv specifically binding to a second antigen, and an Fc heterodimer, wherein
the Fab fragment comprises VH-CH1 and VL-CL, and the scFv comprises VH-linker-VL or VL-linker-VH;
a first Fc region constitutes a first heavy chain;
the C-terminus of the CH1 of the Fab fragment is fused to the N-terminus of the scFv fragment, and the C-terminus of the scFv fragment is fused to a CH2 or a hinge region of a second Fc region, thereby constituting a second heavy chain;
   and
the VL-CL of the Fab fragment constitutes a light chain.

In some other embodiments, the present invention relates to a bispecific antibody which is an IgG-like bispecific antibody comprising two Fab fragments specifically binding to a first antigen, one scFv specifically binding to a second antigen, and an Fc heterodimer, wherein
the Fab fragment comprises VH-CH1 and VL-CL, and the scFv comprises VH-linker-VL or VL-linker-VH;
the C-terminus of the CH1 of the first Fab fragment is fused to a CH2 or a hinge region of a first Fc region, thereby constituting a first heavy chain;
the C-terminus of the CH1 of the second Fab fragment is fused to the N-terminus of the VH of the scFv fragment, and the C-terminus of the scFv fragment is fused to a CH2 or a hinge region of a second Fc region, thereby constituting a second heavy chain;
   and
the VL-CL of the first and second Fab fragments constitute two light chains;
wherein the first Fab and second Fab fragments are identical or different.

In some other embodiments, the present invention relates to a bispecific antibody which is an IgG-like bispecific antibody comprising one Fab fragments specifically binding to a first antigen, one scFv specifically binding to a second antigen, and an Fc heterodimer, wherein
the Fab fragment comprises VH-CH1 and VL-CL, and the scFv comprises VH-linker-VL or VL-linker-VH;
the C-terminus of the CH1 of the Fab fragment is fused to a CH2 or a hinge region of a first Fc region, thereby constituting a first heavy chain;
the C-terminus of the scFv fragment is fused to a CH2 or a hinge region of a second Fc region, thereby constituting a second heavy chain;
   and
the VL-CL of the Fab fragment constitutes a light chain.

In some other embodiments, the present invention relates to a bispecific antibody which is an IgG-like bispecific antibody comprising two Fab fragments specifically binding to a first antigen, one scFv specifically binding to a second antigen, and an Fc heterodimer, wherein
the Fab fragment comprises VH-CH1 and VL-CL, and the scFv comprises VH-linker-VL or VL-linker-VH;
the C-terminus of the CH1 of the first Fab fragment is fused to the N-terminus of the VH of the second Fab fragment, and the C-terminus of the CH1 of the second Fab fragment is fused to a CH2 or a hinge region of a first Fc region, thereby constituting a first heavy chain;
the C-terminus of the scFv fragment is fused to a CH2 or a hinge region of a second Fc region, thereby constituting a second heavy chain; and
the VL-CL of the first and second Fab fragments constitute two light chains;
wherein the first Fab and second Fab fragments are identical or different.

In some embodiments, the first antigen is selected from GPRC5D or MUC16 (e.g., human GPRC5D or human MUC16). In some embodiments, the second antigen is CD3.

In some embodiments, the first Fc region comprises a knob mutation, and the second Fc region comprises a hole mutation. In some embodiments, the first Fc region comprises a hole mutation, and the second Fc region comprises a knob mutation.

In some embodiments, the C-terminus of the scFv fragment is the C-terminus of the VL of the ScFv fragment. In some embodiments, the N-terminus of the scFv fragment is the N-terminus of the VH of the ScFv fragment.

### ➢ Fab fragment suitable for use in bispecific antibody of the present invention

In some embodiments, the Fab fragment that is one of the antigen-binding regions of the bispecific antibody consists of two polypeptide chains comprising VH, CH1, VL, and CL domains of the antibody, wherein the VH is paired with the VL, and the CH1 is paired with the CL to form the antigen-binding region. In some embodiments, in the Fab, one chain comprises, from N-terminus to C-terminus, VH and CH1 (i.e., VH-CH1), and the other chain comprises, from N-terminus to C-terminus, VL and CL (i.e., VL-CL).

In some embodiments, in the bispecific antibody, the Fab may be fused to the N-terminus of the Fc domain of the antibody via the C-terminus of the chain comprising the VH, or fused to the N-terminus of the Fc domain of the antibody via another antigen-binding region, such as an scFv fragment, wherein the Fc domain may or may not comprise a hinge region (e.g., EPKSS or EPKSC). Preferably, the Fab comprises a VH-CH1 chain and a VL-CL chain and is fused to the Fc domain of the antibody via the C-terminus of the CH1 of the VH-CH1 chain, wherein the Fc domain may or may not comprise a hinge region (e.g., EPKSS or EPKSC). Herein, a Fab chain linked to an Fc dimer is also referred to as a Fab heavy chain, and a Fab chain not linked to an Fc dimer is also referred to as a Fab light chain. In some embodiments, the fusion is direct fusion or fusion through a linker.

In some embodiments, the Fab fragment contained in the bispecific antibody of the present invention specifically binds to GPRC5D. In some embodiments, the Fab fragment contained in the bispecific antibody of the present invention is from the anti-GPRC5D antibody of the present invention and comprises the heavy chain variable region VH and the light chain variable region VL of the anti-GPRC5D antibody of the present invention.

In some embodiments, the Fab heavy chain comprises VH and CH1, wherein the VH is the VH of the anti-GPRC5D antibody of the present invention. In some embodiments, the CH1 is a CH1 from IgG1, IgG2, IgG3, or IgG4, preferably a CH1 from IgG1. In some embodiments, the CH1
(i) comprises or consists of an amino acid sequence having at least 85%, 90%, 91%, 92%, 93%, 94%, 95%, 96%, 97%, 98% or 99% identity to an amino acid sequence selected from SEQ ID NO: 44;
(ii) comprises or consists of an amino acid sequence selected from SEQ ID NO: 44; or
(iii) comprises or consists of an amino acid sequence having one or more (preferably no more than 10 or 10, and more preferably no more than 5, 4, 3, 2, or 1) amino acid changes (preferably amino acid replacements, and more preferably conservative amino acid replacements) compared to an amino acid sequence selected from SEQ ID NO: 44.

In some embodiments, the Fab light chain comprises VL-CL, wherein the VL is the VL of the anti-GPRC5D antibody of the present invention. In some embodiments, the CL is a light chain constant region from an antibody kappa or lambda light chain, preferably a light chain constant region from a kappa light chain. In some embodiments, the CL is the light chain constant region of the anti-GPRC5D antibody of the present invention. In some embodiments, the Fab light chain is the light chain of the anti-GPRC5D antibody of the present invention.

In some embodiments, the Fab fragment contained in the bispecific antibody of the present invention specifically binds to MUC16. In some embodiments, the Fab fragment contained in the bispecific antibody of the present invention is from the anti-MUC16 antibody of the present invention and comprises the heavy chain variable region VH and the light chain variable region VL of the anti-MUC16 antibody of the present invention.

In some embodiments, the Fab heavy chain comprises VH and CH1, wherein the VH is the VH of the anti-MUC16 antibody of the present invention. In some embodiments, the CH1 is a CH1 from IgG1, IgG2, IgG3, or IgG4, preferably a CH1 from IgG1. In some embodiments, the CH1
(i) comprises or consists of an amino acid sequence having at least 85%, 90%, 91%, 92%, 93%, 94%, 95%, 96%, 97%, 98% or 99% identity to an amino acid sequence selected from SEQ ID NO: 44;
(ii) comprises or consists of an amino acid sequence selected from SEQ ID NO: 44; or
(iii) comprises or consists of an amino acid sequence having one or more (preferably no more than 10 or 10, and more preferably no more than 5, 4, 3, 2, or 1) amino acid changes (preferably amino acid replacements, and more preferably conservative amino acid replacements) compared to an amino acid sequence selected from SEQ ID NO: 44.

In some embodiments, the Fab light chain comprises VL-CL, wherein the VL is the VL of the anti-MUC16 antibody of the present invention. In some embodiments, the CL is a light chain constant region from an antibody kappa or lambda light chain, preferably a light chain constant region from a kappa light chain. In some embodiments, the CL is the light chain constant region of the anti-MUC16 antibody of the present invention. In some embodiments, the Fab light chain is the light chain of the anti-MUC16 antibody of the present invention.

### ➢ scFv suitable for use in bispecific antibody of the present invention

In some embodiments, the scFv fragment that is one of the binding regions of the bispecific antibody consists of one polypeptide chain comprising VH and VL domains of the antibody, wherein the VH and the VL are linked (e.g., via a linker) to be paired to form the antigen-binding site. In some embodiments, the scFv is a trans-configuration comprising: a VH, a linker and a VL (VH-linker-VL), from N-terminus to C-terminus. In some other embodiments, the scFv is a cis-configuration comprising: a VL, a linker and a VH (VL-linker-VH), from N-terminus to C-terminus. In some embodiments, the linker is a peptide linker consisting of amino acid residues. Suitable peptide linkers are known to those skilled in the art. In one embodiment, the linker has a length of 5-50 amino acids, such as 5-30 amino acids, for example, 15 amino acids or 20 amino acids. In one embodiment, the linker comprises an amino acid sequence (G4S)n, wherein n = 1, 2, 3, 4, or 5, preferably n = 3 or 4, more preferably n = 3.

In some preferred embodiments, the scFv antigen-binding site contained in the antibody molecule of the present invention is a disulfide-stabilized scFv.

In some embodiments, in the bispecific antibody, the scFv may be fused via the C-terminus of the chain to the N-terminus of the Fc domain of the antibody. In some embodiments, in the bispecific antibody, the scFv is fused at the N-terminus of the chain to the C-terminus of another antigen-binding region (e.g., the Fab heavy chain) and fused at the C-terminus of the chain to the N-terminus of the Fc domain of the antibody.

In some embodiments, in the bispecific antibody, the scFv may be fused via the C-terminus of the chain comprising the VL to the N-terminus of the Fc domain of the antibody. In some embodiments, in the bispecific antibody, the scFv is fused at the N-terminus of the VH chain to the C-terminus of another antigen-binding region (e.g., the Fab heavy chain) and fused at the C-terminus of the VL chain to the N-terminus of the Fc domain of the antibody.

In some embodiments, in the bispecific antibody, the scFv may be fused via the C-terminus of the chain comprising the VH to the N-terminus of the Fc domain of the antibody. In some embodiments, in the bispecific antibody, the scFv is fused at the N-terminus of the VL chain to the C-terminus of another antigen-binding region (e.g., the Fab heavy chain) and fused at the C-terminus of the VH chain to the N-terminus of the Fc domain of the antibody.

In some embodiments, the scFv contained in the bispecific antibody of the present invention specifically binds to CD3. In some embodiments, the scFv contained in the bispecific antibody of the present invention is from the anti-CD3 antibody of the present invention and comprises the heavy chain variable region VH and the light chain variable region VL of the anti-CD3 antibody of the present invention.

### ➢ Fc dimer suitable for use in bispecific antibody of the present invention

In one embodiment, two Fc regions in the bispecific antibody of the present invention form a dimeric Fc by dimerization. Preferably, the two Fc regions form a heterodimeric Fc by heterodimerization.

In some embodiments, the first and second Fc regions are identical. In some other embodiments, the first Fc region and the second Fc region are different, and the two are paired and heterodimerized.

The Fc region fragment suitable for use in the antibody molecule of the present invention may be any antibody Fc region. The Fc region may include a native sequence Fc region and a variant Fc region. The native sequence Fc domain encompasses naturally occurring Fc sequences of various immunoglobulins, such as Fc regions of various Ig subtypes and allotypes thereof (Gestur Vidarsson et al., IgG subclasses and allotypes: from structure to effector functions, 20 October 2014, doi: 10.3389/fimmu.2014.00520.). For example, the Fc region of the antibody of the present invention may comprise two or three constant domains, i.e., a CH2 domain, a CH3 domain, and optionally a CH4 domain. In some embodiments, the antibody Fc region may also bear an IgG hinge region or a portion of the IgG hinge region, such as an IgG1 hinge region or a portion of the IgG1 hinge region, at the N-terminus. The hinge region may comprise a mutation. In some embodiments, the hinge region may be EPKSS or EPKSC.

Preferably, the Fc region of the antibody of the present invention comprises CH2-CH3 from N-terminus to C-terminus, or comprises hinge region-CH2-CH3 from N-terminus to C-terminus. In some embodiments, the Fc region suitable for use in the antibody or the bispecific antibody of the present invention is a human IgG Fc, such as human IgG1 Fc, human IgG2 Fc, or human IgG3 or human IgG4 Fc. In one embodiment, the Fc region comprises or consists of an amino acid sequence set forth in SEQ ID NO: 49, or an amino acid sequence having at least 90% identity, e.g., 95%, 96%, 97%, 99% or more identity, thereto.

The Fc region in the antibody or the bispecific antibody of the present invention may be mutated to obtain desired properties. Mutations for the Fc region are known in the art.

In one embodiment, the Fc region is modified in properties of the effector function of the Fc region (e.g., complement activation function of the Fc region). In one embodiment, the effector function has been reduced or eliminated relative to a wild-type isotype Fc region. In one embodiment, the effector function is reduced or eliminated by using a method selected from: using an Fc isotype that naturally has a reduced or an eliminated effector function, and performing Fc region modification.

In a preferred embodiment, the Fc region has a reduced effector function mediated by the Fc region, such as a reduced or an eliminated ADCC or ADCP or CDC effector function, for example, comprising a mutation for achieving the above function.

As understood by those skilled in the art, according to the expected use of the antibody molecule of the present invention, the antibody molecule of the present invention may further comprise a modification in the Fc domain that alters the binding affinity to one or more Fc receptors. In one embodiment, the Fc receptor is an Fcy receptor, in particular a human Fcγ receptor. In some embodiments, the Fc region comprises a mutation that reduces binding to the Fcy receptor. For example, in some embodiments, the Fc region used in the present invention has one or more of an L234A/L235A mutation, a D265A mutation and a P329A mutation, which reduce binding to the Fcγ receptor. In some embodiments, the Fc region used in the present invention has an L234A/L235A mutation, a D265A mutation and a P329A mutation, which reduce binding to the Fcγ receptor. In yet another preferred embodiment, the Fc fragment may have a mutation that leads to an increased serum half-life, e.g., a mutation that improves the binding of the Fc fragment to FcRn. In some embodiments, the Fc region comprising a mutation that reduces binding to the Fcy receptor comprises or consists of an amino acid sequence set forth in SEQ ID NO: 46, or an amino acid sequence having at least 90% identity, e.g., 95%, 96%, 97%, 99% or more identity, thereto. In some embodiments, the Fc region comprises an amino acid sequence having at least 90% identity, e.g., 95%, 96%, 97%, 99% or more identity, to SEQ ID NO: 46 and comprises an L234A/L235A mutation, a D265A mutation and a P329A mutation.

As understood by those skilled in the art, to facilitate the formation of the bispecific antibody of the present invention as a heterodimer, the Fc region contained in the bispecific antibody of the present invention may comprise mutations that favor the heterodimerization. In one embodiment, mutations are introduced into the CH3 regions of the two Fc regions.

Methods for facilitating the heterodimerization of the Fc regions are known in the art. For example, the CH3 region of the first Fc region and the CH3 region of the second Fc region are engineered in a complementary manner, such that each CH3 region (or the heavy chain comprising the same) can be no longer homodimerized with itself but forced to be heterodimerized with other CH3 regions that are complementarily engineered (such that heterodimerization occurs between the first and second CH3 regions and no homodimers are formed between the two first CH3 regions or the two second CH3 regions).

Preferably, based on the knob-in-hole technique, a corresponding knob mutation and a corresponding hole mutation are introduced into the first monomeric Fc region and the second monomeric Fc region, respectively. For this technique, see, e.g., Merchant, A. M., et al. (1998). "An efficient route to human bispecific IgG." Nat Biotechnol 16(7): 677-681.

In a particular embodiment, in the CH3 region of one Fc region, the threonine residue at position 366 is substituted with a tryptophan residue (T366W) (knob mutation); while in the CH3 region of the other Fc region, the tyrosine residue at position 407 is substituted with a valine residue (Y407V) (hole mutation), optionally the threonine residue at position 366 is substituted with a serine residue (T366S), and the leucine residue at position 368 is substituted with an alanine residue (L368A) (numbering according to the EU index).

In yet another embodiment, in the CH3 region of one Fc region, the knob mutations comprise or consist of: a substitution of the threonine residue at position 366 with a tryptophan residue (T366W) and a substitution of the serine residue at position 354 with a cysteine residue (S354C) or a substitution of the glutamic acid residue at position 356 with a cysteine residue (E356C) (in particular, a substitution of the serine residue at position 354 with a cysteine residue); while in the CH3 region of the other Fc region, the hole mutations comprise or consist of: a substitution of the tyrosine residue at position 407 with a valine residue (Y407V), optionally a substitution of the threonine residue at position 366 with a serine residue (T366S) and a substitution of the leucine residue at position 368 with an alanine residue (L368A) (numbering according to the EU index), and optionally a substitution of the tyrosine residue at position 349 with a cysteine residue (Y349C) (numbering according to the EU index).

In a specific embodiment, one Fc region comprises amino acid substitutions S354C and T366W (knob mutations), and the other Fc region comprises amino acid substitutions Y349C, T366S, L368A and Y407V (hole mutations) (numbering according to the EU index).

Thus, in a specific embodiment, the two Fc regions contained in the bispecific antibody of the present invention are heterodimerized, wherein
a) one Fc-region polypeptide comprises a mutation T366W, and the other Fc-region polypeptide comprises mutations T366S, L368A and Y407V, or
b) one Fc-region polypeptide comprises mutations T366W and Y349C, and the other Fc-region polypeptide comprises mutations T366S, L368A, Y407V and S354C, or
c) one Fc-region polypeptide comprises mutations T366W and S354C, and the other Fc-region polypeptide comprises mutations T366S, L368A, Y407V and Y349C;
optionally, the Fc region further comprises a mutation that reduces binding to the Fcy receptor, e.g., one or more of an L234A/L235A mutation, a D265A mutation and a P329A mutation, for example, an L234A/L235A mutation, a D265A mutation and a P329A mutation.

In some embodiments, the Fc region further comprises additional mutations that favor the purification of the heterodimer. For example, an H435R mutation (Eric J. Smith, *Scientific Reports* 15:17943 | DOI: 10.1038/srep17943) can be introduced into one of the Fc regions of the heterodimer (e.g., an Fc region with hole mutations) to facilitate the purification of the heterodimer using protein A. In some other embodiments, for heterodimeric monomers comprising a hinge region, mutations such as C220S may also be introduced into the hinge region to facilitate the formation of the heterodimer.

In a specific embodiment, the two Fc regions of the bispecific antibody of the present invention are heterodimerized, wherein
the first Fc region comprises a knob mutation, and comprises or consists of an amino acid sequence set forth in SEQ ID NO: 43 or 89, or an amino acid sequence having at least 90% identity, e.g., 95%, 96%, 97%, 99% or more identity, thereto; in some embodiments, the Fc region comprises an amino acid sequence having at least 90% identity, e.g., 95%, 96%, 97%, 99% or more identity, to SEQ ID NO: 43 or 89 and comprises an L234A/L235A mutation, a D265A mutation, a P329A mutation, and a knob mutation (e.g., S354C and T366W); in some embodiments, the Fc region comprises or does not comprise a hinge region EPKSS or EPKSC;
the second Fc region comprises a hole mutation, and comprises or consists of an amino acid sequence set forth in SEQ ID NO: 30, 42 or 59, or an amino acid sequence having at least 90% identity, e.g., 95%, 96%, 97%, 99% or more identity, thereto. In some embodiments, the Fc region comprises an amino acid sequence having at least 90% identity, e.g., 95%, 96%, 97%, 99% or more identity, to SEQ ID NO: 30, 42 or 59 and comprises an L234A/L235A mutation, a D265A mutation, a P329A mutation, and a knob mutation; in some embodiments, the Fc region comprises or does not comprise a hinge region EPKSS or EPKSC.

### ➢ Exemplary bispecific antibody molecule

### (I) Bispecific antibody specifically binding to GPRC5D and CD3

In some preferred embodiments, the present invention provides a bispecific antibody, comprising one or two Fab fragments of an anti-GPRC5D antibody, one scFv of an anti-CD3 antibody, and an Fc heterodimer, wherein
(1) the anti-GPRC5D Fab fragment is fused at the C-terminus of a CH1 of a Fab heavy chain to the N-terminus of a VH of the scFv fragment of the anti-CD3 antibody, and the C-terminus of a VL of the scFv fragment is fused to a CH2 or a hinge region of one of Fc regions of the Fc heterodimer (e.g., an Fc region comprising a knob), for example, as shown in the structure of FIG. 4A;
(2) the first anti-GPRC5D Fab fragment is fused at the C-terminus of a CH1 of a Fab heavy chain to a CH2 or a hinge region of one of Fc regions of the Fc heterodimer (e.g., an Fc region comprising a hole); the second anti-GPRC5D Fab fragment is fused at the C-terminus of a CH1 of a Fab heavy chain to the N-terminus of a VH of the scFv fragment of the anti-CD3 antibody, and the C-terminus of a VL of the scFv fragment is fused to a CH2 or a hinge region of another Fc region of the Fc heterodimer (e.g., an Fc region comprising a knob), for example, as shown in the structure of FIG. 4B;
(3) the anti-GPRC5D Fab fragment is fused at the C-terminus of a CH1 of a Fab heavy chain to a CH2 or a hinge region of one of Fc regions of the Fc heterodimer (e.g., an Fc region comprising a hole); the C-terminus of a VL of the scFv fragment of the anti-CD3 antibody is fused to a CH2 or a hinge region of another Fc region of the Fc heterodimer (e.g., an Fc region comprising a knob), for example, as shown in the structure of FIG. 4C.

In some embodiments, the fusion includes direct fusion or fusion through a linker.

Thus, in some embodiments, the present invention relates to a bispecific antibody which is an IgG-like bispecific antibody comprising one Fab fragment of an anti-GPRC5D antibody, one scFv of an anti-CD3 antibody, and an Fc heterodimer, wherein
the Fab fragment comprises VH-CH1 and VL-CL, and the scFv comprises VH-linker-VL;
an Fc region comprising a hole mutation constitutes a first heavy chain;
the C-terminus of the CH1 of the Fab fragment is fused to the N-terminus of the VH of the scFv fragment, and the C-terminus of the VL of the scFv fragment is fused to a CH2 or a hinge region of an Fc region comprising a knob mutation, thereby constituting a second heavy chain;
   and
the VL-CL of the Fab fragment constitutes a light chain.

In some embodiments, the first heavy chain comprises or consists of an amino acid sequence set forth in SEQ ID NO: 30, or an amino acid sequence having at least 85%, 90%, 91%, 92%, 93%, 94%, 95%, 96%, 97%, 98% or 99% identity thereto;
the second heavy chain comprises or consists of an amino acid sequence set forth in SEQ ID NO: 31, 34 or 35, or an amino acid sequence having at least 85%, 90%, 91%, 92%, 93%, 94%, 95%, 96%, 97%, 98% or 99% identity thereto; and/or
the light chain comprises or consists of an amino acid sequence set forth in SEQ ID NO: 20, or an amino acid sequence having at least 85%, 90%, 91%, 92%, 93%, 94%, 95%, 96%, 97%, 98% or 99% identity thereto.

In some other embodiments, the present invention relates to a bispecific antibody which is an IgG-like bispecific antibody comprising two Fab fragments of an anti-GPRC5D antibody, one scFv of an anti-CD3 antibody, and an Fc heterodimer, wherein
the Fab fragment comprises VH-CH1 and VL-CL, and the scFv comprises VH-linker-VL;
the C-terminus of the CH1 of the first Fab fragment is fused to a CH2 or a hinge region of an Fc region comprising a hole mutation, thereby constituting a first heavy chain;
the C-terminus of the CH1 of the second Fab fragment is fused to the N-terminus of the VH of the scFv fragment, and the C-terminus of the VL of the scFv fragment is fused to a CH2 or a hinge region of an Fc region comprising a knob mutation, thereby constituting a second heavy chain;
   and
the VL-CL of the first and second Fab fragments constitute two light chains;
wherein the first Fab and second Fab fragments are identical or different.

In some embodiments, the first heavy chain comprises or consists of an amino acid sequence set forth in SEQ ID NO: 32, 33, or 36, or an amino acid sequence having at least 85%, 90%, 91%, 92%, 93%, 94%, 95%, 96%, 97%, 98% or 99% identity thereto;
the second heavy chain comprises or consists of an amino acid sequence set forth in SEQ ID NO: 31, 34 or 35, or an amino acid sequence having at least 85%, 90%, 91%, 92%, 93%, 94%, 95%, 96%, 97%, 98% or 99% identity thereto; and/or
the light chain comprises or consists of an amino acid sequence set forth in SEQ ID NO: 20, or an amino acid sequence having at least 85%, 90%, 91%, 92%, 93%, 94%, 95%, 96%, 97%, 98% or 99% identity thereto.

In some embodiments, the first heavy chain comprises or consists of an amino acid sequence set forth in SEQ ID NO: 33, or an amino acid sequence having at least 85%, 90%, 91%, 92%, 93%, 94%, 95%, 96%, 97%, 98% or 99% identity thereto;
the second heavy chain comprises or consists of an amino acid sequence set forth in SEQ ID NO: 31, or an amino acid sequence having at least 85%, 90%, 91%, 92%, 93%, 94%, 95%, 96%, 97%, 98% or 99% identity thereto; and/or
the light chain comprises or consists of an amino acid sequence set forth in SEQ ID NO: 20, or an amino acid sequence having at least 85%, 90%, 91%, 92%, 93%, 94%, 95%, 96%, 97%, 98% or 99% identity thereto.

In some embodiments, the first heavy chain comprises or consists of an amino acid sequence set forth in SEQ ID NO: 32, or an amino acid sequence having at least 85%, 90%, 91%, 92%, 93%, 94%, 95%, 96%, 97%, 98% or 99% identity thereto;
the second heavy chain comprises or consists of an amino acid sequence set forth in SEQ ID NO: 34, or an amino acid sequence having at least 85%, 90%, 91%, 92%, 93%, 94%, 95%, 96%, 97%, 98% or 99% identity thereto; and/or
the light chain comprises or consists of an amino acid sequence set forth in SEQ ID NO: 20, or an amino acid sequence having at least 85%, 90%, 91%, 92%, 93%, 94%, 95%, 96%, 97%, 98% or 99% identity thereto.

In some embodiments, the first heavy chain comprises or consists of an amino acid sequence set forth in SEQ ID NO: 36, or an amino acid sequence having at least 85%, 90%, 91%, 92%, 93%, 94%, 95%, 96%, 97%, 98% or 99% identity thereto;
the second heavy chain comprises or consists of an amino acid sequence set forth in SEQ ID NO: 35, or an amino acid sequence having at least 85%, 90%, 91%, 92%, 93%, 94%, 95%, 96%, 97%, 98% or 99% identity thereto; and/or
the light chain comprises or consists of an amino acid sequence set forth in SEQ ID NO: 20, or an amino acid sequence having at least 85%, 90%, 91%, 92%, 93%, 94%, 95%, 96%, 97%, 98% or 99% identity thereto.

In some other embodiments, the present invention relates to a bispecific antibody which is an IgG-like bispecific antibody comprising one Fab fragment of an anti-GPRC5D antibody, one scFv of an anti-CD3 antibody, and an Fc heterodimer, wherein
the Fab fragment comprises VH-CH1 and VL-CL, and the scFv comprises VH-linker-VL;
the C-terminus of the CH1 of the Fab fragment is fused to a CH2 or a hinge region of an Fc region comprising a hole mutation, thereby constituting a first heavy chain;
the C-terminus of the VL of the scFv fragment is fused to a CH2 or a hinge region of an Fc region comprising a knob mutation, thereby constituting a second heavy chain;
   and
the VL-CL of the Fab fragment constitutes a light chain.

In some embodiments, the first heavy chain comprises or consists of an amino acid sequence set forth in SEQ ID NO: 32, 33, or 36, or an amino acid sequence having at least 85%, 90%, 91%, 92%, 93%, 94%, 95%, 96%, 97%, 98% or 99% identity thereto;
the second heavy chain comprises or consists of an amino acid sequence set forth in SEQ ID NO: 37, or an amino acid sequence having at least 85%, 90%, 91%, 92%, 93%, 94%, 95%, 96%, 97%, 98% or 99% identity thereto; and/or
the light chain comprises or consists of an amino acid sequence set forth in SEQ ID NO: 20, or an amino acid sequence having at least 85%, 90%, 91%, 92%, 93%, 94%, 95%, 96%, 97%, 98% or 99% identity thereto.

### (II) Bispecific antibody specifically binding to CD3 and MUC16

The present invention provides a bispecific antibody (anti-CD3×MUC16 bispecific antibody) targeting both human CD3 and human MUC16, which has the following advantages:
(1) binding to human CD3 and target cells expressing human CD3 with high specificity, while binding to human MUC16 and target cells expressing human MUC16 with high affinity;
(2) not binding to circulating free CA125, while targeting more T cells to cancer cells expressing MUC 16;
(3) activating T cells and eliciting cytotoxic activity against the cancer cells expressing MUC16, thereby effectively killing the cancer cells;
(4) low immunogenicity; and
(5) an excellent anti-tumor effect.

In one embodiment, the present invention provides an anti-CD3×MUC16 bispecific antibody, comprising:
(i) one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule; or
(ii) one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule; or
(iii) one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule.

In one embodiment, the present invention provides an anti-CD3×MUC16 bispecific antibody, comprising:
(i) one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 97, an LCDR2 set forth in SEQ ID NO: 98, and an LCDR3 set forth in SEQ ID NO: 99; the scFv recognizing the CD3 molecule comprises a VH recognizing CD3 (referred to herein as VH_{CD3} for distinction from the VH recognizing MUC16) and a VL recognizing CD3 (referred to herein as VL_{CD3} for distinction from the VL recognizing MUC16), wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
   one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 101, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
   one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 103, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10;
      or
(ii) one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 97, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
   one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 101, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
   one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 103, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
(iii) one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 97, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
   one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 101, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
   one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 103, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10.

In a specific embodiment, the present invention provides an anti-CD3×MUC16 bispecific antibody, comprising:
(i) one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises a sequence selected from SEQ ID NO: 92 or 113, and the VL recognizing the MUC16 molecule comprises a sequence selected from SEQ ID NO: 96, 100, 102 or 114; the scFv recognizing the CD3 molecule comprises a VH recognizing CD3 (referred to herein as VH_{CD3} for distinction from the VH recognizing MUC16) and a VL recognizing CD3 (referred to herein as VL_{CD3} for distinction from the VL recognizing MUC16), wherein the VH_{CD3} comprises a sequence set forth in SEQ ID NO: 3, and the VL_{CD3} comprises a sequence set forth in SEQ ID NO: 4;
(ii) one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises a sequence selected from SEQ ID NO: 92 or 113, and the VL recognizing the MUC16 molecule comprises a sequence selected from SEQ ID NO: 96, 100, 102 or 114; the scFv recognizing the CD3 molecule comprises a VH_{CD3} and a VL_{CD3}, wherein the VH_{CD3} comprises a sequence set forth in SEQ ID NO: 3, and the VL_{CD3} comprises a sequence set forth in SEQ ID NO: 4; or
(iii) one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises a sequence selected from SEQ ID NO: 92 or 113, and the VL recognizing the MUC16 molecule comprises a sequence selected from SEQ ID NO: 96, 100, 102 or 114; the scFv recognizing the CD3 molecule comprises a VH_{CD3} and a VL_{CD3}, wherein the VH_{CD3} comprises a sequence set forth in SEQ ID NO: 3, and the VL_{CD3} comprises a sequence set forth in SEQ ID NO: 4.

In one embodiment, the two antigen recognition sites recognizing the MUC16 molecule contained in the bispecific antibody described above can have either identical VH/VL sequences or different VH/VL sequences.

In another embodiment, the present invention provides an anti-CD3×MUC16 bispecific antibody, comprising:
(i) one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises a sequence of SEQ ID NO: 113, or a sequence having at least 90%, 91%, 92%, 93%, 94%, 95%, 96%, 97%, 98%, 99% or more sequence identity and the same CDRs as compared to SEQ ID NO: 113, and the VL recognizing the MUC16 molecule comprises a sequence selected from SEQ ID NO: 114, or a sequence having at least 90%, 91%, 92%, 93%, 94%, 95%, 96%, 97%, 98%, 99% or more sequence identity and the same CDRs as compared to SEQ ID NO: 114; the scFv recognizing the CD3 molecule comprises a VH_{CD3} and a VL_{CD3}, wherein the VH_{CD3} comprises a sequence set forth in SEQ ID NO: 3, or a sequence having at least 90%, 91%, 92%, 93%, 94%, 95%, 96%, 97%, 98%, 99% or more sequence identity and the same CDRs as compared to SEQ ID NO: 3, and the VL_{CD3} comprises a sequence set forth in SEQ ID NO: 4, or a sequence having at least 90%, 91%, 92%, 93%, 94%, 95%, 96%, 97%, 98%, 99% or more sequence identity and the same CDRs as compared to SEQ ID NO: 4; or
(ii) one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises a sequence selected from SEQ ID NO: 113, or a sequence having at least 90%, 91%, 92%, 93%, 94%, 95%, 96%, 97%, 98%, 99% or more sequence identity and the same CDRs as compared to SEQ ID NO: 113, and the VL recognizing the MUC16 molecule comprises a sequence selected from SEQ ID NO: 114, or a sequence having at least 90%, 91%, 92%, 93%, 94%, 95%, 96%, 97%, 98%, 99% or more sequence identity and the same CDRs as compared to SEQ ID NO: 114; the scFv recognizing the CD3 molecule comprises a VH_{CD3} and a VL_{CD3}, wherein the VH_{CD3} comprises a sequence set forth in SEQ ID NO: 3, or a sequence having at least 90%, 91%, 92%, 93%, 94%, 95%, 96%, 97%, 98%, 99% or more sequence identity and the same CDRs as compared to SEQ ID NO: 3, and the VL_{CD3} comprises a sequence set forth in SEQ ID NO: 4, or a sequence having at least 90%, 91%, 92%, 93%, 94%, 95%, 96%, 97%, 98%, 99% or more sequence identity and the same CDRs as compared to SEQ ID NO: 4; or
(iii) one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises a sequence selected from SEQ ID NO: 113, or a sequence having at least 90%, 91%, 92%, 93%, 94%, 95%, 96%, 97%, 98%, 99% or more sequence identity and the same CDRs as compared to SEQ ID NO: 113, and the VL recognizing the MUC16 molecule comprises a sequence selected from SEQ ID NO: 114, or a sequence having at least 90%, 91%, 92%, 93%, 94%, 95%, 96%, 97%, 98%, 99% or more sequence identity and the same CDRs as compared to SEQ ID NO: 114; the scFv recognizing the CD3 molecule comprises a VH_{CD3} and a VL_{CD3}, wherein the VH_{CD3} comprises a sequence set forth in SEQ ID NO: 3, or a sequence having at least 90%, 91%, 92%, 93%, 94%, 95%, 96%, 97%, 98%, 99% or more sequence identity and the same CDRs as compared to SEQ ID NO: 3, and the VL_{CD3} comprises a sequence set forth in SEQ ID NO: 4, or a sequence having at least 90%, 91%, 92%, 93%, 94%, 95%, 96%, 97%, 98%, 99% or more sequence identity and the same CDRs as compared to SEQ ID NO: 4.

In one embodiment, in the anti-CD3×MUC16 bispecific antibody as provided herein above, the Fc region may be selected from native Fc regions of any IgG, IgA or IgM class antibodies known in the art, such as a consensus or germline IgG Fc region. In a specific embodiment, the IgG Fc region may be from an isotype human IgG1, IgG2, IgG3 or IgG4. In a specific embodiment, the IgG Fc region is an Fc sequence of human IgG1. In a further embodiment, the Fc region comprises a mutation, e.g., a mutation that increases the dimer stability of the bispecific antibody (e.g., the knob-in-hole structure) and a mutation that reduces or increases an effector function.

In any one of the above embodiments, the scFv of the anti-CD3×MUC16 bispecific antibody of the present invention is linked to the Fc or the CH1 via a linker, wherein the linker may be any common flexible sequence known in the art or available in the future, typically a short flexible amino acid sequence, such as GGGSG; GGSGG; GSGGG; SGGGG; GGTGS; GTSPGG; GNGGGS; G4S-GGSGG-G4S-SGGGG; GGG; DGGGS; TGEKP; GGRR; EGKSSGSGSESKVD; KESGSVSSEQLAQFRSLD; GGRRGGGS; LRQRDGERP; LRQKDGGGSERP; GSTSGSGKPGSGEGSTKG; and the like. In a specific embodiment, the linker is (G4S)n, wherein n is an integer equal to or greater than 1, for example, n is an integer of 1, 2, 3, 4, 5, 6, 7, 8, or 9. In a specific embodiment, the linker is selected from (G4S)3.

In yet another embodiment, the present invention provides an anti-CD3×MUC16 bispecific antibody, comprising:
(i) one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the heavy chain 1 comprises a sequence set forth in SEQ ID NO: 118, the heavy chain 2 comprises a sequence set forth in SEQ ID NO: 121, and the light chains comprise a sequence set forth in SEQ ID NO: 116;
(ii) one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the heavy chain 1 comprises a sequence set forth in SEQ ID NO: 118, the heavy chain 2 comprises a sequence set forth in SEQ ID NO: 117, and the light chain comprises a sequence set forth in SEQ ID NO: 116; or
(iii) one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the heavy chain 1 comprises a sequence set forth in SEQ ID NO: 119, the heavy chain 2 comprises a sequence set forth in SEQ ID NO: 117, and the light chains comprise a sequence set forth in SEQ ID NO: 116.

### VI. Nucleic acid encoding antibody and host cell comprising the same

In one aspect, the present invention provides a nucleic acid encoding any of the above anti-CD3 antibodies or anti-GPRC5D antibodies or anti-MUC16 antibodies or bispecific antibodies or any one of the chains thereof.

For example, the nucleic acid of the present invention includes a nucleic acid encoding an amino acid sequence selected from any one of SEQ ID NOs: 1, 2, 3, 4, 11, 13, 14-29, 31-38, 40, 60-63, 70-73, 80-83, 92, 96, 100, 102, 106-109, 113-119 and 121, or a nucleic acid encoding an amino acid sequence having at least 85%, 90%, 91%, 92%, 93%, 94%, 95%, 96%, 97%, 98% or 99% identity to an amino acid sequence selected from any one of SEQ ID NOs: 1, 2, 3, 4, 11, 13, 14-29, 31-38, 40, 60-63, 70-73, 80-83, 92, 96, 100, 102, 106-109, 113-119 and 121. As will be understood by those skilled in the art, each antibody or polypeptide amino acid sequence can be encoded by a variety of nucleic acid sequences because of codon degeneracy. Nucleic acid sequences encoding the molecules of the present invention may be produced using methods well known in the art, for example by *de novo* solid phase DNA synthesis, or by PCR amplification.

In one aspect, the present invention provides a nucleic acid encoding any of the above antibodies or any of the chains of the antibodies. When expressed in a suitable expression vector, a polypeptide encoded by the nucleic acid is capable of exhibiting binding capacity to human (and/or monkey (e.g., rhesus monkey or cynomolgus monkey)) CD3 and/or GPRC5D and/or MUC16 antigens.

In a further aspect, the present invention provides a nucleic acid encoding any of the above bispecific antibodies. When expressed in a suitable expression vector, a polypeptide encoded by the nucleic acid is capable of exhibiting binding capacity to human or monkey (e.g., rhesus monkey or cynomolgus monkey) CD3 and another antigen (e.g., human or monkey (e.g., rhesus monkey or cynomolgus monkey) GPRC5D or MUC16 antigen). In one embodiment, the nucleic acids encoding the chains of the bispecific antibody may be in the same vector or in different vectors. In yet another embodiment, the nucleic acids encoding the chains of the bispecific antibody may be introduced into the same or different host cells for expression. Thus, in some embodiments, the method for producing the bispecific antibody of the present invention comprises the step of: culturing a host cell comprising a nucleic acid encoding each chain of the molecule under a condition suitable for expressing the chain to produce the bispecific antibody of the present invention.

In one embodiment, provided is a vector comprising the nucleic acid. In one embodiment, the vector is an expression vector, e.g., a eukaryotic expression vector. The vector includes, but is not limited to, a virus, a plasmid, a cosmid, a λ phage, or a yeast artificial chromosome (YAC). In one embodiment, the vector is, for example, a pcDNA vector, such as pcDNA3.1.

In one embodiment, provided is a host cell comprising the nucleic acid or the vector, e.g., for cloning or expressing a vector encoding the anti-CD3 antibody or the anti-GPRC5D antibody or the anti-MUC16 antibody or the bispecific antibody. In one embodiment, the host cell is eukaryotic. In another embodiment, the host cell is selected from a yeast cell, a mammalian cell (e.g., a CHO cell (e.g., CHO-S, such as ExpiCHO-S) or 293 cell (e.g., 293F or HEK293 cell)), or other cells suitable for preparing an antibody or a fragment thereof. In one embodiment, the host cell is prokaryotic, e.g., a bacterium, such as *E. coli.*

In one embodiment, the host cell is eukaryotic. In another embodiment, the host cell is selected from a yeast cell, a mammalian cell, and other cells suitable for producing an antibody or a fragment thereof. For example, eukaryotic microorganisms, such as filamentous fungi or yeast, are suitable cloning or expression hosts for the vector encoding the antibody. For example, fungus and yeast strains in which a glycosylation pathway has been "humanized" produce antibodies having a partial or full human glycosylation pattern. Host cells suitable for expressing a glycosylated antibody are also derived from multicellular organisms (invertebrates and vertebrates). Vertebrate cells may also be used as hosts. For example, a mammalian cell line engineered to be suitable for suspension growth may be used. Other examples of useful mammalian host cell lines are monkey kidney CV1 line (COS-7) transformed with SV40, human embryonic kidney line (HEK293, 293F, or 293T cells), and the like. Other useful mammalian host cell lines include Chinese hamster ovary (CHO) cells, including DHFR-CHO cells, CHO-S cells, ExpiCHO, and the like; and myeloma cell lines such as Y0, NS0, and Sp2/0. Mammalian host cell lines suitable for producing antibodies are known in the art.

### VII. Production and purification of anti-CD3 antibody or anti-GPRC5D antibody or anti-MUC16 antibody or bispecific antibody of the present invention

In one embodiment, provided is a method for preparing the anti-CD3 antibody or the anti-GPRC5D antibody or the anti-MUC16 antibody or the bispecific antibody of the present invention, wherein the method comprises culturing the host cell comprising a nucleic acid encoding the anti-CD3 antibody or the anti-GPRC5D antibody or the anti-MUC16 antibody or the bispecific antibody (e.g., any one and/or more polypeptide chains) or an expression vector comprising the nucleic acid, as provided above, under a condition suitable for expressing the anti-CD3 antibody or the anti-GPRC5D antibody or the anti-MUC16 antibody or the bispecific antibody or the chain thereof, and optionally recovering the anti-CD3 antibody or the anti-GPRC5D antibody or the anti-MUC16 antibody or the bispecific antibody from the host cell (or the host cell medium).

Polynucleotides encoding the polypeptide chains of the anti-CD3 antibody or the anti-GPRC5D antibody or the anti-MUC16 antibody or the bispecific antibody of the present invention may be inserted into one or more vectors for further cloning and/or expression in host cells. Methods known to those skilled in the art can be used to construct expression vectors. Once the expression vector comprising one or more nucleic acid molecules of the present invention has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, liposome-based transfection, or other conventional techniques.

The anti-CD3 antibody or the anti-GPRC5D antibody or the anti-MUC16 antibody or the bispecific antibody prepared as described herein can be purified by known prior art such as high performance liquid chromatography, ion exchange chromatography, gel electrophoresis, affinity chromatography, size exclusion chromatography, and the like. The actual conditions used for purifying a particular protein also depend on factors such as net charge, hydrophobicity, hydrophilicity, and the like, and these will be apparent to those skilled in the art.

The purity of the antibody molecule of the present invention can be determined by any one of a variety of well-known analytical methods including size exclusion chromatography, gel electrophoresis, high performance liquid chromatography, and the like.

### VIII. Assays for anti-CD3 antibody or anti-GPRC5D antibody or bispecific antibody

The anti-CD3 antibody or the anti-GPRC5D antibody or the anti-MUC16 antibody or the bispecific antibody provided herein can be identified, screened, or characterized for its physical/chemical properties and/or biological activity through a variety of assays known in the art.

In one aspect, the anti-CD3 antibody or the anti-GPRC5D antibody or the anti-MUC16 antibody or the bispecific antibody of the present invention is assayed for its binding activity to a target (e.g., an antigen, e.g., a free antigen or an antigen expressed on a cell), for example, by known methods such as bio-layer interferometry, ELISA, flow cytometry, and the like. The binding to CD3 and/or GPRC5D and/or MUC16 (or CD3 and/or GPRC5D and/or MUC16 expressed on a cell) can be assayed using methods known in the art, and exemplary methods are disclosed herein. In some embodiments, the binding is determined by radioimmunoassay (RIA), bio-layer interferometry (BLI), electrochemiluminescence (ECL), surface plasmon resonance (SPR), or flow cytometry (FACS).

The present invention further provides an assay for identifying the biological activity of the anti-CD3 antibody or the anti-GPRC5D antibody or the anti-MUC16 antibody or the bispecific antibody. The biological activity is selected from the properties of the anti-CD3 antibody or the anti-GPRC5D antibody or the anti-MUC16 antibody or the bispecific antibody of the present invention.

For example, the binding activity of the antibody molecule of the present invention to a cell expressing CD3 and/or GPRC5D and/or MUC16 can be determined by methods known in the art, such as fluorescent reporter molecule assay and flow cytometry, or by the exemplary methods disclosed in the examples herein. For example, the binding of the antibody molecule of the present invention to CD3 and/or GPRC5D and/or MUC16 expressed on the cell can be determined by methods as shown in Example 5, 6, 7, 8 or 10.

For example, the activation activity of the antibody molecule of the present invention on a T cell can be determined by methods known in the art, such as a T cell activation assay system (e.g., an NFAT-luc reporter gene system such as a Jurkat/NFAT-luc reporter gene system). For example, the activation activity can be determined by the methods shown in Example 1, 6, 10, 12, 13 or 14, through detecting a CD3 signaling pathway in the T cell or through detecting the release of cytokines (e.g., interferons such as IFNγ; tumor necrosis factors such as TNFα and/or interleukins such as IL-6) following activation of the T cell.

For example, the inhibitory activity or structural safety of the antibody molecule of the present invention against a tumor can be determined by methods known in the art, such as a tumor inhibition experiment conducted on a mouse tumor model, for example, by the methods shown in Example 7 or 11.

The cells for use in any of the above *in-vitro* assays are primary cells or cell lines, including cells that naturally express or overexpress CD3 (e.g., human or monkey (e.g., cynomolgus monkey)), GPRC5D (e.g., human or monkey (e.g., cynomolgus monkey) GPRC5D) or MUC16 (e.g., human or monkey (e.g., cynomolgus monkey) MUC16), for example, cells that overexpress CD3, GPRC5D or MUC16, e.g., T cells, 293 cells, CHO cells, Jurkat cells or myeloma cells, such as HEK293 or CHO-K1 or Jurkat/NFAT-Luc cells or NCI-H929 cells.

It will be appreciated that any of the above assays can be performed using a combination of the antibody of the present invention and other active agents.

### IX. Immunoconjugate, pharmaceutical composition, pharmaceutical combination product, and kit of anti-CD3 antibody or anti-GPRC5D antibody or anti-MUC16 antibody or bispecific antibody of the present invention

In some embodiments, the present invention provides an immunoconjugate comprising any of the anti-CD3 antibodies or the anti-GPRC5D antibodies or the anti-MUC16 antibodies or the bispecific antibodies described herein. Preferably, the immunoconjugate comprises one or more additional therapeutic agents (e.g., cytotoxins or small molecule compounds) or markers.

In some embodiments, the present invention provides a composition or a medicament or a formulation comprising any of the anti-CD3 antibodies or the anti-GPRC5D antibodies or the anti-MUC16 antibodies or the bispecific antibodies described herein, wherein preferably, the composition is a pharmaceutical composition.

In one embodiment, the composition further comprises a pharmaceutical supplementary material. In one embodiment, the composition, e.g., the pharmaceutical composition, comprises a combination of the anti-CD3 antibody or the anti-GPRC5D antibody or the anti-MUC16 antibody or the bispecific antibody of the present invention and one or more additional therapeutic agents.

The composition or the medicament or the formulation may further comprise a suitable pharmaceutical supplementary materialsupplementary material, such as a pharmaceutical carrier and a pharmaceutical excipient known in the art, including buffers.

As used herein, the "pharmaceutical carrier" includes any and all solvents, dispersion media, isotonic agents and absorption delaying agents, and the like that are physiologically compatible.

For use and application of pharmaceutical supplementary materials, see also *"*Handbook of Pharmaceutical Excipients", 8th edition, R. C. Rowe, P. J. Seskey and S.C. Owen, Pharmaceutical Press, London, Chicago.

The composition or the medicament or the formulation of the present invention may be in a variety of forms. These forms include, for example, liquid, semi-solid, and solid dosage forms, such as liquid solutions (e.g., injectable solutions and eye drops), pulvis or suspensions, liposomes, and suppositories. The preferred form depends on the intended mode of administration and therapeutic use.

The medicament or the formulation, e.g., in the form of a lyophilized formulation or an aqueous solution, comprising the anti-CD3 antibody or the anti-GPRC5D antibody or the anti-MUC16 antibody or the bispecific antibody described herein may be prepared by mixing the anti-CD3 antibody or the anti-GPRC5D antibody or the anti-MUC16 antibody or the bispecific antibody of the present invention having a desired purity with one or more optional pharmaceutical supplementary materials.

The composition or the medicament or the formulation of the present invention may further comprise more than one active ingredient required by a particular indication treated, preferably those having complementarity activity without adversely affecting one another. For example, it is desirable to further provide additional therapeutic agents.

The present invention further provides a pharmaceutical combination or a pharmaceutical combination product, comprising the anti-CD3 antibody or the anti-GPRC5D antibody or the anti-MUC16 antibody or the bispecific antibody of the present invention, and one or more additional therapeutic agents.

The present invention further provides a kit of parts, comprising the pharmaceutical combination, wherein, for example, the kit of parts comprises in the same package:
- a first container containing a pharmaceutical composition comprising the anti-CD3 antibody or the anti-GPRC5D antibody or the anti-MUC16 antibody or the bispecific antibody of the present invention; and
- a second container containing a pharmaceutical composition comprising an additional therapeutic agent.

In some embodiments, the additional therapeutic agent is, for example, a chemotherapeutic agent, a cytokine, a cytotoxic agent, an additional antibody, a small molecule drug, or an immunomodulatory agent (e.g., an immunosuppressive agent).

### X. Use of the anti-CD3 antibody or the anti-GPRC5D antibody or the anti-MUC16 antibody or the bispecific antibody and method for using the same.

In one aspect, the present invention provides a method for preventing or treating a disease in a subject, comprising administering to the subject the anti-CD3 antibody or the anti-GPRC5D antibody or the anti-MUC16 antibody or the bispecific antibody, or the immunoconjugate, the composition or the medicament or the formulation comprising the same of the present invention. In some embodiments, the present invention provides a method for specifically activating a T cell in a subject (e.g., thereby enhancing, stimulating, or increasing an immunoreaction in the subject), comprising administering to the subject the bispecific antibody, or the immunoconjugate, the composition or the medicament or the formulation comprising the same of the present invention.

In some embodiments, the disease is, for example, a tumor, e.g., a cancer. The cancer may be at an early, intermediate or advanced stage, or may be a metastatic cancer. In some embodiments, the cancer may be a solid tumor or a hematological tumor. In some embodiments, the cancer is ovarian cancer, myeloma, colon cancer, rectal cancer, or colorectal cancer.

In some embodiments, the ovarian cancer is a cancer associated with abnormal proliferation of ovarian cells, comprising a group of heterogeneous lesions, most commonly those derived from epithelial cell types. In one embodiment, the ovarian cancer is, for example, ovarian carcinoma, particularly serous ovarian carcinoma, such as ovarian serous cystadenocarcinoma, ovarian serous adenocarcinoma, ovarian mucinous adenocarcinoma, endometrioid adenocarcinoma, or ovarian clear cell adenocarcinoma.

In some embodiments, the treatment of the disease will benefit from activation of a CD3 signaling pathway and/or activation of T cells.

In some embodiments, the cancer is a cancer characterized as having an elevated protein level and/or nucleic acid level (e.g., elevated expression) of GPRC5D, e.g., having an elevated protein level and/or nucleic acid level (e.g., elevated expression) of GPRC5D in tumor cells thereof, for example, as compared to the protein level and/or nucleic acid level of GPRC5D in the same tissue of a healthy subject, or as compared to the protein level and/or nucleic acid level of GPRC5D in a healthy tissue adjacent to a tumor tissue of a patient.

In some embodiments, the cancer is a cancer characterized as having an elevated protein level and/or nucleic acid level (e.g., elevated expression) of MUC16, e.g., having an elevated protein level and/or nucleic acid level (e.g., elevated expression) of MUC16 in tumor cells thereof, for example, as compared to the protein level and/or nucleic acid level of MUC16 in the same tissue of a healthy subject, or as compared to the protein level and/or nucleic acid level of MUC16 in a healthy tissue adjacent to a tumor tissue of a patient.

The patient or the subject may be a mammal, e.g., a primate, preferably a higher primate, such as a human (e.g., a patient suffering from or at risk of suffering from the cancer described herein). In certain embodiments, the subject is receiving or has received additional therapies, e.g., chemotherapy and/or radiotherapy.

In one embodiment, the anti-MUC16 antibody disclosed herein does not bind to a free CA125 molecule in circulation, but only to a cell membrane-bound MUC16 molecule expressed on the surface of a cell. It is known that MUC16 molecules are molecules specifically expressed in ovarian cancer. Therefore, a therapeutic effective amount of the anti-MUC16 antibody or the anti-CD3×MUC16 bispecific antibody can specifically bind to ovarian cancer cells expressing the MUC16 molecules, which helps to improve the killing of the cancer cells by the immune system, thereby achieving the therapeutic purpose.

Thus, the present invention provides use of the anti-MUC16 antibody or the anti-CD3×MUC16 bispecific antibody in the preparation of a medicament for treating a tumor (e.g., ovarian cancer).

In another aspect, the present invention relates to a method for preventing or treating a tumor (e.g., ovarian cancer) in a subject, wherein the method comprises administering to the subject an effective amount of the anti-MUC16 antibody or the anti-CD3×MUC16 bispecific antibody, or the pharmaceutical composition comprising the same disclosed herein.

In one embodiment, the anti-GPRC5D antibody or the anti-GPRC5D×CD3 bispecific antibody disclosed herein can be used to treat a cancer, such as myeloma, colon cancer, rectal cancer, or colorectal cancer. Thus, the present invention provides use of the anti-GPRC5D antibody or the anti-GPRC5D×CD3 bispecific antibody in the preparation of a medicament for treating a cancer, such as myeloma, colon cancer, rectal cancer, or colorectal cancer.

In another aspect, the present invention relates to a method for preventing or treating a tumor (e.g., a cancer such as myeloma, colon cancer, rectal cancer, or colorectal cancer) in a subject, wherein the method comprises administering to the subject an effective amount of the anti-GPRC5D antibody or the anti-CD3× GPRC5D bispecific antibody, or the pharmaceutical composition comprising the same disclosed herein.

The anti-CD3 antibody or the anti-GPRC5D antibody or the anti-MUC16 antibody or the bispecific antibody (as well as the immunoconjugate, the composition, the pharmaceutical composition, the formulation, the combination product or the like comprising the same) of the present invention can be administered by any suitable method, including parenteral administration, intrapulmonary administration, intranasal administration, and, if required by locoregional treatment, intralesional administration. Parenteral injection or infusion includes intramuscular, intravenous, intra-arterial, intraperitoneal or subcutaneous injection or infusion. The administration may be performed by any suitable route, such as injection, e.g., intravenous or subcutaneous injection, to some extent depending on short-term or long-term treatment. Various administration schedules are encompassed herein, including, but not limited to, single administration or multiple administrations at multiple time points, bolus injection, and pulse infusion.

In order to prevent or treat a disease, the appropriate dosage of the anti-CD3 antibody or the anti-GPRC5D antibody or the anti-MUC16 antibody or the bispecific antibody (as well as the immunoconjugate, the composition, the pharmaceutical composition, the formulation, the combination product or the like comprising the same) of the present invention (when used alone or in combination with one or more additional therapeutic agents) will depend on the type of the disease to be treated, the type of the antibody, the severity and progression of the disease, purpose of administration (prophylactic or therapeutic), previous therapies, clinical histories of patients, responses to the antibody, and the discretion of an attending physician. The antibody is suitably administered to a patient through a single treatment or through a series of treatments. In other aspects, the present invention provides use of the anti-CD3 antibody or the anti-GPRC5D antibody or the anti-MUC16 antibody or the bispecific antibody, or the immunoconjugate or the composition or the combination product comprising the same of the present invention in the manufacture or preparation of a medicament for the use described herein, e.g., for use in preventing or treating the related disease or condition mentioned herein.

In some embodiments, the anti-CD3 antibody or the anti-GPRC5D antibody or the anti-MUC16 antibody or the bispecific antibody (as well as the immunoconjugate, the composition, the pharmaceutical composition, the formulation or the like comprising the same) can also be administered in combination with one or more additional therapies, e.g., therapeutic modalities and/or additional therapeutic agents, for the use described herein, e.g., for use in preventing or treating the related disease or condition mentioned herein.

In some embodiments, the therapeutic modality is, for example, surgical therapy or radiotherapy.

In some embodiments, the additional therapeutic agent is, for example, a chemotherapeutic agent, a cytokine, a cytotoxic agent, an additional antibody, a small molecule drug, or an immunomodulatory agent (e.g., an immunosuppressive agent).

### XI. Diagnosis and detection

In one aspect, the present invention further relates to methods for diagnosis and detection (e.g., for diagnostic or non-diagnostic purposes) of the anti-CD3 antibody or the anti-GPRC5D antibody or the anti-MUC16 antibody or the bispecific antibody, and a composition for diagnosis and detection comprising the antibody.

In certain embodiments, the anti-GPRC5D antibody provided herein can be used to detect the presence of GPRC5D in a biological sample. In certain embodiments, the bispecific antibody provided herein can be used to detect the presence of CD3 and/or GPRC5D in a biological sample.

In certain embodiments, the anti-MUC16 antibody provided herein can be used to detect the presence of MUC16 in a biological sample. In certain embodiments, the bispecific antibody provided herein can be used to detect the presence of CD3 and/or MUC16 in a biological sample.

In certain embodiments, the bispecific antibody provided herein can be used to detect the presence of a first antigen and/or a second antigen in a biological sample. In certain embodiments, the bispecific antibody provided herein can be used to detect the presence of a first antigen and/or a second antigen in a biological sample.

The term "detection" used herein includes quantitative and qualitative detections, and exemplary detections may involve immunohistochemistry, immunocytochemistry, flow cytometry (e.g., FACS), magnetic beads complexed with antibody molecules, ELISA, and PCR techniques (e.g., RT-PCR). In certain embodiments, the biological sample is a body fluid, such as blood, serum, or plasma.

In certain embodiments, the method comprises contacting a biological sample with the anti-GPRC5D antibody or the bispecific antibody described herein under a condition that allows the anti-GPRC5D antibody or the bispecific antibody to bind to GPRC5D, and detecting whether a complex is formed by the anti-GPRC5D antibody or the bispecific antibody and GPRC5D. The formation of the complex indicates the presence of GPRC5D. The method may be an *in-vitro* or *in-vivo* method.

In certain embodiments, the method comprises contacting a biological sample with the anti-MUC16 antibody or the bispecific antibody described herein under a condition that allows the anti-MUC16 antibody or the bispecific antibody to bind to MUC16, and detecting whether a complex is formed by the anti-MUC16 antibody or the bispecific antibody and MUC16. The formation of the complex indicates the presence of MUC16. The method may be an *in-vitro* or *in-vivo* method.

In certain embodiments, provided is a labeled anti-GPRC5D antibody or anti-MUC16 antibody or bispecific antibody. The label includes, but is not limited to, a label or moiety that is detected directly (such as a fluorescent label, a chromophoric label, an electron-dense label, a chemiluminescent label, and a radioactive label), and a moiety that is detected indirectly, such as an enzyme or a ligand, for example, by enzymatic reaction or molecular interaction. In some embodiments, the label is a marker such as biotin or hFc.

In some embodiments provided herein, the sample is obtained prior to treatment with the anti-CD3 antibody or the anti-GPRC5D antibody or the anti-MUC16 antibody or the bispecific antibody of the present invention. In some embodiments, the sample is obtained prior to application of other therapies. In some embodiments, the sample is obtained during treatment with other therapies, or after treatment with other therapies.

In some embodiments, GPRC5D or MUC16 is detected prior to treatment, e.g., prior to an initial treatment or prior to a treatment after an interval from a certain treatment.

In some embodiments, the first antigen and/or the second antigen are detected prior to treatment, e.g., prior to an initial treatment or prior to a treatment after an interval from a certain treatment.

### XII. DETAILED DESCRIPTION

In some aspects, the present invention relates to the following specific embodiments:
1. A bispecific antibody, comprising
   a first antigen-binding region and a second antigen-binding region, wherein the first antigen-binding region specifically binds to a tumor-associated antigen, and/or the second antigen-binding region specifically binds to CD3,
   wherein the second antigen-binding region is an scFv of an anti-CD3 antibody, and the scFv comprises a VH and a VL optionally linked via a linker comprising, for example, an amino acid sequence (G4S)n, wherein n = 1, 2, 3, 4, or 5, preferably n = 3 or 4, more preferably n = 3.
2. The bispecific antibody according to embodiment 1, wherein the VH contained in the scFv comprises 3 complementarity determining regions from a heavy chain variable region (HCDRs): HCDR1, HCDR2 and HCDR3, and the VL contained in the scFv comprises 3 complementarity determining regions from a light chain variable region (LCDRs): LCDR1, LCDR2 and LCDR3, wherein
   (i) the HCDR1, the HCDR2 and the HCDR3 are selected from three complementarity determining regions HCDR1, HCDR2 and HCDR3 contained in a VH set forth in SEQ ID NO: 3, respectively, and the LCDR1, the LCDR2 and the LCDR3 are selected from three complementarity determining regions LCDR1, LCDR2 and LCDR3 contained in a VL set forth in SEQ ID NO: 4, respectively; or
   (ii) the HCDR1 consists of an amino acid sequence set forth in SEQ ID NO: 5, the HCDR2 consists of an amino acid sequence set forth in SEQ ID NO: 6, the HCDR3 consists of an amino acid sequence set forth in SEQ ID NO: 7, the LCDR1 consists of an amino acid sequence set forth in SEQ ID NO: 8, the LCDR2 consists of an amino acid sequence set forth in SEQ ID NO: 9, and the LCDR3 consists of an amino acid sequence set forth in SEQ ID NO: 10.
3. The bispecific antibody according to embodiment 2, wherein
   the VH comprises or consists of an amino acid sequence set forth in SEQ ID NO: 3, or an amino acid sequence having at least 90%, 91%, 92%, 93%, 94%, 95%, 96%, 97%, 98% or 99% identity thereto,
   and/or the VL comprises or consists of an amino acid sequence set forth in SEQ ID NO: 4, or an amino acid sequence having at least 90%, 91%, 92%, 93%, 94%, 95%, 96%, 97%, 98% or 99% identity thereto.
4. The bispecific antibody according to any one of embodiments 1-3, wherein the first antigen-binding region is a Fab specifically binding to a first antigen.
5. The bispecific antibody according to embodiment 4, wherein the Fab comprises a CH1, wherein the CH1 is a CH1 from IgG1, IgG2, IgG3, or IgG4, preferably a CH1 from IgG1.
6. The bispecific antibody according to embodiment 5, wherein the CH1 (i) comprises or consists of an amino acid sequence having at least 85%, 90%, 91%, 92%, 93%, 94%, 95%, 96%, 97%, 98% or 99% identity to an amino acid sequence set forth in SEQ ID NO: 44; or (ii) comprises or consists of the amino acid sequence set forth in SEQ ID NO: 44.
7. The bispecific antibody according to any one of embodiments 1-6, wherein the bispecific antibody is an IgG-like bispecific antibody comprising an Fc dimer, wherein two Fc regions constituting the Fc dimer are identical or different.
8. The bispecific antibody according to embodiment 7, wherein one or both of the Fc regions comprise a mutation that reduces binding to an Fcy receptor, e.g., one or more of an L234A/L235A mutation, a D265A mutation and a P329A mutation, for example, an L234A/L235A mutation, a D265A mutation and a P329A mutation.
9. The bispecific antibody according to embodiment 8, wherein one or both of the Fc regions comprise or consist of an amino acid sequence set forth in SEQ ID NO: 46 or 49, or an amino acid sequence having at least 90%, 91%, 92%, 93%, 94%, 95%, 96%, 97%, 98%, 99% or more identity thereto.
10. The bispecific antibody according to any one of embodiments 7-9, wherein the two Fc regions are different, and preferably, a corresponding knob mutation and a corresponding hole mutation are introduced into a first monomeric Fc region and a second monomeric Fc region, respectively.
11. The bispecific antibody according to embodiment 10, wherein
   a) one Fc-region polypeptide comprises a knob mutation T366W, and the other Fc-region polypeptide comprises hole mutations T366S, L368A and Y407V, or
   b) one Fc-region polypeptide comprises knob mutations T366W and Y349C, and the other Fc-region polypeptide comprises hole mutations T366S, L368A, Y407V and S354C, or
   c) one Fc-region polypeptide comprises knob mutations T366W and S354C, and the other Fc-region polypeptide comprises hole mutations T366S, L368A, Y407V and Y349C;
   optionally, the Fc region further comprises a mutation that reduces binding to the Fcy receptor, e.g., one or more of an L234A/L235A mutation, a D265A mutation and a P329A mutation, for example, an L234A/L235A mutation, a D265A mutation and a P329A mutation.
12. The bispecific antibody according to any one of embodiments 7-11, wherein one or both of the Fc regions comprise a hinge region, e.g., EPKSS or EPKSC.
13. The bispecific antibody according to embodiment 12, wherein
   (i) the first Fc region comprises a knob mutation, and
      a) comprises or consists of an amino acid sequence set forth in SEQ ID NO: 43 or 89; or
      b) comprises or consists of an amino acid sequence having at least 90% identity, e.g., 95%, 96%, 97%, 99% or more identity, to SEQ ID NO: 43 or 89 and comprising an L234A/L235A mutation, a D265A mutation, a P329A mutation, and a knob mutation (e.g., S354C and T366W); and/or
   (ii) the second Fc region comprises a hole mutation, and
      a) comprises or consists of an amino acid sequence set forth in SEQ ID NO: 30, 42 or 59; or
      b) comprises or consists of an amino acid sequence having at least 90% identity, e.g., 95%, 96%, 97%, 99% or more identity, to SEQ ID NO: 30, 42 or 59 and comprising an L234A/L235A mutation, a D265A mutation, a P329A mutation, and a hole mutation (e.g., Y349C, T366S, L368A, and Y407V).
14. The bispecific antibody according to any one of embodiments 1-13, wherein the bispecific antibody is an IgG-like bispecific antibody comprising one Fab fragment specifically binding to the first antigen, one scFv specifically binding to a second antigen, and an Fc heterodimer, wherein
   the Fab fragment comprises VH-CH1 and VL-CL, and the scFv comprises VH-linker-VL or VL-linker-VH;
   the Fc region comprising the hole mutation constitutes a first heavy chain;
   the C-terminus of the CH1 of the Fab fragment is fused to the N-terminus of the scFv fragment, and the C-terminus of the scFv fragment is fused to a CH2 or the hinge region of the Fc region comprising the knob mutation, thereby constituting a second heavy chain;
      and
   the VL-CL of the Fab fragment constitutes a light chain.
15. The bispecific antibody according to any one of embodiments 1-13, wherein the bispecific antibody is an IgG-like bispecific antibody comprising two Fab fragments specifically binding to the first antigen, one scFv specifically binding to a second antigen, and an Fc heterodimer, wherein
   the Fab fragment comprises VH-CH1 and VL-CL, and the scFv comprises VH-linker-VL or VL-linker-VH;
   the C-terminus of the CH1 of the first Fab fragment is fused to a CH2 or the hinge region of the Fc region comprising the hole mutation, thereby constituting a first heavy chain;
   the C-terminus of the CH1 of the second Fab fragment is fused to the N-terminus of the scFv fragment, and the C-terminus of the scFv fragment is fused to a CH2 or the hinge region of the Fc region comprising the knob mutation, thereby constituting a second heavy chain;
      and
   the VL-CL of the first and second Fab fragments constitute two light chains;
   wherein the first Fab and second Fab fragments are identical or different.
16. The bispecific antibody according to any one of embodiments 1-13, wherein the bispecific antibody is an IgG-like bispecific antibody comprising one Fab fragment specifically binding to the first antigen, one scFv specifically binding to a second antigen, and an Fc heterodimer, wherein
   the Fab fragment comprises VH-CH1 and VL-CL, and the scFv comprises VH-linker-VL or VL-linker-VH;
   the C-terminus of the CH1 of the Fab fragment is fused to a CH2 or the hinge region of the Fc region comprising the hole mutation, thereby constituting a first heavy chain;
   the C-terminus of the scFv fragment is fused to a CH2 or the hinge region of the Fc region comprising the knob mutation, thereby constituting a second heavy chain;
      and
   the VL-CL of the Fab fragment constitutes a light chain.
17. The bispecific antibody according to any one of embodiments 1-13, wherein the bispecific
   antibody is an IgG-like bispecific antibody comprising two Fab fragments specifically binding to the first antigen, one scFv specifically binding to a second antigen, and an Fc heterodimer, wherein
   the Fab fragment comprises VH-CH1 and VL-CL, and the scFv comprises VH-linker-VL or VL-linker-VH;
   the C-terminus of the CH1 of the first Fab fragment is fused to the N-terminus of the VH of the second Fab fragment, and the C-terminus of the CH1 of the second Fab fragment is fused to a CH2 or the hinge region of the Fc region comprising the hole mutation, thereby constituting a first heavy chain;
   the C-terminus of the scFv fragment is fused to a CH2 or the hinge region of the Fc region comprising the knob mutation, thereby constituting a second heavy chain; and
   the VL-CL of the first and second Fab fragments constitute two light chains;
   wherein the first Fab and second Fab fragments are identical or different.
18. The bispecific antibody according to any one of embodiments 1-18, wherein the first antigen is a tumor-associated antigen, for example, selected from GPRC5D or MUC16 (e.g., human GPRC5D or human MUC16).
19. The bispecific antibody according to embodiment 18, wherein the first antigen is human GPRC5D, and the first antigen-binding region comprises the 3 CDRs of the heavy chain variable region VH: HCDR1, HCDR2 and HCDR3, and the 3 CDRs of the light chain variable region VL: LCDR1, LCDR2 and LCDR3, wherein
   (i) the HCDR1, the HCDR2 and the HCDR3 are selected from three complementarity determining regions HCDR1, HCDR2 and HCDR3 contained in a VH set forth in any one of SEQ ID NOs: 62, 38 and 40, respectively; and the LCDR1, the LCDR2 and the LCDR3 are three complementarity determining regions LCDR1, LCDR2 and LCDR3 contained in a VL set forth in SEQ ID NO: 63, respectively;
   (ii) the HCDR1, the HCDR2 and the HCDR3 are selected from three complementarity determining regions HCDR1, HCDR2 and HCDR3 contained in a VH set forth in SEQ ID NO: 72, respectively; and the LCDR1, the LCDR2 and the LCDR3 are three complementarity determining regions LCDR1, LCDR2 and LCDR3 contained in a VL set forth in SEQ ID NO: 73, respectively; or
   (iii) the HCDR1, the HCDR2 and the HCDR3 are selected from three complementarity determining regions HCDR1, HCDR2 and HCDR3 contained in a VH set forth in SEQ ID NO: 82, respectively; and the LCDR1, the LCDR2 and the LCDR3 are three complementarity determining regions LCDR1, LCDR2 and LCDR3 contained in a VL set forth in SEQ ID NO: 83, respectively.
20. The bispecific antibody according to embodiment 19, wherein the first antigen-binding region comprises
   (i) an HCDR1 set forth in SEQ ID NO: 64, an HCDR2 set forth in SEQ ID NO: 65, 39, or 41, an HCDR3 set forth in SEQ ID NO: 66, an LCDR1 set forth in SEQ ID NO: 67, an LCDR2 set forth in SEQ ID NO: 68, and an LCDR3 set forth in SEQ ID NO: 69;
   (ii) an HCDR1 set forth in SEQ ID NO: 74, an HCDR2 set forth in SEQ ID NO: 75, an HCDR3 set forth in SEQ ID NO: 76, an LCDR1 set forth in SEQ ID NO: 77, an LCDR2 set forth in SEQ ID NO: 78, and an LCDR3 set forth in SEQ ID NO: 79; or
   (iii) an HCDR1 set forth in SEQ ID NO: 84, an HCDR2 set forth in SEQ ID NO: 85, an HCDR3 set forth in SEQ ID NO: 86, an LCDR1 set forth in SEQ ID NO: 87, an LCDR2 set forth in SEQ ID NO: 78, and an LCDR3 set forth in SEQ ID NO: 88.
21. The bispecific antibody according to embodiment 19 or 20, wherein the first antigen-binding region comprises a heavy chain variable region VH, wherein the heavy chain variable region
   (i) comprises or consists of an amino acid sequence having at least 90%, 91%, 92%, 93%, 94%, 95%, 96%, 97%, 98%, or 99% identity to an amino acid sequence of SEQ ID NO: 62, 72, 82, 16, 23, 25, 27, 29, 38, or 40; or
   (ii) comprises or consists of an amino acid sequence of SEQ ID NO: 62, 72, 82, 16, 23, 25, 27, 29, 38 or 40.
22. The bispecific antibody according to any one of embodiments 19-21, wherein the first antigen-binding region comprises a light chain variable region VL, wherein the light chain variable region
   (i) comprises or consists of an amino acid sequence having at least 90%, 91%, 92%, 93%, 94%, 95%, 96%, 97%, 98%, or 99% identity to an amino acid sequence of SEQ ID NO: 63, 73, 83, 17, 19, or 21; or
   (ii) comprises or consists of an amino acid sequence of SEQ ID NO: 63, 73, 83, 17, 19 or 21.
23. The bispecific antibody according to any one of embodiments 19-22, wherein the first antigen-binding region comprises a heavy chain variable region VH and a light chain variable region VL, wherein
   (i) the heavy chain variable region comprises or consists of an amino acid sequence set forth in SEQ ID NO: 62, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 63, or an amino acid sequence having at least 90%, 91%, 92%, 93%, 94%, 95%, 96%, 97%, 98% or 99% identity thereto;
   (ii) the heavy chain variable region comprises or consists of an amino acid sequence set forth in SEQ ID NO: 72, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 73, or an amino acid sequence having at least 90%, 91%, 92%, 93%, 94%, 95%, 96%, 97%, 98% or 99% identity thereto;
   (iii) the heavy chain variable region comprises or consists of an amino acid sequence set forth in SEQ ID NO: 82, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 83, or an amino acid sequence having at least 90%, 91%, 92%, 93%, 94%, 95%, 96%, 97%, 98% or 99% identity thereto; or
   (iv) the heavy chain variable region comprises or consists of an amino acid sequence set forth in SEQ ID NO: 16, 23, 25, 27, 29, 38 or 40, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 17, 19 or 21, or an amino acid sequence having at least 90%, 91%, 92%, 93%, 94%, 95%, 96%, 97%, 98% or 99% identity thereto.
24. The bispecific antibody according to any one of embodiments 19-23, wherein the first antigen-binding region comprises a heavy chain variable region VH and a light chain variable region VL, wherein the VH and the VL comprise or consist of amino acid sequences set forth below, respectively:
   (i). SEQ ID NO: 62 and SEQ ID NO: 63;
   (ii). SEQ ID NO: 72 and SEQ ID NO: 73;
   (iii). SEQ ID NO: 82 and SEQ ID NO: 83;
   (iv). SEQ ID NO: 16, 23, 25, 27, 29, 38, or 40 and SEQ ID NO: 21;
   (v). SEQ ID NO: 16 or 25 and SEQ ID NO: 17; or
   (vi). SEQ ID NO: 16 or 27 and SEQ ID NO: 19.
25. The bispecific antibody according to embodiment 18, wherein the first antigen is human MUC16, and the first antigen-binding region comprises the 3 CDRs of the heavy chain variable region VH: HCDR1, HCDR2 and HCDR3, and the 3 CDRs of the light chain variable region VL: LCDR1, LCDR2 and LCDR3, wherein
   the HCDR1, the HCDR2 and the HCDR3 are selected from three complementarity determining regions HCDR1, HCDR2 and HCDR3 contained in a VH set forth in SEQ ID NO: 92 or 113, respectively; and the LCDR1, the LCDR2 and the LCDR3 are three complementarity determining regions LCDR1, LCDR2 and LCDR3 contained in a VL set forth in SEQ ID NO: 96, 100, 102 or 114, respectively.
26. The bispecific antibody according to embodiment 25, wherein the first antigen-binding region comprises
   an HCDR1 set forth in SEQ ID NO: 93, an HCDR2 set forth in SEQ ID NO: 94, an HCDR3 set forth in SEQ ID NO: 95, an LCDR1 set forth in SEQ ID NO: 97, 101, or 103, an LCDR2 set forth in SEQ ID NO: 98, and an LCDR3 set forth in SEQ ID NO: 99.
27. The bispecific antibody according to embodiment 25 or 26, wherein the first antigen-binding region comprises a heavy chain variable region VH, wherein the heavy chain variable region
   comprises or consists of an amino acid sequence having at least 90%, 91%, 92%, 93%, 94%, 95%, 96%, 97%, 98% or 99% identity to an amino acid sequence of SEQ ID NO: 92 or 113, or comprises or consists of an amino acid sequence of SEQ ID NO: 92 or 113.
28. The bispecific antibody according to any one of embodiments 25-27, wherein the first antigen-binding region comprises a light chain variable region VL, wherein the light chain variable region comprises or consists of an amino acid sequence having at least 90%, 91%, 92%, 93%, 94%, 95%, 96%, 97%, 98% or 99% identity to an amino acid sequence of SEQ ID NO: 96, 100, 102 or 114, or comprises or consists of an amino acid sequence of SEQ ID NO: 96, 100, 102 or 114.
29. The bispecific antibody according to any one of embodiments 25-28, wherein the first antigen-binding region comprises a heavy chain variable region VH and a light chain variable region VL, wherein
   the heavy chain variable region comprises or consists of an amino acid sequence set forth in SEQ ID NO: 92 or 113, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 96, 100, 102 or 114, or an amino acid sequence having at least 90%, 91%, 92%, 93%, 94%, 95%, 96%, 97%, 98% or 99% identity thereto.
30. The bispecific antibody according to any one of embodiments 25-29, wherein the first antigen-binding region comprises a heavy chain variable region VH and a light chain variable region VL, wherein the VH comprises or consists of the amino acid sequence set forth in SEQ ID NO: 92, and the VL comprises or consists of the amino acid sequence set forth in SEQ ID NO: 96, 100 or 102; or
   the first antigen-binding region comprises a heavy chain variable region VH and a light chain variable region VL, wherein the VH comprises or consists of the amino acid sequence set forth in SEQ ID NO: 113, and the VL comprises or consists of the amino acid sequence set forth in SEQ ID NO: 114.
31. A nucleic acid molecule, encoding any one of the chains in the bispecific antibody according to any one of embodiments 1-30, or consisting of a nucleic acid sequence.
32. An expression vector, comprising the nucleic acid molecule according to embodiment 31, wherein preferably, the expression vector is pCDNA, e.g., pCDNA3.1.
33. A host cell, comprising the nucleic acid molecule according to embodiment 31 or the expression vector according to embodiment 32, wherein preferably, the host cell is prokaryotic or eukaryotic, e.g., a 293 cell or a CHO cell, such as a 293F cell or a 293T cell or a CHO-S cell.
34. A method for preparing the bispecific antibody according to any one of embodiments 1-30, wherein the method comprises culturing the host cell of the nucleic acid molecule according to embodiment 31 or the expression vector according to embodiment 32 under a condition suitable for expressing the chains of the antibody, and optionally recovering the antibody from the host cell (or the host cell medium).
35. An immunoconjugate, comprising the bispecific antibody according to any one of embodiments 1-30.
36. A pharmaceutical composition or a medicament or a formulation, comprising the bispecific antibody according to any one of embodiments 1-30 or the immunoconjugate according to embodiment 35, and optionally a pharmaceutical supplementary material.
37. A pharmaceutical combination product, comprising the bispecific antibody according to any one of embodiments 1-30 or the immunoconjugate according to embodiment 35, and one or more additional therapeutic agents (e.g., a chemotherapeutic agent, a cytokine, a cytotoxic agent, an additional antibody, a small molecule drug, or an immunomodulatory agent).
38. A method for preventing or treating a cancer in a subject, comprising administering to the subject an effective amount of the bispecific antibody according to any one of embodiments 1-30, or the immunoconjugate according to embodiment 35, or the pharmaceutical composition or the formulation according to embodiment 36, or the pharmaceutical combination product according to embodiment 37.
39. The method according to embodiment 38, wherein tumor cells of the cancer have an elevated protein level and/or nucleic acid level (e.g., elevated expression) of a first antigen.
40. The method according to embodiment 38, wherein the first antigen is selected from GPRC5D or MUC16.
41. The method according to any one of embodiments 38-40, wherein the cancer is a solid tumor or a hematological tumor, such as ovarian carcinoma (e.g., serous ovarian carcinoma, such as ovarian serous cystadenocarcinoma, ovarian serous adenocarcinoma, ovarian mucinous adenocarcinoma, endometrioid adenocarcinoma, or ovarian clear cell adenocarcinoma), myeloma, colon cancer, rectal cancer, or colorectal cancer.
42. The method according to any one of embodiments 38-41, wherein the method further comprises administering in combination with an additional therapy such as a therapeutic modality (e.g., surgical therapy or radiotherapy) and/or an additional therapeutic agent (e.g., a chemotherapeutic agent, a cytokine, a cytotoxic agent, an additional antibody, a small molecule drug, or an immunomodulatory agent).
43. A method for detecting the presence of a first antigen in a biological sample, comprising
   (i) contacting the biological sample with the bispecific antibody according to any one of embodiments 1-30 under a condition that allows the bispecific antibody to bind to the first antigen, and
   (ii) detecting whether a complex is formed by the antibody or the bispecific antibody and the first antigen,
   wherein the formation of the complex indicates the presence of the first antigen.

In some aspects, the present invention relates to the following specific embodiments:
1. An anti-CD3 antibody or an antigen-binding fragment thereof that specifically binds to human CD3, comprising:
   1) a heavy chain variable region (VH_{CD3}) having a sequence set forth in SEQ ID NO: 3, or a heavy chain variable region having at least 90%, 91%, 92%, 93%, 94%, 95%, 96%, 97%, 98%, 99% or more sequence identity and the same CDRs as compared to SEQ ID NO: 3; and
   2) a light chain variable region (VL_{CD3}) having a sequence set forth in SEQ ID NO: 4, or a light chain variable region having at least 90%, 91%, 92%, 93%, 94%, 95%, 96%, 97%, 98%, 99% or more sequence identity and the same CDRs as compared to SEQ ID NO: 4.
2. The anti-CD3 antibody or the antigen-binding fragment thereof according to embodiment 1, comprising:
   1) a heavy chain (H) having a sequence set forth in SEQ ID NO: 1, or a heavy chain having at least 90%, 91%, 92%, 93%, 94%, 95%, 96%, 97%, 98%, 99% or more sequence identity and the same CDRs as compared to SEQ ID NO: 1; and
   2) a light chain (L) having a sequence set forth in SEQ ID NO: 2, or a light chain having at least 90%, 91%, 92%, 93%, 94%, 95%, 96%, 97%, 98%, 99% or more sequence identity and the same CDRs as compared to SEQ ID NO: 2.
3. An anti-MUC16 antibody or an antigen-binding fragment thereof that specifically binds to human MUC16, comprising a VH and a VL, wherein
   1) the VH comprises an HCDR1 set forth in SEQ ID NO: 93, an HCDR2 set forth in SEQ ID NO: 94, and an HCDR3 set forth in SEQ ID NO: 95, and the VL comprises an LCDR1 set forth in SEQ ID NO: 97, an LCDR2 set forth in SEQ ID NO: 98, and an LCDR3 set forth in SEQ ID NO: 99; or
   2) the VH comprises an HCDR1 set forth in SEQ ID NO: 93, an HCDR2 set forth in SEQ ID NO: 94, and an HCDR3 set forth in SEQ ID NO: 95, and the VL comprises an LCDR1 set forth in SEQ ID NO: 101, an LCDR2 set forth in SEQ ID NO: 98, and an LCDR3 set forth in SEQ ID NO: 99; or
   3) the VH comprises an HCDR1 set forth in SEQ ID NO: 93, an HCDR2 set forth in SEQ ID NO: 94, and an HCDR3 set forth in SEQ ID NO: 95, and the VL comprises an LCDR1 set forth in SEQ ID NO: 103, an LCDR2 set forth in SEQ ID NO: 98, and an LCDR3 set forth in SEQ ID NO: 99.
4. The anti-MUC16 antibody or the antigen-binding fragment thereof that specifically binds to human MUC16 according to embodiment 3, comprising:
   1) a VH having a sequence set forth in SEQ ID NO: 92, or a VH having at least 90%, 91%, 92%, 93%, 94%, 95%, 96%, 97%, 98%, 99% or more sequence identity and the same CDRs as compared to SEQ ID NO: 92; and a VL having a sequence set forth in SEQ ID NO: 96, or a VL having at least 90%, 91%, 92%, 93%, 94%, 95%, 96%, 97%, 98%, 99% or more sequence identity and the same CDRs as compared to SEQ ID NO: 96; or
   2) a VH having a sequence set forth in SEQ ID NO: 92, or a VH having at least 90%, 91%, 92%, 93%, 94%, 95%, 96%, 97%, 98%, 99% or more sequence identity and the same CDRs as compared to SEQ ID NO: 92; and a VL having a sequence set forth in SEQ ID NO: 100, or a VL having at least 90%, 91%, 92%, 93%, 94%, 95%, 96%, 97%, 98%, 99% or more sequence identity and the same CDRs as compared to SEQ ID NO: 100; or
   3) a VH having a sequence set forth in SEQ ID NO: 92, or a VH having at least 90%, 91%, 92%, 93%, 94%, 95%, 96%, 97%, 98%, 99% or more sequence identity and the same CDRs as compared to SEQ ID NO: 92; and a VL having a sequence set forth in SEQ ID NO: 102, or a VL having at least 90%, 91%, 92%, 93%, 94%, 95%, 96%, 97%, 98%, 99% or more sequence identity and the same CDRs as compared to SEQ ID NO: 102; or
   4) a VH having a sequence set forth in SEQ ID NO: 113, or a VH having at least 90%, 91%, 92%, 93%, 94%, 95%, 96%, 97%, 98%, 99% or more sequence identity and the same CDRs as compared to SEQ ID NO: 113; and a VL having a sequence set forth in SEQ ID NO: 114, or a VL having at least 90%, 91%, 92%, 93%, 94%, 95%, 96%, 97%, 98%, 99% or more sequence identity and the same CDRs as compared to SEQ ID NO: 114.
5. The anti-MUC16 antibody or the antigen-binding fragment thereof according to embodiment 3 or 4, comprising:
   1) a heavy chain (H) having a sequence set forth in SEQ ID NO: 106, or a heavy chain having at least 90%, 91%, 92%, 93%, 94%, 95%, 96%, 97%, 98%, 99% or more sequence identity and the same CDRs as compared to SEQ ID NO: 106; and a light chain (L) having a sequence set forth in SEQ ID NO: 107, or a light chain having at least 90%, 91%, 92%, 93%, 94%, 95%, 96%, 97%, 98%, 99% or more sequence identity and the same CDRs as compared to SEQ ID NO: 107; or
   2) a heavy chain (H) having a sequence set forth in SEQ ID NO: 106, or a heavy chain having at least 90%, 91%, 92%, 93%, 94%, 95%, 96%, 97%, 98%, 99% or more sequence identity and the same CDRs as compared to SEQ ID NO: 106; and a light chain (L) having a sequence set forth in SEQ ID NO: 108, or a light chain having at least 90%, 91%, 92%, 93%, 94%, 95%, 96%, 97%, 98%, 99% or more sequence identity and the same CDRs as compared to SEQ ID NO: 108; or
   3) a heavy chain (H) having a sequence set forth in SEQ ID NO: 106, or a heavy chain having at least 90%, 91%, 92%, 93%, 94%, 95%, 96%, 97%, 98%, 99% or more sequence identity and the same CDRs as compared to SEQ ID NO: 106; and a light chain (L) having a sequence set forth in SEQ ID NO: 109, or a light chain having at least 90%, 91%, 92%, 93%, 94%, 95%, 96%, 97%, 98%, 99% or more sequence identity and the same CDRs as compared to SEQ ID NO: 109; or
   4) a heavy chain (H) having a sequence set forth in SEQ ID NO: 115, or a heavy chain having at least 90%, 91%, 92%, 93%, 94%, 95%, 96%, 97%, 98%, 99% or more sequence identity and the same CDRs as compared to SEQ ID NO: 115; and a light chain (L) having a sequence set forth in SEQ ID NO: 116, or a light chain having at least 90%, 91%, 92%, 93%, 94%, 95%, 96%, 97%, 98%, 99% or more sequence identity and the same CDRs as compared to SEQ ID NO: 116.
6. The anti-CD3 antibody or the antigen-binding fragment thereof according to any one of embodiments 1-2 or the anti-MUC16 antibody or the antigen-binding fragment thereof according to any one of embodiments 3-5, wherein the antigen-binding fragment is an Fv, a Fab, a Fab', a Fab'-SH, an F(ab')₂, a linear antibody, or an scFv.
7. The anti-CD3 antibody or the antigen-binding fragment thereof according to embodiment 6, wherein the scFv comprises a sequence set forth in SEQ ID NO: 11, or a sequence having at least 90%, 91%, 92%, 93%, 94%, 95%, 96%, 97%, 98%, 99% or more sequence identity and the same CDRs as compared to SEQ ID NO: 11.
8. An anti-CD3×MUC16 bispecific antibody, comprising:
   (i) one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 96, an LCDR2 set forth in SEQ ID NO: 97, and an LCDR3 set forth in SEQ ID NO: 98; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
      one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 101, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
      one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 103, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10;
         or
   (ii) one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 97, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
      one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 101, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
      one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 103, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
   (iii) one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 97, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or

   one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 101, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10; or
   one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises an HCDR1 set forth in SEQ ID NO: 93, an HCDR2 set forth in SEQ ID NO: 94, and an HCDR3 set forth in SEQ ID NO: 95, and the VL recognizing the MUC16 molecule comprises an LCDR1 set forth in SEQ ID NO: 103, an LCDR2 set forth in SEQ ID NO: 98, and an LCDR3 set forth in SEQ ID NO: 99; the scFv recognizing the CD3 molecule comprises a VH_{CD3} and a VL_{CD3}, wherein the VH_{CD3} comprises an HCDR1 set forth in SEQ ID NO: 5, an HCDR2 set forth in SEQ ID NO: 6, and an HCDR3 set forth in SEQ ID NO: 7, and the VL_{CD3} comprises an LCDR1 set forth in SEQ ID NO: 8, an LCDR2 set forth in SEQ ID NO: 9, and an LCDR3 set forth in SEQ ID NO: 10.
9. The anti-CD3×MUC16 bispecific antibody according to embodiment 8, comprising:
   (i) one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises a sequence selected from SEQ ID NO: 92 or 113, and the VL recognizing the MUC16 molecule comprises a sequence selected from SEQ ID NO: 96, 100, 102 or 114; the scFv recognizing the CD3 molecule comprises a VH_{CD3} and a VL_{CD3}, wherein the VH_{CD3} comprises a sequence set forth in SEQ ID NO: 3, and the VL_{CD3} comprises a sequence set forth in SEQ ID NO: 4;
   (ii) one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises a sequence selected from SEQ ID NO: 92 or 113, and the VL recognizing the MUC16 molecule comprises a sequence selected from SEQ ID NO: 96, 100, 102 or 114; the scFv recognizing the CD3 molecule comprises a VH_{CD3} and a VL_{CD3}, wherein the VH_{CD3} comprises a sequence set forth in SEQ ID NO: 3, and the VL_{CD3} comprises a sequence set forth in SEQ ID NO: 4; or
   (iii) one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises a sequence selected from SEQ ID NO: 92 or 113, and the VL recognizing the MUC16 molecule comprises a sequence selected from SEQ ID NO: 96, 100, 102 or 114; the scFv recognizing the CD3 molecule comprises a VH_{CD3} and a VL_{CD3}, wherein the VH_{CD3} comprises a sequence set forth in SEQ ID NO: 3, and the VL_{CD3} comprises a sequence set forth in SEQ ID NO: 4.
10. The anti-CD3×MUC16 bispecific antibody according to embodiment 8 or 9, comprising:
   (i) one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the VH recognizing the MUC16 molecule comprises a sequence of SEQ ID NO: 113, and the VL recognizing the MUC16 molecule comprises a sequence selected from SEQ ID NO: 114; the scFv recognizing the CD3 molecule comprises a VH_{CD3} and a VL_{CD3}, wherein the VH_{CD3} comprises a sequence set forth in SEQ ID NO: 3, and the VL_{CD3} comprises a sequence set forth in SEQ ID NO: 4;
   (ii) one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the VH recognizing the MUC16 molecule comprises a sequence selected from SEQ ID NO: 113, and the VL recognizing the MUC16 molecule comprises a sequence selected from SEQ ID NO: 114; the scFv recognizing the CD3 molecule comprises a VH_{CD3} and a VL_{CD3}, wherein the VH_{CD3} comprises a sequence set forth in SEQ ID NO: 3, and the VL_{CD3} comprises a sequence set forth in SEQ ID NO: 4; or
   (iii) one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the VH recognizing the MUC16 molecule comprises a sequence selected from SEQ ID NO: 113, and the VL recognizing the MUC16 molecule comprises a sequence selected from SEQ ID NO: 114; the scFv recognizing the CD3 molecule comprises a VH_{CD3} and a VL_{CD3}, wherein the VH_{CD3} comprises a sequence set forth in SEQ ID NO: 3, and the VL_{CD3} comprises a sequence set forth in SEQ ID NO: 4.
11. The anti-CD3×MUC16 bispecific antibody according to any one of embodiments 8-10, wherein the two antigen recognition sites recognizing the MUC16 molecule contained in the bispecific antibody can have either identical VH/VL sequences or different VH/VL sequences.
12. The anti-CD3×MUC16 bispecific antibody according to any one of embodiments 8-11, wherein the Fc region is selected from an Fc region of an IgG class antibody, e.g., an Fc region from an isotype IgG1, IgG2, IgG3 or IgG4, and preferably, the Fc region comprises a mutation, e.g., a mutation that increases the dimer stability of the bispecific antibody (e.g., a knob-in-hole structure) and a mutation that reduces or increases an effector function.
13. The anti-CD3×MUC16 bispecific antibody according to any one of embodiments 8-12, wherein the scFv is linked to the Fc or the CH1 via a linker, e.g., a flexible peptide linker.
14. The anti-CD3×MUC16 bispecific antibody according to any one of embodiments 8-13, comprising:
   (i) one heavy chain 1 with a structure of VH-CH1-Fc, one heavy chain 2 with a structure of VH-CH1-scFv-Fc, and two light chains with a structure of VL-CL, wherein the two light chains are respectively paired with the structures of VH-CH1 of the heavy chains 1 and 2 to form two antigen recognition sites recognizing an MUC16 molecule, and the scFv forms an antigen recognition site recognizing a CD3 molecule, wherein the heavy chain 1 comprises a sequence set forth in SEQ ID NO: 118, the heavy chain 2 comprises a sequence set forth in SEQ ID NO: 121, and the light chains comprise a sequence set forth in SEQ ID NO: 116;
   (ii) one heavy chain 1 with a structure of VH-CH1-Fc, one heavy chain 2 with a structure of scFv-Fc, and one light chain with a structure of VL-CL, wherein the light chain is paired with the structure of VH-CH1 of the heavy chain 1 to form an antigen recognition site recognizing an MUC16 molecule, and the scFv forms an antigen recognition site recognizing a CD3 molecule, wherein the heavy chain 1 comprises a sequence set forth in SEQ ID NO: 118, the heavy chain 2 comprises a sequence set forth in SEQ ID NO: 117, and the light chain comprises a sequence set forth in SEQ ID NO: 116; or
   (iii) one heavy chain 1 with a structure of VH-CH1-VH-CH1-Fc, one heavy chain 2 with a structure of scFv-Fc, and two light chains with a structure of VL-CL, wherein the two light chains are respectively paired with the two structures of VH-CH1 in the heavy chain 1 to form two antigen recognition sites recognizing an MUC16 molecule, and the scFv forms an antigen recognition site recognizing a CD3 molecule, wherein the heavy chain 1 comprises a sequence set forth in SEQ ID NO: 119, the heavy chain 2 comprises a sequence set forth in SEQ ID NO: 117, and the light chains comprise a sequence set forth in SEQ ID NO: 116.
15. A nucleic acid, encoding the anti-CD3 antibody or the antigen-binding fragment thereof according to any one of embodiments 1-2 or 6-7, the anti-MUC16 antibody or the antigen-binding fragment thereof according to any one of embodiments 3-6, or the anti-CD3×MUC16 bispecific antibody according to any one of embodiments 8-13.
16. A vector, comprising the nucleic acid according to embodiment 15, wherein preferably, the vector is an expression vector.
17. A host cell, comprising the nucleic acid according to embodiment 15 or the vector according to embodiment 16.
18. A method for producing the anti-CD3 antibody or the antigen-binding fragment thereof according to any one of embodiments 1-2 or 6-7, the anti-MUC16 antibody or the antigen-binding fragment thereof according to any one of embodiments 3-6, or the anti-CD3×MUC16 bispecific antibody according to any one of embodiments 8-13, comprising the steps of:
   (i) culturing the host cell described herein under a condition suitable for expressing the anti-CD3 antibody, the anti-MUC16 antibody or the anti-CD3×MUC16 bispecific antibody described herein, and optionally,
   (ii) recovering the anti-CD3 antibody, the anti-MUC16 antibody or the anti-CD3×MUC16 bispecific antibody described herein.
19. A pharmaceutical composition, comprising the anti-CD3 antibody or the antigen-binding fragment thereof according to any one of embodiments 1-2 or 6-7, the anti-MUC16 antibody or the antigen-binding fragment thereof according to any one of embodiments 3-6, or the anti-CD3×MUC16 bispecific antibody according to any one of embodiments 8-13, and a pharmaceutically acceptable carrier.
20. The pharmaceutical composition according to embodiment 19, further comprising an additional therapeutic agent, wherein preferably, the additional therapeutic agent is selected from a chemotherapeutic agent and a cytotoxic agent.
21. Use of the anti-CD3 antibody or the antigen-binding fragment thereof according to any one of embodiments 1-2 or 6-7, the anti-MUC16 antibody or the antigen-binding fragment thereof according to any one of embodiments 3-6 or the anti-CD3×MUC16 bispecific antibody according to any one of embodiments 8-13, or the pharmaceutical composition according to any one of embodiments 19-20 in the preparation of a medicament for treating, preventing and/or diagnosing ovarian cancer.
22. A method for treating, preventing and/or diagnosing ovarian cancer, comprising administering to a subject in need a therapeutically or diagnostically effective amount of the anti-CD3 antibody or the antigen-binding fragment thereof according to any one of embodiments 1-2 or 6-7, the anti-MUC16 antibody or the antigen-binding fragment thereof according to any one of embodiments 3-6 or the anti-CD3×MUC16 bispecific antibody according to any one of embodiments 8-13, or the pharmaceutical composition according to any one of embodiments 19-20.
23. The use according to embodiment 21 or the method according to embodiment 22, wherein the ovarian cancer is ovarian carcinoma, e.g., serous ovarian carcinoma, such as ovarian serous cystadenocarcinoma, ovarian serous adenocarcinoma, ovarian mucinous adenocarcinoma, endometrioid adenocarcinoma, or ovarian clear cell adenocarcinoma.

In some aspects, the present invention relates to the following specific embodiments:
1. An anti-GPRC5D antibody or an antigen-binding fragment thereof, comprising 3 CDRs of a heavy chain variable region VH: HCDR1, HCDR2 and HCDR3, and 3 CDRs of a light chain variable region VL: LCDR1, LCDR2 and LCDR3, wherein
   (i) the HCDR1, the HCDR2 and the HCDR3 are selected from three complementarity determining regions HCDR1, HCDR2 and HCDR3 contained in a VH set forth in any one of SEQ ID NOs: 62, 38 and 40, respectively; and the LCDR1, the LCDR2 and the LCDR3 are three complementarity determining regions LCDR1, LCDR2 and LCDR3 contained in a VL set forth in SEQ ID NO: 63, respectively;
   (ii) the HCDR1, the HCDR2 and the HCDR3 are selected from three complementarity determining regions HCDR1, HCDR2 and HCDR3 contained in a VH set forth in SEQ ID NO: 72, respectively; and the LCDR1, the LCDR2 and the LCDR3 are three complementarity determining regions LCDR1, LCDR2 and LCDR3 contained in a VL set forth in SEQ ID NO: 73, respectively; or
   (iii) the HCDR1, the HCDR2 and the HCDR3 are selected from three complementarity determining regions HCDR1, HCDR2 and HCDR3 contained in a VH set forth in SEQ ID NO: 82, respectively; and the LCDR1, the LCDR2 and the LCDR3 are three complementarity determining regions LCDR1, LCDR2 and LCDR3 contained in a VL set forth in SEQ ID NO: 83, respectively.
2. An anti-GPRC5D antibody or an antigen-binding fragment thereof, comprising
   (i) an HCDR1 set forth in SEQ ID NO: 64, an HCDR2 set forth in SEQ ID NO: 65, 39, or 41, an HCDR3 set forth in SEQ ID NO: 66, an LCDR1 set forth in SEQ ID NO: 67, an LCDR2 set forth in SEQ ID NO: 68, and an LCDR3 set forth in SEQ ID NO: 69;
   (ii) an HCDR1 set forth in SEQ ID NO: 74, an HCDR2 set forth in SEQ ID NO: 75, an HCDR3 set forth in SEQ ID NO: 76, an LCDR1 set forth in SEQ ID NO: 77, an LCDR2 set forth in SEQ ID NO: 78, and an LCDR3 set forth in SEQ ID NO: 79; or
   (iii) an HCDR1 set forth in SEQ ID NO: 84, an HCDR2 set forth in SEQ ID NO: 85, an HCDR3 set forth in SEQ ID NO: 86, an LCDR1 set forth in SEQ ID NO: 87, an LCDR2 set forth in SEQ ID NO: 78, and an LCDR3 set forth in SEQ ID NO: 88.
3. The antibody or the antigen-binding fragment thereof according to embodiment 1 or 2, comprising a heavy chain variable region VH, wherein the heavy chain variable region
   (i) comprises or consists of an amino acid sequence having at least 90%, 91%, 92%, 93%, 94%, 95%, 96%, 97%, 98%, or 99% identity to an amino acid sequence of SEQ ID NO: 62, 72, 82, 16, 23, 25, 27, 29, 38, or 40; or
   (ii) comprises or consists of an amino acid sequence of SEQ ID NO: 62, 72, 82, 16, 23, 25, 27, 29, 38 or 40.
4. The antibody or the antigen-binding fragment thereof according to any one of embodiments 1-3, comprising a light chain variable region VL, wherein the light chain variable region
   (i) comprises or consists of an amino acid sequence having at least 90%, 91%, 92%, 93%, 94%, 95%, 96%, 97%, 98%, or 99% identity to an amino acid sequence of SEQ ID NO: 63, 73, 83, 17, 19, or 21; or
   (ii) comprises or consists of an amino acid sequence of SEQ ID NO: 63, 73, 83, 17, 19 or 21.
5. The antibody or the antigen-binding fragment thereof according to embodiment 1 or 2, comprising a heavy chain variable region VH and a light chain variable region VL, wherein
   (i) the heavy chain variable region comprises or consists of an amino acid sequence set forth in SEQ ID NO: 62, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 63, or an amino acid sequence having at least 90%, 91%, 92%, 93%, 94%, 95%, 96%, 97%, 98% or 99% identity thereto;
   (ii) the heavy chain variable region comprises or consists of an amino acid sequence set forth in SEQ ID NO: 72, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 73, or an amino acid sequence having at least 90%, 91%, 92%, 93%, 94%, 95%, 96%, 97%, 98% or 99% identity thereto;
   (iii) the heavy chain variable region comprises or consists of an amino acid sequence set forth in SEQ ID NO: 82, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 83, or an amino acid sequence having at least 90%, 91%, 92%, 93%, 94%, 95%, 96%, 97%, 98% or 99% identity thereto; or
   (iv) the heavy chain variable region comprises or consists of an amino acid sequence set forth in SEQ ID NO: 16, 23, 25, 27, 29, 38 or 40, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 17, 19 or 21, or an amino acid sequence having at least 90%, 91%, 92%, 93%, 94%, 95%, 96%, 97%, 98% or 99% identity thereto.
6. The antibody or the antigen-binding fragment thereof according to embodiment 1 or 2, comprising a heavy chain variable region VH and a light chain variable region VL, wherein the VH and the VL comprise or consist of amino acid sequences set forth below, respectively:
   (i). SEQ ID NO: 62 and SEQ ID NO: 63;
   (ii). SEQ ID NO: 72 and SEQ ID NO: 73;
   (iii). SEQ ID NO: 82 and SEQ ID NO: 83;
   (iv). SEQ ID NO: 16, 23, 25, 27, 29, 38, or 40 and SEQ ID NO: 21;
   (v). SEQ ID NO: 16 or 25 and SEQ ID NO: 17; or
   (vi). SEQ ID NO: 16 or 27 and SEQ ID NO: 19.
7. The antibody or the antigen-binding fragment thereof according to any one of embodiments 1-6, further comprising a heavy chain constant region HC, wherein, for example, the heavy chain constant region HC of the antibody is a heavy chain constant region of IgG1, IgG2, IgG3, or IgG4, preferably a heavy chain constant region of IgG1.
8. The antibody or the antigen-binding fragment thereof according to embodiment 7,
   (i) comprising or consisting of an amino acid sequence having at least 85%, 90%, 91%, 92%, 93%, 94%, 95%, 96%, 97%, 98% or 99% identity to an amino acid sequence selected from SEQ ID NO: 45 or 48; or
   (ii) comprising or consisting of an amino acid sequence selected from SEQ ID NO: 45 or 48.
9. The antibody or the antigen-binding fragment thereof according to embodiment 7 or 8, wherein the heavy chain constant region comprises a mutation that reduces binding to an Fcy receptor, e.g., an LALA mutation, a D265A mutation and/or a P329A mutation, preferably an LALA mutation, a D265A mutation and a P329A mutation.
10. The antibody or the antigen-binding fragment thereof according to any one of embodiments 1-9, comprising a light chain constant region, wherein, for example, the light chain constant region is a lambda or kappa light chain constant region.
11. The antibody or the antigen-binding fragment thereof according to embodiment 10, wherein the light chain constant region
   (i) comprises or consists of an amino acid sequence having at least 85%, 90%, 91%, 92%, 93%, 94%, 95%, 96%, 97%, 98% or 99% identity to an amino acid sequence selected from SEQ ID NO: 47 or 50; or
   (ii) comprises or consists of an amino acid sequence selected from SEQ ID NO: 47 or 50.
12. The antibody or the antigen-binding fragment thereof according to any one of embodiments 1-11, comprising a heavy chain, wherein the heavy chain comprises or consists of an amino acid sequence of SEQ ID NO: 60, 70, 80, 14, 22, 24, 26 or 28, or an amino acid sequence having at least 85%, 90%, 91%, 92%, 93%, 94%, 95%, 96%, 97%, 98% or 99% identity thereto.
13. The antibody or the antigen-binding fragment thereof according to any one of embodiments 1-12, comprising a light chain, wherein the light chain comprises or consists of an amino acid sequence of SEQ ID NO: 61, 71, 81, 15, 18 or 20, or an amino acid sequence having at least 85%, 90%, 91%, 92%, 93%, 94%, 95%, 96%, 97%, 98% or 99% identity thereto.
14. The antibody or the antigen-binding fragment thereof according to embodiment 12 or 13, comprising a heavy chain and a light chain, wherein
   (i) the heavy chain comprises or consists of an amino acid sequence of SEQ ID NO: 60, or an amino acid sequence having at least 85%, 90%, 91%, 92%, 93%, 94%, 95%, 96%, 97%, 98% or 99% identity thereto, and the light chain comprises or consists of an amino acid sequence of SEQ ID NO: 61, or an amino acid sequence having at least 85%, 90%, 91%, 92%, 93%, 94%, 95%, 96%, 97%, 98% or 99% identity thereto;
   (ii) the heavy chain comprises or consists of an amino acid sequence of SEQ ID NO: 70, or an amino acid sequence having at least 85%, 90%, 91%, 92%, 93%, 94%, 95%, 96%, 97%, 98% or 99% identity thereto, and the light chain comprises or consists of an amino acid sequence of SEQ ID NO: 71, or an amino acid sequence having at least 85%, 90%, 91%, 92%, 93%, 94%, 95%, 96%, 97%, 98% or 99% identity thereto;
   (iii) the heavy chain comprises or consists of an amino acid sequence of SEQ ID NO: 80, or an amino acid sequence having at least 85%, 90%, 91%, 92%, 93%, 94%, 95%, 96%, 97%, 98% or 99% identity thereto, and the light chain comprises or consists of an amino acid sequence of SEQ ID NO: 81, or an amino acid sequence having at least 85%, 90%, 91%, 92%, 93%, 94%, 95%, 96%, 97%, 98% or 99% identity thereto;
   (iv) the heavy chain comprises or consists of an amino acid sequence set forth in SEQ ID NO: 14, 22, 24, 26 or 28, or an amino acid sequence having at least 85%, 90%, 91%, 92%, 93%, 94%, 95%, 96%, 97%, 98% or 99% identity thereto, and the light chain comprises or consists of an amino acid sequence set forth in SEQ ID NO: 15, 18 or 20, or an amino acid sequence having at least 85%, 90%, 91%, 92%, 93%, 94%, 95%, 96%, 97%, 98% or 99% identity thereto;
   (v) the heavy chain comprises or consists of an amino acid sequence set forth in SEQ ID NO: 14 or 24, or an amino acid sequence having at least 85%, 90%, 91%, 92%, 93%, 94%, 95%, 96%, 97%, 98% or 99% identity thereto, and the light chain comprises or consists of an amino acid sequence set forth in SEQ ID NO: 15, or an amino acid sequence having at least 85%, 90%, 91%, 92%, 93%, 94%, 95%, 96%, 97%, 98% or 99% identity thereto;
   (vi) the heavy chain comprises or consists of an amino acid sequence set forth in SEQ ID NO: 14 or 26, or an amino acid sequence having at least 85%, 90%, 91%, 92%, 93%, 94%, 95%, 96%, 97%, 98% or 99% identity thereto, and the light chain comprises or consists of an amino acid sequence set forth in SEQ ID NO: 18, or an amino acid sequence having at least 85%, 90%, 91%, 92%, 93%, 94%, 95%, 96%, 97%, 98% or 99% identity thereto;
   (vii) the heavy chain comprises or consists of an amino acid sequence set forth in SEQ ID NO: 14, 22, 24, 26, or 28, or an amino acid sequence having at least 85%, 90%, 91%, 92%, 93%, 94%, 95%, 96%, 97%, 98%, or 99% identity thereto, and the light chain comprises or consists of an amino acid sequence set forth in SEQ ID NO: 20, or an amino acid sequence having at least 85%, 90%, 91%, 92%, 93%, 94%, 95%, 96%, 97%, 98%, or 99% identity thereto.
15. The antibody or the antigen-binding fragment thereof according to embodiment 14, comprising a heavy chain and a light chain, wherein the heavy chain and the light chain comprise or consist of amino acid sequences set forth in SEQ ID NOs shown below, respectively:
   (i). SEQ ID NO: 60 and SEQ ID NO: 61;
   (ii). SEQ ID NO: 70 and SEQ ID NO: 71;
   (iii). SEQ ID NO: 80 and SEQ ID NO: 81;
   (iv). SEQ ID NO: 14 or 24 and SEQ ID NO: 15;
   (v). SEQ ID NO: 14 or 26 and SEQ ID NO: 18; or
   (vi). SEQ ID NO: 14, 22, 24, 26, or 28 and SEQ ID NO: 20.
16. The antibody or the antigen-binding fragment thereof according to any one of embodiments 1-15, wherein the antibody is a humanized antibody or a chimeric antibody.
17. The antibody or the antigen-binding fragment thereof according to any one of embodiments 1-16, wherein the antibody is a monoclonal antibody.
18. The antibody or the antigen-binding fragment thereof according to any one of embodiments 1-17, wherein the antigen-binding fragment is an antibody fragment selected from: a Fab, a Fab', a Fab'-SH, an Fv, a single-chain antibody (e.g., scFv), an (Fab')₂, a single-domain antibody (e.g., VHH), a domain antibody (dAb), or a linear antibody.
19. The antibody or the antigen-binding fragment thereof according to any one of embodiments 1-6, wherein the antibody is a bispecific antibody or a multispecific antibody comprising a first binding specificity for GPRC5D and other binding specificities for one or more molecules.
20. The antibody or the antigen-binding fragment thereof according to embodiment 19, wherein the antibody is a bispecific antibody comprising the first binding specificity for GPRC5D and a second binding specificity for CD3.
21. The bispecific antibody according to embodiment 20, comprising a first antigen-binding region and a second antigen-binding region,
   wherein the first antigen-binding region specifically binds to GPRC5D and comprises a VH and a VL, wherein
   the VH comprises the HCDR1, HCDR2 and HCDR3 as defined in embodiment 1 or 2, and the VL comprises the LCDR1, LCDR2 and LCDR3 as defined in embodiment 1 or 2; or
   the VH and the VL are the VH and the VL as defined in any one of embodiments 3-6;
   the second antigen-binding region specifically binds to CD3.
22. The bispecific antibody according to embodiment 21, wherein the second antigen-binding region comprises a VH and a VL, wherein the VH comprises 3 complementarity determining regions from a heavy chain variable region (HCDRs): HCDR1, HCDR2 and HCDR3, and the VL comprises 3 complementarity determining regions from a light chain variable region (LCDRs): LCDR1, LCDR2 and LCDR3, wherein
   (i) the HCDR1, HCDR2 and HCDR3 are selected from the three complementarity determining regions HCDR1, HCDR2 and HCDR3 contained in the VH set forth in any one of SEQ ID NO: 3, and the LCDR1, LCDR2 and LCDR3 are selected from the three complementarity determining regions LCDR1, LCDR2 and LCDR3 contained in the VL set forth in any one of SEQ ID NO: 4;
   (ii) the HCDR1 consists of an amino acid sequence set forth in SEQ ID NO: 5, the HCDR2 consists of an amino acid sequence set forth in SEQ ID NO: 6, the HCDR3 consists of an amino acid sequence set forth in SEQ ID NO: 7, the LCDR1 consists of an amino acid sequence set forth in SEQ ID NO: 8, the LCDR2 consists of an amino acid sequence set forth in SEQ ID NO: 9, and the LCDR3 consists of an amino acid sequence set forth in SEQ ID NO: 10.
23. The bispecific antibody according to embodiment 22, wherein the second antigen-binding region comprises a VH and a VL, wherein
   the VH comprises or consists of an amino acid sequence set forth in SEQ ID NO: 3, or an amino acid sequence having at least 90%, 91%, 92%, 93%, 94%, 95%, 96%, 97%, 98% or 99% identity thereto,
   and/or the VL comprises or consists of an amino acid sequence set forth in SEQ ID NO: 4, or an amino acid sequence having at least 90%, 91%, 92%, 93%, 94%, 95%, 96%, 97%, 98% or 99% identity thereto.
24. The bispecific antibody according to any one of embodiments 21-23, wherein the first antigen-binding region is the Fab of the anti-GPRC5D antibody as defined in any one of embodiments 1-18.
25. The bispecific antibody according to embodiment 24, wherein the Fab comprises a CH1, wherein the CH1 is a CH1 from IgG1, IgG2, IgG3, or IgG4, preferably a CH1 from IgG1.
26. The bispecific antibody according to embodiment 25, wherein the CH1 (i) comprises or consists of an amino acid sequence having at least 85%, 90%, 91%, 92%, 93%, 94%, 95%, 96%, 97%, 98%, or 99% identity to an amino acid sequence selected from SEQ ID NO: 44; or (ii) comprises or consists of an amino acid sequence selected from SEQ ID NO: 44.
27. The bispecific antibody according to any one of embodiments 21-26, wherein the second antigen-binding region is an scFv.
28. The bispecific antibody according to embodiment 27, wherein the scFv comprises a linker comprising, for example, an amino acid sequence (G4S)n, wherein n = 1, 2, 3, 4, or 5, preferably n = 3 or 4, and more preferably n = 3.
29. The bispecific antibody according to any one of embodiments 21-28, wherein the bispecific antibody is an IgG-like bispecific antibody comprising an Fc dimer, wherein two Fc regions constituting the Fc dimer are identical or different.
30. The bispecific antibody according to embodiment 29, wherein one or both of the Fc regions comprise a mutation that reduces binding to an Fcy receptor, e.g., one or more of an L234A/L235A mutation, a D265A mutation and a P329A mutation, for example, an L234A/L235A mutation, a D265A mutation and a P329A mutation.
31. The bispecific antibody according to embodiment 30, wherein one or both of the Fc regions comprise or consist of an amino acid sequence set forth in SEQ ID NO: 46 or 49, or an amino acid sequence having at least 90%, for example, 95%, 96%, 97%, 99% or more, identity thereto.
32. The bispecific antibody according to any one of embodiments 29-31, wherein the two Fc regions are different, and preferably, a corresponding knob mutation and a corresponding hole mutation are introduced into a first monomeric Fc region and a second monomeric Fc region, respectively.
33. The bispecific antibody according to embodiment 32, wherein
   a) one Fc-region polypeptide comprises a knob mutation T366W, and the other Fc-region polypeptide comprises hole mutations T366S, L368A and Y407V, or
   b) one Fc-region polypeptide comprises knob mutations T366W and Y349C, and the other Fc-region polypeptide comprises hole mutations T366S, L368A, Y407V and S354C, or
   c) one Fc-region polypeptide comprises knob mutations T366W and S354C, and the other Fc-region polypeptide comprises hole mutations T366S, L368A, Y407V and Y349C;
   optionally, the Fc region further comprises a mutation that reduces binding to the Fcy receptor, e.g., one or more of an L234A/L235A mutation, a D265A mutation and a P329A mutation, for example, an L234A/L235A mutation, a D265A mutation and a P329A mutation.
34. The bispecific antibody according to any one of embodiments 29-33, wherein one or both of the Fc regions comprise a hinge region, e.g., EPKSS or EPKSC.
35. The bispecific antibody according to embodiment 33, wherein
   (i) the first Fc region comprises a knob mutation, and
      a) comprises or consists of an amino acid sequence set forth in SEQ ID NO: 43 or 89; or
      b) comprises or consists of an amino acid sequence having at least 90% identity, e.g., 95%, 96%, 97%, 99% or more identity, to SEQ ID NO: 43 or 89 and comprising an L234A/L235A mutation, a D265A mutation, a P329A mutation, and a knob mutation (e.g., S354C and T366W); and/or
   (ii) the second Fc region comprises a hole mutation, and
      a) comprises or consists of an amino acid sequence set forth in SEQ ID NO: 30, 42 or 59; or
      b) comprises or consists of an amino acid sequence having at least 90% identity, e.g., 95%, 96%, 97%, 99% or more identity, to SEQ ID NO: 30, 42 or 59 and comprising an L234A/L235A mutation, a D265A mutation, a P329A mutation, and a hole mutation (e.g., Y349C, T366S, L368A, and Y407V).
36. The bispecific antibody according to any one of embodiments 21-35, comprising the first antigen-binding region, the second antigen-binding region, and the Fc dimer, wherein the first antigen-binding region is a Fab fragment specifically binding to GPRC5D, and the second antigen-binding region is an scFv specifically binding to CD3.
37. The bispecific antibody according to embodiment 36, comprising one first antigen-binding region Fab, one second antigen-binding region scFv, and the Fc dimer, wherein the Fab comprises VH-CH1 and VL-CL, the scFv comprises VH-linker-VL, one Fc region of the Fc dimer comprises a hole mutation, and the other comprises a knob mutation; wherein
   the Fc region comprising the hole mutation constitutes a first heavy chain;
   the C-terminus of the CH1 of the Fab fragment is fused to the N-terminus of the VH of the scFv fragment, and the C-terminus of the VL of the scFv fragment is fused to a CH2 or the hinge region of the Fc region comprising the knob mutation, thereby constituting a second heavy chain;
      and
   the VL-CL of the Fab fragment constitutes a light chain.
38. The bispecific antibody according to embodiment 37, wherein
   the first heavy chain comprises or consists of an amino acid sequence set forth in SEQ ID NO: 30, or an amino acid sequence having at least 85%, 90%, 91%, 92%, 93%, 94%, 95%, 96%, 97%, 98%, or 99% identity thereto;
   the second heavy chain comprises or consists of an amino acid sequence set forth in SEQ ID NO: 31, 34, or 35, or an amino acid sequence having at least 85%, 90%, 91%, 92%, 93%, 94%, 95%, 96%, 97%, 98%, or 99% identity thereto; and/or
   the light chain comprises or consists of an amino acid sequence set forth in SEQ ID NO: 20, or an amino acid sequence having at least 85%, 90%, 91%, 92%, 93%, 94%, 95%, 96%, 97%, 98% or 99% identity thereto.
39. The bispecific antibody according to embodiment 36, comprising two first antigen-binding regions Fab, one second antigen-binding region scFv, and the Fc dimer, wherein the Fab comprises VH-CH1 and VL-CL, the scFv comprises VH-linker-VL, one Fc region of the Fc dimer comprises a hole mutation, and the other comprises a knob mutation; wherein
   the C-terminus of the CH1 of the first Fab fragment is fused to a CH2 or the hinge region of the Fc region comprising the hole mutation, thereby constituting a first heavy chain;
   the C-terminus of the CH1 of the second Fab fragment is fused to the N-terminus of the VH of the scFv fragment, and the C-terminus of the VL of the scFv fragment is fused to a CH2 or the hinge region of the Fc region comprising the knob mutation, thereby constituting a second heavy chain;
      and
   the VL-CL of the first and second Fab fragments constitute two light chains;
   wherein the first Fab and second Fab fragments are identical or different.
40. The bispecific antibody according to embodiment 39, wherein
   (i) the first heavy chain comprises or consists of an amino acid sequence set forth in SEQ ID NO: 33, or an amino acid sequence having at least 85%, 90%, 91%, 92%, 93%, 94%, 95%, 96%, 97%, 98%, or 99% identity thereto;
      the second heavy chain comprises or consists of an amino acid sequence set forth in SEQ ID NO: 31, or an amino acid sequence having at least 85%, 90%, 91%, 92%, 93%, 94%, 95%, 96%, 97%, 98% or 99% identity thereto; and/or
      the light chain comprises or consists of an amino acid sequence set forth in SEQ ID NO: 20, or an amino acid sequence having at least 85%, 90%, 91%, 92%, 93%, 94%, 95%, 96%, 97%, 98%, or 99% identity thereto;
   (ii) the first heavy chain comprises or consists of an amino acid sequence set forth in SEQ ID NO: 32, or an amino acid sequence having at least 85%, 90%, 91%, 92%, 93%, 94%, 95%, 96%, 97%, 98%, or 99% identity thereto;
      the second heavy chain comprises or consists of an amino acid sequence set forth in SEQ ID NO: 34, or an amino acid sequence having at least 85%, 90%, 91%, 92%, 93%, 94%, 95%, 96%, 97%, 98%, or 99% identity thereto; and/or
      the light chain comprises or consists of an amino acid sequence set forth in SEQ ID NO: 20, or an amino acid sequence having at least 85%, 90%, 91%, 92%, 93%, 94%, 95%, 96%, 97%, 98%, or 99% identity thereto; or
   (iii) the first heavy chain comprises or consists of an amino acid sequence set forth in SEQ ID NO: 36, or an amino acid sequence having at least 85%, 90%, 91%, 92%, 93%, 94%, 95%, 96%, 97%, 98%, or 99% identity thereto;
      the second heavy chain comprises or consists of an amino acid sequence set forth in SEQ ID NO: 35, or an amino acid sequence having at least 85%, 90%, 91%, 92%, 93%, 94%, 95%, 96%, 97%, 98%, or 99% identity thereto; and/or
      the light chain comprises or consists of an amino acid sequence set forth in SEQ ID NO: 20, or an amino acid sequence having at least 85%, 90%, 91%, 92%, 93%, 94%, 95%, 96%, 97%, 98% or 99% identity thereto.
41. The bispecific antibody according to embodiment 36, comprising one first antigen-binding region Fab, one second antigen-binding region scFv, and the Fc dimer, wherein the Fab comprises VH-CH1 and VL-CL, the scFv comprises VH-linker-VL, one Fc region of the Fc dimer comprises a hole mutation, and the other comprises a knob mutation; wherein
   the C-terminus of the CH1 of the Fab fragment is fused to a CH2 or the hinge region of the Fc region comprising the hole mutation, thereby constituting a first heavy chain;
   the C-terminus of the VL of the scFv fragment is fused to a CH2 or the hinge region of the Fc region comprising the knob mutation, thereby constituting a second heavy chain;
      and
   the VL-CL of the Fab fragment constitutes a light chain.
42. The bispecific antibody according to embodiment 41, wherein
   the first heavy chain comprises or consists of an amino acid sequence set forth in SEQ ID NO: 32, 33, or 36, or an amino acid sequence having at least 85%, 90%, 91%, 92%, 93%, 94%, 95%, 96%, 97%, 98%, or 99% identity thereto;
   the second heavy chain comprises or consists of an amino acid sequence set forth in SEQ ID NO: 37, or an amino acid sequence having at least 85%, 90%, 91%, 92%, 93%, 94%, 95%, 96%, 97%, 98%, or 99% identity thereto; and/or
   the light chain comprises or consists of an amino acid sequence set forth in SEQ ID NO: 20, or an amino acid sequence having at least 85%, 90%, 91%, 92%, 93%, 94%, 95%, 96%, 97%, 98% or 99% identity thereto.
43. A nucleic acid molecule, encoding any one of the chains of the antibody or the antigen-binding fragment thereof according to any one of embodiments 1-20 or any one of the chains in the bispecific antibody according to any one of embodiments 21-42, or consisting of a nucleic acid sequence.
44. An expression vector, comprising the nucleic acid molecule according to embodiment 43, wherein preferably, the expression vector is pCDNA, e.g., pCDNA3.1.
45. A host cell, comprising the nucleic acid molecule according to embodiment 43 or the expression vector according to embodiment 44, wherein preferably, the host cell is prokaryotic or eukaryotic, e.g., a 293 cell or a CHO cell, such as a 293F cell or a 293T cell or a CHO-S cell.
46. A method for preparing the antibody or the antigen-binding fragment thereof according to any one of embodiments 1-20 or the bispecific antibody according to any one of embodiments 21-42, wherein the method comprises culturing the host cell of the nucleic acid molecule according to embodiment 43 or the expression vector according to embodiment 44 under a condition suitable for expressing the chains of the antibody, and optionally recovering the antibody from the host cell (or the host cell medium).
47. An immunoconjugate, comprising the antibody or the antigen-binding fragment thereof according to any one of embodiments 1-20 or the bispecific antibody according to any one of embodiments 21-42.
48. A pharmaceutical composition or a medicament or a formulation, comprising the antibody or the antigen-binding fragment thereof according to any one of embodiments 1-20 or the bispecific antibody according to any one of embodiments 21-42 or the immunoconjugate according to embodiment 47, and optionally a pharmaceutical supplementary material.
49. A pharmaceutical combination product, comprising the antibody or the antigen-binding fragment thereof according to any one of embodiments 1-20 or the bispecific antibody according to any one of embodiments 21-42 or the immunoconjugate according to embodiment 47, and one or more additional therapeutic agents (e.g., a chemotherapeutic agent, a cytokine, a cytotoxic agent, an additional antibody, a small molecule drug, or an immunomodulatory agent).
50. A method for preventing or treating a cancer in a subject, comprising administering to the subject an effective amount of the antibody or the antigen-binding fragment thereof according to any one of embodiments 1-20 or the bispecific antibody according to any one of embodiments 21-42, or the immunoconjugate according to embodiment 47, or the pharmaceutical composition or the formulation according to embodiment 48, or the pharmaceutical combination product according to embodiment 49.
51. The method according to embodiment 50, wherein tumor cells of the cancer have an elevated protein level and/or nucleic acid level (e.g., elevated expression) of GPRC5D.
52. The method according to embodiment 50 or 51, wherein the cancer is a solid tumor or a hematological tumor, such as myeloma, colon cancer, rectal cancer, or colorectal cancer.
53. The method according to any one of embodiments 50-52, wherein the method further comprises administering in combination with an additional therapy such as a therapeutic modality (e.g., surgical therapy or radiotherapy) and/or an additional therapeutic agent (e.g., a chemotherapeutic agent, a cytokine, a cytotoxic agent, an additional antibody, a small molecule drug, or an immunomodulatory agent).
54. A method for detecting the presence of GPRC5D in a biological sample, comprising
   (i) contacting the biological sample with the antibody or the antigen-binding fragment thereof according to any one of embodiments 1-20 or the bispecific antibody according to any one of embodiments 21-42 under a condition that allows the antibody to bind to GPRC5D, and
   (ii) detecting whether a complex is formed by the antibody or the bispecific antibody and GPRC5D,
   wherein the formation of the complex indicates the presence of GPRC5D.

### EXAMPLES

### Example 1: Preparation and Characterization of Humanized CD3 Antibody

### 1.1 Preparation of CD3 antibody humanization

The SP34 antibody is a murine antibody specifically binding to a CD3 epsilon subunit (CD3ε), which has species cross-activity with humans and monkeys (Pessano, S., et al. (1985). "The T3/T cell receptor complex: antigenic distinction between the two 20-kd T3 (T3-delta and T3-epsilon) subunits." EMBO J 4(2): 337-344). In this example, the antibody SP34 was used as a candidate molecule for humanization construction.

The murine antibody SP34 was humanized using CDR grafting technology. Briefly, the SP34 VH and SP34 VL sequences described above were searched and aligned in the IMGT database, thereby obtaining human germline gene sequences IGHV3-73 *01 and IGLV7-46*01 which had high homology to the heavy chain variable region and the light chain variable region of SP34, respectively, and then using them as frameworks of humanized antibody variable regions; the CDRs of the murine antibody SP34 were grafted into the corresponding heavy and light chain variable region frameworks of the humanized antibody, respectively, to form humanized anti-CD3 antibody variable regions. In order to maintain the affinity of the murine antibody SP34, the humanized antibody variable regions obtained were back-mutated.

Thus, a humanized heavy chain variable region sequence (hu34H1) and a light chain variable region sequence (hu34L2) of the SP34 antibody were obtained, and the humanized SP34 antibody was further obtained. The specific sequences are shown in Table 1.

**Table 1: Sequences of humanized anti-CD3 antibody (CDR sequences are numbered according to the Kabat numbering scheme**

| Name | SEQ ID NO |
|---|---|
| Heavy chain of humanized SP34 antibody hu34 hu34H1 | 1 |
| Light chain of humanized SP34 antibody hu34 hu34L2 | 2 |
| Heavy chain variable region VH of humanized SP34 antibody hu34 (hu34H1) | 3 |
| Light chain variable region VL of humanized SP34 antibody hu34 (hu34L2) | 4 |
| HCDR1 | 5 |
| HCDR2 | 6 |
| HCDR3 | 7 |
| LCDR1 | 8 |
| LCDR2 | 9 |
| LCDR3 | 10 |

The sequence of the humanized control antibody (Roche-CD3/017-Roche-CD3) was derived from the patent US 2016/0075785 A1, the heavy chain amino acid sequence of the control antibody was set forth in SEQ ID NO: 51, and the light chain was set forth in SEQ ID NO: 52. See the sequence listing for details.

The variable region amino acid sequences in Table 1 and the control antibody Roche-CD3 were sent to General Biology System (Anhui) Co., Ltd. for codon optimization and gene synthesis. The genes encoding the VH region and the VL region of the Roche-CD3 antibody were inserted sequentially into an expression vector pcDNA3.1(+) comprising a coding gene of a human IgG1 heavy chain constant region (the amino acid sequence set forth in SEQ ID NO: 45) and a coding gene of a lambda light chain constant region (the amino acid sequence set forth in SEQ ID NO: 47) to obtain a plasmid expressing the anti-CD3 humanized antibody 017-2 full-length heavy chain hu34H1 (SEQ ID NO: 1) and a plasmid expressing the full-length light chain hu34L2 (SEQ ID NO: 2). Meanwhile, the synthesized VH region (hu34H1) and VL region (hu34L2) were linked via a linker peptide (G4S)3 (SEQ ID NO: 12) to constitute a single-chain antibody 23L2 (23L2ScFv) (SEQ ID NO: 11), and inserted into the expression vector pcDNA3.1(+) comprising a coding gene of a human IgG1 heavy chain constant region Fc region (the amino acid sequence set forth in SEQ ID NO: 46) to obtain a plasmid expressing DA023AH23L2 (scFv-Fc) (SEQ ID NO: 13). The heavy chain constant regions used both contained mutations at sites L234A, L235A, D265A, and P329A (according to Eu numbering) to eliminate effector functions.

The single-chain antibody (23L2) was named 23L2ScFv, and its amino acid sequence was set forth in SEQ ID NO: 11. The synthesized anti-CD3 single-chain antibody comprising the constant region Fc domain was named DA023AH23L2 (VH-linker peptide-VL-Fc) having a sequence set forth in SEQ ID NO: 13, wherein the amino acid sequence of VH-linker peptide-VL was set forth in SEQ ID NO: 11, and the amino acid sequence of the Fc region was set forth in SEQ ID NO: 46.

The plasmids obtained above were co-transfected with ExpiCHO-S cells expressing the control antibodies Roche-CD3, 017-2 and DA023AH23L2 using an ExpiCHO^{™} expression system (ThermoFisher, Cat# A29133) according to the manufacturer's product instructions. The cells were cultured for 10-12 days after transfection. When the cell viability decreased to 60%-70%, the supernatant was collected, and the antibody expressed in the supernatant was purified using a MabSelect Sure protein A affinity chromatography system (GE healthcare). The purified antibody was concentrated, and sterile filtration was performed. The purity of the antibody protein was detected by SDS-PAGE and size exclusion chromatography. The results indicated that the purity of the antibody met the requirements, and the antibody could be used in the next step.

### 1.2 Detection of activation of humanized CD3 antibody

The activation of the CD3 antibody was detected using a Jurkat/NFAT-luc (luciferase) reporter gene system. According to the manufacturer's instructions, reporter gene elements in NFAT-luc2P (plasmid: Promega, Cat# E8481) were transfected into Jurkat cells (Cell Bank of Type Culture Collection Committee of the Chinese Academy of Sciences, Cat# SCSP-513) by Gene Pul Ser X Cell TM (BIO-RAD) electroporation, and then the cells were screened with hygromycin B (Invitrogen, Cat# 10687010) to obtain an NFAT-luc2P polyclonal cell strain. The polyclonal cells were subjected to limiting dilution to obtain a stable monoclonal cell strain Jurkat/NFAT-luc highly expressing NFAT-luc2P.

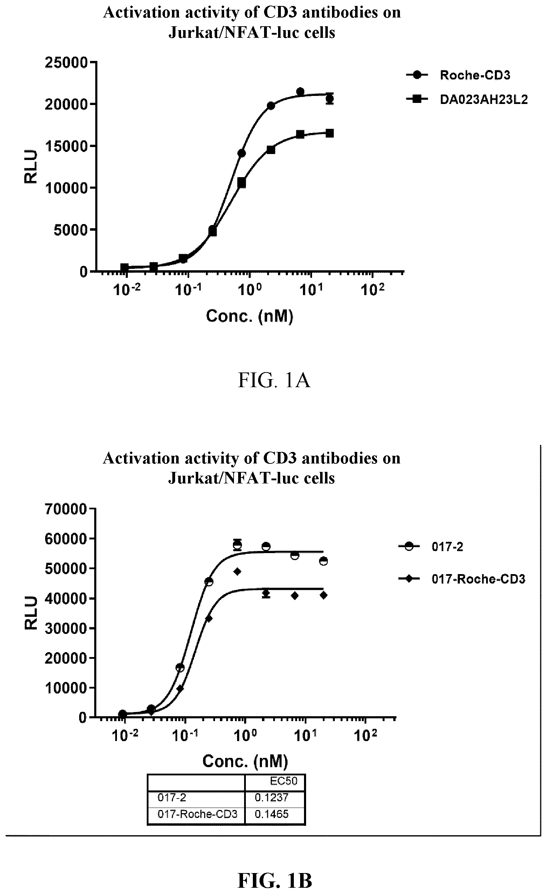

The cells were collected and centrifuged, and the supernatant was removed. The cells were resuspended in an assay buffer (99% 1640 medium + 1% FBS) with a cell density of 5 × 10⁵ cells/mL. The cells were seeded into a 384-well plate (Corning, Cat# 3570) at 40 µL per well. Then, 10 µL of the humanized anti-CD3 antibodies DA023AH23L2 and Roche-CD3 diluted serially (at a final concentration of 20 nM, 3-fold dilution, 8 gradients) was added per well. The 384-well plate was placed in an incubator at 37 °C. After 6 h, 30 µL of One-Glo (Promega, Cat# E6130) reagent was added into each well, and finally, luminescence signals were detected using a multifunctional microplate reader (Tecan Infinite F200PRO). The activation of the CD3 antibody was directly reflected by the expression level of luciferase. The results are shown in FIG. 1, indicating that 017-2 and DA023AH23L2 have activating effects.

### Example 2: Immunogen

Due to the difficulty in producing a recombinant GPRC5D antigen, a transfected cell line of GPRC5D [human and cyno] was produced using standard methods for use as a whole cell antigen for antibody production and characterization studies (Table 2).

The gene sequence of the human GPRC5D protein (NP_061124.1 (NCBI sequence number); sp|Q9NZD1 (Uniprot sequence number), SEQ ID NO: 57) was subjected to codon optimization and gene synthesis by General Biology System (Anhui) Co., Ltd. The gene sequence encoding the human GPRC5D protein was transfected into CHO-K1 (ACTT, Cat# CCL-61) and HEK293T (Cell Bank of Type Culture Collection Committee of the Chinese Academy of Sciences, Cat# SCSP-502) cells, respectively, using a Lipofectamine^{™} 2000 (Invitrogen, Cat# 11668019) transfection reagent. The gene sequence of the cynomolgus monkey GPRC5D protein (XP_005570249.2 (NCBI sequence number), SEQ ID NO: 58) was subjected to codon optimization and gene synthesis by General Biology System (Anhui) Co., Ltd. The gene sequence encoding the cynomolgus monkey GPRC5D protein was transfected into HEK293T cells using Lipofectamine^{™} 2000. The transfected cells were screened with 0.3 µg/mL of puromycin (Gibco, Cat# A1113802) and subjected to monoclonal seeding to obtain CHO-K1 (CHO-K1-HuGPRCSD) and HEK293T engineered monoclonal cells (HEK293T-HuGPRC5D) highly expressing human GPRC5D and HEK293T polyclonal cells (HEK293T-CynoGPRC5D) highly expressing cynomolgus monkey GPRC5D (Table 2). The amino acid sequences encoding the human GPRC5D and cynomolgus monkey GPRC5D proteins are shown in the sequence listing.

**Table 2: GPRC5D expression cell lines**

| | Protein | Cell line | Promoter | Resistance |
|---|---|---|---|---|
| CHO-K1-HuGPRC5D cell line | HuGPRC5D | CHO-K1 | CMV | Puromycin |
| HEK293T-HuGPRC5D cell line | HuGPRC5D | HEK293T | CMV | Puromycin |
| HEK293T-CynoGPRC5D | CynoGPRC5D | HEK293T | CMV | Puromycin |

### Example 3 Production of Anti-Human GPRC5D Antibodies

### 3.1 Hybridoma

### 3.1.1 Animal immunization

Five BALB/c mice (Zhejiang Vital River Laboratory Animal Technology Co., Ltd.) were immunized with the CHO-K1-HuGPRCSD cell line (Table 2). The mice were injected alternately once every two weeks for a total of four sessions (Table 3). Blood samples of the mice were collected intravenously 7 days after the first immunization and 7 days after the second booster immunization, respectively. Mouse immune sera were determined by flow cytometry (FACS) using the HEK293T-HuGPRC5D cell line and HEK293T-CynoGPRC5D cell line (Table 2). Two mice were selected for animal fusion. Four days after the last booster immunization, spleens of the mice were taken, and lymphocytes isolated from the spleens were used for fusion to produce hybridomas.

**Table 3. Mouse immunization procedures**

| Procedures | Route of administration | Dose |
|---|---|---|
| Primary Immunization | Subcutaneous injection | 5 × 10^6 CHO-K1-HuGPRCSD cells/mouse |
| 1st Boost | Intraperitoneal injection | 5 × 10^6 CHO-K1-HuGPRCSD cells/mouse |
| 2nd Boost | Subcutaneous injection | 5 × 10^6 CHO-K1-HuGPRCSD cells/mouse |
| Final Boost | Intraperitoneal injection | 5 × 10^6 CHO-K1-HuGPRCSD cells/mouse |

### 3.1.2 Hybridoma screening

Tissue culture supernatants from 249 hybridomas were subjected to a primary screening test by the FACS assay, in which 31 clones binding to the HEK293T-HuGPRC5D cell line or the HEK293T-CynoGPRC5D cell line were picked for the next round of confirmation test. Positive cultures were retested with the screened antigen to confirm secretion.

### 3.1.3 Subcloning

The screened hybridoma cell lines (parental clones) were subcloned to ensure monoclonality. Subcloning was performed by reseeding the parental clones using a single step cloning system. The 24 subclones were transferred to a 96-well culture plate. The subclones were screened by FACS, and antibodies binding to both HuGPRC5D and CynoGPRC5D were screened.

### 3.1.4 Hybridoma sequencing

The light and heavy chain variable region sequences of the obtained murine anti-GPRC5D antibodies were obtained by the polymerase chain reaction (PCR) amplification technique. Total RNA of 24 antibodies was isolated using RNAprep pure Cell Kit (TIANGEN, Cat# DP430) and reversely transcribed into cDNA using RevertAid First Strand cDNA Synthesis Kit (Thermo, K1622). The heavy and light chain variable regions were cloned by PCR, and all PCRs were performed using high fidelity polymerases. The antibody heavy and light chain variable domain sequence fragments amplified by PCR were sent to Suzhou Genewiz Biological Technology Co., Ltd. for sequencing. The amino acid sequences encoding the light chain variable domains and the heavy chain variable domains of the produced antibodies were determined. The Kabat light and heavy chain variable region sequences of the antibodies are shown in Table 4.

**Table 4: Amino acid sequences of heavy chain variable regions and light chain variable regions of anti-GPRC5D antibodies**

| Antibody name | 29H12C9 | 4B9E9 | 39A9F1 |
|---|---|---|---|
| VH | SEQ ID NO:62 | SEQ ID NO:72 | SEQ ID NO:82 |
| VL | SEQ ID NO:63 | SEQ ID NO:73 | SEQ ID NO:83 |
| HCDR1 | SEQ ID NO:64 | SEQ ID NO:74 | SEQ ID NO:84 |
| HCDR2 | SEQ ID NO:65 | SEQ ID NO:75 | SEQ ID NO:85 |
| HCDR3 | SEQ ID NO:66 | SEQ ID NO:76 | SEQ ID NO:86 |
| LCDR1 | SEQ ID NO:67 | SEQ ID NO:77 | SEQ ID NO:87 |
| LCDR2 | SEQ ID NO:68 | SEQ ID NO:78 | SEQ ID NO:78 |
| LCDR3 | SEQ ID NO:69 | SEQ ID NO:79 | SEQ ID NO:88 |

### Example 4: Characterization of GPRC5D Antibodies Obtained by Hybridoma Technique

### 4.1 Synthesis and expression of anti-GPRC5D chimeric antibodies

The DNA sequences of the 3 murine antibodies in Table 4 were subj ected to codon optimization and gene synthesis by General Biology System (Anhui) Co., Ltd. The genes encoding the VH regions and the VL regions of the antibodies were inserted sequentially into an expression vector pcDNA3.1(+) comprising genes encoding a human IgG1 heavy chain constant region (SEQ ID NO: 48) and a kappa light chain constant region (SEQ ID NO: 50) to obtain murine chimeric antibodies.

The humanized control antibody GPRC5D (clone No. GC5B596, the heavy chain amino acid sequence was set forth in SEQ ID NO: 55, and the light chain amino acid sequence was set forth in SEQ ID NO: 56) and CD3 (clone No. CD3B219, the heavy chain amino acid sequence was set forth in SEQ ID NO: 53, and the light chain amino acid sequence was set forth in SEQ ID NO: 54) sequences were derived from the patent WO2018017786A2.

Plasmids encoding the heavy and light chains of the anti-GPRC5D antibodies were co-transfected into ExpiCHO-S cells to express the murine chimeric antibodies as described in Table 5 as well as the control antibodies GC5B596 and CD3B219, and then the corresponding purification was performed. The plasmids encoding the heavy and light chains of the anti-GPRC5D antibodies as described in Table 5 were co-transfected into the ExpiCHO-S cells using an ExpiCHO^{™} expression system (ThermoFisher, Cat# A29133) to express the anti-GPRC5D chimeric antibodies, and similarly to express the control antibodies GC5B596 and CD3B219, according to the manufacturer's product instructions. The cells were cultured for 10-12 days after transfection. When the cell viability decreased to 60%-70%, the supernatant was collected, and the antibody expressed in the supernatant was purified using a MabSelect Sure protein A affinity chromatography system (GE healthcare). The purified antibody was concentrated, and sterile filtration was performed. The purity of the antibody protein was detected by SDS-PAGE and size exclusion chromatography. The results indicated that the purity of the antibody was more than 90%, and the antibody could be used in the next step.

**Table 5: Chimeric antibody names and combinations of corresponding heavy and light chains**

| Chimeric antibody name | Heavy chain | Light chain |
|---|---|---|
| 29H12C9 | 29H12C9 VH-IgG1(SEQ ID NO:60) | 29H12C9 VL-kappa(SEQ ID NO:61) |
| 4B9E9 | 4B9E9 VH-IgG1(SEQ ID NO:70) | 4B9E9 VL-kappa(SEQ ID NO:71) |
| 39A9F1 | 39A9F1 VH-IgG1(SEQ ID NO:80) | 39A9F1 VL-kappa(SEQ ID NO:81) |

### 4.2 Binding of anti-GPRC5D chimeric antibodies to cells expressing human GPRC5D protein or cynomolgus monkey GPRC5D protein

Whether the anti-GPRC5D chimeric antibodies in the present invention could bind to the human GPRC5D protein or cynomolgus monkey GPRC5D protein stably expressed on the cell surface was determined by cell binding experiments.

The HEK293T-HuGPRC5D cell line and HEK293T-CynoGPRC5D shown in Table 2 were subjected to enzymatic digestion to obtain single cell suspensions of the two types of cells. The single cell suspensions were centrifuged at room temperature at 400× g, and then the media were discarded. The resulting cell pellets were washed once with PBS and then resuspended in the serially diluted anti-GPRC5D chimeric antibodies shown in Table 5 and the control antibody GC5B596 (at an initial concentration of 133.3 nM, 4-fold gradient dilution, 8 concentrations in total), and the cells were incubated at 4 °C for 30 min. The cells were washed once with PBS, and then fluorescent secondary antibody R-PE-conjugated AffiniPure Goat Anti-Human IgG, Fcy Fragment Specific (Jackson ImmunoResearch, Cat# 109-116-098) diluted in a 1:200 ratio was added. The cells were incubated in the dark at 4 °C for 30 min. The cells were washed twice with PBS and then resuspended, and finally, two-channel fluorescence signals were detected using a BD Accuri C5 (BD Bioscience) flow cytometer.

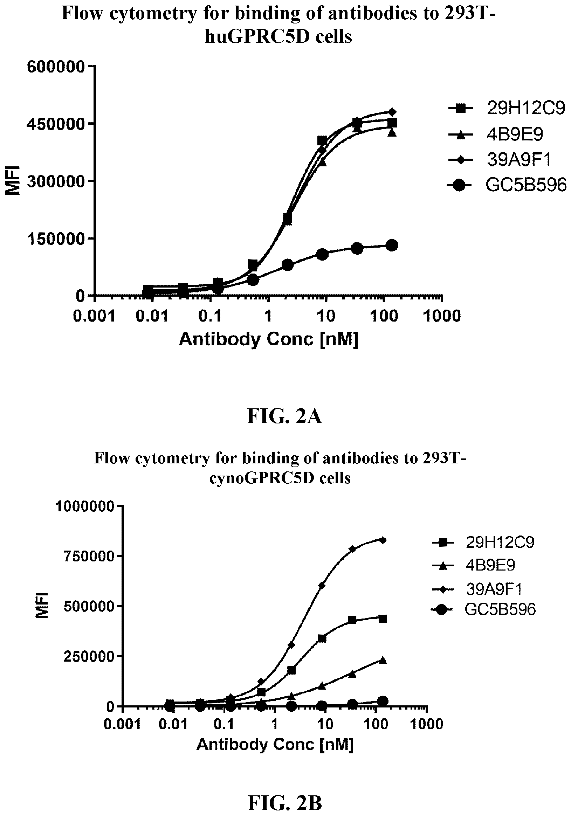

From the results shown in FIG. 2, it is shown that the murine chimeric anti-GPRC5D antibodies could bind to cells expressing the human GPRC5D protein (FIG. 2A) and cynomolgus monkey GPRC5D protein (FIG. 2B), and that the binding capacity of the chimeric antibody 29H12C9 and 39A9F1 clones to the cells was superior to that of the control antibody (GC5B596).

### Example 5: Humanization of Murine Anti-GPRC5D Antibodies and Characterization Thereof

### 5.1 Humanization of murine anti-GPRC5D antibodies

The antibody 29H12C9 was humanized by the method as described in Example 1. The sequences of 29H12C9 were searched and aligned in the IMGT database, thereby obtaining a human germline gene sequence IGHV1-46*01 with high homology to the 29H12C9 heavy chain variable region for use as a humanized framework of the heavy chain variable region, and selecting a human germline gene sequence IGKV4-1*01 with high homology to the light chain variable region for use as a humanized framework of the light chain variable region. The CDRs of the 29H12C9 heavy and light chain variable regions were grafted into the corresponding humanized frameworks to form humanized 29H12C9 antibody variable regions. In order to maintain the affinity of the antibodies for GPRC5D, the humanized antibody variable regions obtained were back-mutated.

Therefore, the sequences of the humanized heavy chain variable regions and humanized light chain variable regions were obtained. The amino acid sequences of the humanized variable regions of the murine antibody 29H12C9 are listed in Table 6.

The variable region amino acid sequences were sent to General Biology System (Anhui) Co., Ltd. for codon optimization and gene synthesis. The genes encoding the VH regions and the VL regions of the antibodies were inserted sequentially into an expression vector pcDNA3.1(+) comprising a coding gene of a human IgG1 heavy chain constant region (SEQ ID NO: 48) and a coding gene of a kappa light chain constant region (SEQ ID NO: 50) to obtain plasmids expressing the anti-GPRC5D humanized antibody full-length heavy chains and plasmids encoding the full-length light chains. Names, variable region sequences, and heavy and light chain sequences of each antibody are listed in Table 6.

**Table 6: Humanized antibody names and SEQ ID NOs of corresponding sequences**

| Antibody name | Heavy chain | Light chain | Heavy chain variable region | Light chain variable region | HCDR1 | HCDR2 | HCDR3 | LCDR1 | LCDR2 | LCDR3 |
|---|---|---|---|---|---|---|---|---|---|---|
| 29H1 | 14 | 15 | 16 | 17 | 64 | 65 | 66 | **67** | 68 | **69** |
| 29H2 | 14 | 18 | 16 | 19 | | | | | | |
| 29H3 | 14 | 20 | 16 | 21 | | | | | | |
| 29H6 | 22 | 20 | 23 | 21 | | | | | | |
| 29H10 | 24 | 15 | 25 | 17 | | | | | | |
| 29H12 | 24 | 20 | 25 | 21 | | | | | | |
| 29H20 | 26 | 18 | 27 | 19 | | | | | | |
| 29H21 | 26 | 20 | 27 | 21 | | | | | | |
| 29H24 | 28 | 20 | 29 | 21 | | | | | | |

In addition, the amino acids at positions 55 and 56 of the CDR2 of the antibody could be mutated to obtain the following antibodies:

| Antibody name | Heavy chain variable region | Light chain variable region | HCDR1 | HCDR2 | HCDR3 | LCDR1 | LCDR2 | LCDR3 |
|---|---|---|---|---|---|---|---|---|
| 29H6NS (N55S mutation) | 38 | 21 | 64 | 39 | 66 | 67 | 68 | 69 |
| 29H6GE (G56E mutation) | 40 | 21 | 64 | 41 | 66 | 67 | 68 | 69 |

### 5.2 Expression and purification of humanized anti-GPRC5D antibodies

The plasmid combinations encoding the heavy and light chains (Table 6) were co-transfected into ExpiCHO-S cells as described in Example 4.1 to express the anti-GPRC5D humanized antibodies, and then the corresponding purification was performed as described in Example 1.1. The purified antibodies were concentrated, and sterile filtration was performed. The purities of the antibodies were detected by SDS-PAGE and size exclusion chromatography (SEC).

### 5.3 Physicochemical analysis of humanized anti-GPRC5D antibodies

For the humanized anti-GPRC5D antibodies obtained, the purities of the antibodies were determined by size exclusion chromatography. Specifically, 20 µg of antibody sample was injected onto a TSK G3000SWXI, column using 100 mM sodium phosphate + 100 mM Na₂SO₄ (pH 7.0) as a running buffer for 30 min. The collected effluent was measured using Agilent 1220 HPLC and the data were analyzed using an OpenLAB software. The results indicated that the purity of the antibody was more than 90%, and the antibody could be used in the next step.

### 5.4 Binding of humanized anti-GPRC5D antibodies to engineered cells expressing human GPRC5D protein

Whether the anti-GPRC5D humanized antibodies in the present invention could bind to the human GPRC5D protein stably expressed on the cell surface was determined by cell binding experiments.

The HEK293T-HuGPRC5D cell line shown in Table 2 was subjected to enzymatic digestion to obtain a single cell suspension of the HEK293T-huGPRC5D cells. The single cell suspension was centrifuged at room temperature at 400× g, and then the medium was discarded. The cell pellet was washed once with PBS and then resuspended in the serially diluted anti-GPRC5D humanized antibodies and the murine chimeric antibody 29H12C9 (at an initial concentration of 166.67 nM, 4-fold gradient dilution, 8 concentration points in total), and the cells were incubated at 4 °C for 30 min. The cells were washed once with PBS, and then fluorescent secondary antibody R-PE-conjugated AffiniPure Goat Anti-Human IgG, Fcy Fragment Specific (Jackson ImmunoResearch, Cat# 109-116-098) diluted in a 1:200 ratio was added. The cells were incubated in the dark at 4 °C for 30 min. The cells were washed twice with PBS and then resuspended, and finally, two-channel fluorescence signals were detected using a Beckman Cytoflex flow cytometer.

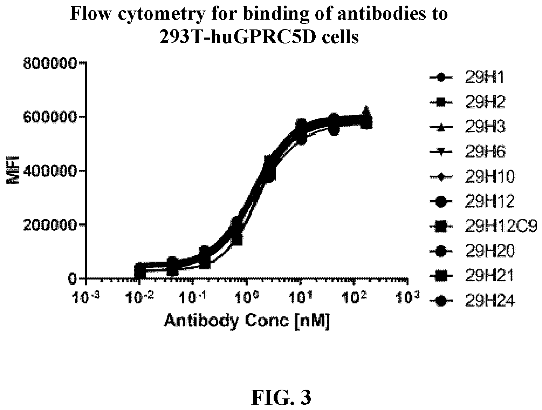

From the results shown in FIG. 3, it is shown that the anti-GPRC5D humanized antibodies could bind to cells expressing the human GPRC5D protein, and that the binding capacity of the antibodies was close to that of the murine control antibody (29H12C9).

### Example 6: Anti-CD3/GPRC5D Bispecific Antibodies

### 6.1 Construction of bispecific antibodies

In the present invention, three types of bispecific antibodies with different asymmetric structures were constructed as shown in FIG. 4, wherein in each bispecific antibody, the anti-GPRC5D antigen-binding region was derived from the humanized GPRC5D antibodies produced in Example 5 described above, the anti-CD3 antigen-binding region was derived from the humanized CD3 antibody produced in Example 1, and the constant regions of the bispecific antibodies each contained a knob-in-hole (KIH) structure (Merchant, A. M., et al. (1998). "An efficient route to human bispecific IgG." Nat Biotechnol 16(7): 677-681.), and contained mutations at sites L234A, L235A, D265A, and P329A (according to Eu numbering) to eliminate effector functions. Such bispecific antibodies are also referred to herein as "anti-CD3/GPRC5D bispecific antibodies" or "anti-CD3/anti-GPRC5D bispecific antibodies", sometimes simply referred to as "bispecific antibody molecules" or "diabodies".

The anti-GPRC5D moiety of the anti-CD3/GPRC5D bispecific antibody targeted cells expressing GPRC5D, and the anti-CD3 moiety activated T cells. The diabody simultaneously bound to GPRC5D on tumor cells and CD3 on T cells to facilitate targeted killing of the tumor cells by the activated T cells.

Three types of anti-CD3/GPRC5D bispecific antibodies with the structures shown in FIG. 4 were obtained using standard construction methods in the present invention, the amino acid sequences of which are listed in Table 7 below. The heavy chain of the bispecific antibody was named sequentially from the N-terminus to the C-terminus according to the source sequence. For example, 23L2-Rknob (also referred to as Hu34Scfv-Rknob) means that the sequence sequentially comprises, from the N-terminus to the C-terminus, an ScFv derived from 23L2 and a constant region comprising a knob mutation; 29H6-Rhole means that the sequence sequentially comprises, from the N-terminus to the C-terminus, a sequence derived from 29H6 and a constant region comprising a hole mutation. In order to eliminate post-translational modification (PTM) sites of the 29H6 heavy chain molecule, asparagine (N) at position 55 or glycine (G) at position 56 of the 29H6 heavy chain was mutated into serine (S) or glutamic acid (E), respectively. After mutation, the amino acid was simply referred to as NS or GE (Kabat) in the molecule.

The construction and related sequences of each bispecific antibody are summarized in Table 7 below:

**Table 7: Construction and sequences of bispecific antibody molecules of the present invention**

| Bispecific antibody in FIG. 4A | Bi-29H6-1 | Bi-29H6-3 | Bi-29H6-4 |
|---|---|---|---|
| Heavy chain 1: Fc-hole Hinge region-CH2-CH3hole | SEQ ID NO: 30 (Fc-Rhole) Comprising a hinge region EPKSS and CH2-CH3hole (SEQ ID NO: 42) | | |
| Heavy chain 2: anti-GPRC5D VH-CH1-anti-CD3 scFv-Fcknob | SEQ ID NO:31 (29H6-23L2 Rknob) | SEQ ID NO:34 (29H6NS-23L2 Rknob) | SEQ ID NO:35 (29H6GE-23L2 Rknob) |
| Anti-GPRC5D VH | 29H6-VH: SEQ ID NO:23 | 29H6NS VH : SEQ ID NO:38 | 29H6GE VH : SEQ ID NO:40 |
| CH1 | SEQ ID NO:44 | | |
| Anti-CD3 scFv | SEQ ID NO:11 | | |
| Fcknob | SEQ ID NO: 43 | | |
| Hinge region-CH2-CH3knob | Comprising a hinge region EPKSS and CH2-CH3 (SEQ ID NO: 89) | | |
| Light chain: anti-GPRC5D VL-CL Anti-GPRC5D VL | 29H6-LC:SEQ ID NO:20 (29HL3) | | |
| | 29H6-VL:SEQ ID NO:21 | | |

| Bispecific antibody in FIG. 4B | Bi-29H6-2 | Bi-29H6-5 | Bi-29H6-6 |
|---|---|---|---|
| Heavy chain 1: anti-GPRC5D VH-CH1-Fc-hole | SEQ ID NO:33 (29H6- Rhole) | SEQ ID NO:32 (29H6NS -Rhole) | SEQ ID NO:36 (29H6GE-Rhole) |
| Anti-GPRC5D VH | 29H6-VH: SEQ ID NO:23 | 29H6NS VH : SEQ ID NO:38 | 29H6GE VH : SEQ ID NO:40 |
| CH1 | SEQ ID NO:44 | | |
| Fc-hole | SEQ ID NO: 59 | | |
| | Comprising a hinge region EPKSC and CH2-CH3hole (SEQ ID NO: 42) | | |
| Heavy chain 2: anti-GPRC5D VH-CH1-anti-CD3 scFv-Fcknob | SEQ ID NO:31 (29H6-23L2 Rknob) | SEQ ID NO:34 (29H6NS-23L2 Rknob) | SEQ ID NO:35 (29H6GE-23L2 Rknob) |
| Anti-GPRC5D VH | 29H6-VH: SEQ ID NO:23 | 29H6NS VH : SEQ ID NO:38 | 29H6GE VH : SEQ ID NO:40 |
| CH1 | SEQ ID NO:44 | | |
| Anti-CD3 scFv | SEQ ID NO:11 | | |
| Fcknob | SEQ ID NO: 43 | | |
| Hinge region-CH2-CH3knob | Comprising a hinge region EPKSS and CH2-CH3 (SEQ ID NO: 89) | | |
| Light chain: anti-GPRC5D VL-CL | 29H6-LC:SEQ ID NO:20 (29HL3) | | |

| Anti-GPRC5D VL | 29H6-VL:SEQ ID NO:21 | | |
|---|---|---|---|
| Antibody in FIG. 4C | Bi-29H6/23L2 | Bi-29H6GE/23L2 | |
| Heavy chain 1: anti-GPRC5D VH-CH1-Fc-hole | SEQ ID NO:33 (29H6-Rhole) | SEQ ID NO:36 (29H6GE-Rhole) | |
| Anti-GPRC5D VH | 29H6-VH: SEQ ID NO:23 | 29H6GE VH : SEQ ID NO:40 | |
| CH1 | SEQ ID NO:44 | | |
| Fc-hole | SEQ ID NO: 59 | | |
| | Comprising a hinge region EPKSC and CH2-CH3hole (SEQ ID NO: 42) | | |
| Heavy chain 2: anti-CD3 scFv-Fcknob | SEQ ID NO:37 (23L2Scfv-Rknob) | | |
| Anti-CD3 scFv | SEQ ID NO:11 | | |
| Fcknob | SEQ ID NO: 43 | | |
| Hinge region-CH2-CH3knob | Comprising a hinge region EPKSS and CH2-CH3 (SEQ ID NO: 89) | | |
| Light chain: anti-GPRC5D VL-CL | 29H6-LC:SEQ ID NO:20 (29HL3) | | |
| Anti-GPRC5D VL | 29H6-VL:SEQ ID NO:21 | | |

Each chain in each bispecific antibody molecule was constructed as follows:
23L2-Rknob (also known as Hu34Scfv-Rknob): the nucleotide sequence encoding the anti-CD3 single-chain antibody 23L2 was cloned into a pcDNA3.1(+) vector with Fc (Knob) for expression to obtain the 23L2-Knob molecule.

29H6-23L2-Rknob: the coding sequence of the humanized anti-GPRC5D antibody heavy chain was used as a template to amplify a nucleotide sequence encoding the heavy chain variable region and the heavy chain constant region CH1 segment, and the sequence was directly spliced with the nucleotide sequence encoding the complete 23L2-Rknob (there was no linker peptide between the two sequences) using an overlapping PCR technique; the spliced nucleotide sequence was cloned into an expression vector pcDNA3.1(+) vector for expression to obtain the 29H6-23L2-Rknob molecule.

29H6NS-23L2-Rknob: the constructed 29H6-23L2-Knob was used as a template, in which asparagine (N) at position 55 was mutated into serine (S) using the overlapping PCR technique, and the sequence was cloned into an expression vector pcDNA3.1(+) vector for expression to obtain the 29H6NS-23L2-Rknob molecule.

29H6GE-23L2-Rknob: the constructed 29H6-23L2-Rknob was used as a template, in which glycine (G) at position 56 was mutated into glutamic acid (E) using the overlapping PCR technique, and the sequence was cloned into an expression vector pcDNA3.1(+) vector for expression to obtain the 29H6GE-23L2-Rknob molecule.

29H6-Rhole: the nucleotide sequence encoding the heavy chain variable region and the heavy chain constant region CH1 segment of the humanized anti-GPRC5D antibody was cloned into a pcDNA3.1(+) vector with Fc (hole) for expression to obtain the 29H6-Rhole molecule.

29H6NS-Rhole: the constructed 29H6-Rhole was used as a template, in which asparagine (N) at position 55 was mutated into serine (S) using the overlapping PCR technique, and the sequence was cloned into an expression vector pcDNA3.1(+) vector for expression to obtain the 29H6NS-Rhole molecule.

29H6GE-Rhole: the constructed 29H6-Hole was used as a template, in which glycine (G) at position 56 was mutated into glutamic acid (E) using the overlapping PCR technique, and the sequence was cloned into an expression vector pcDNA3.1(+) vector for expression to obtain the 29H6GE-Rhole molecule.

29HL3: the nucleotide sequence encoding the light chain of the humanized anti-29H6 antibody was cloned into a pcDNA3.1(+) vector for expression to obtain the 29HL3 molecule.

The bispecific antibodies in FIG. 4 were obtained by accordingly combining the expression vectors described above according to the specific composition shown in Table 7 and expressing them under appropriate conditions.

The construction, expression, purification, and preliminary analysis steps of the bispecific antibodies were the same as those in Example 1.1 or 4.1.

### 6.2 Binding experiments of bispecific antibodies to cells expressing human CD3 protein, human GPRC5D protein, and cynomolgus monkey GPRC5D

(1) Whether the anti-CD3/anti-GPRC5D bispecific antibodies in the present invention could bind to the CD3 protein endogenously expressed on Jurkat cells (Cell Bank of Type Culture Collection Committee of the Chinese Academy of Sciences, Cat# SCSP-513) was determined by cell binding experiments.

The experiments were performed using flow cytometry. Jurkat cells were collected by centrifugation, resuspended in PBS, and seeded into a 96-well plate (Corning, Cat# 3799) at 1 × 10⁵ cells/well. The 96-well plate was centrifuged and then the supernatant was discarded. 100 µL of the serially diluted test bispecific antibody (at a maximum concentration of 166.67 nM, 4-fold gradient dilution, 8 concentration points in total) was added to the Jurkat cells, and the plate was incubated at 4 °C for 30 min. The plate was centrifuged at room temperature at 400× g, and then the supernatant was discarded. The cells were washed twice with PBS and centrifuged. The supernatant was discarded. The cell pellet was resuspended in fluorescent secondary antibody R-PE-conjugated AffiniPure Goat Anti-Human IgG, Fcy Fragment Specific (Jackson ImmunoResearch, Cat# 109-116-098) diluted in a 1:200 ratio. The cells were incubated in the dark at 4 °C for 30 min. The cells were washed twice with PBS, and 100 µL of PBS was added to each well to resuspend the cells. Fluorescence signals were detected using a Beckman Cytoflex flow cytometer.

From the results shown in FIG. 5, it can be seen that the tested bispecific antibodies could bind to the CD3 protein expressed on the Jurkat cells at high concentrations.

(2) Whether the anti-CD3/anti-GPRC5D bispecific antibodies in the present invention could bind to HEK293T cells stably expressing the human GPRC5D protein was determined by cell binding experiments. Referring to the flow cytometry described above, 100 µL of the serially diluted test bispecific antibody (at a maximum concentration of 166.67 nM, 4-fold gradient dilution, 8 concentration points in total) was added to the 293T-huGPRC5D cells described in Table 2, and the plate was incubated at 4 °C for 30 min. The plate was centrifuged at room temperature at 400× g, and then the supernatant was discarded. The cells were washed twice with PBS and centrifuged. The supernatant was discarded. The cell pellet was resuspended in fluorescent secondary antibody R-PE-conjugated AffiniPure Goat Anti-Human IgG, Fcy Fragment Specific (Jackson ImmunoResearch, Cat# 109-116-098) diluted in a 1:200 ratio. The cells were incubated in the dark at 4 °C for 30 min. The cells were washed twice with PBS, and 100 µL of PBS was added to each well to resuspend the cells. Fluorescence signals were detected using a Beckman Cytoflex flow cytometer.

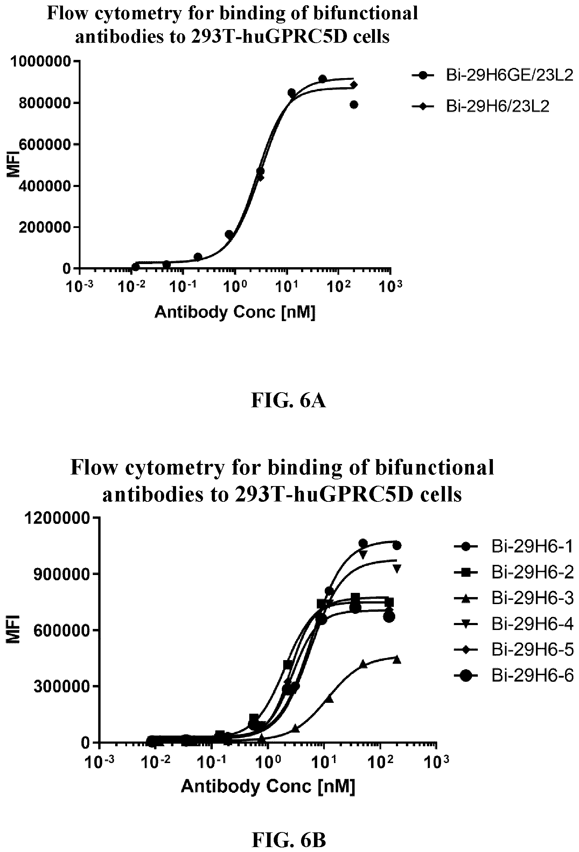

From the results shown in FIGs. 6A and 6B, it can be seen that the tested bispecific antibodies could bind to the HEK293T cells expressing the human GPRC5D protein.

(3) Whether the anti-GPRC5D/CD3 bifunctional antibodies in the present invention could bind to the cynomolgus monkey GPRC5D protein stably expressed on the cell surface was determined by cell binding experiments.

The HEK293T-CynoGPRC5D cell line shown in Table 2 was subjected to enzymatic digestion to obtain a single cell suspension. The single cell suspension was centrifuged at room temperature at 400× g, and then the medium was discarded. The cell pellet was washed once with PBS and then resuspended in the serially diluted bifunctional antibodies shown in Table 5 (at an initial concentration of 115.6 nM, 4-fold gradient dilution, 8 concentration points in total), and the cells were incubated at 4 °C for 30 min. The cells were washed once with PBS, and then fluorescent secondary antibody R-PE-conjugated AffiniPure Goat Anti-Human IgG, Fcy Fragment Specific (Jackson ImmunoResearch, Cat# 109-116-098) diluted in a 1:200 ratio was added. The cells were incubated in the dark at 4 °C for 30 min. The cells were washed twice with PBS and then resuspended, and finally, two-channel fluorescence signals were detected using a Beckman Cytoflex flow cytometer.

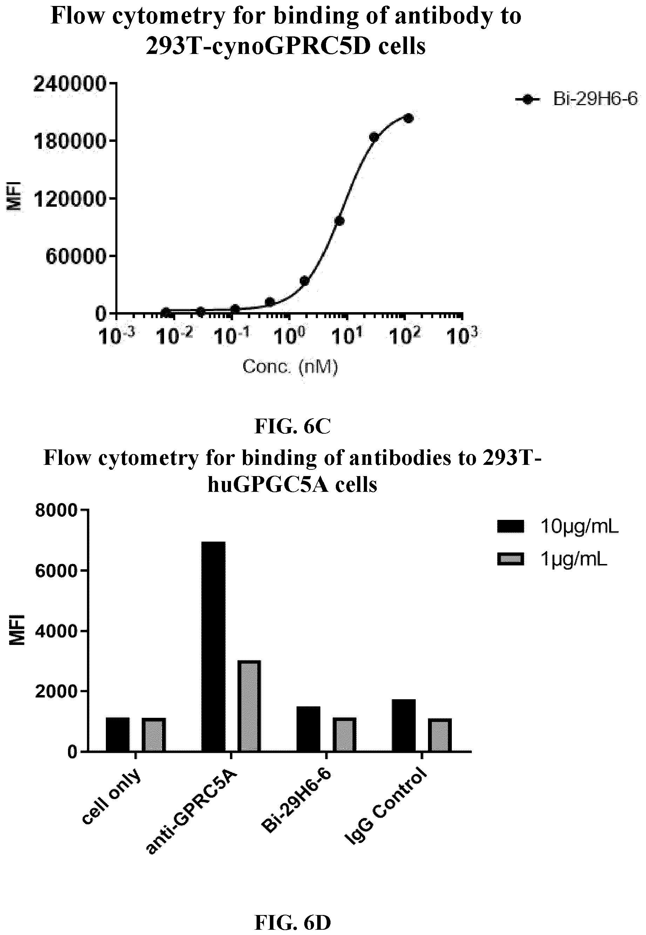

The results are shown in FIG. 6C, indicating that Bi-29H6-6 is capable of specifically binding to the cells expressing cynomolgus monkey GPRC5D.

(4) Binding experiment of anti-CD3/anti-GPRC5D bispecific antibody to cells expressing the same family protein of human GPRC5

GPRC5D is a member of the GPRC5 family of the G protein-coupled receptor, as well as GPRC5A, GPRC5B, and GPRC5C. In this example, the binding of the anti-GPRC5D/CD3 bifunctional antibody to 293T cells overexpressing human GPRC5A, GPRC5B, and GPRC5C was separately tested to determine the binding specificity of the anti-GPRC5D/CD3 bifunctional antibody to GPRC5D.

293T-huGPRC5A, 293T-huGPRC5B, and 293T-huGPRC5C cell lines (obtained by separately transfecting human GPRC5A (UniProtKB/Swiss-Prot: Q8NFJ5), human GPRC5B (UniProtKB/Swiss-Prot: Q9NZH0), and human GPRC5C (UniProtKB/Swiss-Prot: Q9NQ84) into 293T cells, and performing pressurized screening on the cells with 0.3 µg/mL of puromycin (Gibco, Cat# A1113802)) to obtain single cell suspensions. The single cell suspensions were centrifuged at room temperature at 400× g, and then the media were discarded. The cell pellet was washed once with PBS and then resuspended in the serially diluted bifunctional antibody (at an initial concentration of 10 µg/mL, 10-fold gradient dilution, 2 concentration points in total), and the cells were incubated at 4 °C for 30 min. The cells were washed once with PBS, and then fluorescent secondary antibody R-PE-conjugated AffiniPure Goat Anti-Human IgG, Fcy Fragment Specific (Jackson ImmunoResearch, Cat# 109-116-098) diluted in a 1:200 ratio was added. The cells were incubated in the dark at 4 °C for 30 min. The cells were washed twice with PBS and then resuspended, and finally, two-channel fluorescence signals were detected using a Beckman Cytoflex flow cytometer.

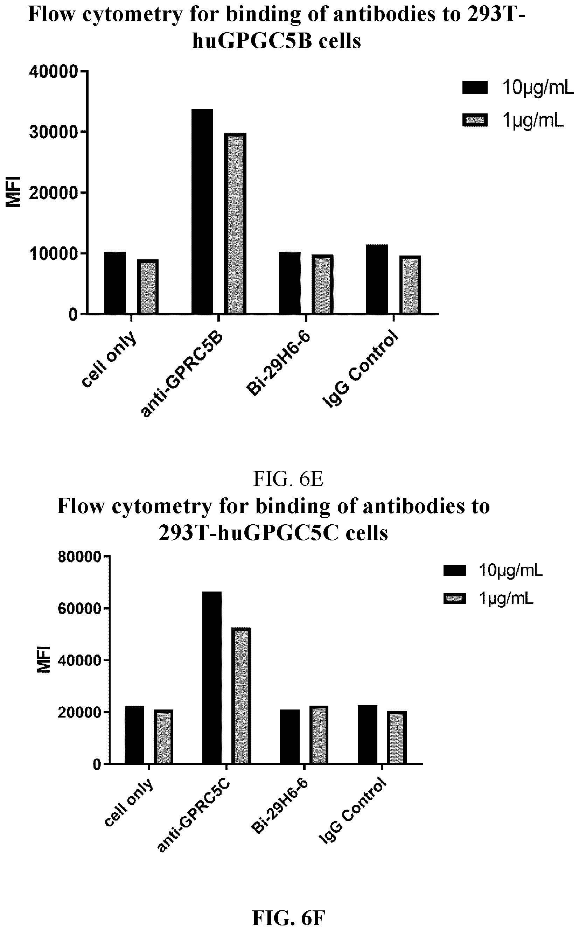

The results are shown in FIGs. 6D, 6E, and 6F and show that anti-GPRC5A(R&D, MAB5239), anti-GPRC5B (R&D, MAB10253), and anti-GRPC5C (R&D, MAB6594) were used as positive controls (R-Phycoerythrin AffiniPure Goat Anti-Mouse IgG (subclasses 1+2a+2b+3) and Fcy Fragment Specific (min X Hu, Bov, Rb Sr Prot) (Jackson ImmunoResearch, 115-115-164) were used as secondary antibodies), and the negative control IgG control (Sino, MA16SE3081) was used as a reference; the anti-GPRC5D/CD3 bifunctional antibody Bi-29H6-6 had no binding to the cells expressing GPRC5A, GPRC5B, and GPRC5C, indicating that the bifunctional antibody has binding specificity to GPRC5D.

### 6.3 Activation of anti-CD3/GPRC5D bispecific antibody on T cells and killing activity thereof on tumor cells

In this example, the activating effect of the anti-CD3/GPRC5D bispecific antibody dependent on the binding to the GPRC5D antigen in activating a Jurkat/NFAT-luc reporter gene system (luciferase, Promega, Cat# E8481) was detected. The target cell in the detection system was NCI-H929 (Nanjing Cobioer Biosciences Co., Ltd., CBP60243) tumor cell strain endogenously expressing human GPRC5D or HEK293T negative cell strain.

The anti-CD3/anti-GPRC5D bispecific antibody bound to both GPRC5D on the surface of NCI-H929 cells and CD3 on the surface of Jurkat cells, and then activated CD3 downstream signals on the Jurkat cells by cross-linking with the antibody dependent on the binding to the GPRC5D antigen. The expression level of luciferase directly reflected the activating effect of the anti-CD3/anti-GPRC5D bispecific antibody on Jurkat/NFAT-luc cells.

The Jurkat/NFAT-luc cells (effector cells, prepared as described in Example 1.2) were collected and centrifuged, and the supernatant was removed. The cells were resuspended in an assay buffer (99% 1640 medium + 1% FBS) with an effector cell density of 1E6 cells/mL. The NCI-H929 tumor cell strain and 293T negative cell strain (target cells) were digested, collected, and centrifuged, and the supernatant was removed. The cells were resuspended in the assay buffer with a target cell density of 5E5 cells/mL. The effector cell and target cell suspensions were then seeded into a white 384-well assay plate (Corning, Cat# 3570) with 20 µL of effector cells and 20 µL of target cells per well. 20 µL of the serially diluted test bispecific antibody (at a maximum assay concentration of 116 nM, 4-fold gradient dilution, 9 concentration points in total) was added into each well. The 384-well plate was placed in an incubator at 37 °C. After 6 h, 25 µL of One-Glo (Promega, Cat# E6130) reagent was added into each well, and finally, self-luminescence signals were detected using a multifunctional microplate reader (Tecan, Cat# F200).

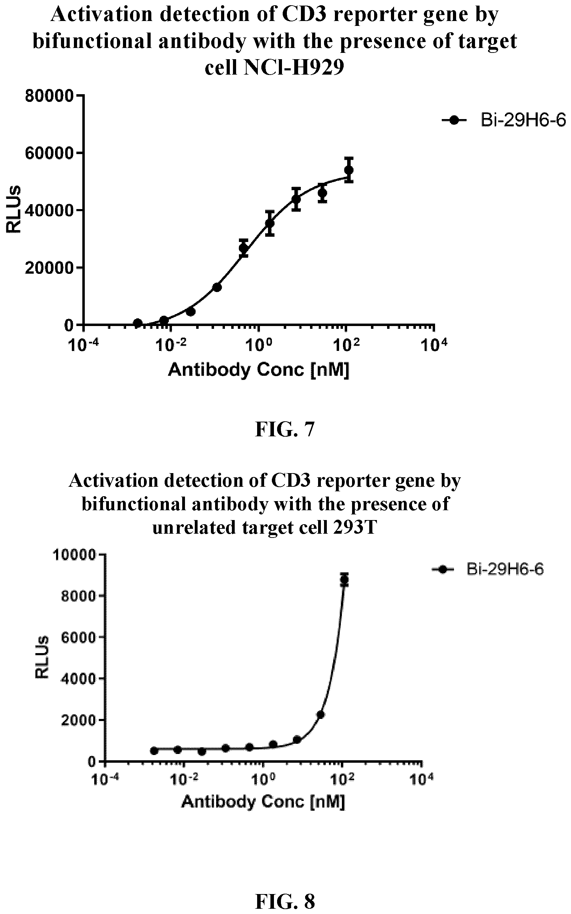

The results are shown in FIG. 7 and show that the anti-CD3/GPRC5D bispecific antibody was capable of activating the CD3 signaling pathway in the presence of the cells expressing human GPRC5D (NCI-H929 cells) and was antibody concentration-dependent.

FIG. 8 shows that the anti-CD3/GPRC5D bispecific antibody was capable of activating the CD3 signaling pathway only at high concentrations when there was no GPRC5D present in the assay system (HEK293T negative cell strain). Therefore, the bispecific antibody of the present invention was concentration dependent for the activation of the CD3 signaling pathway with little non-specific activation, indicating that the bispecific antibody of the present invention is capable of specifically activating the CD3 signaling pathway.

In order to detect the activity of the anti-CD3/anti-GPRC5D bispecific antibody in mediating T cells to kill tumor cells, we established a primary T cell-dependent cytotoxicity assay system with NCI-H929 endogenously expressing human GPRC5D as the target cells and human peripheral blood mononuclear cells (PBMCs, obtained from healthy human blood) as the effector cells.

The PBMCs were thawed, and the cell density was adjusted to 1-2 × 10⁶ cells/mL using a 1640 complete medium (adding 10% FBS). The cells were activated overnight with IL2 (Jiangsu Kingsley Pharmaceutical Co., Ltd.) at a final concentration of 100 IU/mL. The target cells NCI-H929 and GPRC5D negative cells Raji (Cell Bank of Type Culture Collection Committee of the Chinese Academy of Sciences, Cat# TCHu 44) were centrifuged at 400× g for 5 min. The cells were collected, and the supernatant was removed. The target cell density was adjusted to 2 × 10⁵ cells/mL with an MEM-α (Gibco, Cat# 41061-029) assay buffer containing 1% FBS (Gibco, Cat# 10099-141) and 100 IU/mL of IL2. The cells were seeded into a 96-well plate (Corning, Cat# 3599) at 50 µL/well (i.e., 10,000 cells/well).

A dilution of the serially diluted test antibody (at a maximum assay concentration of 30 nM, 5-fold gradient dilution, 8 concentration points in total) was prepared, and 50 µL of the dilution was added into each well. In the experiment, the maximal killing of target cells (2% Triton 100 lysis solution was added to the target cells), minimal killing (the assay buffer was added to the target cells), and natural killing (the effector cells was added to the target cells) controls were set at 50 µL/well. The ratio of effector cells E to target cells T was finally 10:1, and then the plate was placed in an incubator for another 24 h. After the incubation was completed, the experimental plate was taken out and centrifuged at 400× g for 3 min to enable all cells to settle to the bottom of the plate. 50 µL of supernatant was carefully pipetted into a new 96-well plate, 50 µL of LDH detection solution (Roche, Cat# 11644793001) was added, and the plate was incubated at room temperature. When the color changed, the plate was read on an F50 microplate reader. The detection wavelength was 492 nm, the reference wavelength was 650 nm, and the detection analysis was performed when the OD492 value of the maximum killing well was between 0.4 and 1.0.

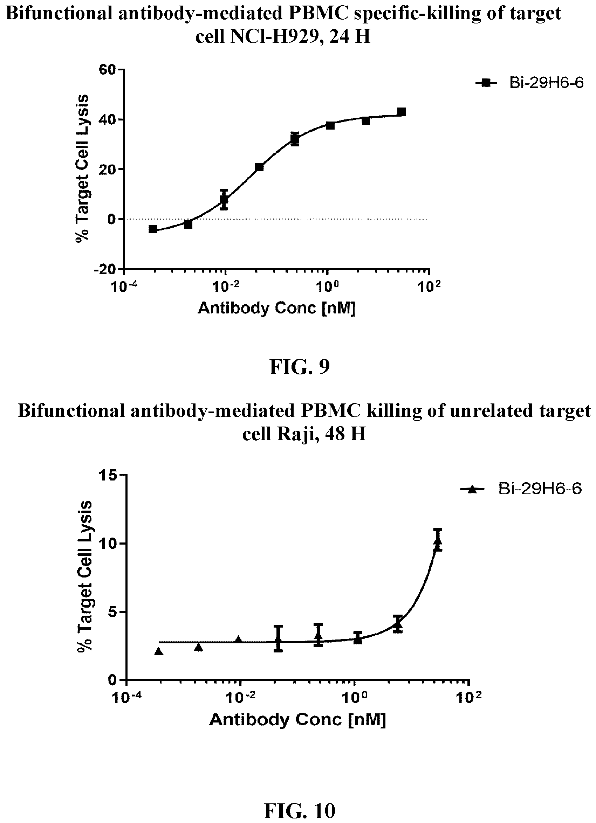

The results are shown in FIGs. 9 and 10 and show that the anti-CD3/anti-GPRC5D bispecific antibody was capable of specifically inducing T cells to kill the NCI-H929 tumor cells; however, there was no killing effect at all on the GPRC5D negative cells Raji at low concentrations, and only a slight killing effect at high concentrations of the antibody. Therefore, the bispecific antibody of the present invention is capable of specifically inducing the killing by the T cells expressing GPRC5D without visible non-specific activation.

### 6.4 Induction of cytokine release after activation of T cells by anti-CD3/anti-GPRC5D bispecific antibody

In order to detect the killing of tumor cells by T cells mediated by the anti-CD3/anti-GPRC5D bispecific antibody and the induction of factor secretion at the same time, we established a primary T cell-dependent cytotoxicity assay system with NCI-H929 endogenously expressing human GPRC5D as the target cells and human peripheral blood mononuclear cells (PBMCs) as the effector cells. The killing and incubation were performed as shown in 6.3. At the endpoint of the killing, the supernatant was collected for the detection of IL-6, TNF-α and IFN-γ in the system.

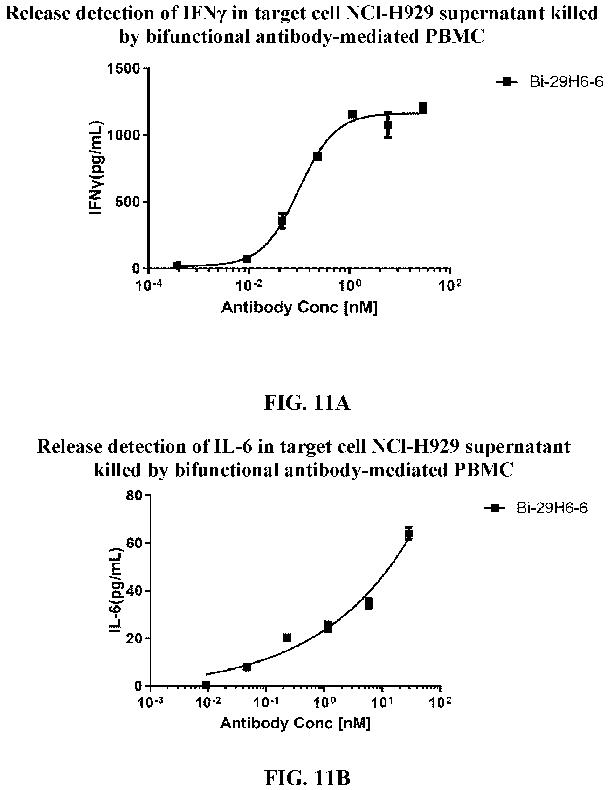

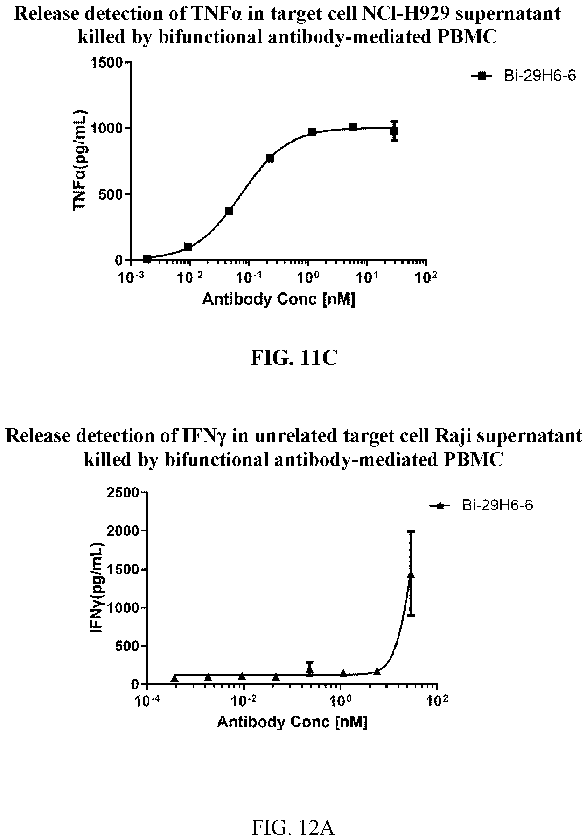

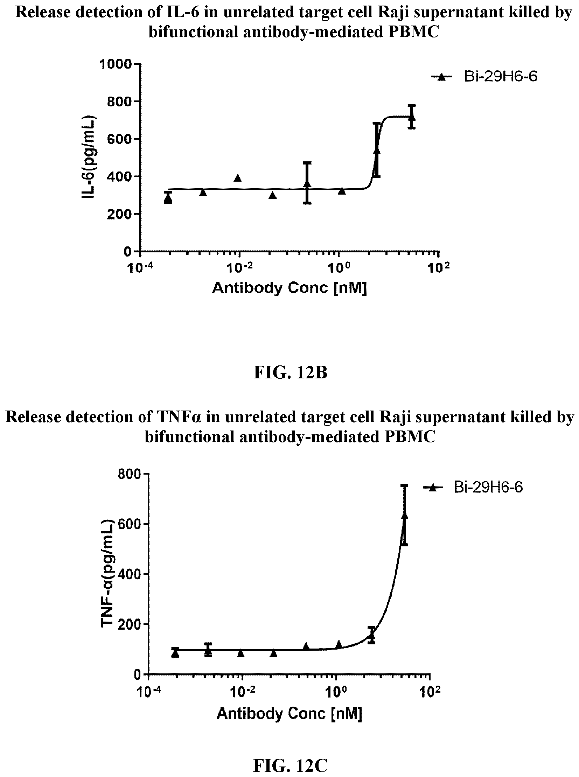

After the killing and incubation were completed, the experimental plate was taken out and centrifuged at 400× g for 3 min to enable all cells to settle to the bottom of the plate. 100 µL of supernatant was carefully pipetted into a new 96-well plate, and the human IFNγ (detection kit: Cisbio, Cat# 62HIFNGPEH), human TNFα (detection kit: R&D, Cat# DY210), and human IL-6 (detection kit: R&D, Cat# DY206) factor levels in the supernatant were detected, respectively. The results are shown in FIG. 11 and show that the anti-CD3/anti-GPRC5D bispecific antibody was capable of inducing the T cells to release the human IFNγ (FIG. 11A), human TNFα (FIG. 11B), and human IL-6 (FIG. 11C) factors in the NCI-H929 system. FIG. 12 shows that the anti-CD3/anti-GPRC5D bispecific antibody in the Raji system not expressing GPRC5D could not induce the release of IL-6 at low concentrations, and could induce the release of IFNγ, TNFα, and IL-6 in a small amount only at high concentrations of the antibody (FIGs. 12A-12C), thereby indicating that the anti-CD3/anti-GPRC5D bispecific antibody has better induction specificity.

In conclusion, in the T cell-mediated negative cell killing and factor secretion experiments, Bi-29H6-6 induces PBMCs to have weaker killing activity on unrelated target cells Raji, induces immune cells to generate lower levels of cytokines, thereby suggesting that the anti-CD3/anti-GPRC5D bifunctional antibody has lower risk of causing cytokine storm and better safety.

### 6.5 Affinity Assay of bispecific antibody for human/monkey CD3 protein

In this experiment, the binding affinity of the bispecific antibody
for human CD3D & CD3E heterodimer protein (Sino Biological, Cat# CT038-H2508H) and monkey CD3D & CD3E heterodimer protein (Acro biosystems, Cat# CDD-C52W4) was detected using ForteBio Octet RED96 according to the manufacturer's instructions. Briefly, an AHC sensor (ForteBio, Cat# 18-5060) was placed in a running buffer (1× PBS, Hyclone, Cat# SH30256.01, containing 0.02% Tween 20, pH 7.0) and pre-equilibrated at room temperature for 10 min. In a 96-well plate, the kinetic assay was performed according to the following steps:
a) equilibrating the baseline for 100 s with the running buffer;
b) adding the bispecific antibody diluted with the running buffer at a final concentration of 5 µg/mL and immobilizing for 200 s;
c) equilibrating the baseline for 300 s with the running buffer; and
d) adding 100 nM of human/monkey CD3 protein diluted with the running buffer into each well, binding for 200 s, and dissociating for 600 s.

The experimental data were fitted and calculated using a Fortebio Data Analysis software 1:1 binding model. The binding affinity of the bispecific antibody Bi-29H6-6 for the human/monkey CD3 protein is summarized in Table 8.

**Table 8: Binding affinity of Bi-29H6-6 for human/monkey CD3 protein**

| Antibody | Analyte | KD (M) | Kon (M-1S-1) | Koff (S-1) | Full R^2 |
|---|---|---|---|---|---|
| Bi-29H6-6 | Human CD3D & CD3E heterodimer protein | 7.27E-07 | 2.17E+04 | 1.58E-02 | 0.9906 |
| | Monkey CD3D & CD3E heterodimer protein | 7.59E-08 | 8.32E+04 | 6.31E-03 | 0.99 |

Table 8 shows that the bispecific antibody constructed in the present application could specifically bind to the CD3 protein from the human/monkey, and had species cross-activity. Generally, the affinity for the CD3 protein was preferably lower than those for other targets, which facilitates the formation of clustered T cells by the production of a cascade effect, thereby exponentially inducing changes in downstream signaling pathways. It can be seen that the anti-CD3/anti-GPRC5D bispecific antibody obtained in the present application retained the appropriate affinity for CD3.

### Example 7. Anti-CD3/Anti-GPRC5D Bispecific Antibody for In Vivo Tumor Inhibition

Example 7.1 In this example, the inhibitory effect of the anti-CD3/anti-GPRC5D bispecific antibody on a mouse subcutaneous xenograft tumor was evaluated in C57BL/6-hCD3e transgenic mice carrying a mouse colon cancer cell line MC38.

First, a mouse colon cancer cell line MC38 highly expressing human GPRC5D was constructed. MC38 cells were infected with the nucleotide encoding the human GPRC5D protein (NP_061124.1 (NCBI sequence number); sp|Q9NZD1 (Uniprot sequence number), SEQ ID NO: 57) (synthesized by General Biology System (Anhui) Co., Ltd.) using a lentivirus infection method. Then, 4 µg/mL of puromycin (Gibco, Cat# A1113802) was added to the medium for screening, and monoclonal seeding was performed, thereby obtaining polyclonal cells highly expressing human GPRC5D. Moreover, a monoclonal MC38 cell strain highly expressing human GPRC5D was obtained by limiting dilution and named MC38-huGPRC5D.

The MC38-huGPRC5D cells were collected and counted, and matrigel was added according to a volume ratio of 1:1 and mixed uniformly. The mixture was placed in a wet ice box and stored at a low temperature for use. The experimental animals were 6- to 8-week-old C57BL/6-hCD3e mice (Biocytogen). 100 µL of PBS suspension containing 2 × 10⁶ cells and an equal volume of matrigel were mixed uniformly, and then the mixture was inoculated subcutaneously at the back of 35 mice with an inoculation volume of 200 µL. The mice were anesthetized with 3-4% isoflurane before inoculation. Among them, 5 mice were administrated on the day of tumor cell inoculation (G5). When tumors grew to an average of about 60-80 mm³, 25 mice were randomly divided into 3 groups (G1, G3, and G4: 5 mice per group) according to the tumor size and body weight, and the day of administration for each group was defined as day 0. The grouping and administration regimens are shown in Table 9:

**Table 9: Grouping and administration regimens**

| Group | Test drug | Number | Dose | Route | Frequency of administration |
|---|---|---|---|---|---|
| G1 | PBS | 5 | na | i.v. | BIW * 6 times |
| G3 | Bi-29H6-6 | 5 | 5 mg/kg | i.v. | BIW * 6 times |
| G4 | Bi-29H6-6 | 5 | 15 mg/kg | i.v. | BIW * 6 times |
| G5 | Bi-29H6-6 | 5 | 5 mg/kg | i.v | BIW * 6 times |

The appearance and behavior of the mice were observed and recorded daily, with up to 4 weeks of continuous observation starting from the grouping. Tumor volumes were measured twice a week after grouping.

The tumor volume (V) was calculated as follows: Tumor volume (mm3) = 1/2 × (a × b2) (where a represents long diameter and b represents short diameter)

The relative tumor volume (RTV) per mouse was calculated as: RTV = Vt/V₀, where Vt is the measured volume for each time and V0 is the volume at the start of the treatment. Mouse body weights were measured and recorded twice a week after grouping. The results are presented as mean ± S.E.M. Two groups were compared using Dunnett's multi-comparison test, and a statistically significant difference was considered if p < 0.05.

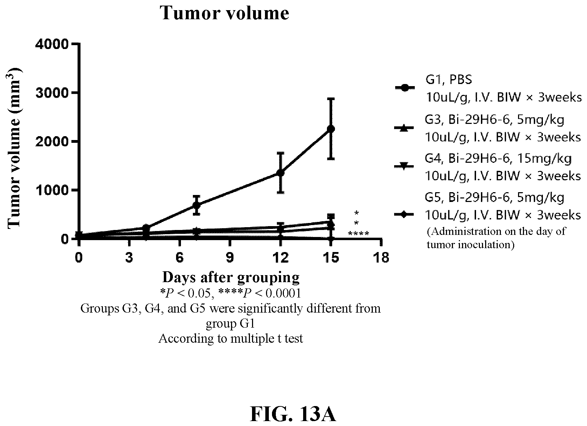

The results are shown in FIG. 13A and show that compared to the negative control (group G1), the groups G3, G4, and G5 all had good tumor inhibitory effects, and the differences were extremely significant. There were individuals with complete regression of tumors in the mice in groups G4 and G5.

**Table 25:**

| Group | Dose | Number | Tumor volume (mm³)^{a} (day 15) | Tumor growth inhibition (%)^{b} | Significance^{c} |
|---|---|---|---|---|---|
| G1, PBS | / | 5 | 2260 ± 619.32 | / | / |
| G3, Bi-29H6-6 | 5mpk | 5 | 350 ± 146.25 | 84.94% | <0.05 |
| G4, Bi-29H6-6 | 15mpk | 5 | 227 ± 214.25 | 89.15% | <0.05 |
| G5, Bi-29H6-6 | 5mpk | 5 | 0 | 100.00% | <0.0001 |
| Administration on the day of tumor inoculation | | | | | |

| | | | | | |
|---|---|---|---|---|---|
| a: presentation of tumor data as mean ± standard deviation b: tumor growth inhibition = (1 - relative volume of administration group/relative volume of PBS group) × 100% c: comparison with group G1 | | | | | |

### Example 7.2 Anti-Tumor Effect of Anti-CD3/Anti-GPRC5D Bispecific Antibody in Human Myeloma NCI-H929 Cell Strain NCG Mouse Mixeno Model

In this example, the inhibitory effect of the anti-CD3/anti-GPRC5D bispecific antibody on the mouse subcutaneous xenograft tumor was evaluated in NCG mice transplanted with PBMCs and inoculated with a human myeloma NCI-H929 tumor model.

Firstly, the human myeloma NCI-H929 cell strain was inoculated subcutaneously to the right side of the back of NCG mice (Jiangsu GemPharmatech Co., Ltd.) of an appropriate age (7-9 weeks) near the axilla, and the cells were inoculated after being resuspended in PBS (5 × 10⁶ cells/mice/100 µL) and mixed with 100 µL of matrigel in a ratio, by volume, of 1:1. On the day of tumor inoculation, PBMCs (donor Z0080) were intraperitoneally transplanted at 1 × 10⁷ cells/mouse/100 µL. When tumors grew to an average of about 70-90 mm³ (for about 7 days), 18 mice were randomly divided into 3 groups (G1-G3: 6 mice per group) according to the tumor size and body weight, and the day of administration for each group was defined as day 0. The grouping and administration regimens are shown in Table 22:

**Table 22: Grouping and administration regimens**

| Group | Test drug | Number | Dose | Route | Frequency of administration |
|---|---|---|---|---|---|
| G1 | PBS | 6 | na | i.v. | BIW * 6 times |
| G2 | Talquetamab | 6 | 3 mg/kg | i.v. | BIW * 6 times |
| G3 | Bi-29H6-6 | 6 | 3 mg/kg | i.v. | BIW * 6 times |

After inoculation with tumor cells, routine monitoring included the effect of tumor growth and treatment on the normal behavior of the animals, specifically the activity, food and water intake, weight gain or loss, the eyes, the hair and other abnormal conditions. Clinical symptoms observed during the experiment were all recorded in the raw data.

After the administration was started, the body weight and tumor size of the mice were measured twice a week. Calculation formula for tumor volume: Tumor volume (mm3) = 1/2 × (a × b2) (where a represents long diameter and b represents short diameter).

In the experiment, data collection, including measurement of tumors' long and short diameters and animals' body weight, was performed using StudyDirectorTM (version: 3.1.399.19; supplier: Studylog System, Inc.) software. Raw data measured by scales and vernier calipers were directly imported into the software. Any changes in the data were recorded in the software. All the processes of administration, tumor measurement, body weight weighing, and the like were performed in a biosafety cabinet or a superclean bench.

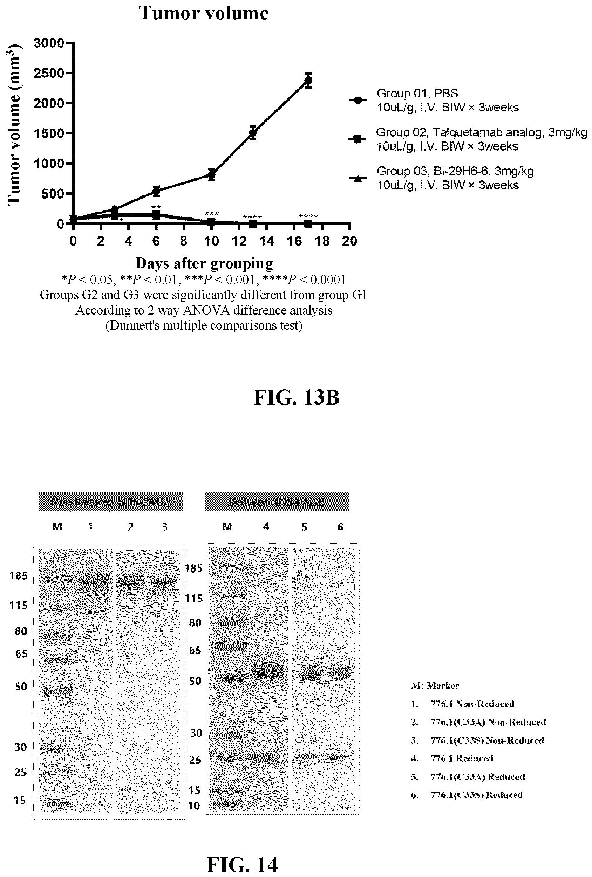

FIG. 13B shows that before the 5^{th} administration in the experiment, the positive control antibody Talquetamab (GC5B596 + CD3B219) (WO2018017786A2) in group G2 and the test antibody Bi-29H6-6 in group G3 were both capable of completely regressing the tumors, and no increase in the tumors was observed even after one week. Compared with the PBS in group G1, the antibodies in groups G2 and G3 had extremely significant anti-tumor effects. There was no significant difference in the anti-tumor effects between the antibodies in group G2 and group G3.

**Table 23:**

| Group | Dose | Number | Tumor volume (mm³) a (day 17) | Tumor growth inhibition (%) b | Significance c |
|---|---|---|---|---|---|
| G1, PBS | / | 6 | 2378±117.99 | / | / |
| G2, Talquetamab | 3mpk | 6 | 0 | 100% | <0.0001 |
| G3, Bi-29H6-6 | 3mpk | 6 | 0 | 100% | <0.0001 |

| | | | | | |
|---|---|---|---|---|---|
| a: presentation of tumor data as mean ± standard deviation b: tumor growth inhibition = (1 - relative volume of administration group/relative volume of PBS group) × 100% c: comparison with group G1 | | | | | |

### Example 8. Preparation and Characterization of Humanized MUC16 Antibodies

### 8.1 Preparation of murine anti-MUC16 antibodies and sequence optimization

The sequence of the murine MUC16 antibody 776.1 was disclosed in the patent US 7429382 B2. 776.1 could bind to the C-terminus of the extracellular domain of the human MUC16 protein and had human-monkey cross-reactivity. By analyzing the sequences of FIG. 8C and FIG. 8D in the patent US 7429382 B2, it was found that there was an unpaired cysteine (C) in the light chain of 776.1, and the third framework region of the heavy chain contained an N-glycosylation site. During the sequence optimization stage, cysteine (C) at position 33 (according to kabat numbering) of the light chain was selectively mutated into serine (S) or alanine (A), thereby obtaining the corresponding light chains 776.1LC (C33S) and 776.1LC (C33A). Their DNA sequences were subjected to codon optimization and gene synthesis by General Biology System (Anhui) Co., Ltd. The variable region sequences of each murine anti-MUC16 antibody obtained as described above are given in Table 24.

**Table 24: Amino acid sequences of heavy chain variable regions and light chain variable regions of MUC16 antibodies**

| Name | Amino acid sequence |
|---|---|
| 776.1 VH | EVQLQQSGPELVKPGASVKISCKASGYTFTDYNIHWVKQSHGKILEWIGY IYPYNGVSDYNQNFKSKATLIVDNSSNTAYMELRSLTSEDSAVYYCARWD FGSGYYFDYWGQGTTLTVSS (SEQ ID NO: 92) Comprising the following HCDRs |
| | HCDR1: DYNIH (SEQ ID NO: 93) |
| | HCDR2: YIYPYNGVSDYNQNFKS (SEQ ID NO: 94) |
| | HCDR3: WDFGSGYYFDY (SEQ ID NO: 95) |
| 776.1 VL | QIVLSQSPAILFASPGETVTMTCRASSSVIYMCWNQQKPGSSPKPWIYGTS TLASGVPTRFSGSGSGTSYSLTISRVEAEDAATYYCQQWSSNPFTFGSGTK LEIK (SEQ ID NO: 96) Comprises the following LCDRs |
| | LCDR1: RASSSVIYMC (SEQ ID NO: 97) |
| | LCDR2: GTSTLAS (SEQ ID NO: 98) |
| | LCDR3: QQWSSNPFT (SEQ ID NO: 99) |
| 776.1 VL(C33A) | QIVLSQSPAILFASPGETVTMTCRASSSVIYMAWNQQKPGSSPKPWIYGTS TLASGVPTRFSGSGSGTSYSLTISRVEAEDAATYYCQQWSSNPFTFGSGTK LEIK (SEQ ID NO: 100) Comprises the following LCDRs |
| | LCDR1: RASSSVIYMA (SEQ ID NO: 101) |
| | LCDR2: GTSTLAS (SEQ ID NO: 98) |
| | LCDR3: QQWSSNPFT (SEQ ID NO: 99) |
| 776.1 VL(C33S) | QIVLSQSPAILFASPGETVTMTCRASSSVIYMSWNQQKPGSSPKPWIYGTS TLASGVPTRFSGSGSGTSYSLTISRVEAEDAATYYCQQWSSNPFTFGSGTK LEIK (SEQ ID NO:102) Comprises the following LCDRs |
| | LCDR1: RASSSVIYMS (SEQ ID NO: 103) |
| | LCDR2: GTSTLAS (SEQ ID NO: 98) |
| | LCDR3: QQWSSNPFT (SEQ ID NO: 99) |

### Kappa light chain constant region: SEQ ID NO: 50

The genes encoding the VH regions and the VL regions described above were inserted sequentially into an expression vector pcDNA3.1(+) comprising genes encoding a human IgG1 heavy chain constant region and a kappa light chain constant region to obtain the corresponding heavy chains and light chains of the antibodies. The heavy chain constant regions used all contained mutations at sites L234A, L235A, D265A, and P329A (according to Eu numbering) to eliminate effector functions.

Plasmids encoding the heavy and light chains of the anti-MUC16 antibodies described above were combined according to Table 25 and co-transfected into ExpiCHO-S cells to express the antibodies as described in Table 25, followed by corresponding purification (see Example 1.1 for details). The purified antibodies were concentrated, and sterile filtration was performed. The purities of the antibody proteins were detected by SDS-PAGE and size exclusion chromatography.

**Table 25: Antibody names and combinations of corresponding heavy and light chains**

| Antibody name | Heavy chain name | Light chain name |
|---|---|---|
| 776.1 | 776.1 HC (SEQ ID NO:106) | 776.1 LC (SEQ ID NO:107) |
| 776.1(C33A) | 776.1 HC (SEQ ID NO:106) | 776.1 LC(C33A) (SEQ ID NO:108) |
| 776.1(C33S) | 776.1 HC (SEQ ID NO:106) | 776.1 LC(C33S) (SEQ ID NO:109) |

N-glycosylation of the heavy chains was determined by reduced SDS-PAGE and non-reduced SDS-PAGE. Under reducing conditions, the heavy chains of all antibodies had two bands at around 50 kDa as shown in FIG. 14, indicating the possibility of glycosylation at the N-glycosylation site in the third framework region of the heavy chain.

The amino acid sequences of the membrane-proximal ends of the extracellular regions of the human, cynomolgus monkey and rhesus monkey MUC16 proteins were sent to General Biology System (Anhui) Co., Ltd. for gene synthesis, and each protein was fused with a 6×His tag at the C-terminus. The amino acid sequence of each fusion protein having the His tag at the C-terminus is given in the table below.

**Table 26: Amino acid sequences of recombinant MUC 16**

| Name | Amino acid sequence |
|---|---|
| Human MUC16-His | SEQ ID NO:110 |
| Rhesus monkey MUC16-His | SEQ ID NO:111 |
| Cynomolgus monkey MUC 16-His | SEQ ID NO:112 |

Plasmids comprising proteins encoding the membrane-proximal ends of the extracellular regions of the human, cynomolgus monkey and rhesus monkey MUC16 proteins described in Table 26 were separately transfected into ExpiCHO-S cells to express the antigens as described in Table 26 (see Example 1.1 for details) and purified using HisTrap^{™} excel (GE, Cat# 29048586) according to the manufacturer's instructions. The purified proteins were concentrated, and sterile filtration was performed. The purities of the purified proteins were detected by SDS-PAGE.

8.2 Binding affinity of anti-MUC-16 antibodies for human and rhesus monkey MUC16 proteins The binding affinity of the anti-MUC-16 antibodies for the membrane-proximal end of the extracellular region of the human MUC16 protein (human MUC16-His, Table 26) and the membrane-proximal end of the extracellular region of the rhesus monkey MUC16 protein (rhesus monkey MUC16-His, Table 26) was detected by ForteBio Octet RED96, respectively. According to the manufacturer's instructions, an AHC sensor (ForteBio, Cat# 18-5060) was placed in a running buffer (1× PBS, Hyclone, Cat# SH30256.01, containing 0.02% Tween 20, pH 7.0) and pre-equilibrated at room temperature for 10 min. In a 96-well plate, the kinetic assay was performed according to the following steps: a) equilibrating the baseline for 100 s with the running buffer; b) diluting the anti-MUC-16 antibodies with the running buffer to 5 µg/mL, immobilizing for 200 s, and stopping; c) equilibrating the baseline for 300 s with the running buffer; and d) diluting the human or rhesus monkey MUC16 protein with the running buffer to 50 nM, binding for 200 s, and dissociating for 600 s. The experimental data were fitted and calculated using a Fortebio Data Analysis software 1: 1 binding model.

The results are shown in Table 27.

**Table 27. Binding affinity ofMUC-16 antibodies for membrane-proximal ends of extracellular regions of human and rhesus monkey MUC16 proteins**

| Antibody | Antigen | Ka (M-1S-1) | Kd (S-1) | KD (M) |
|---|---|---|---|---|
| 776.1 | Human MUC16 | 4.68E+05 | 1.68E-04 | 3.59E-10 |
| | Rhesus monkey MUC 16 | 8.56E+05 | 1.56E-03 | 1.82E-09 |
| 776.1(C33A) | Human MUC16 | 2.55E+05 | 3.48E-04 | 1.36E-09 |
| | Rhesus monkey MUC16 | 7.91E+05 | 9.45E-04 | 1.20E-09 |
| 776.1(C33S) | Human MUC16 | 2.66E+05 | 2.45E-04 | 9.21E-10 |
| | Rhesus monkey MUC16 | 8.03E+05 | 8.26E-04 | 1.03E-09 |

### 8.3 Binding of anti-human MUC16 antibodies to human MUC16 protein or rhesus monkey MUC16 protein expressed on surface of 293T cells

Whether the anti-human MUC16 antibodies in the present invention could bind to the human or monkey MUC16 protein stably expressed on the surface of 293T cells was determined by cell binding experiments. Firstly, the coding human MUC16 protein (ECT_TM huMuc16: SEQ ID NO: 127, comprising the membrane-proximal end, transmembrane region, and intracellular region, subjected to codon optimization and gene synthesis by General Biology System (Anhui) Co., Ltd., and cloned into a stably transfected vector) and rhesus monkey MUC16 protein (ECT_TM RhMuc16: SEQ ID NO: 128, comprising the membrane-proximal end, transmembrane region, and intracellular region, subjected to codon optimization and gene synthesis by General Biology System (Anhui) Co., Ltd., and cloned into a stably transfected vector) were separately transfected into 293T cells using a Lipofectamine^{™} 2000 (Invitrogen, Cat# 11668019) transfection reagent. The cells were screened with 0.3 µg/mL of puromycin (Gibco, Cat# A1113802) to obtain 293T cells highly expressing human MUC16 (also referred to as 293T/hMUC16 cells) or 293T cells highly expressing rhesus monkey MUC16 (also referred to as 293T/rhesus monkey MUC16 cells or 293T/rhesus MUC16 cells). The serially diluted anti-human MUC16 antibodies (at an initial concentration of 200 nM, 4-fold dilution, 8 points in total) obtained in the present application were added to the cells, and the cells were incubated at 4 °C for 30 min. The cells were washed twice with PBS, and then fluorescent secondary antibody R-PE-conjugated AffiniPure Goat Anti-Human IgG, Fcy Fragment Specific (Jackson ImmunoResearch, Cat# 109-116-098) was added. The cells were incubated at 4 °C for 30 min. The cells were washed twice with PBS and then resuspended, and finally, fluorescence signals were detected using a BD Accuri C5 (BD Bioscience) flow cytometer.

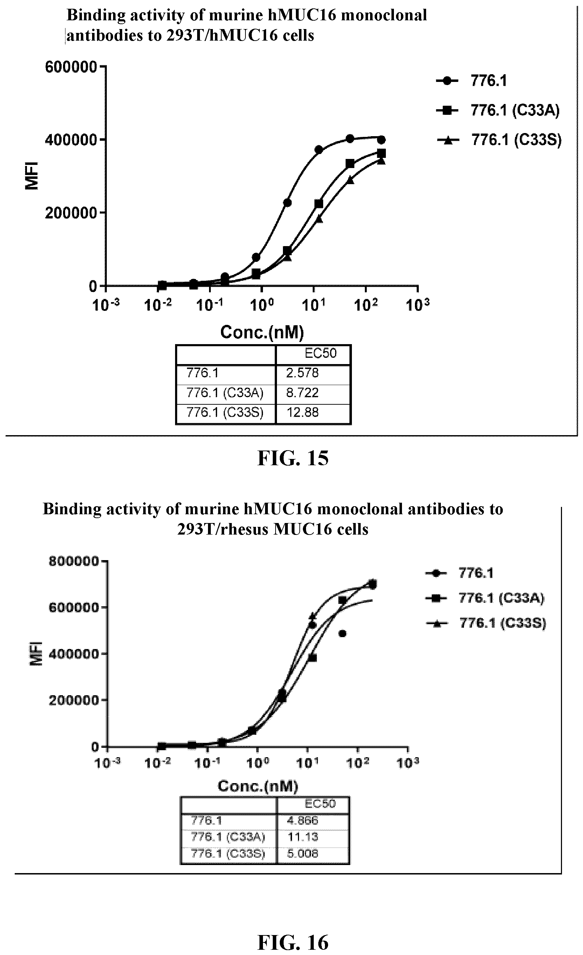

From the results shown in FIGs. 15-16, it can be seen that 776.1(C33A) and 776.1(C33S) bound to the human Muc16 protein and rhesus monkey Muc16 protein expressed on the 293T cells, and had binding capacity comparable to that of the unmodified antibody (776.1).

### 8.4 Humanization of MUC16 antibody

The murine antibody 776.1(C33S) was humanized by the method as described in Example 1. Briefly, the sequence of 776.1(C33S) was searched and aligned in the IMGT database, thereby obtaining a human germline gene sequence IGHV1-18*01 with high homology to the 776.1(C33S) heavy chain variable region for use as a humanized framework of the heavy chain variable region, and selecting a human germline gene sequence IGKV1-39*01 with high homology to the light chain variable region for use as a humanized framework of the light chain variable region. The CDRs of the 776.1(C33S) heavy and light chain variable regions were grafted into the corresponding humanized frameworks to form humanized anti-MUC16 antibody variable regions. In order to maintain the affinity of the murine antibody 776.1(C33S), the humanized antibody variable regions obtained were back-mutated. In order to eliminate potential N-glycosylation sites, N73T (according to kabat numbering) was introduced into the third framework region.

Therefore, the sequences of the corresponding humanized heavy chain variable region and humanized light chain variable region were obtained as shown in Table 28.

| Table 28 | |
|---|---|
| Name | Amino acid sequence |
| Heavy chain variable region (MCH7) | QVQLVQSGAEVKKPGASVKVSCKASGYTFTDYNIHWVRQAPG QGLEWMGYIYPYNGVSDYNQNFKSRVTMTTDTSTSTAYMELR SLRSDDTAVYYCARWDFGSGYYFDYWGQGTLVTVSS (SEQ ID NO: 113) |
| Light chain variable region (MCL4) | EIVLTQSPATLSLSPGERATLSCRASSSVIYMSWYQQKPGQAPRL LIYGTSTLASGIPARFSGSGSGTDFTLTISSLEPEDFAVYYCQQWS SNPFTFGQGTKLEIK (SEQ ID NO: 114) |

The amino acid sequence of the heavy chain variable region was sent to Suzhou Genewiz Biological Technology Co., Ltd. and the amino acid sequence of the light chain variable region was sent to Nanjing GenScript Biotech Co., Ltd. for codon optimization and gene synthesis. The genes encoding the VH region and the VL region of the antibody were inserted sequentially into an expression vector pcDNA3.1(+) comprising a coding gene of a human IgG1 heavy chain constant region and a coding gene of a kappa light chain constant region to obtain a plasmid expressing the anti-MUC16 humanized antibody full-length heavy chain and a plasmid encoding the full-length light chain. The heavy chain constant region used by the humanized MUC16 antibody contained mutations at sites L234A, L235A, D265A, and P329A (according to Eu numbering) to eliminate effector functions.

The plasmid combination encoding the heavy and light chains was co-transfected into ExpiCHO-S cells to express the anti-MUC16 humanized antibody (Hu-L4H7, the heavy chain sequence thereof was set forth in SEQ ID NO: 115, and the light chain sequence thereof was set forth in SEQ ID NO: 116), followed by corresponding purification (see Example 1.1 for details). The purified antibody was concentrated, and sterile filtration was performed. The purity of the protein was detected by SDS-PAGE and size exclusion chromatography (SEC).

### 8.5 Physicochemical analysis and affinity assay of humanized anti-MUC16 antibody

For the humanized anti-MUC16 antibody (Hu-L4H7) obtained, the purity of the antibody was determined by size exclusion chromatography. Specifically, 20 µg of sample was injected onto a TSK G3000SWXI, column using 100 mM sodium phosphate + 100 mM Na₂SO₄ (pH 7.0) as a running buffer for 30 min. The collected effluent was measured using Agilent 1220 HPLC and the data were analyzed using an OpenLAB software.

The results in Table 29 show that the humanized anti-MUC16 antibody Hu-L4H7 had a main peak on SEC of more than 99%, while the main peak of the control 776.1(C33S) was 56.9%, indicating that the purified humanized antibody has higher purity and integrity, which also shows that the humanized antibody has successfully removed N-glycosylation.

Table 29. Purity of humanized antibody

| mAb ID | Purity (%) |
|---|---|
| Hu-L4H7 | 99.1 |
| 776.1(C33S) | 56.9 |

The binding affinity of the humanized anti-MUC-16 antibody for the human and rhesus monkey MUC16 proteins was separately detected using ForteBio Octet RED96. See Example 8.1 for detection and analysis methods, and see Table 30 for detection results.

**Table 30. Binding affinity of humanized anti-MUC-16 antibody for human and rhesus monkey MUC16 proteins**

| Antibody | Antigen | Ka (M⁻¹S⁻¹) | Kd (S⁻¹) | KD (M) |
|---|---|---|---|---|
| Hu-L4H7 | Human MUC16-His | 3.14E+05 | 9.11E-04 | 2.90E-09 |
| | Rhesus monkey MUC16-His | 2.54E+05 | 2.63E-04 | 1.04E-09 |

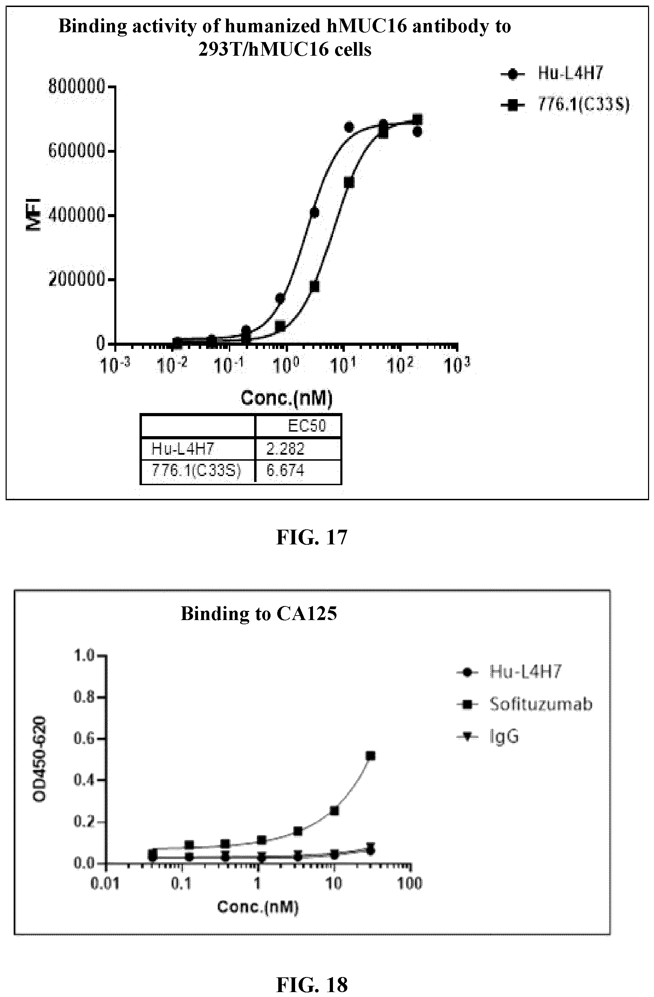

Whether the humanized anti-MUC 16 antibody in the present invention could bind to the human MUC16 protein stably expressed on the surface of 293T cells was determined by cell binding experiments. See Example 8.1 for specific detection and analysis steps. FIG. 17 shows that the humanized MUC-16 antibody was capable of binding to the human MUC16 protein expressed on the surface of the 293T cells.

In order to further verify that the humanized anti-MUC16 antibody was capable of specifically binding to the membrane-proximal end of MUC16 without binding to free CA125, 4000 U/mL of human CA125 (Guilin Immunetech International, LLC, Cat# KAY0023, CA125 extracted from ascites of patients) was immobilized on a 96-well plate at 50 µL/well, and the plate was incubated at 4 °C overnight. The plate was incubated with a blocking buffer (a PBS solution containing 1% BSA) at 37 °C and blocked for 1 h. After blocking, the plate was washed once with a PBST solution (a PBS solution containing 0.05% Tween 20). Biotinylated Hu-L4H7, Sofituzumab (heavy chain: SEQ ID NO: 125; light chain: SEQ ID NO: 126; positive control), and IgG (negative control) were serially diluted with a dilution and separately added to the plate. The plate was incubated at 37 °C for 1 h. After the incubation, the plate was washed with the PBST solution. The secondary antibody (horseradish peroxidase HRP-labeled streptavidin, Jackson Immuno Research, Cat# 016-030-084) was diluted with a dilution and added to the plate. The plate was incubated at 37 °C for 1 h. After the incubation, the plate was washed again, and subjected to color development with TMB. Then the color development was stopped with 1 M H₂SO₄. The absorbance values of OD450 nm-OD620 nm were read in a microplate reader. The results are shown in FIG. 18. The results show that the humanized antibody Hu-L4H7 did not bind to human CA125.

### Example 9: Immunohistochemical Assay of MUC16 and CD8 in Tumor Tissues

Ovarian carcinoma tissue sections (Bioyeargene, Cat# FOV1006) were dewaxed with xylene and ethanol and rehydrated for 5 min. The tissue sections were immersed in an antigen retrieval solution (Servicebio, Cat# G1202-250ML) and boiled for 15 min. The tissue sections were cooled and incubated with 3% hydrogen peroxide at room temperature for 10 min, then washed twice with a PBS solution (Solarbio, Cat# P1010) and blocked in 5% BSA blocking buffer (Solarbio, Cat# SW3015) at room temperature for 1 h. The tissue sections were then incubated in the blocking buffer with 8.7 µg/mL of Hu-L4H7 or a CD8 antibody (Abcam, Cat# ab209775) diluted in a ratio of 1:300 at 4 °C overnight. After 3 washes with the PBS solution, the tissue sections were incubated with a goat anti-human IgG Fc-specific antibody (Jackson ImmunoReasearch, Cat# 109-035-098) diluted with the blocking buffer in a ratio of 1:3000 at room temperature for 1 h. The tissue sections were then washed twice with the PBS solution and detected at room temperature using DAB (Servicebio, Cat# G1212-200T) for color development for 10 min. The tissue sections were then counterstained with hematoxylin (Servicebio, Cat# G1004-100ML). The tissue sections were dehydrated with ethanol and xylene, the coverslips were coated with neutral balsam (Servicebio, Cat# WG10004160) for sealing, and finally, the sections were scanned.

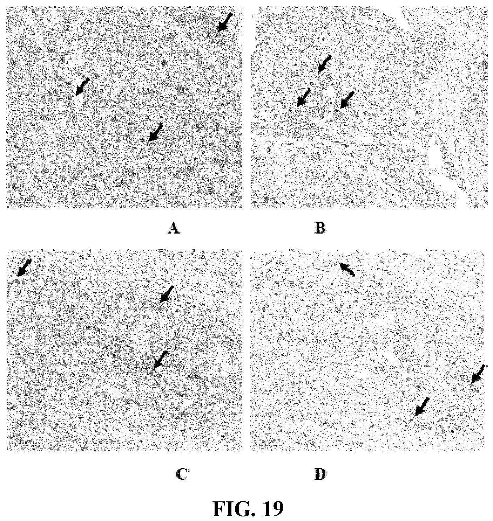

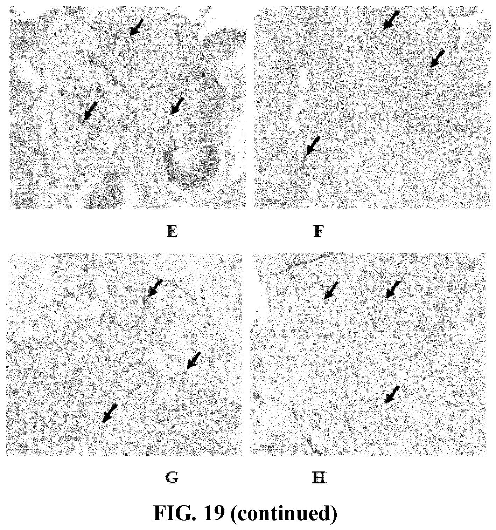

The results are shown in FIG. 19, in which FIGs. 19A and 19B show section samples from ovarian serous adenocarcinoma (T1aN0M0, Grade 3), FIGs. 19C and 19D show section samples from ovarian mucinous adenocarcinoma (T3aN0M0, Grade 3), FIGs. 19E and 19F show section samples from endometrioid adenocarcinoma (T1aN0M0, Grade 3), and FIGs. 19G and 19H show section samples from ovarian clear cell adenocarcinoma (T1aN0M0, Grade 2). Samples in FIGs. 19A, 19C, 19E, and 19G were stained with the CD8 antibody, and samples in FIGs. 19B, 19D, 19F, and 19H were stained with the Hu-L4H7 antibody obtained in the present application. The positive results are shown by arrows. The results in FIG. 19 show that there were a certain amount of CD8 T cells in ovarian carcinoma, and that the antibody Hu-L4H7 obtained in the present application could bind to the cells expressing MUC16 in ovarian carcinoma. Therefore, the bispecific antibody of the Hu-L4H7 and CD3 antibodies obtained in the present application is predicted to improve the tumor cell killing efficiency through bridging in ovarian carcinoma, thereby achieving the effect of inhibiting tumor growth.

### Example 10: Anti-CD3/MUC16 Bispecific Antibodies

### 10.1 Construction of bispecific antibodies

In the present invention, three types of bispecific antibodies with different asymmetric structures were constructed as shown in FIG. 20, wherein in each bispecific antibody, the anti-MUC16 moiety was derived from the humanized MUC16 antibody described above, the anti-CD3 moiety was derived from the humanized CD3 antibody described above, and the constant regions of the bispecific antibodies each contained a knob-in-hole (KIH) structure (Merchant, A. M., et al. (1998). "An efficient route to human bispecific IgG." Nat Biotechnol 16(7): 677-681.). Such bispecific antibodies are also referred to herein as "anti-CD3/MUC16 bispecific antibodies" or "anti-CD3/anti-MUC16 bispecific antibodies", sometimes simply referred to as "bispecific antibody molecules" or "diabodies". The anti-MUC16 moiety of the anti-CD3/MUC16 bispecific antibody targeted cells expressing MUC16, and the anti-CD3 moiety activated T cells. The diabody simultaneously bound to MUC16 on tumor cells and CD3 on T cells to facilitate targeted killing of the tumor cells by the activated T cells.

Three types of anti-CD3/MUC16 bispecific antibodies with the structures shown in FIG. 20 were obtained using standard construction methods in the present application, the amino acid sequences of which are listed in Table 31 below. The heavy chain of the bispecific antibody was named sequentially from the N-terminus to the C-terminus according to the source sequence. For example, 23L2ScFv-Hole means that the sequence sequentially comprises, from the N-terminus to the C-terminus, an ScFv derived from 23L2 and a constant region comprising a hole structure; 2MCH7-KIH-Knob means that the sequence sequentially comprises, from the N-terminus to the C-terminus, 2 sequences derived from MCH7 and a constant region comprising a knob structure.

Each bispecific antibody molecule was constructed as follows:
23L2ScFv-Hole: the nucleotide sequence encoding the anti-CD3 single-chain antibody 23L2 was cloned into a pcDNA3.1(+) vector with Fc (Hole) for expression to obtain the 23L2ScFv-Hole molecule.
MCH7-23L2ScFv-Hole: the coding sequence of the humanized anti-MUC16 antibody heavy chain was used as a template to amplify a nucleotide sequence encoding the heavy chain variable region and the heavy chain constant region CH1 segment, and the sequence was directly spliced with the nucleotide sequence encoding the complete 23L2ScFv-Hole (there was no linker peptide between the two sequences) using an overlapping PCR technique; the spliced nucleotide sequence was cloned into an expression vector pcDNA3.1(+) vector for expression to obtain the MCH7-23L2ScFv-Hole molecule.
MCH7-KIH-Knob: the nucleotide sequence encoding the heavy chain variable region and the heavy chain constant region CH1 segment of the humanized anti-MUC16 antibody was cloned into a pcDNA3.1(+) vector with Fc (Knob) for expression to obtain the MCH7-KIH-Knob molecule.
MCL4 LC: the nucleotide sequence encoding the light chain of the humanized anti-MUC16 antibody was cloned into a pcDNA3.1(+) vector for expression to obtain the MCL4 LC molecule.
2MCH7-KIH-Knob: 2 nucleotide sequences coding the heavy chain variable region and the heavy chain constant region CH1 segment of the humanized anti-MUC16 antibody were directly spliced in series (there was no linker peptide between the 2 coding fragments) by adopting the overlapping PCR technique, and then the spliced sequence was cloned into a pcDNA3.1(+) vector with Fc (Knob) for expression to obtain the 2MCH7-KIH-Knob molecule. The bispecific antibodies in FIG. 8 were obtained by accordingly combining the expression vectors described above according to the specific composition shown in Table 31 and expressing them under appropriate conditions.

The control antibody REGN4018 was derived from the patent US20190389966A1, subjected to codon optimization and gene synthesis by General Biology System (Anhui) Co., Ltd., and cloned into an expression vector.

The construction, expression, purification, and preliminary analysis steps of the bispecific antibodies were the same as those in Example 1.1.

**Table 31. Composition of anti-CD3/MUC16 bispecific antibodies**

| Clone | SEQ ID NO | |
|---|---|---|
| MCH7-23L2 (FIG. 8B) | 117 | Heavy chain 23L2ScFv-Hole |
| | 118 | Heavy chain MCH7-KIH-Knob |
| | 116 | Light chain MCL4 LC |
| 2MCH7-23L2 (FIG. 8C) | 119 | Heavy chain 2MCH7-KIH-Knob |
| | 117 | Heavy chain 23L2ScFv-Hole |
| | 116 | Light chain MCL4 LC |
| MCH7-23L2-MCH7 (FIG. 8A) | 121 | Heavy chain MCH7-23L2ScFv-Hole |
| | 118 | Heavy chain MCH7-KIH-Knob |
| | 116 | Light chain MCL4 LC |
| REGN4018 | 122 | Heavy chain REGN4018-8767pH |
| | 123 | Heavy chain REGN4018-G20 |
| | 124 | Light chain REGN4018-8767pL |

### 10.2 Affinity Assay of bispecific antibody for human/cynomolgus monkey CD3 protein and MUC16 protein

In this experiment, the binding affinity of the bispecific antibody for the human CD3 protein (Cat# CT038-H2508H, Sinobiological), cynomolgus monkey CD3 protein (Cat# CDD-C52W4, Acrobiosystems), human MUC16 protein (full-length human MUC16, the membrane-proximal end of the extracellular region of MUC16 (Table 26)), and cynomolgus monkey MUC16 protein (full-length cynomolgus monkey MUC16, the membrane-proximal end of the extracellular region of MUC16 (Table 26)) was detected using ForteBio Octet RED96 according to the manufacturer's instructions.

Briefly, an AHC sensor (ForteBio, Cat# 18-5060) was placed in a running buffer (1× PBS, Hyclone, Cat# SH30256.01, containing 0.02% Tween 20, pH 7.0) and pre-equilibrated at room temperature for 10 min. In a 96-well plate, the kinetic assay was performed according to the following steps: a) equilibrating the baseline for 100 s with the running buffer; b) adding the anti-human CD3 and MUC-16 bispecific antibody diluted with the running buffer at a final concentration of 5 µg/mL, immobilizing for 200 s; c) equilibrating the baseline for 300 s with the running buffer; and d) adding the human and cynomolgus monkey CD3 and MUC16 proteins diluted with the running buffer at the following concentrations: 100 nM, 50 nM, 25 nM, 12.5 nM, 6.25 nM, and 3.13 nM, to each well, binding for 200 s, and dissociating for 600 s. The experimental data were fitted and calculated using a Fortebio Data Analysis software 1: 1 binding model.

The binding affinity of the bispecific antibody MCH7-23L2-MCH7 for the human and cynomolgus monkey CD3 proteins is summarized in Table 32. The binding affinity of the bispecific antibody MCH7-23L2-MCH7 for the human and cynomolgus monkey MUC16 proteins is summarized in Table 33.

**Table 32. Binding affinity of MCH7-23L2-MCH7 for human and cynomolgus monkey CD3 proteins**

| Antibody | Analyte | KD (M) | Kon (M⁻¹S⁻¹) | Koff (S⁻¹) | Full R^² |
|---|---|---|---|---|---|
| MCH7-23L2-MCH7 | Human CD3D & CD3E heterodimer protein | 4.43E-08 | 2.11E+05 | 9.33E-03 | 0.9033 |
| | Cynomolgus monkey CD3D & CD3E heterodimer protein | 3.04E-08 | 1.24E+05 | 3.78E-03 | 0.9795 |

**Table 33. Binding affinity of MCH7-23L2-MCH7 for human and cynomolgus monkey MUC16 proteins**

| Antibody | Analyte | KD (M) | Kon (M⁻¹S⁻¹) | Koff (S⁻¹) | Full R^² |
|---|---|---|---|---|---|
| MCH7-23L2-MCH7 | Human MUC 16 protein | 3.17E-09 | 1.66E+05 | 5.24E-04 | 0.9873 |
| | Cynomolgus monkey MUC16 protein | 2.91E-09 | 1.88E+05 | 5.46E-04 | 0.9405 |

Tables 32-33 show that the bispecific antibody constructed in the present application could specifically bind to the CD3 protein and the MUC16 protein from the human or cynomolgus monkey, and had species cross-activity. The bispecific antibody obtained in the present application had an order of magnitude lower KD value for the CD3 protein than that for the MUC16 protein.

At present, the affinity level of the CD3 antibody for the ε chain of the CD3 complex is a key factor for the success of the construction of the CD3 diabody. The optimal affinity for CD3 should be maintained in the range of 1-100 nM, and the activation of T cells should be in an appropriate range. Generally, the affinity for the CD3 protein was preferably lower than those for other targets. It can be seen that the anti-CD3/anti-MUC16 bispecific antibody obtained in the present application retained the optimal affinity for CD3, meeting the requirements described above.

### 10.3 Binding experiments of bispecific antibodies to cells expressing human CD3 protein and MUC16 protein

(1) Whether the anti-CD3/anti-MUC16 bispecific antibodies in the present invention could bind to the MUC16 protein endogenously expressed on human ovarian carcinoma cells (OVCAR3) (Cell Bank of Type Culture Collection Committee of the Chinese Academy of Sciences, Cat# TCHu228) was determined by cell binding experiments.

The experiments were performed using flow cytometry. OVCAR3 cells were digested, centrifuged and collected. The cells were resuspended in a PBS solution, and seeded into a 96-well plate (Corning, Cat# 3799) at 1 × 10⁵ cells/well. The 96-well plate was centrifuged and then the supernatant was discarded. 100 µL of the serially diluted test bispecific antibody (at a maximum concentration of 200 nM, 5-fold dilution from 100 nM, 9 concentration points in total) was added to the OVCAR3 cells. The plate was incubated at 4 °C for 30 min and centrifuged (1000 rpm, 5 min), and then the supernatant was discarded. 100 µL of PBS solution was added to each well, and the cells were washed twice. 100 µL of fluorescent secondary antibody R-PE-conjugated AffiniPure Goat Anti-Human IgG, Fcy Fragment Specific (Jackson ImmunoResearch, Cat# 109-116-098) diluted in a ratio of 1:200 was then added. The cells were incubated at 4 °C for 30 min. After the cells were washed twice with the PBS solution, 100 µL of PBS solution was added to each well to resuspend the cells, and finally, fluorescence signals were detected using a BD Accuri C5 (BD Bioscience) flow cytometer according to the manufacturer's instructions. REGN4018 was used as a positive control.

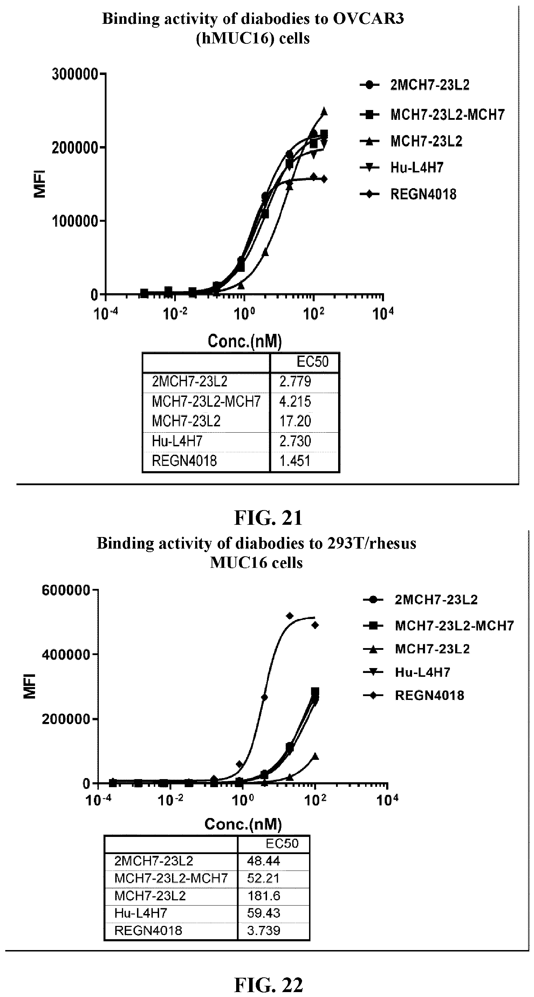

From the results shown in FIG. 21, it can be seen that the tested bispecific antibodies could bind to the MUC16 molecule expressed on the OVCAR3 cells. The tested bispecific antibodies had better binding strength to the MUC16 molecule expressed on the OVCAR3 cells than that of the control REGN4018 at high concentrations.

(2) Whether the anti-CD3/anti-MUC16 bispecific antibodies in the present invention could bind to the cynomolgus monkey MUC16 protein stably expressed on the surface of 293T cells (Cell Bank of Type Culture Collection Committee of the Chinese Academy of Sciences, Cat# SCSP-502) was determined by cell binding experiments. Firstly, a eukaryotic expression vector encoding the cynomolgus monkey MUC16 protein (ECT_TM cyMuc16: SEQ ID NO: 120, comprising the membrane-proximal end, transmembrane region, and intracellular region, subjected to codon optimization and gene synthesis by General Biology System (Anhui) Co., Ltd., and cloned into a stably transfected vector pIRESpuro3) was transfected into 293T cells using a Lipofectamine^{™} 2000 (Invitrogen, Cat# 11668019) transfection reagent according to the manufacturer's instructions. 48 h after the transfection, the cells were screened with 0.3 µg/mL of puromycin (puromycin dihydrochloride, Gibco, Cat# A1113802) for 3-5 days to obtain 293T cells highly expressing the cynomolgus monkey MUC16 protein. Referring to the flow cytometry described above, the serially diluted test bispecific antibody (at an initial concentration of 100 nM, 5-fold dilution, 9 points) was added to the cells, and the plate was incubated at 4 °C for 30 min. The cells were washed twice with PBS, and then fluorescent secondary antibody R-PE-conjugated AffiniPure Goat Anti-Human IgG, Fcy Fragment Specific was added. The cells were incubated at 4 °C for 30 min. The cells were washed twice with PBS and then resuspended, and finally, fluorescence signals were detected using a BD Accuri C5 (BD Bioscience) flow cytometer.

From the results shown in FIG. 22, it can be seen that the tested bispecific antibodies could bind to the cynomolgus monkey MUC16 protein expressed on the 293T cells.

(3) Whether the anti-CD3/anti-MUC16 bispecific antibodies in the present invention could bind to the CD3 protein endogenously expressed on Jurkat cells (Cell Bank of Type Culture Collection Committee of the Chinese Academy of Sciences, Cat# SCSP-513) was determined by cell binding experiments. Detection was performed using the flow cytometry described above, with the following differences: the dilution gradient for the test bispecific antibody was: a maximum concentration of 200 nM, starting from a second concentration of 100 nM, 5-fold dilution, 10 points; the bispecific antibodies were added to Jurkat cells, and the cells were incubated at 4 °C for 30 min. The cells were washed twice with PBS, and then fluorescent secondary antibody R-PE-conjugated AffiniPure Goat Anti-Human IgG, Fcy Fragment Specific was added. The cells were incubated at 4 °C for 30 min. The cells were washed twice with PBS and then resuspended, and finally, fluorescence signals were detected using a BD Accuri C5 (BD Bioscience) flow cytometer.

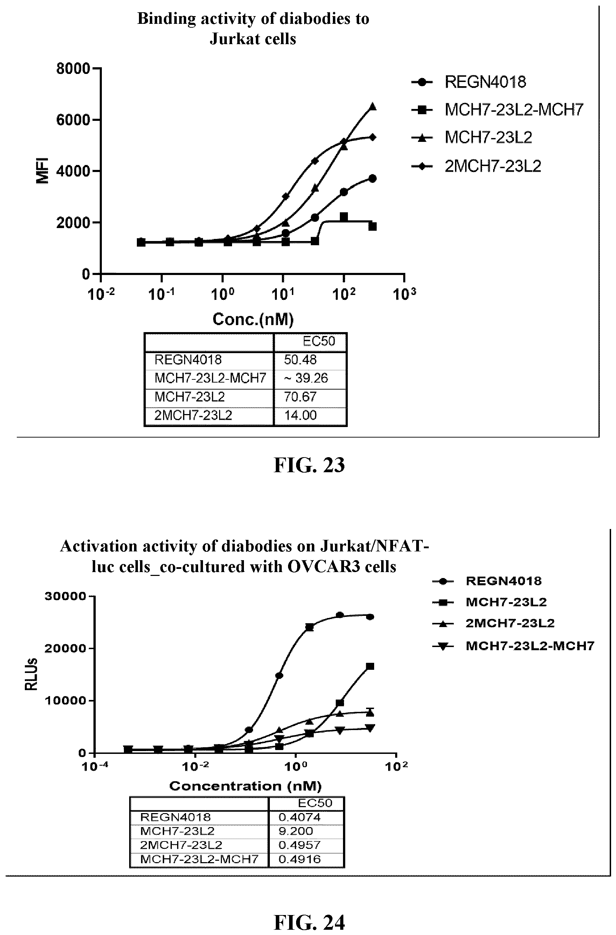

From the results shown in FIG. 23, it can be seen that the tested bispecific antibodies could bind to the human CD3 molecule expressed by the cells.

### 10.4 Activation of anti-CD3/anti-MUC16 bispecific antibodies on T cells and killing activity thereof on tumor cells

In this example, the MUC16 antigen-dependent activating effect of the anti-CD3/anti-MUC16 bispecific antibodies on a Jurkat / NFAT-luc reporter gene system (plasmid: Promega, Cat# E8481) was detected. The target cells in the detection system were an OVCAR3 tumor cell strain endogenously expressing human MUC16 or 293T negative cell strain (Cell Bank of Type Culture Collection Committee of the Chinese Academy of Sciences, Cat# SCSP-502).

The anti-CD3/anti-MUC16 bispecific antibodies bound to both MUC16 on the surface of the OVCAR3 cells and CD3 on the surface of the Jurkat T cells, and activated the T cells by cross-linking with MUC16 antigen-dependent CD3. The expression level of luciferase directly reflected the activating effect of the anti-CD3/anti-MUC16 bispecific antibodies on Jurkat/NFAT-luc cells.

According to the manufacturer's instructions, the Jurkat/NFAT-luc cells were collected and centrifuged, and the supernatant was removed. The cells were resuspended in an assay buffer (90% 1640 medium + 10% FBS) with an effector cell density of 1E6 cells/mL. The OVCAR3 tumor cell strain and 293T negative cell strain were digested, collected, and centrifuged, and the supernatant was removed. The cells were resuspended in the assay buffer (90% 1640 medium + 10% FBS) with a target cell density of 5E5 cells/mL. The effector cell and target cell suspensions were then seeded into a 384-well plate (Corning, Cat# 3570) with 20 µL of effector cells and 20 µL of target cells per well. 10 µL of the serially diluted test bispecific antibody (at a maximum assay concentration of 30 nM, 3-fold dilution in sequence, 9 concentration points in total) was added into each well. The 384-well plate was placed in an incubator at 37 °C. After 6 h, 30 µL of One-Glo (Promega, Cat# E6130) reagent was added into each well, and finally, luminescence signals were detected using a multifunctional microplate reader (Tecan, Cat# F200).

The results are shown in FIGs. 24-25 and show that all the anti-CD3/anti-MUC16 bispecific antibodies were capable of activating the CD3 signaling pathway in the presence of the cells expressing human MUC16 (OVCAR3 cells).

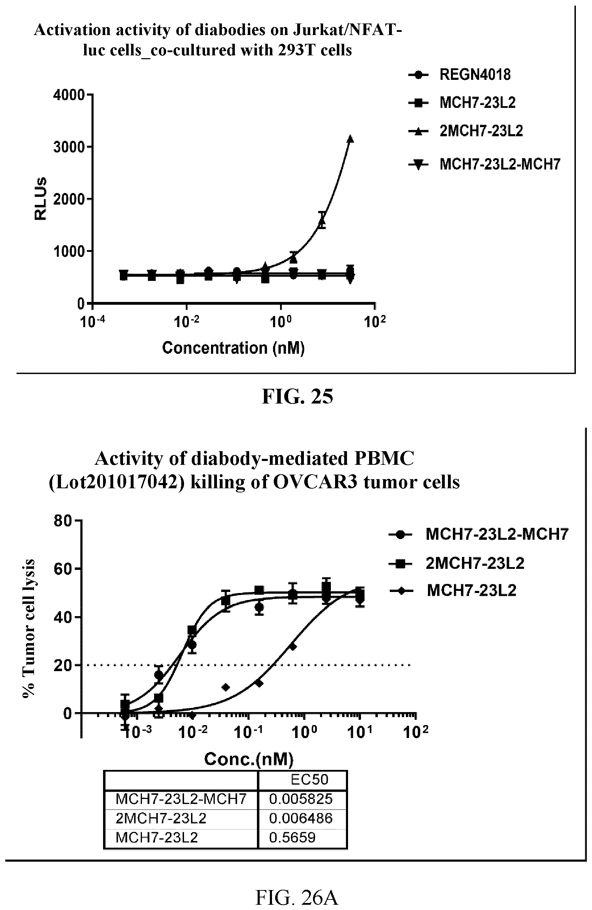

FIG. 25 shows that all the anti-CD3/anti-MUC16 bispecific antibodies were not capable of activating the CD3 signaling pathway when there was no MUC16 present in the assay system (293T negative cell strain).

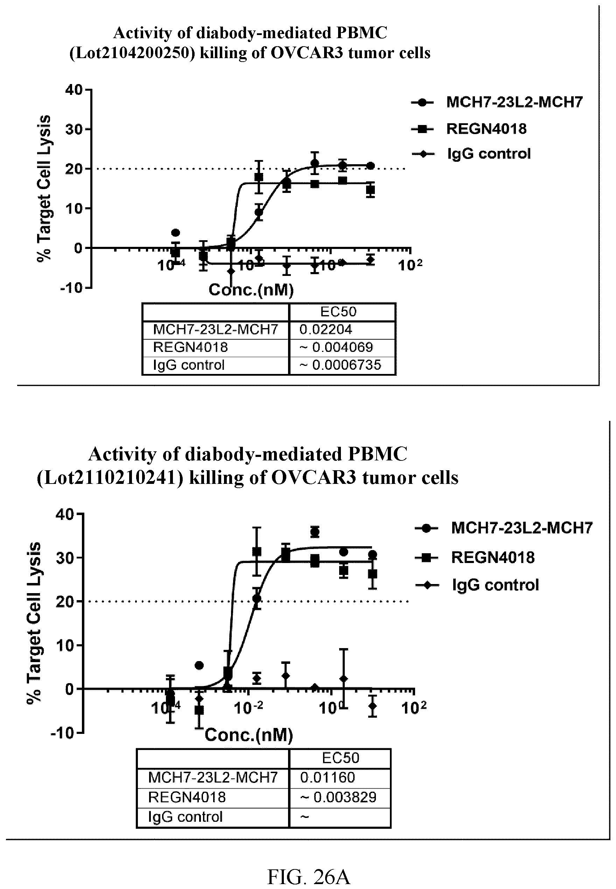

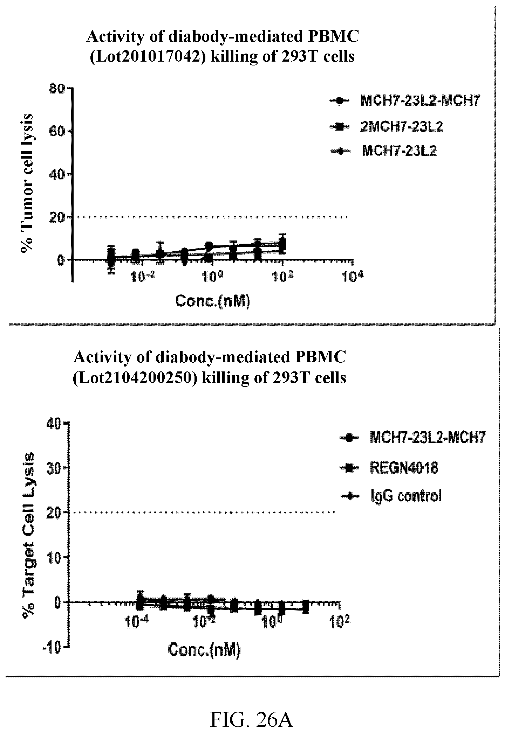

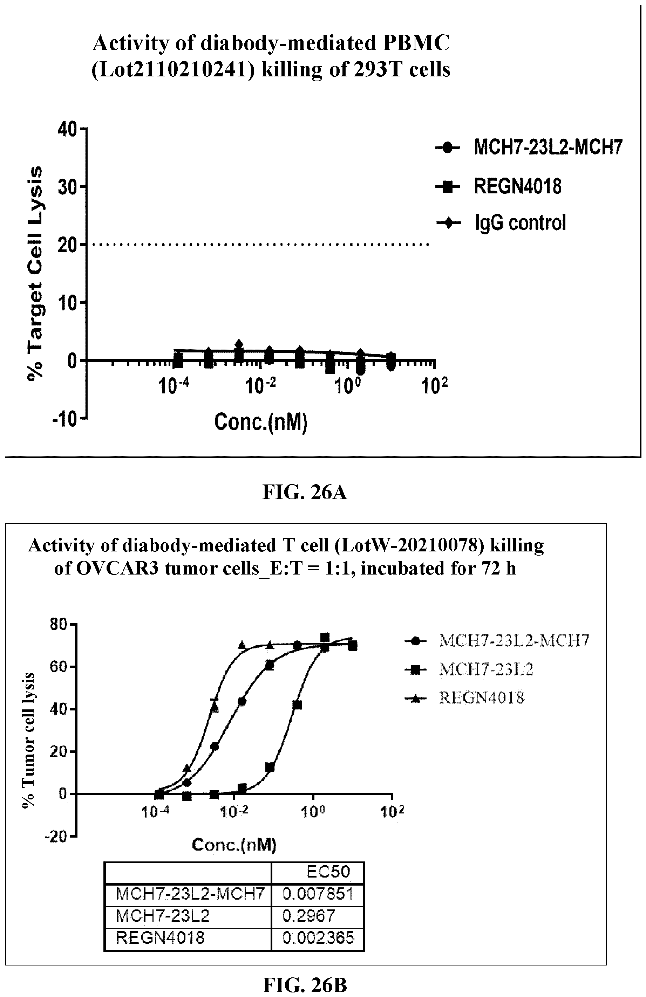

In order to detect the activity of the anti-CD3/anti-MUC16 bispecific antibodies in mediating T cells to kill tumor cells, we established a primary T cell-dependent cytotoxicity assay system with OVCAR3 endogenously expressing human MUC16 as the target cells and human peripheral blood mononuclear cells (PBMCs) as the effector cells.
(1) The PBMCs were thawed, and the cell density was adjusted to 1-2E6 cells/mL using 1640 + 10% FBS complete medium. IL2 at a final concentration of 100 IU/mL was added, and the cells were activated overnight. The next morning, the cultured target cells OVCAR3 and MUC16 negative cells 293T were digested with trypsin, centrifuged at 1000 rpm for 5 min, and collected, and the supernatant was discarded. The target cell density was adjusted to 2 × 10⁵ cells/mL with an MEM-α (Gibco, Cat# 41061-029) assay buffer containing 1% FBS (Gibco, Cat# 10099-141). The cells were seeded into a 96-well plate (Corning, Cat# 3599) at 50 µL/well (i.e., 10,000 cells/well). A test antibody at a 4-fold final detection concentration was prepared. The maximum detection concentration of the test antibody was 10 nM, and the test antibody was serially diluted in 3-fold gradient to obtain 8 concentration points. 50 µL of the test antibody was added into each well. In the experiment, the maximal killing of target cells (2% Triton 100 lysis solution was added to the target cells), minimal killing (the assay buffer was added to the target cells), and natural killing (the effector cells was added to the target cells) controls were set at 50 µL/well. Finally, 100 µL of PBMC effector cell suspension containing IL2 was added to enable the final cell density to be 1 × 10⁶ cells/mL (effector cell E:target cell T = 10:1), and the cells were incubated in the incubator for another 24 h, wherein the PBMC effector cell suspension contained 200 IU/mL of IL2 (Jiangsu Kingsley Pharmaceutical Co., Ltd.). After the incubation was completed, the experimental plate was taken out and centrifuged at 2000 rpm for 3 min to enable all cells to settle to the bottom of the plate. 50 µL of supernatant was carefully pipetted into a new 96-well plate, 50 µL of LDH detection solution (Roche, Cat# 11644793001) was added, and the plate was incubated at room temperature. When the color changed, the plate was read on an F50 microplate reader. The detection wavelength was OD492 nm, the background wavelength was OD650 nm, and the detection analysis was performed when the OD492 nm value of the maximum killing well was between 0.4 and 1.0. The results are shown in FIG. 26A and show that the anti-CD3/anti-MUC16 bispecific antibodies were capable of specifically inducing T cells to kill OVCAR3 tumor cells; however, there was no ADCC effect at all on the MUC16-negative cells 293T.
(2) The PBMCs were thawed, and the cell density was adjusted to 1-2E6 cells/mL using 1640 + 10% FBS complete medium. IL2 at a final concentration of 100 IU/mL was added, and the cells were activated overnight. The next morning, the PBMC cell suspensions were collected, and all T cells were sorted out for use by a T cell isolation kit (Stemcell, Cat# 17951RF). The cultured target cells OVCAR3 were digested with trypsin, centrifuged at 1000 rpm for 5 min, and collected, and the supernatant was discarded. The target cell density was adjusted to 2 × 10⁵ cells/mL with the MEM-α (Gibco, Cat# 41061-029) assay buffer containing 1% FBS (Gibco, Cat# 10099-141). The cells were seeded into a 96-well plate (Corning, Cat# 3599) at 50 µL/well (i.e., 10,000 cells/well). A test antibody at a 4-fold final detection concentration was prepared. The maximum detection concentration of the test antibody was 10 nM, and the test antibody was serially diluted in 5-fold gradient to obtain 8 concentration points. 50 µL of the test antibody was added into each well. In the experiment, the maximal killing of target cells (2% Triton100 lysis solution was added to the target cells), minimal killing (the assay buffer was added to the target cells), and natural killing (the effector cells was added to the target cells) controls were set at 50 µL/well. Finally, 100 µL of T effector cell suspension containing IL2 was added to enable the final cell density to be 1 × 10⁵ cells/mL (effector cell E:target cell T = 1:1), and the cells were incubated in the incubator for another 72 h, wherein the T effector cell suspension contained 200 IU/mL of IL2 (Jiangsu Kingsley Pharmaceutical Co., Ltd.). After the incubation was completed, the experimental plate was taken out and centrifuged at 2000 rpm for 3 min to enable all cells to settle to the bottom of the plate. 50 µL of supernatant was carefully pipetted into a new 96-well plate, 50 µL of LDH detection solution (Roche, Cat# 11644793001) was added, and the plate was incubated at room temperature. When the color changed, the plate was read on an F50 microplate reader. The detection wavelength was OD492 nm, the background wavelength was OD650 nm, and the detection analysis was performed when the OD492 nm value of the maximum killing well was between 0.4 and 1.0. The results are shown in FIG. 26B and show that the anti-CD3/anti-MUC16 bispecific antibodies were still capable of specifically inducing T cells to kill OVCAR3 tumor cells at a low E:T ratio under the condition of extended incubation time.

### 10.5 Induction of cytokine release after activation of T cells by anti-CD3/anti-MUC16 bispecific antibodies

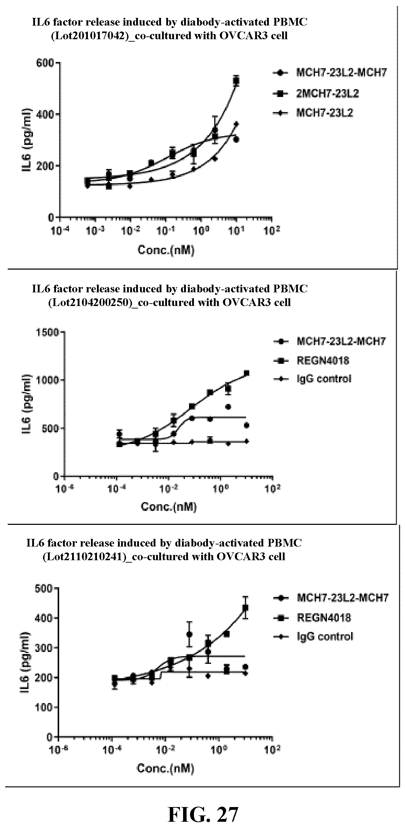

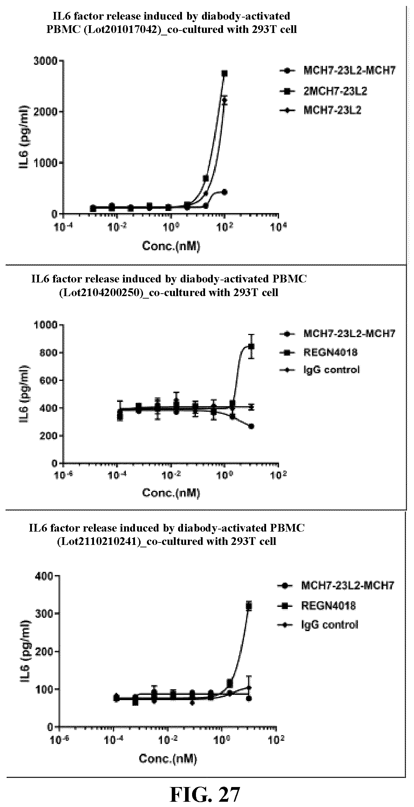

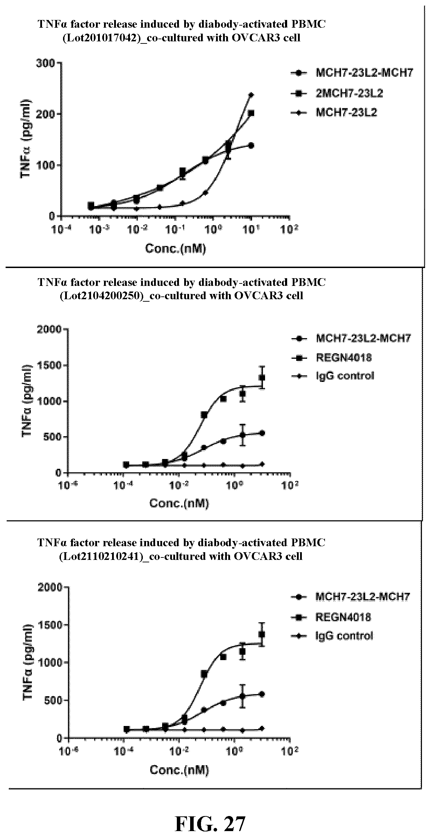

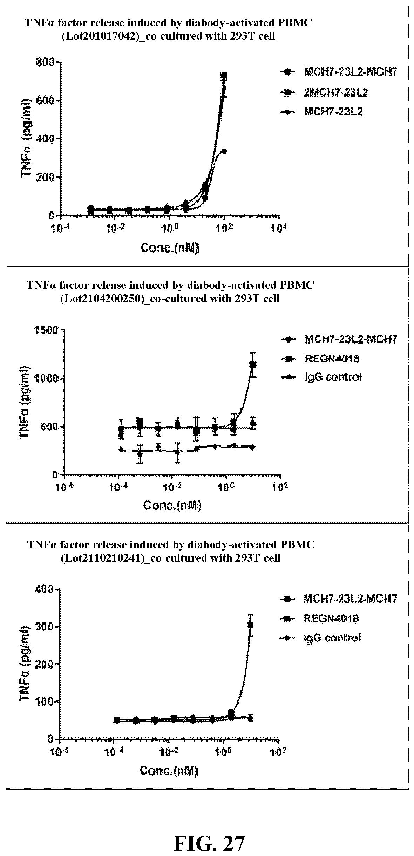

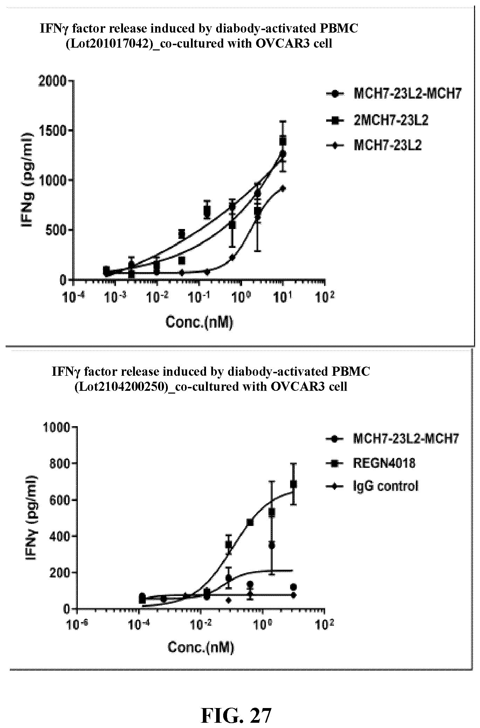

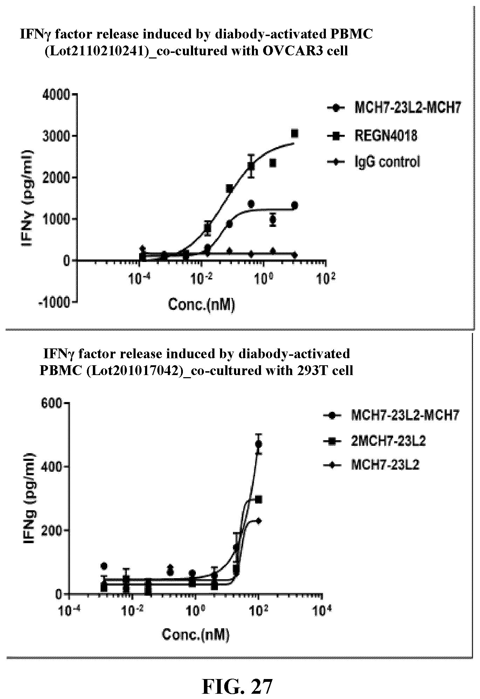

In order to detect the killing of tumor cells by T cells mediated by the anti-CD3/anti-MUC16 bispecific antibodies and the factor secretion at the same time, we established a primary T cell-dependent cytotoxicity assay system with OVCAR3 endogenously expressing human MUC16 as the target cells and human peripheral blood mononuclear cells (PBMCs) as the effector cells. The PBMCs were thawed, and the cell density was adjusted to 1-2E6 cells/mL using 1640 + 10% FBS complete medium. IL2 at a final concentration of 100 IU/mL was added, and the cells were activated overnight. The next morning, the cultured target cells OVCAR3 and MUC16 negative cells 293T were digested with trypsin, centrifuged at 1000 rpm for 5 min, and collected, and the supernatant was discarded. The target cell density was adjusted to 2 × 10⁵ cells/mL with an MEM-α (Gibco, Cat# 41061-029) assay buffer containing 1% FBS (Gibco, Cat# 10099-141). The cells were seeded into a 96-well plate (Corning, Cat# 3599) at 50 µL/well (i.e., 10,000 cells/well). A test antibody at a 4-fold final detection concentration was prepared. The maximum detection concentration of the test antibody was 10 nM, and the test antibody was serially diluted in 3-fold gradient to obtain 8 concentration points. 200 IU/mL of IL2 (Jiangsu Kingsley Pharmaceutical Co., Ltd.) was added to the PBMC effector cell suspension, and finally, 100 µL of PBMC effector cell suspension containing IL2 was added into each well to enable the final cell density to be 1 × 10⁶ cells/mL (effector cell E:target cell T = 10:1). The cells were incubated in the incubator for another 24 h. After the incubation was completed, the experimental plate was taken out and centrifuged at 2000 rpm for 3 min to enable all cells to settle to the bottom of the plate. 100 µL of supernatant was carefully pipetted into a new 96-well plate, and the human IFNγ (Cisbio, Cat# 62HIFNGPEH), human TNFα (R&D, Cat# DY210), and human IL-6 (R&D, Cat# DY206) factor levels in the supernatant were detected, respectively. The results are shown in FIG. 27 and show that the anti-CD3/anti-MUC16 bispecific antibodies and the positive control antibody REGN4018 were capable of inducing T cells to release different levels of human IFNγ, human TNFα, and human IL-6 factors in the OVCAR3 or 293T systems, wherein the human IFNγ factor was released at a level below the lower limit of detection in two batches of PBMCs (Lot2104200250 and Lot2110210241) and 293 co-culture systems, so that the profile is not shown.

### Example 11. Anti-CD3/Anti-MUC16 Bispecific Antibody for In Vivo Tumor Inhibition

In this example, the inhibitory effect of the anti-CD3/anti-MUC16 bispecific antibody on a mouse subcutaneous xenograft tumor was evaluated in C57BL/6-hCD3 transgenic mice carrying a mouse colon cancer cell line MC38.

First, a mouse colon cancer cell line MC38 highly expressing human MUC16 was constructed. MC38 cells were infected with the nucleotide encoding the human MUC16 protein (ECT_TM huMuc16, synthesized by General Biology System (Anhui) Co., Ltd.) using a lentivirus infection method. 4 µg/mL of puromycin dihydrochloride (Gibco, Cat# A1113802) was used for screening to obtain polyclonal cells highly expressing human MUC16, thereby obtaining a monoclonal MC38 cell strain highly expressing human MUC16 by limiting dilution, which was named MC38-hMUC16.

The MC38-hMUC16 cells were collected and counted, and matrigel was added according to a volume ratio of 1:1 and mixed uniformly. The mixture was placed in a wet ice box and stored at a low temperature for use. The experimental animals were 42 C57BL/6-hCD3 mice (Biocytogen, Cat# 110008) at 6-8 weeks of age, which were anesthetized with 3-4% isoflurane before inoculation. The cells were inoculated to the right side of the back of the mice near the axilla by subcutaneous injection (i.v.) at 1.5 × 10⁶ MC38-hMUC16 cells per mouse with an inoculation volume of 200 µL. Among them, 5 mice were administrated on the day of tumor cell inoculation (group G6). Twenty-five mice with tumor volume growing to about 60-80 mm³ were selected. The 25 mice were randomly divided into 5 groups (G1-G5: 5 mice per group) according to the tumor size and body weight, and the day of administration for each group was defined as day 0. The grouping and administration regimens are shown in Table 34:

**Table 34. Grouping and administration regimens**

| Group | Test substance | Number | Dose (mg/kg) | Administration regimen |
|---|---|---|---|---|
| 1 | Vehicle control | 5 | N/A | i.v., 2 times a week for 3 weeks |
| 2 | MCH7-23L2-MCH7 | 5 | 15 | i.v., 2 times a week for 3 weeks |
| 3 | MCH7-23L2-MCH7 | 5 | 5 | i.v., 2 times a week for 3 weeks |
| 4 | MCH7-23L2-MCH7 | 5 | 5 | i.v., once a day for 10 days |
| 5 | REGN4018 | 5 | 5 | i.v., 2 times a week for 3 weeks |
| 6 | MCH7-23L2-MCH7 | 5 | 5 | Administration on the day of tumor cell inoculation, i.v., 2 times a week for 3 weeks |

The appearance and behavior of the mice were observed and recorded daily, with up to 4 weeks of continuous observation starting from the grouping. Tumor volumes were measured twice a week after grouping. The tumor volume (V) was calculated as follows: v = (length × width²)/2. The relative tumor volume (RTV) per mouse was calculated as: RTV = Vₜ/V₀, where Vₜ is the measured volume for each time and V₀ is the volume at the start of the treatment. The results are presented as mean ± S.E.M. Two groups were compared using Dunnett's multi-comparison test, and a statistically significant difference was considered if p < 0.05.

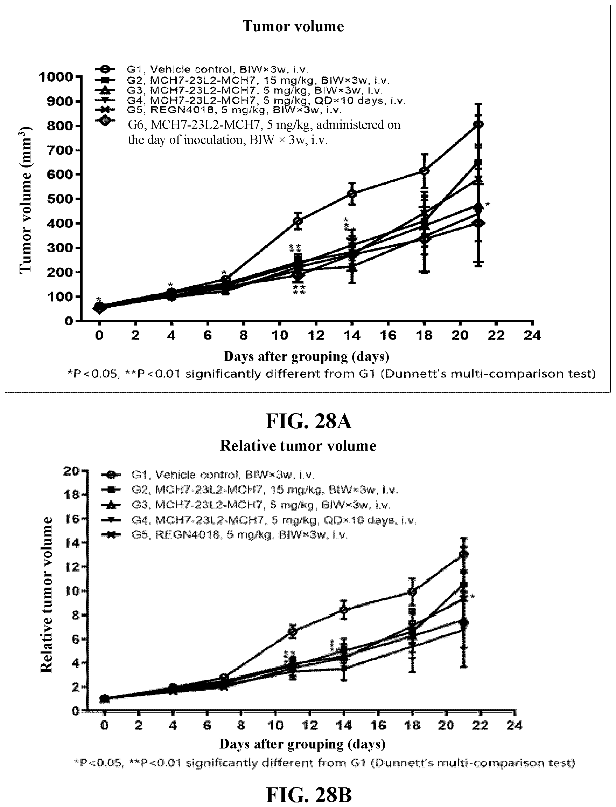

The results are shown in FIGs. 28A and 28B and show that each group had a good tumor inhibitory effect relative to the negative control (group G1), wherein group G3 (MCH7-23L2-MCH7 5 mg/kg) was significantly different from group G1 on day 21 (P < 0.05), and furthermore the tumor inhibitory effect of group G3 was better than that of group G5 (relative tumor growth inhibition TGI_{TV}: 41.52% vs 28.32%). Groups G2-G6 were significantly different from group G1 on day 11 (P < 0.05), and groups G3-G6 were significantly different from group G1 on day 14 (P < 0.05).

### Example 12. Effects of Anti-CD3/Anti-MUC16 Bispecific Antibodies on T Cell Function in TDCC Experiments

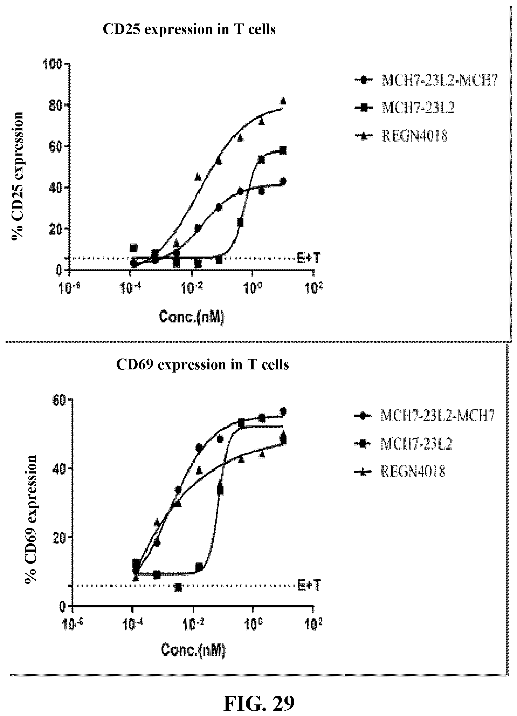

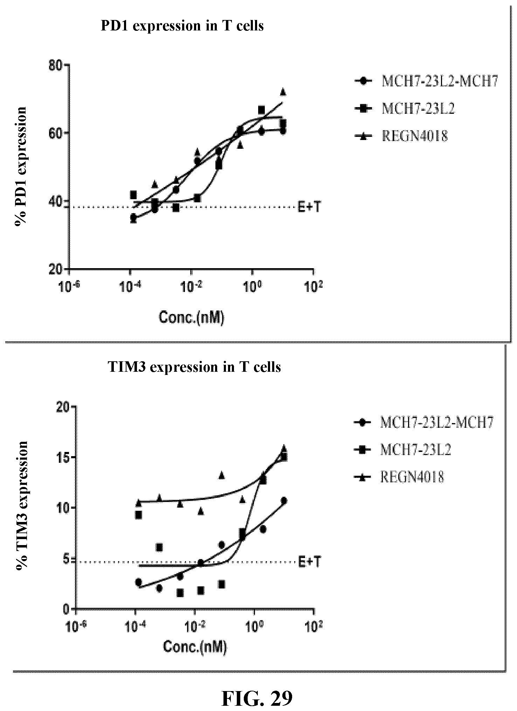

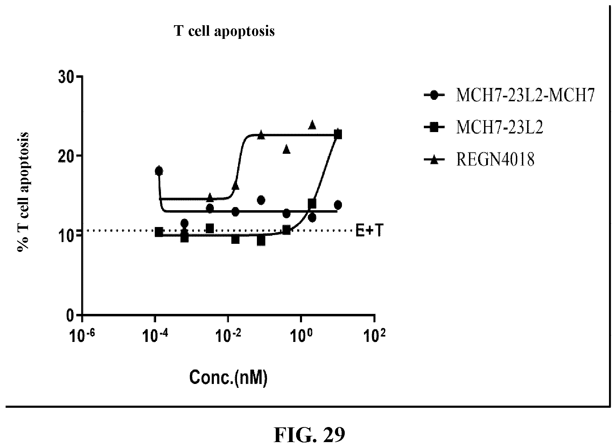

In order to detect the effects of the anti-CD3/anti-MUC16 bispecific antibodies on T cell function in the TDCC killing system, we tested indicators of the expression of CD25 (Biolegend, Cat# 302612), CD69 (Biolegend, Cat# 310904), PD1 (Biolegend, Cat# 329908), and TIM3 (Biolegend, Cat# 345006) in T cells and T cell apoptosis (Biolegend, Cat# 423114). The PBMCs were thawed, and the cell density was adjusted to 1-2E6 cells/mL using 1640 + 10% FBS complete medium. IL2 at a final concentration of 100 IU/mL was added, and the cells were activated overnight. The next morning, the PBMC cell suspensions were collected, and all T cells were sorted out for use by a T cell isolation kit (Stemcell, Cat# 17951RF). The cultured target cells OVCAR3 were digested with trypsin, centrifuged at 1000 rpm for 5 min, and collected, and the supernatant was discarded. The target cell density was adjusted to 2 × 10⁵ cells/mL with the MEM-α (Gibco, Cat# 41061-029) assay buffer containing 1% FBS (Gibco, Cat# 10099-141). The cells were seeded into a 96-well plate (Corning, Cat# 3599) at 50 µL/well (i.e., 10,000 cells/well). A test antibody at a 4-fold final detection concentration was prepared. The maximum detection concentration of the test antibody was 10 nM, and the test antibody was serially diluted in 5-fold gradient to obtain 8 concentration points. 50 µL of the test antibody was added into each well. Finally, 100 µL of T effector cell suspension containing IL2 was added to enable the final cell density to be 1 × 10⁵ cells/mL (effector cell E:target cell T = 1:1), and the cells were incubated in the incubator for another 72 h, wherein the T effector cell suspension contained 200 IU/mL of IL2 (Jiangsu Kingsley Pharmaceutical Co., Ltd.). After the incubation, the plate was taken out, and cells from each well were collected for flow cytometry to test the indicators of T cell activation (the expression of CD25 and CD69), T cell failure (the expression of PD1 and TIM3), and changes in T cell apoptosis. The results are shown in FIG. 29 and show that the anti-CD3/anti-MUC16 bispecific antibodies were less active on T cells, but less active in inducing T cell failure and inducing T cell apoptosis, compared to the control antibody REGN4018 at a low E:T ratio under the condition of extended incubation time.

### Example 13. Repeated Killing Activity of Anti-CD3/Anti-MUC16 Bispecific Antibodies on Tumor Cells

In order to detect the activity of the anti-CD3/anti-MUC16 bispecific antibodies in mediating T cells to repeatedly kill tumor cells, we established a primary T cell-dependent cytotoxicity assay system with OVCAR3 endogenously expressing human MUC16 as the target cells and T cells isolated from human peripheral blood mononuclear cells (PBMCs) as the effector cells. The PBMCs were thawed, and the cell density was adjusted to 1-2E6 cells/mL using 1640 + 10% FBS complete medium. IL2 at a final concentration of 100 IU/mL was added, and the cells were activated overnight. The next morning, the PBMC cell suspensions were collected, and all T cells were sorted out for use by a T cell isolation kit (Stemcell, Cat# 17951RF). The cultured target cells OVCAR3 were digested with trypsin, centrifuged at 1000 rpm for 5 min, and collected, and the supernatant was discarded. The target cell density was adjusted to 2 × 10⁵ cells/mL with the MEM-α (Gibco, Cat# 41061-029) assay buffer containing 1% FBS (Gibco, Cat# 10099-141). The cells were seeded into a 96-well plate (Corning, Cat# 3599) at 50 µL/well (i.e., 10,000 cells/well). A test antibody at a 4-fold final detection concentration was prepared. The maximum detection concentration of the test antibody was 10 nM, and the test antibody was serially diluted in 5-fold gradient to obtain 8 concentration points. 50 µL of the test antibody was added into each well. In the experiment, the maximal killing of target cells (2% Triton100 lysis solution was added to the target cells), minimal killing (the assay buffer was added to the target cells), and natural killing (the effector cells was added to the target cells) controls were set at 50 µL/well. Finally, 100 µL of T effector cell suspension containing IL2 was added to enable the final cell density to be 5 × 10⁵ cells/mL (effector cell E:target cell T = 5:1), and the cells were incubated in the incubator for another 24 h, wherein the T effector cell suspension contained 200 IU/mL of IL2 (Jiangsu Kingsley Pharmaceutical Co., Ltd.). After the incubation was completed, 2 identical experimental plates were taken out and centrifuged at 2000 rpm for 3 min to enable all cells to settle to the bottom of the plate. In one of the experimental plates, 50 µL of supernatant was carefully pipetted into a new 96-well plate, 50 µL of LDH detection solution (Roche, Cat# 11644793001) was added, and the plate was incubated at room temperature. When the color changed, the plate was read on an F50 microplate reader. The detection wavelength was OD492 nm, the background wavelength was OD650 nm, and the detection analysis was performed when the OD492 nm value of the maximum killing well was between 0.4 and 1.0. In another plate, 100 µL of supernatant was discarded, and 50 µL of OVCAR3 cell suspension (10,000 cells/well) and 50 µL of test antibody were added into each well under the preceding experimental conditions. 48 h after incubation, the supernatant was collected for killing detection.

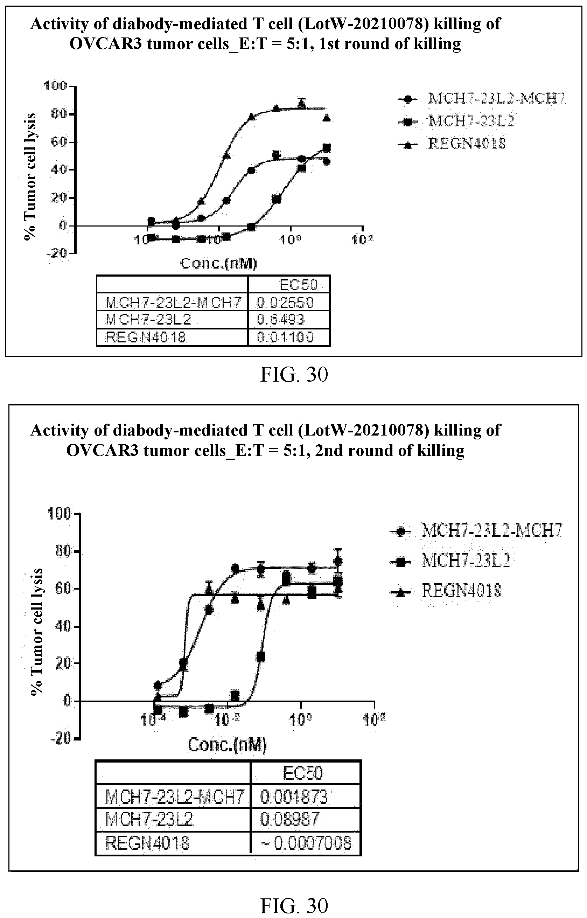

The results are shown in FIG. 30 and show that the anti-CD3/anti-MUC16 bispecific antibodies could induce T cells to continuously kill OVCAR3 tumor cells, with a weaker killing strength in the first round than the positive control antibody but a slightly stronger killing strength in the second round than the positive control REGN4018 under the condition of high E:T.

### Example 14. Effects of Anti-CD3/Anti-MUC16 Bispecific Antibodies on T Cell Function in TDCC Repeated Killing Experiments

In order to detect the effects of the anti-CD3/anti-MUC16 bispecific antibodies on T cell function in the TDCC repeated killing system, we tested indicators of the expression of CD25 (Biolegend, Cat# 302612), PD1 (Biolegend, Cat# 367604 or Cat# 329908), and TIM3 (Biolegend, Cat# 345006) in T cells and T cell apoptosis (Biolegend, Cat# 423114).

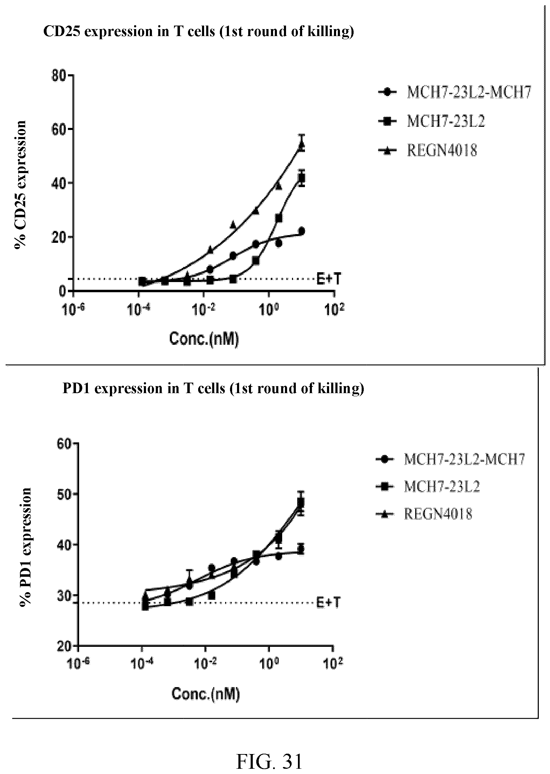

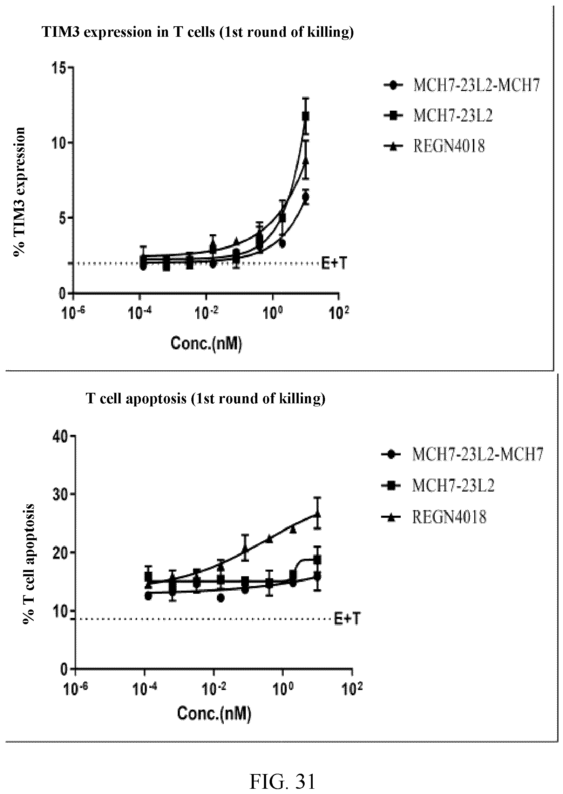

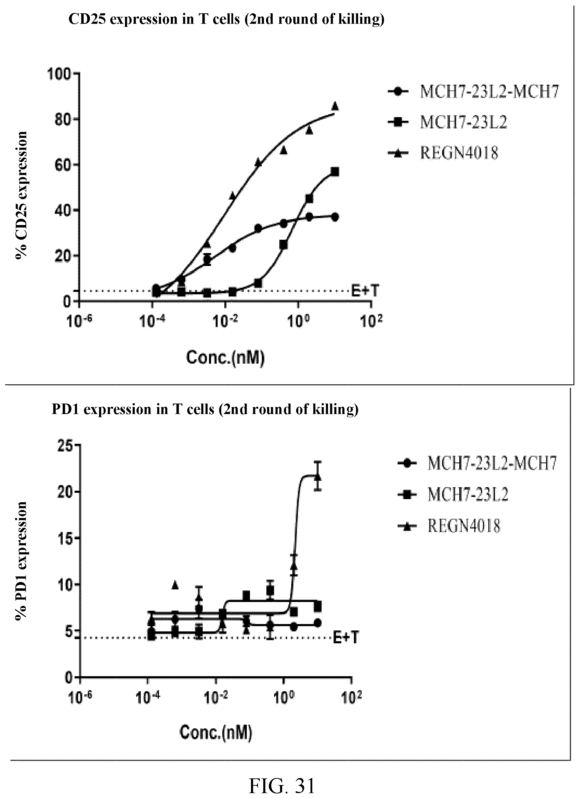

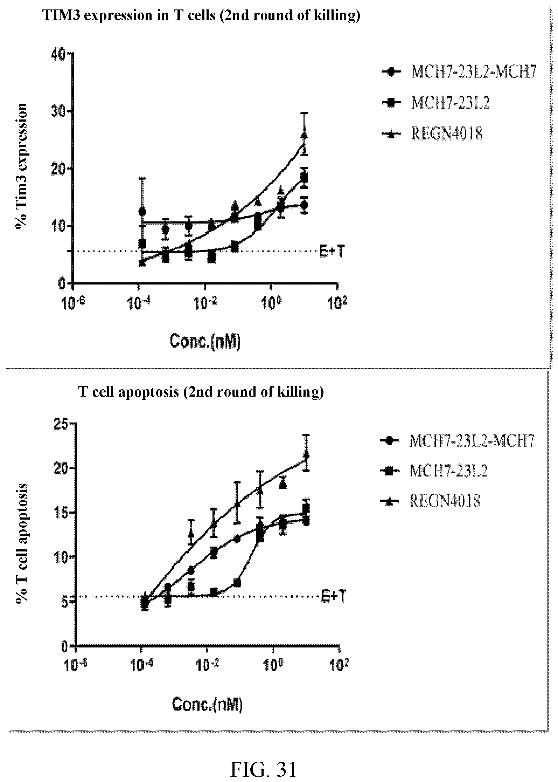

The PBMCs were thawed, and the cell density was adjusted to 1-2E6 cells/mL using 1640 + 10% FBS complete medium. IL2 at a final concentration of 100 IU/mL was added, and the cells were activated overnight. The next morning, the PBMC cell suspensions were collected, and all T cells were sorted out for use by a T cell isolation kit (Stemcell, Cat# 17951RF). The cultured target cells OVCAR3 were digested with trypsin, centrifuged at 1000 rpm for 5 min, and collected, and the supernatant was discarded. The target cell density was adjusted to 2 × 10⁵ cells/mL with the MEM-α (Gibco, Cat# 41061-029) assay buffer containing 1% FBS (Gibco, Cat# 10099-141). The cells were seeded into a 96-well plate (Corning, Cat# 3599) at 50 µL/well (i.e., 10,000 cells/well). A test antibody at a 4-fold final detection concentration was prepared. The maximum detection concentration of the test antibody was 10 nM, and the test antibody was serially diluted in 5-fold gradient to obtain 8 concentration points. 50 µL of the test antibody was added into each well. Finally, 100 µL of T effector cell suspension containing IL2 was added to enable the final cell density to be 5 × 10⁵ cells/mL (effector cell E:target cell T = 5:1), and the cells were incubated in the incubator for another 24 h, wherein the T effector cell suspension contained 200 IU/mL of IL2 (Jiangsu Kingsley Pharmaceutical Co., Ltd.). After the incubation, 2 identical experimental plates were taken out, and cells in one of the plates were collected for flow cytometry. In another plate, 100 µL of supernatant per well was discarded, and 50 µL of OVCAR3 cell suspension (10,000 cells/well) and 50 µL of test antibody were added into each well under the preceding experimental conditions. 48 h after incubation, cells in each well were collected for flow cytometry. The test indicators were T cell activation (the expression of CD25), T cell failure (the expression of PD1 and TIM3), and T cell apoptosis. The results are shown in FIG. 31 and show that the anti-CD3/anti-MUC16 bispecific antibodies were less active on T cells, but less active in inducing T cell failure and inducing T cell apoptosis, compared to the control antibody REGN4018 in two rounds of killing experiments, which may be the reason why it had a stronger killing effect than that of the control antibody in the second round of killing.

All publications, patent applications, patents, and other references mentioned herein are incorporated by reference in their entireties. Any or all of the features described above and throughout the present application may be combined in various embodiments of the present invention. In addition, the materials, methods, and examples described herein are illustrative only and are not intended to be limiting. Other features, objectives, and advantages of the present invention will be apparent from the specification and drawings, and from the appended claims.

### SEQUENCE LISTING:

| SEQ ID NO | Description | (CDRs are all defined using the Kabat scheme; the grey moiety is the sequence in the constant region) |
|---|---|---|
| 1 | SP34 antibody humanized antibody heavy chain hu34H1 | EVQLVESGGGLVQPGGSLKLSCAASGFTFSTYAMNWVRQAPGKGLEWVGRIRSKYNNYATYYA DSVKDRFTISRDDSKNTLYLQMNSLKTEDTAVYYCTRHGNFGNSYVSWFAYWGQGTTVTVSSAS TKGPSVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPEAAGGPSVFLFPPKPKDTL MISRTPEVTCVVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVKGFYPSDIAVE WESNGQPENNYKTTPPVLDSDGSFFLYSKLTVDKSRWQQGNVFSCSVMHEALHNHYTQKSLSLS PGK |
| 2 | SP34 antibody humanized antibody light chain hu34L2 | QAVVTQEPSLTVSPGGTVTLTCRSSTGAVTTSNYANWVQQKPGQAPRGLIGGTNKRAPGVPARFS GSLLGGKAALTLSGAQPEDEAEYFCALWYSNLWVFGGGTKLTVLGQPKAAPSVTLFPPSSEELQ ANKATLVCLISDFYPGAVTVAWKADSSPVKAGVETTTPSKQSNNKYAASSYLSLTPEQWKSHRSY SCQVTHEGSTVEKTVAPTEC |
| 3 | SP34 antibody humanized antibody heavy chain variable region hu34H1 | EVQLVESGGGLVQPGGSLKLSCAASGFTFSTYAMNWVRQAPGKGLEWVGRIRSKYNNYATYYA DSVKDRFTISRDDSKNTLYLQMNSLKTEDTAVYYCTRHGNFGNSYVSWFAYWGQGTTVTVSS |
| 4 | SP34 antibody humanized antibody light chain variable region hu34L2 | QAVVTQEPSLTVSPGGTVTLTCRSSTGAVTTSNYANWVQQKPGQAPRGLIGGTNKRAPGVPARFS GSLLGGKAALTLSGAQPEDEAEYFCALWYSNLWVFGGGTKLTVL |
| 5 | SP34 antibody humanized antibody HCDR1 | TYAMN |
| 6 | SP34 antibody humanized antibody HCDR2 | RIRSKYNNYATYYAD SVKD |
| 7 | SP34 antibody humanized antibody HCDR3 | HGNFGNSYVSWFAY |
| 8 | SP34 antibody humanized antibody LCDR1 | RSSTGAVTTSNYAN |
| 9 | SP34 antibody humanized antibody LCDR2 | GTNKRAP |
| 10 | SP34 antibody humanized antibody LCDR3 | ALWYSNLWV |
| 11 | 23L2 ScFv | EVQLVESGGGLVQPGGSLKLSCAASGFTFSTYAMNWVRQAPGKGLEWVGRIRSKYNNYATYYA DSVKDRFTISRDDSKNTLYLQMNSLKTEDTAVYYCTRHGNFGNSYVSWFAYWGQGTTVTVSSG GGGSGGGGSGGGGSQAVVTQEPSLTVSPGGTVTLTCRSSTGAVTTSNYANWVQQKPGQAPRGL IGGTNKRAPGVPARFSGSLLGGKAALTLSGAQPEDEAEYFCALWYSNLWVFGGGTKLTVLG |
| 12 | scFv linker peptide | GGGGSGGGGSGGGGS |
| 13 | DA023AH23L2 heavy chain (the linker peptide is in bold and underlined) | EVQLVESGGGLVQPGGSLKLSCAASGFTFSTYAMNWVRQAPGKGLEWVGRIRSKYNNYATYYA DSVKDRFTISRDDSKNTLYLQMNSLKTEDTAVYYCTRHGNFGNSYVSWFAYWGQGTTVTVSSG GGGSGGGGSGGGGSQAVVTQEPSLTVSPGGTVTLTCRSSTGAVTTSNYANWVQQKPGQAPRGL IGGTNKRAPGVPARFSGSLLGGKAALTLSGAQPEDEAEYFCALWYSNLWVFGGGTKLTVLGDKT HTCPPCPAPEAAGGPSVFLFPPKPKDTLMISRTPEVTCVVVAVSHEDPEVKFNWYVDGVEVHNAK TKPREEQYNSTYRVVSVLTVLHQDWLNGKEYKCKVSNKALAAPIEKTISKAKGQPREPQVYTLP PSRDELTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 14 | Humanized antibody heavy chain 29HH1-1 | QVQLVQSGAEVKKPGASVKVSCKASGYTFTTHYMYWVRQAPGQGLEWMGGINPSNGATNFNE KFKTRVTLTVDKSTSTAYMELSSLRSEDTAVYYCTRVGGLSYTMDYWGQGTLVTVSSASTKGPS VFPLAPSSKSTSGGTAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSVFLFPPKPKDTLMISRT PEVTCVVVDVSHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFYPSDIAVEWESN GQPENNYKTTPPVLDSDGSFFLYSKLTVDKSRWQQGNVFSCSVMHEALHNHYTQKSLSLSPGK |
| 15 | Humanized antibody light chain 29HL 1 | DIVMTQSPDSLAVSLGERATINCKSSQSLLYSSNQKNYLAWYQQKPGQPPKLLIYWASTRESGVP DRFSGSGYGTDFTLTISSLQAEDVAVYYCQQYYSYPRTFGGGTKVEIKRTVAAPSVFIFPPSDEQLK SGTASVVCLLNNFYPREAKVQWKVDNALQSGNSQESVTEQDSKDSTYSLSSTLTLSKADYEKHK VYACEVTHQGLSSPVTKSFNRGEC |
| 16 | Humanized antibody heavy chain variable region 29HH1-1 | QVQLVQSGAEVKKPGASVKVSCKASGYTFTTHYMYWVRQAPGQGLEWMGGINPSNGATNFNE KFKTRVTLTVDKSTSTAYMELSSLRSEDTAVYYCTRVGGLSYTMDYWGQGTLVTVSS |
| 17 | Humanized antibody light chain variable region 29HL1 | DIVMTQSPDSLAVSLGERATINCKSSQSLLYSSNQKNYLAWYQQKPGQPPKLLIYWASTRESGVP DRFSGSGYGTDFTLTISSLQAEDVAVYYCQQYYSYPRTFGGGTKVEIK |
| 18 | Humanized antibody light chain 29HL2 | DIVMTQSPDSLAVSLGERATINCKSSQSLLYSSNQKNYLAWYQQKPGQPPKLLIYWASTRESGVP DRFSGSGSGTDFTLTISSLQAEDVAVYYCQQYYSYPRTFGGGTKVEIKRTVAAPSVFIFPPSDEQLK SGTASVVCLLNNFYPREAKVQWKVDNALQSGNSQESVTEQDSKDSTYSLSSTLTLSKADYEKHK VYACEVTHQGLSSPVTKSFNRGEC |
| 19 | Humanized antibody light chain variable region 29HL2 | DIVMTQSPDSLAVSLGERATINCKSSQSLLYSSNQKNYLAWYQQKPGQPPKLLIYWASTRESGVP DRFSGSGSGTDFTLTISSLQAEDVAVYYCQQYYSYPRTFGGGTKVEIK |
| 20 | Humanized antibody light chain 29HL3 | DIVMTQSPDSLAVSLGERVTMNCKSSQSLLYSSNQKNYLAWYQQKPGQPPKLLIYWASTRESGVP DRFSGSGYGTDFTLTISSLQAEDVAVYYCQQYYSYPRTFGGGTKLEIKRTVAAPSVFIFPPSDEQLK SGTASVVCLLNNFYPREAKVQWKVDNALQSGNSQESVTEQDSKDSTYSLSSTLTLSKADYEKHK VYACEVTHQGLSSPVTKSFNRGEC |
| 21 | Humanized antibody light chain variable region 29HL3 | DIVMTQSPDSLAVSLGERVTMNCKSSQSLLYSSNQKNYLAWYQQKPGQPPKLLIYWASTRESGVP DRFSGSGYGTDFTLTISSLQAEDVAVYYCQQYYSYPRTFGGGTKLEIK |
| 22 | Humanized antibody heavy chain 29HH1-2 | QVQLVQSGAEVKKPGASVKVSCKASGYTFTTHYMYWVRQAPGQGLEWIGGINPSNGATNFNEK FKTKATLTVDKSTSTAYMELSSLRSEDTAVYYCTRVGGLSYTMDYWGQGTLVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKC KVSNKALPAPIEKTISKAKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYTQKSLSLSPGK |
| 23 | Humanized antibody heavy chain variable region 29HH1-2 | QVQLVQSGAEVKKPGASVKVSCKASGYTFTTHYMYWVRQAPGQGLEWIGGINPSNGATNFNEK FKTKATLTVDKSTSTAYMELSSLRSEDTAVYYCTRVGGLSYTMDYWGQGTLVTVSS |
| 24 | Humanized antibody heavy chain 29HH1-4 | QVQLVQSGAEVKKPGASVKVSCKASGYTFTTHYMYWVRQAPGQGLEWMGGINPSNGATNFNE KFKTRVTMTVDKSTSTAYMELSSLRSEDTAVYYCTRVGGLSYTMDYWGQGTLVTVSSASTKGPS VFPLAPSSKSTSGGTAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSVFLFPPKPKDTLMISRT PEVTCVVVDVSHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFYPSDIAVEWESN GQPENNYKTTPPVLDSDGSFFLYSKLTVDKSRWQQGNVFSCSVMHEALHNHYTQKSLSLSPGK |
| 25 | Humanized antibody heavy chain variable region 29HH1-4 | QVQLVQSGAEVKKPGASVKVSCKASGYTFTTHYMYWVRQAPGQGLEWMGGINPSNGATNFNE KFKTRVTMTVDKSTSTAYMELSSLRSEDTAVYYCTRVGGLSYTMDYWGQGTLVTVSS |
| 26 | Humanized antibody heavy chain 29HH2-3 | QVQLVQSGAEVKKPGASVKVSCKASGYTFTTHYMYWVRQAPGQGLEWIGGINPSNGATNFNEK FKTRVTLTVDKSISTAYMELSRLRSDDTAVYYCTRVGGLSYTMDYWGQGTLVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKC KVSNKALPAPIEKTISKAKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYTQKSLSLSPGK |
| 27 | Humanized antibody heavy chain variable region 29HH2-3 | QVQLVQSGAEVKKPGASVKVSCKASGYTFTTHYMYWVRQAPGQGLEWIGGINPSNGATNFNEK FKTRVTLTVDKSISTAYMELSRLRSDDTAVYYCTRVGGLSYTMDYWGQGTLVTVSS |
| 28 | Humanized antibody heavy chain 29HH2-4 | QVQLVQSGAEVKKPGASVKVSCKASGYTFTTHYMYWVKQAPGQGLEWIGGINPSNGATNFNEK FKTKATLTVDKSISTAYMELSRLRSDDTAVYYCTRVGGLSYTMDYWGQGTLVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKC KVSNKALPAPIEKTISKAKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYTQKSLSLSPGK |
| 29 | Humanized antibody heavy chain variable region 29HH2-4 | QVQLVQSGAEVKKPGASVKVSCKASGYTFTTHYMYWVKQAPGQGLEWIGGINPSNGATNFNEK FKTKATLTVDKSISTAYMELSRLRSDDTAVYYCTRVGGLSYTMDYWGQGTLVTVSS |
| 30 | Bispecific antibody HC1 (Fc-Rhole) | EPKSSDKTHTCPPCPAPEAAGGPSVFLFPPKPKDTLMISRTPEVTCVVVAVSHEDPEVKFNWYVD GVEVHNAKTKPREEQYNSTYRVVSVLTVLHQDWLNGKEYKCKVSNKALAAPIEKTISKAKGQP REPQVCTLPPSRDELTKNQVSLSCAVKGFYPSDIAVEWESNGQPENNYKTTPPVLDSDGSFFLVSK LTVDKSRWQQGNVFSCSVMHEALHNHYTQKSLSLSPGK |
| 31 | Bispecific antibody HC2 (29H6-23L2 Rknob) | QVQLVQSGAEVKKPGASVKVSCKASGYTFTTHYMYWVRQAPGQGLEWIGGINPSNGATNFNEK FKTKATLTVDKSTSTAYMELSSLRSEDTAVYYCTRVGGLSYTMDYWGQGTLVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCEVQLVESGGGLVQPGGSLKLSCAASGFTFSTYAMNWV RQAPGKGLEWVGRIRSKYNNYATYYADSVKDRFTISRDDSKNTLYLQMNSLKTEDTAVYYCTRH GNFGNSYVSWFAYWGQGTTVTVSSGGGGSGGGGSGGGGSQAVVTQEPSLTVSPGGTVTLTCRSS TGAVTTSNYANWVQQKPGQAPRGLIGGTNKRAPGVPARFSGSLLGGKAALTLSGAQPEDEAEYF CALWYSNLWVFGGGTKLTVLGEPKSSDKTHTCPPCPAPEAAGGPSVFLFPPKPKDTLMISRTPEVT CVVVAVSHEDPEVKFNWYVDGVEVHNAKTKPREEQYNSTYRVVSVLTVLHQDWLNGKEYKCK VSNKALAAPIEKTISKAKGQPREPQVYTLPPCRDELTKNQVSLWCLVKGFYPSDIAVEWESNGQP ENNYKTTPPVLDSDGSFFLYSKLTVDKSRWQQGNVFSCSVMHEALHNHYTQKSLSLSPGK |
| 32 | Bispecific antibody heavy chain HC1 (29H6NS-Rhole) (N55S) | QVQLVQSGAEVKKPGASVKVSCKASGYTFTTHYMYWVRQAPGQGLEWIGGINPSSGATNFNEK FKTKATLTVDKSTSTAYMELSSLRSEDTAVYYCTRVGGLSYTMDYWGQGTLVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPEAAGGPSVFLFPPKPKDTLMISRTPEV TCVVVAVSHEDPEVKFNWYVDGVEVHNAKTKPREEQYNSTYRVVSVLTVLHQDWLNGKEYKC KVSNKALAAPIEKTISKAKGQPREPQVCTLPPSRDELTKNQVSLSCAVKGFYPSDIAVEWESNGQP ENNYKTTPPVLDSDGSFFLVSKLTVDKSRWQQGNVFSCSVMHEALHNHYTQKSLSLSPGK |
| 33 | Bispecific antibody HC1 (29H6-Rhole) | QVQLVQSGAEVKKPGASVKVSCKASGYTFTTHYMYWVRQAPGQGLEWIGGINPSNGATNFNEK FKTKATLTVDKSTSTAYMELSSLRSEDTAVYYCTRVGGLSYTMDYWGQGTLVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPEAAGGPSVFLFPPKPKDTLMISRTPEV TCVVVAVSHEDPEVKFNWYVDGVEVHNAKTKPREEQYNSTYRVVSVLTVLHQDWLNGKEYKC KVSNKALAAPIEKTISKAKGQPREPQVCTLPPSRDELTKNQVSLSCAVKGFYPSDIAVEWESNGQP ENNYKTTPPVLDSDGSFFLVSKLTVDKSRWQQGNVFSCSVMHEALHNHYTQKSLSLSPGK |
| 34 | Bispecific antibody HC2 (29H6NS-23L2 Rknob) (N55S) | QVQLVQSGAEVKKPGASVKVSCKASGYTFTTHYMYWVRQAPGQGLEWIGGINPSSGATNFNEK FKTKATLTVDKSTSTAYMELSSLRSEDTAVYYCTRVGGLSYTMDYWGQGTLVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCEVQLVESGGGLVQPGGSLKLSCAASGFTFSTYAMNWV RQAPGKGLEWVGRIRSKYNNYATYYADSVKDRFTISRDDSKNTLYLQMNSLKTEDTAVYYCTRH GNFGNSYVSWFAYWGQGTTVTVSSGGGGSGGGGSGGGGSQAVVTQEPSLTVSPGGTVTLTCRSS TGAVTTSNYANWVQQKPGQAPRGLIGGTNKRAPGVPARFSGSLLGGKAALTLSGAQPEDEAEYF CALWYSNLWVFGGGTKLTVLGEPKSSDKTHTCPPCPAPEAAGGPSVFLFPPKPKDTLMISRTPEVT CVVVAVSHEDPEVKFNWYVDGVEVHNAKTKPREEQYNSTYRVVSVLTVLHQDWLNGKEYKCK VSNKALAAPIEKTISKAKGQPREPQVYTLPPCRDELTKNQVSLWCLVKGFYPSDIAVEWESNGQP ENNYKTTPPVLDSDGSFFLYSKLTVDKSRWQQGNVFSCSVMHEALHNHYTQKSLSLSPGK |
| 35 | Bispecific antibody HC2 (29H6GE-23L2 Rknob) | QVQLVQSGAEVKKPGASVKVSCKASGYTFTTHYMYWVRQAPGQGLEWIGGINPSNEATNFNEK FKTKATLTVDKSTSTAYMELSSLRSEDTAVYYCTRVGGLSYTMDYWGQGTLVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCEVQLVESGGGLVQPGGSLKLSCAASGFTFSTYAMNWV RQAPGKGLEWVGRIRSKYNNYATYYADSVKDRFTISRDDSKNTLYLQMNSLKTEDTAVYYCTRH GNFGNSYVSWFAYWGQGTTVTVSSGGGGSGGGGSGGGGSQAVVTQEPSLTVSPGGTVTLTCRSS TGAVTTSNYANWVQQKPGQAPRGLIGGTNKRAPGVPARFSGSLLGGKAALTLSGAQPEDEAEYF CALWYSNLWVFGGGTKLTVLGEPKSSDKTHTCPPCPAPEAAGGPSVFLFPPKPKDTLMISRTPEVT CVVVAVSHEDPEVKFNWYVDGVEVHNAKTKPREEQYNSTYRVVSVLTVLHQDWLNGKEYKCK VSNKALAAPIEKTISKAKGQPREPQVYTLPPCRDELTKNQVSLWCLVKGFYPSDIAVEWESNGQP ENNYKTTPPVLDSDGSFFLYSKLTVDKSRWQQGNVFSCSVMHEALHNHYTQKSLSLSPGK (G56E) |
| 36 | Bispecific antibody heavy chain HC1 (29H6GE-Rhole) (G56E) | QVQLVQSGAEVKKPGASVKVSCKASGYTFTTHYMYWVRQAPGQGLEWIGGINPSNEATNFNEK FKTKATLTVDKSTSTAYMELSSLRSEDTAVYYCTRVGGLSYTMDYWGQGTLVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPEAAGGPSVFLFPPKPKDTLMISRTPEV TCVVVAVSHEDPEVKFNWYVDGVEVHNAKTKPREEQYNSTYRVVSVLTVLHQDWLNGKEYKC KVSNKALAAPIEKTISKAKGQPREPQVCTLPPSRDELTKNQVSLSCAVKGFYPSDIAVEWESNGQP ENNYKTTPPVLDSDGSFFLVSKLTVDKSRWQQGNVFSCSVMHEALHNHYTQKSLSLSPGK |
| 37 | Bispecific antibody heavy chain HC2 (23L2Scfv-Rknob) | EVQLVESGGGLVQPGGSLKLSCAASGFTFSTYAMNWVRQAPGKGLEWVGRIRSKYNNYATYYA DSVKDRFTISRDDSKNTLYLQMNSLKTEDTAVYYCTRHGNFGNSYVSWFAYWGQGTTVTVSSG GGGSGGGGSGGGGSQAVVTQEPSLTVSPGGTVTLTCRSSTGAVTTSNYANWVQQKPGQAPRGLI GGTNKRAPGVPARFSGSLLGGKAALTLSGAQPEDEAEYFCALWYSNLWVFGGGTKLTVLGEPKS SDKTHTCPPCPAPEAAGGPSVFLFPPKPKDTLMISRTPEVTCVVVAVSHEDPEVKFNWYVDGVEV HNAKTKPREEQYNSTYRVVSVLTVLHQDWLNGKEYKCKVSNKALAAPIEKTISKAKGQPREPQV YTLPPCRDELTKNQVSLWCLVKGFYPSDIAVEWESNGQPENNYKTTPPVLDSDGSFFLYSKLTVD KSRWQQGNVFSCSVMHEALHNHYTQKSLSLSPGK |
| 38 | Bispecific antibody heavy chain variable region 29H6NS VH (N55S) | QVQLVQSGAEVKKPGASVKVSCKASGYTFTTHYMYWVRQAPGQGLEWIGGINPSSGATNFNEK FKTKATLTVDKSTSTAYMELSSLRSEDTAVYYCTRVGGLSYTMDYWGQGTLVTVSS |
| 39 | Bispecific antibody heavy chain variable region HCDR2-NS | GINPSSGATNFNEKFKT |
| 40 | Bispecific antibody heavy chain variable region 29H6GE VH | QVQLVQSGAEVKKPGASVKVSCKASGYTFTTHYMYWVRQAPGQGLEWIGGINPSNEATNFNEK FKTKATLTVDKSTSTAYMELSSLRSEDTAVYYCTRVGGLSYTMDYWGQGTLVTVSS (G56E) |
| 41 | Bispecific antibody heavy chain variable region 29H6GE VH-HCDR2 | GINP SNEATNFNEKFKT |
| 42 | Bispecific antibody Fc-hole (CH2-CH3hole) | DKTHTCPPCPAPEAAGGPSVFLFPPKPKDTLMISRTPEVTCVVVAVSHEDPEVKFNWYVDGVEVH NAKTKPREEQYNSTYRVVSVLTVLHQDWLNGKEYKCKVSNKALAAPIEKTISKAKGQPREPQVC TLPPSRDELTKNQVSLSCAVKGFYPSDIAVEWESNGQPENNYKTTPPVLDSDGSFFLVSKLTVDKS RWQQGNVFSCSVMHEALHNHYTQKSLSLSPGK |
| 43 | Bispecific antibody Fc-knob (hinge region-CH2-CH3knob) | EPKSSDKTHTCPPCPAPEAAGGPSVFLFPPKPKDTLMISRTPEVTCVVVAVSHEDPEVKFNWYVD GVEVHNAKTKPREEQYNSTYRVVSVLTVLHQDWLNGKEYKCKVSNKALAAPIEKTISKAKGQP REPQVYTLPPCRDELTKNQVSLWCLVKGFYPSDIAVEWESNGQPENNYKTTPPVLDSDGSFFLYS KLTVDKSRWQQGNVFSCSVMHEALHNHYTQKSLSLSPGK |
| 44 | CH1 | ASTKGPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSC |
| 45 | IgG1 constant region (LALA mutation + D265A + P329A) (the underlined part indicates the sequence of the constant region Fc) | ASTKGPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGGPSVFLFPPKPK DTLMISRTPEVTCVVVAVSHEDPEVKFNWYVDGVEVHNAKTKPREEQYNSTYRVVSVLTVLHQD WLNGKEYKCKVSNKALAAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEALHNHYTQKS LSLSPGK |
| 46 | Fc (without hinge region, CH2-CH3 + LALA mutation + D265A + P329A) | DKTHTCPPCPAPEAAGGPSVFLFPPKPKDTLMISRTPEVTCVVVAVSHEDPEVKFNWYVDGVEVH NAKTKPREEQYNSTYRVVSVLTVLHQDWLNGKEYKCKVSNKALAAPIEKTISKAKGQPREPQVY TLPPSRDELTKNQVSLTCLVKGFYPSDIAVEWESNGQPENNYKTTPPVLDSDGSFFLYSKLTVDKS RWQQGNVFSCSVMHEALHNHYTQKSLSLSPGK |
| 47 | Lambda light chain constant region | GQPKAAPSVTLFPPSSEELQANKATLVCLISDFYPGAVTVAWKADSSPVKAGVETTTPSKQSNNK YAASSYLSLTPEQWKSHRSYSCQVTHEGSTVEKTVAPTEC |
| 48 | IgG1 constant region (without mutations) (the underlined part indicates the sequence of the constant region Fc) | |
| 49 | Fc (without hinge region, without mutations, CH2-CH3) | DKTHTCPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSKLTVDKS RWQQGNVFSCSVMHEALHNHYTQKSLSLSPGK |
| 50 | Kappa light chain constant region | |
| 51 | Roche-CD3 HC (the sequence of the heavy chain variable region is underlined) | EVQLLESGGGLVQPGGSLRLSCAASGFTFSTYAMNWVRQAPGKGLEWVSRIRSKYNNYATYYAD SVKGRFTISRDDSKNTLYLQMNSLRAEDTAVYYCVRHGNFGNSYVSWFAYWGQGTLVTVSSAST KGPSVFPLAPSSKSTSGGTAALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEAAGGPSVFLFPPKPKDTL MISRTPEVTCVVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVKGFYPSDIAVE WESNGQPENNYKTTPPVLDSDGSFFLYSKLTVDKSRWQQGNVFSCSVMHEALHNHYTQKSLSLS PGK |
| 52 | Roche-CD3 LC | QAVVTQEPSLTVSPGGTVTLTCGSSTGAVTTSNYANWVQEKPGQAFRGLIGGTNKRAPGTPARFS GSLLGGKAALTLSGAQPEDEAEYYCALWYSNLWVFGGGTKLTVLGQPKAAPSVTLFPPSSEELQ ANKATLVCLISDFYPGAVTVAWKADSSPVKAGVETTTPSKQSNNKYAASSYLSLTPEQWKSHRSY SCQVTHEGSTVEKTVAPTEC (the light chain variable region is underlined) |
| 53 | CD3B219 HC | EVQLVESGGGLVQPGGSLRLSCAASGFTFNTYAMNWVRQAPGKGLEWVARIRSKYNNYATYYA ASVKGRFTISRDDSKNSLYLQMNSLKTEDTAVYYCARHGNFGNSYVSWFAYWGQGTLVTVSSAS TKGPSVFPLAPCSRSTSESTAALGCLVKDYFPEPVTVSWNSGALTSGVHTFPAVLQSSGLYSLSSV VTVPSSSLGTKTYTCNVDHKPSNTKVDKRVESKYGPPCPPCPAPEAAGGPSVFLFPPKPKDTLMIS RTPEVTCVVVDVSQEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSLTCLVKGFYPSDIAVEWE SNGQPENNYKTTPPVLDSDGSFLLYSKLTVDKSRWQEGNVFSCSVMHEALHNHYTQKSLSLSLG K |
| 54 | CD3B219 LC | QTVVTQEPSLTVSPGGTVTLTCRSSTGAVTTSNYANWVQQKPGQAPRGLIGGTNKRAPGTPARFS GSLLGGKAALTLSGVQPEDEAEYYCALWYSNLWVFGGGTKLTVLGQPKAAPSVTLFPPSSEELQ ANKATLVCLISDFYPGAVTVAWKADSSPVKAGVETTTPSKQSNNKYAASSYLSLTPEQWKSHRSY SCQVTHEGSTVEKTVAPTEC |
| 55 | GC5B596 HC | QVQLVQSGAEVKKPGASVKVSCKASGYSFTGYTMNWVRQAPGQGLEWMGLINPYNSDTNYAQ KLQGRVTMTTDTSTSTAYMELRSLRSDDTAVYYCARVALRVALDYWGQGTLVTVSSASTKGPSV FPLAPCSRSTSESTAALGCLVKDYFPEPVTVSWNSGALTSGVHTFPAVLQSSGLYSLSSVVTVPSSS LGTKTYTCNVDHKPSNTKVDKRVESKYGPPCPPCPAPEAAGGPSVFLFPPKPKDTLMISRTPEVTC VVVDVSQEDPEVQFNWYVDGVEVHNAKTKPREEQFNSTYRVVSVLTVLHQDWLNGKEYKCKV SNKGLPSSIEKTISKAKGQPREPQVYTLPPSQEEMTKNQVSLTCLVKGFYPSDIAVEWESNGQPEN NYKTTPPVLDSDGSFFLYSRLTVDKSRWQEGNVFSCSVMHEALHNHYTQKSLSLSLGK |
| 56 | GC5B596 LC | DIQMTQSPSSLSASVGDRVTITCKASQNVATHVGWYQQKPGKAPKRLIYSASYRYSGVPSRFSGS GSGTEFTLTISNLQPEDFATYYCQQYNRYPYTFGQGTKLEIKRTVAAPSVFIFPPSDEQLKSGTASV VCLLNNFYPREAKVQWKVDNALQSGNSQESVTEQDSKDSTYSLSSTLTLSKADYEKHKVYACE VTHQGLSSPVTKSFNRGEC |
| 57 | HuGPRC5D | MYKDCIESTGDYFLLCDAEGPWGIILESLAILGIVVTILLLLAFLFLMRKIQDCSQWNVLPTQLLFL LSVLGLFGLAFAFIIELNQQTAPVRYFLFGVLFALCFSCLLAHASNLVKLVRGCVSFSWTTILCIAIG CSLLQIIIATEYVTLIMTRGMMFVNMTPCQLNVDFVVLLVYVLFLMALTFFVSKATFCGPCENWK QHGRLIFITVLFSIIIWVVWISMLLRGNPQFQRQPQWDDPVVCIALVTNAWVFLLLYIVPELCILYR SCRQECPLQGNACPVTAYQHSFQVENQELSRARDSDGAEEDVALTSYGTPIQPQTVDPTQECFIPQ AKLSPQQDAGGV |
| 58 | CynoGPRC5D | MYKDCIESTGDYFLPCDSEGPWGIILESLAILGIVVTILLLLAFLFLMRKIQDCSQWNVLPTQLLFL LSVLGLFGLAFAFIIQLNQQTAPVRYFLFGVLFALCFSCLLAHASNLVKLVRGRVSFSWTTILCIAIG CSLLQVIIAIEYVTLIMTRGMMFVHMTPYQLNVDFVVLLVYVLFLMALTFFVSKATFCGPCENWK QHGRLIFITVLFSIIIWVVWISMLLRGNPQFQRQPQWDDPVVCIALVTNAWVFLLLYIVPELCILYR SCRQECPSQGHACPVTAYQRSFQVENQELSRARDSDGAEEDVALTSFGTPIQPQTVDPTQECFIPR AKLSPQQDAGV |
| 59 | Fc-hole (hinge region-CH2-CH3 hole) | EPKSCDKTHTCPPCPAPEAAGGPSVFLFPPKPKDTLMISRTPEVTCVVVAVSHEDPEVKFNWYVD GVEVHNAKTKPREEQYNSTYRVVSVLTVLHQDWLNGKEYKCKVSNKALAAPIEKTISKAKGQP REPQVCTLPPSRDELTKNQVSLSCAVKGFYPSDIAVEWESNGQPENNYKTTPPVLDSDGSFFLVSK LTVDKSRWQQGNVFSCSVMHEALHNHYTQKSLSLSPGK |
| 60 | Chimeric antibody 29H12C9 heavy chain | QVQLQQPGAELVKPGASVKLSCKASGYTFTTHYMYWVKQRPGQGLEWIGGINPSNGATNFNEK FKTKATLTVDKSSSTAYMQLSSLTSEDSAVYYCTRVGGLSYTMDYWGQGTSVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKC KVSNKALPAPIEKTISKAKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYTQKSLSLSPGK |
| 61 | Chimeric antibody 29H12C9 light chain | DIVMSQSPSSLAVSVGEKVSMSCKSSQSLLYSSNQKNYLAWYQQKPGQSPKLLIYWASTRESGVP DRFTGSGYGTDFTLTISSMKAEDLAVYYCQQYYSYPRTFGGGTKLDIKRTVAAPSVFIFPPSDEQL KSGTASVVCLLNNFYPREAKVQWKVDNALQSGNSQESVTEQDSKDSTYSLSSTLTLSKADYEKH KVYACEVTHQGLSSPVTKSFNRGEC |
| 62 | Chimeric antibody 29H12C9 heavy chain variable region | QVQLQQPGAELVKPGASVKLSCKASGYTFTTHYMYWVKQRPGQGLEWIGGINPSNGATNFNEK FKTKATLTVDKSSSTAYMQLSSLTSEDSAVYYCTRVGGLSYTMDYWGQGTSVTVSS |
| 63 | Chimeric antibody 29H12C9 light chain variable region | DIVMSQSPSSLAVSVGEKVSMSCKSSQSLLYSSNQKNYLAWYQQKPGQSPKLLIYWASTRESGVP DRFTGSGYGTDFTLTISSMKAEDLAVYYCQQYYSYPRTFGGGTKLDIK |
| 64 | Chimeric antibody 29H12C9 heavy chain variable region HCDR1 | THYMY |
| 65 | Chimeric antibody 29H12C9 heavy chain variable region HCDR2 | GINPSNGATNFNEKFKT |
| 66 | Chimeric antibody 29H12C9 heavy chain variable region HCDR3 | VGGLSYTMDY |
| 67 | Chimeric antibody 29H12C9 light chain variable region LCDR1 | KSSQSLLYS SNQKNYLA |
| 68 | Chimeric antibody 29H12C9 light chain variable region LCDR2 | WASTRES |
| 69 | Chimeric antibody 29H12C9 light chain variable region LCDR3 | QQYYSYPRT |
| 70 | Chimeric antibody 4B9E9 heavy chain | DVQLVESGGGLVQPRGSRKLSCAASGFTFSSFGMHWVRQAPEKGLEWVAYISSDSNTIYYADTV QGRFTISRDNPKNTLFLQMTRLRSEDTAMYYCARNPWYFDVWGAGTTVTVSSASTKGPSVFPLA PSSKSTSGGTAALGCLVKDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHYTQKSLSLSPGK |
| 71 | Chimeric antibody 4B9E9 light chain | DVLMTQTPLSLPVSLGDQVSISCRSSQSIVHSNGNTYLEWFLQKPGQSPKLLIYKVSNRFSGVPDR FSGSGSGTDFTLKISRVEAEDLGIYYCFQGSHIPPTFGGGTKLEIKRTVAAPSVFIFPPSDEQLKSGT ASVVCLLNNFYPREAKVQWKVDNALQSGNSQESVTEQDSKDSTYSLSSTLTLSKADYEKHKVY ACEVTHQGLSSPVTKSFNRGEC |
| 72 | Chimeric antibody 4B9E9 heavy chain variable region | DVQLVESGGGLVQPRGSRKLSCAASGFTFSSFGMHWVRQAPEKGLEWVAYISSDSNTIYYADTV QGRFTISRDNPKNTLFLQMTRLRSEDTAMYYCARNPWYFDVWGAGTTVTVSS |
| 73 | Chimeric antibody 4B9E9 light chain variable region | DVLMTQTPLSLPVSLGDQVSISCRSSQSIVHSNGNTYLEWFLQKPGQSPKLLIYKVSNRFSGVPDR FSGSGSGTDFTLKISRVEAEDLGIYYCFQGSHIPPTFGGGTKLEIK |
| 74 | Chimeric antibody **4B9E9** heavy chain variable region HCDR1 | SFGMH |
| 75 | Chimeric antibody 4B9E9 heavy chain variable region HCDR2 | YISSDSNTIYYADTVQG |
| 76 | Chimeric antibody 4B9E9 heavy chain variable region HCDR3 | NPWYFDV |
| 77 | Chimeric antibody 4B9E9 light chain variable region LCDR1 | RSSQSIVHSNGNTYLE |
| 78 | Light chain variable region LCDR2 of chimeric antibodies 4B9E9 and 39A9F1 | KVSNRFS |
| 79 | Chimeric antibody 4B9E9 light chain variable region LCDR3 | FQGSHIPPT |
| 80 | Chimeric antibody 39A9F1 heavy chain | QVQLQQPGAELVKPGSSVKLSCKASGYTFTTYWMEWVKQRPGQGLEWIGEINPNNGRSNYNEK FQTKATLTVDKSSSTAYMQFSSLTSEDSAVYYCARERAGLTYWGQGTLVIVSAASTKGPSVFPLAP SSKSTSGGTAALGCLVKDYFPEPVTVSWNSGALTSGVHTFPAVLQSSGLYSLSSVVTVPSSSLGTQ TYICNVNHKPSNTKVDKKVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQKSLSLSPGK |
| 81 | Chimeric antibody 39A9F1 light chain | DVVMTQTPLSLPVSLGDQASISCRSSQSLVHSNGNTYLHWYLQKPGQSPKLLIYKVSNRFSGVPD RFGGSGSGTDFTLKISRVEAEDLGVYFCSQSTHFPYTFGGGTKLEIKRTVAAPSVFIFPPSDEQLKS GTASVVCLLNNFYPREAKVQWKVDNALQSGNSQESVTEQDSKDSTYSLSSTLTLSKADYEKHKV YACEVTHQGLSSPVTKSFNRGEC |
| 82 | Chimeric antibody 39A9F1 heavy chain variable region | QVQLQQPGAELVKPGSSVKLSCKASGYTFTTYWMEWVKQRPGQGLEWIGEINPNNGRSNYNEK FQTKATLTVDKSSSTAYMQFSSLTSEDSAVYYCARERAGLTYWGQGTLVIVSA |
| 83 | Chimeric antibody 39A9F1 light chain variable region | DVVMTQTPLSLPVSLGDQASISCRSSQSLVHSNGNTYLHWYLQKPGQSPKLLIYKVSNRFSGVPD RFGGSGSGTDFTLKISRVEAEDLGVYFCSQSTHFPYTFGGGTKLEIK |
| 84 | Chimeric antibody **39A9F1** heavy chain variable region HCDR1 | TYWME |
| 85 | Chimeric antibody 39A9F1 heavy chain variable region HCDR2 | EINPNNGRSNYNEKFQT |
| 86 | Chimeric antibody 39A9F1 heavy chain variable region HCDR3 | ERAGLTY |
| 87 | Chimeric antibody 39A9F1 light chain variable region LCDR1 | RSSQSLVHSNGNTYLH |
| 88 | Chimeric antibody 39A9F1 light chain variable region LCDR3 | SQSTHFPYT |
| 89 | Bispecific antibody Fc-knob (CH2-CH3knob) | DKTHTCPPCPAPEAAGGPSVFLFPPKPKDTLMISRTPEVTCVVVAVSHEDPEVKFNWYVDGVEVH NAKTKPREEQYNSTYRVVSVLTVLHQDWLNGKEYKCKVSNKALAAPIEKTISKAKGQPREPQVY TLPPCRDELTKNQVSLWCLVKGFYPSDIAVEWESNGQPENNYKTTPPVLDSDGSFFLYSKLTVDK SRWQQGNVFSCSVMHEALHNHYTQKSLSLSPGK |
| 90 | sp34 VH | EVQLVESGGGLVQPKGSLKLSCAASGFTFNTYAMNWVRQAPGKGLEWVARIRSKYNNYATYYA DSVKDRFTISRDDSQSILYLQMNNLKTEDTAMYYCVRHGNFGNSYVSWFAYWGQGTLVTVSS |
| 91 | sp34 VL | QAVVTQESALTTSPGETVTLTCRSSTGAVTTSNYANWVQEKPDHLFTGLIGGTNKRAPGVPARFS GSLIGDKAALTITGAQTEDEAIYFCALWYSNLWVFGGGTKLTVL |
| 92 | 776.1 VH | EVQLQQSGPELVKPGASVKISCKASGYTFTDYNIHWVKQSHGKILEWIGYIYPYNGVSDYNQNF KSKATLIVDNSSNTAYMELRSLTSEDSAVYYCARWDFGSGYYFDYWGQGTTLTVSS |
| 93 | 776.1 VH HCDR1 | DYNIH |
| 94 | 776.1 VH HCDR2 | YIYPYNGVSDYNQNFKS |
| 95 | 776.1 VH HCDR3 | WDFGSGYYFDY |
| 96 | 776.1 VL | QIVLSQSPAILFASPGETVTMTCRASSSVIYMCWNQQKPGSSPKPWIYGTSTLASGVPTRFSGSGS GTSYSLTISRVEAEDAATYYCQQWSSNPFTFGSGTKLEIK |
| 97 | 776.1 VL LCDR1 | RASSSVIYMC |
| 98 | 776.1 VL LCDR2 | GTSTLAS |
| 99 | 776.1 VL LCDR3 | QQWSSNPFT |
| 100 | 776.1 VL(C33A) | QIVLSQSPAILFASPGETVTMTCRASSSVIYMAWNQQKPGSSPKPWIYGTSTLASGVPTRFSGSGS GTSYSLTISRVEAEDAATYYCQQWSSNPFTFGSGTKLEI |
| 101 | 776.1 VL(C33A) LCDR1 | RASSSVIYMA |
| 102 | 776.1 VL(C33S) | QIVLSQSPAILFASPGETVTMTCRASSSVIYMSWNQQKPGSSPKPWIYGTSTLASGVPTRFSGSGSG TSYSLTISRVEAEDAATYYCQQWSSNPFTFGSGTKLEIK |
| 103 | 776.1 VL(C33 S) LCDR1 | RASSSVIYMS |
| 104 | sp34 HC | EVQLVESGGGLVQPKGSLKLSCAASGFTFNTYAMNWVRQAPGKGLEWVARIRSKYNNYATYYA DSVKDRFTISRDDSQSILYLQMNNLKTEDTAMYYCVRHGNFGNSYVSWFAYWGQGTLVTVSSAS TKGPSVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPEAAGGPSVFLFPPKPKDTL MISRTPEVTCVVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVKGFYPSDIAVE WESNGQPENNYKTTPPVLDSDGSFFLYSKLTVDKSRWQQGNVFSCSVMHEALHNHYTQKSLSLS PGK |
| 105 | sp34 LC | QAVVTQESALTTSPGETVTLTCRSSTGAVTTSNYANWVQEKPDHLFTGLIGGTNKRAPGVPARFS GSLIGDKAALTITGAQTEDEAIYFCALWYSNLWVFGGGTKLTVLGQPKAAPSVTLFPPSSEELQA NKATLVCLISDFYPGAVTVAWKADSSPVKAGVETTTPSKQSNNKYAASSYLSLTPEQWKSHRSYS CQVTHEGSTVEKTVAPTEC |
| 106 | 776.1 HC | EVQLQQSGPELVKPGASVKISCKASGYTFTDYNIHWVKQSHGKILEWIGYIYPYNGVSDYNQNF KSKATLIVDNSSNTAYMELRSLTSEDSAVYYCARWDFGSGYYFDYWGQGTTLTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPEAAGGPSVFLFPPKPKDTLMISRTPEV TCVVVAVSHEDPEVKFNWYVDGVEVHNAKTKPREEQYNSTYRVVSVLTVLHQDWLNGKEYKC KVSNKALAAPIEKTISKAKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYTQKSLSLSPGK |
| 107 | 776.1 LC | QIVLSQSPAILFASPGETVTMTCRASSSVIYMCWNQQKPGSSPKPWIYGTSTLASGVPTRFSGSGS GTSYSLTISRVEAEDAATYYCQQWSSNPFTFGSGTKLEIKRTVAAPSVFIFPPSDEQLKSGTASVVC LLNNFYPREAKVQWKVDNALQSGNSQESVTEQDSKDSTYSLSSTLTLSKADYEKHKVYACEVT HQGLSSPVTKSFNRGEC |
| 108 | 776.1 LC(C33A) | QIVLSQSPAILFASPGETVTMTCRASSSVIYMAWNQQKPGSSPKPWIYGTSTLASGVPTRFSGSGS GTSYSLTISRVEAEDAATYYCQQWSSNPFTFGSGTKLEIKRTVAAPSVFIFPPSDEQLKSGTASVVC LLNNFYPREAKVQWKVDNALQSGNSQESVTEQDSKDSTYSLSSTLTLSKADYEKHKVYACEVT HQGLSSPVTKSFNRGEC |
| 109 | 776.1 LC(C33S) | QIVLSQSPAILFASPGETVTMTCRASSSVIYMSWNQQKPGSSPKPWIYGTSTLASGVPTRFSGSGSG TSYSLTISRVEAEDAATYYCQQWSSNPFTFGSGTKLEIKRTVAAPSVFIFPPSDEQLKSGTASVVCL LNNFYPREAKVQWKVDNALQSGNSQESVTEQDSKDSTYSLSSTLTLSKADYEKHKVYACEVTH QGLSSPVTKSFNRGEC |
| 110 | Human MUC16-His | GSRKFNTTERVLQGLLRPLFKNTSVGPLYSGCRLTLLRPEKDGEATGVDAICTHRPDPTGPGLDRE QLYLELSQLTHSITELGPYTLDRDSLYVNGFTHRSSVPTTSTGVVSEEPFTLNFTINNLRYMADMG QPGSLKFNITDNVMQHLLSPLFQRSSLGARYTGCRVIALRSVKNGAETRVDLLCTYLQPLSGPGLP IKQVFHELSQQTHGITRLGPYSLDKDSLYLNGYNEPGPDEPPTTPKPATTFLPPLSEATTAMGYHLK TLTLNFTISNLQYSPDMGKGSATFNSTEGVLQHLLRPLFQKSSMGPFYLGCQLISLRPEKDGAATG VDTTCTYHPDPVGPGLDIQQLYWELSQLTHGVTQLGFYVLDRDSLFINGYAPQNLSIRGEYQINF HIVNWNLSNPDPTSSEYITLLRDIQDKVTTLYKGSQLHDTFRFCLVTNLTMDSVLVTVKALFSSNL DPSLVEQVFLDKTLNASFHWLGSTYQLVDIHVTEMESSVYQPTSSSSTQHFYLNFTITNLPYSQDK AQPGTTNYQRNKRNIEDALNQLFRNSSIKSYFSDCQVSTFRSVPNRHHTGVDSLCNFSPLARRVD RVAIYEEFLRMTRNGTQLQNFTLDRSSVLVDGYSPNRNEPLTGNSDLPHHHHHH |
| 111 | Rhesus monkey MUC16-His | GSRKFNTTERVLQGLLRPLFKNTSVGPLYSGCRLTLLRPEKDGEATGVDVICTHRPDPTGPGLDRE QLYLELSQLTHSITELGPYTLDNDSLFVNGFTHRSSVPTTSTGMVSEEPFTLNFTINNLRYMADMG QPGSLKFNITDNVMQHLLSPLFQRSSVGARYTGCRVIALRSVKNGAKTQVDILCTYLQPLSGPALP IKQVFHELSQQTHGITRLGPYFLDKDSLYLNGYNEPGPDEPPTTPKPATTFLPPLSEATTAMGYHLK TLTLNFTISNLQYSPDMGNGSAAFNSTERVLKHLLRALFQKSSMGPFYLGCQLISLRPEKNGAATG VDTTCTYHTDPVGPGLDIQQLYWELSQLTHGVTQLGFYVLDRDSLFINGYAPQNLSIRGKYQIHF HIVNWNFSNPDPTSSEYIALLRDIQDKVTTLYKGSQLHDTFRYCLVTNLTMDSMLVTVKALFSSD LDPSLVEQVFLDKTLNASSHWLGSTYQLVDIHVTEMEPSVYQPTKPTSSSSTQHFYLNFTITNLPY SQDISQPSTTNYQRNKRNIEDALNQLFRNSSIKSYFSDCQVSTFRSVPNSHHTGVDSLCNFSPLAR RVDRVAIYEEFLRMTRNGSQLQNFTLDRSSVLVDGYSPNRNEPLTGNSDLPHHHHHH |
| 112 | Cynomolgus monkey MUC16-His | GSRKFNTTERVLQGLLRPLFKNTSVGPLYSGCRLTLLRPEKDGEATGVDVICTHRPDPTGPGLDRE QLYLELSQLTHSITELGPYTLDNDSLFVNGFTHRSSVPTTSTGMVSEEPFTLNFTINNLRYMADMG QPGSLKFNITDNVMQHLLSPLFQRSSVGARYTGCRVIALRSVKNGAKTQVDILCTYLQPLSGPGL PIKQVFHELSQQTHGITRLGPYFLDKDSLYLNGYNEPGPDEPPTTPKPATTFLPPLSEATTAMGYHL KTLTLNFTISNLQYSPDMGNGSAAFNSTERVLKHLLRALFQKSSMGPFYLGCQLISLRPEKNGAAT GVDTTCTYHTDPVGPGLDIQQLYWELSQLTHGVTQLGFYVLDRDSLFINGYAPQNLSIRGKYQIH FHIVNWNLGNPDPTSSEYIALLRDIQDKVTTLYKGSRLHDTFCYCLVTNLTMDSMLVTVKALFSS DLDPSLVEQVFLDKTLNASSHWLGSTYQLVDIHVTEMEPSVYQPTKPTSSSTQHFYLNFTITNLP YSQDISQPSTTNYQRNKRNIEDALNQLFRNSSIKSCFSDCQVSTFRSVPNSHHTGVDSLCNFSPLA RRVDRVAIYEEFLRMTRNGSQLQNFTLDRSSVLVDGYSPNRNEPLTGNSDLPHHHHHH |
| 113 | Heavy chain variable region (MCH7) | QVQLVQSGAEVKKPGASVKVSCKASGYTFTDYNIHWVRQAPGQGLEWMGYIYPYNGVSDYNQ NFKSRVTMTTDTSTSTAYMELRSLRSDDTAVYYCARWDFGSGYYFDYWGQGTLVTVSS |
| 114 | Light chain variable region (MCL4) | EIVLTQSPATLSLSPGERATLSCRASSSVIYMSWYQQKPGQAPRLLIYGTSTLASGIPARFSGSGSGT DFTLTISSLEPEDFAVYYCQQWSSNPFTFGQGTKLEIK |
| 115 | MCH7 HC | QVQLVQSGAEVKKPGASVKVSCKASGYTFTDYNIHWVRQAPGQGLEWMGYIYPYNGVSDYNQ NFKSRVTMTTDTSTSTAYMELRSLRSDDTAVYYCARWDFGSGYYFDYWGQGTLVTVSSASTKGP SVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSCDKTHTCPPCPAPEAAGGPSVFLFPPKPKDTLMISR TPEVTCVVVAVSHEDPEVKFNWYVDGVEVHNAKTKPREEQYNSTYRVVSVLTVLHQDWLNGKE YKCKVSNKALAAPIEKTISKAKGQPREPQVYTLPPSRDELTKNQVSLTCLVKGFYPSDIAVEWESN GQPENNYKTTPPVLDSDGSFFLYSKLTVDKSRWQQGNVFSCSVMHEALHNHYTQKSLSLSPGK |
| 116 | MCL4 LC | EIVLTQSPATLSLSPGERATLSCRASSSVIYMSWYQQKPGQAPRLLIYGTSTLASGIPARFSGSGSGT DFTLTISSLEPEDFAVYYCQQWSSNPFTFGQGTKLEIKRTVAAPSVFIFPPSDEQLKSGTASVVCLLN NFYPREAKVQWKVDNALQSGNSQESVTEQDSKDSTYSLSSTLTLSKADYEKHKVYACEVTHQG LSSPVTKSFNRGEC |
| 117 | Heavy chain 23L2ScFv-Hole | EVQLVESGGGLVQPGGSLKLSCAASGFTFSTYAMNWVRQAPGKGLEWVGRIRSKYNNYATYYA DSVKDRFTISRDDSKNTLYLQMNSLKTEDTAVYYCTRHGNFGNSYVSWFAYWGQGTTVTVSSG GGGSGGGGSGGGGSQAVVTQEPSLTVSPGGTVTLTCRSSTGAVTTSNYANWVQQKPGQAPRGLI GGTNKRAPGVPARFSGSLLGGKAALTLSGAQPEDEAEYFCALWYSNLWVFGGGTKLTVLGEPKS SDKTHTCPPCPAPEAAGGPSVFLFPPKPKDTLMISRTPEVTCVVVAVSHEDPEVKFNWYVDGVEV HNAKTKPREEQYNSTYRVVSVLTVLHQDWLNGKEYKCKVSNKALAAPIEKTISKAKGQPREPQV CTLPPSRDELTKNQVSLSCAVKGFYPSDIAVEWESNGQPENNYKTTPPVLDSDGSFFLVSKLTVDK SRWQQGNVFSCSVMHEALHNHYTQKSLSLSPGK |
| 118 | Heavy chain MCH7-KIH-Knob | QVQLVQSGAEVKKPGASVKVSCKASGYTFTDYNIHWVRQAPGQGLEWMGYIYPYNGVSDYNQ NFKSRVTMTTDTSTSTAYMELRSLRSDDTAVYYCARWDFGSGYYFDYWGQGTLVTVSSASTKGP SVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSCDKTHTCPPCPAPEAAGGPSVFLFPPKPKDTLMISR TPEVTCVVVAVSHEDPEVKFNWYVDGVEVHNAKTKPREEQYNSTYRVVSVLTVLHQDWLNGKE YKCKVSNKALAAPIEKTISKAKGQPREPQVYTLPPCRDELTKNQVSLWCLVKGFYPSDIAVEWES NGQPENNYKTTPPVLDSDGSFFLYSKLTVDKSRWQQGNVFSCSVMHEALHNHYTQKSLSLSPGK |
| 119 | Heavy chain 2MCH7-KIH-Knob | QVQLVQSGAEVKKPGASVKVSCKASGYTFTDYNIHWVRQAPGQGLEWMGYIYPYNGVSDYNQ NFKSRVTMTTDTSTSTAYMELRSLRSDDTAVYYCARWDFGSGYYFDYWGQGTLVTVSSASTKGP SVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSCQVQLVQSGAEVKKPGASVKVSCKASGYTFTDYN IHWVRQAPGQGLEWMGYIYPYNGVSDYNQNFKSRVTMTTDTSTSTAYMELRSLRSDDTAVYYC ARWDFGSGYYFDYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEPKSCDK THTCPPCPAPEAAGGPSVFLFPPKPKDTLMISRTPEVTCVVVAVSHEDPEVKFNWYVDGVEVHNA KTKPREEQYNSTYRVVSVLTVLHQDWLNGKEYKCKVSNKALAAPIEKTISKAKGQPREPQVYTL PPCRDELTKNQVSLWCLVKGFYPSDIAVEWESNGQPENNYKTTPPVLDSDGSFFLYSKLTVDKSR WQQGNVFSCSVMHEALHNHYTQKSLSLSPGK |
| 120 | ECT_TM cyMuc 16 | MALPVTALLLPLALLLHAARPGSRKFNTTERVLQGLLRPLFKNTSVGPLYSGCRLTLLRPEKDGE ATGVDVICTHRPDPTGPGLDREQLYLELSQLTHSITELGPYTLDNDSLFVNGFTHRSSVPTTSTGM VSEEPFTLNFTINNLRYMADMGQPGSLKFNITDNVMQHLLSPLFQRSSVGARYTGCRVIALRSVK NGAKTQVDILCTYLQPLSGPGLPIKQVFHELSQQTHGITRLGPYFLDKDSLYLNGYNEPGPDEPPT TPKPATTFLPPLSEATTAMGYHLKTLTLNFTISNLQYSPDMGNGSAAFNSTERVLKHLLRALFQKS SMGPFYLGCQLISLRPEKNGAATGVDTTCTYHTDPVGPGLDIQQLYWELSQLTHGVTQLGFYVL DRDSLFINGYAPQNLSIRGKYQIHFHIVNWNLGNPDPTSSEYIALLRDIQDKVTTLYKGSRLHDTF CYCLVTNLTMDSMLVTVKALFSSDLDPSLVEQVFLDKTLNASSHWLGSTYQLVDIHVTEMEPSV YQPTKPTSSSSTQHFYLNFTITNLPYSQDISQPSTTNYQRNKRNIEDALNQLFRNSSIKSCFSDCQV STFRSVPNSHHTGVDSLCNFSPLARRVDRVAIYEEFLRMTRNGSQLQNFTLDRSSVLVDGYSPNR NEPLTGNSDLPFWAIILICLAVLLGLITCLICGFLATTRWRKKEEYSFRQQCPGYYRSHLDLEDLQ |
| 121 | Heavy chain MCH7-23L2ScFv-Hole | QVQLVQSGAEVKKPGASVKVSCKASGYTFTDYNIHWVRQAPGQGLEWMGYIYPYNGVSDYNQ NFKSRVTMTTDTSTSTAYMELRSLRSDDTAVYYCARWDFGSGYYFDYWGQGTLVTVSSASTKGP SVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSCEVQLVESGGGLVQPGGSLKLSCAASGFTFSTYAM NWVRQAPGKGLEWVGRIRSKYNNYATYYADSVKDRFTISRDDSKNTLYLQMNSLKTEDTAVYY CTRHGNFGNSYVSWFAYWGQGTTVTVSSGGGGSGGGGSGGGGSQAVVTQEPSLTVSPGGTVTL TCRSSTGAVTTSNYANWVQQKPGQAPRGLIGGTNKRAPGVPARFSGSLLGGKAALTLSGAQPED EAEYFCALWYSNLWVFGGGTKLTVLGEPKSSDKTHTCPPCPAPEAAGGPSVFLFPPKPKDTLMIS RTPEVTCVVVAVSHEDPEVKFNWYVDGVEVHNAKTKPREEQYNSTYRVVSVLTVLHQDWLNGK EYKCKVSNKALAAPIEKTISKAKGQPREPQVCTLPPSRDELTKNQVSLSCAVKGFYPSDIAVEWES NGQPENNYKTTPPVLDSDGSFFLVSKLTVDKSRWQQGNVFSCSVMHEALHNHYTQKSLSLSPGK |
| 122 | Heavy chain REGN4018-8767pH | QVQLVESGGGLVKPGGSLRLSCAASGFTFSNYYMSWVRQAPGKGLEWISYISGRGSTIFYADSVK GRITISRDNAKNSLFLQMNSLRAEDTAVYFCVKDRGGYSPYWGQGTLVTVSSASTKGPSVFPLAP CSRSTSESTAALGCLVKDYFPEPVTVSWNSGALTSGVHTFPAVLQSSGLYSLSSVVTVPSSSLGTKT YTCNVDHKPSNTKVDKRVESKYGPPCPPCPAPPVAGPSVFLFPPKPKDTLMISRTPEVTCVVVDVS QEDPEVQFNWYVDGVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIAVEWESNGQPENNYKTTP PVLDSDGSFFLYSRLTVDKSRWQEGNVFSCSVMHEALHNHYTQKSLSLSLGK |
| 123 | Heavy chain REGN4018-G20 | EVQLVESGGGLVQPGRSLRLSCAASGFTFDDYSMHWVRQAPGKGLEWVSGISWNSGSIGYADSV KGRFTISRDNAKNSLYLQMNSLRAEDTALYYCAKYGSGYGKFYYYGMDVWGQGTTVTVSSAST KGPSVFPLAPCSRSTSESTAALGCLVKDYFPEPVTVSWNSGALTSGVHTFPAVLQSSGLYSLSSVV TVPSSSLGTKTYTCNVDHKPSNTKVDKRVESKYGPPCPPCPAPPVAGPSVFLFPPKPKDTLMISRTP EVTCVVVDVSQEDPEVQFNWYVDGVEVHNAKTKPREEQFNSTYRVVSVLTVLHQDWLNGKEY KCKVSNKGLPSSIEKTISKAKGQPREPQVYTLPPSQEEMTKNQVSLTCLVKGFYPSDIAVEWESNG QPENNYKTTPPVLDSDGSFFLYSRLTVDKSRWQEGNVFSCSVMHEALHNRFTQKSLSLSLGK |
| 124 | Light chain REGN4018-8767pL | DIQMTQSPSSLSASVGDRVTITCRASQSISTYLNWYQQKPGKAPKLLIYTASSLQSGVPSRFSGSGS GTDFTLTISSLQPEDFATYYCQQSYSTPPITFGQGTRLEIKRTVAAPSVFIFPPSDEQLKSGTASVVCL LNNFYPREAKVQWKVDNALQSGNSQESVTEQDSKDSTYSLSSTLTLSKADYEKHKVYACEVTH QGLSSPVTKSFNRGEC |
| 125 | sofituzumab HC | EVQLVESGGGLVQPGGSLRLSCAASGYSITNDYAWNWVRQAPGKGLEWVGYISYSGYTTYNPSL KSRFTISRDTSKNTLYLQMNSLRAEDTAVYYCARWTSGLDYWGQGTLVTVSS |
| 126 | sofituzumab LC | DIQMTQSPSSLSASVGDRVTITCKASDLIHNWLAWYQQKPGKAPKLLIYGATSLETGVPSRFSGSG SGTDFTLTISSLQPEDFATYYCQQYWTTPFTFGQGTKVEIK |
| 127 | ECT_TM huMuc16 | MALPVTALLLPLALLLHAARPGSRKFNTTERVLQGLLRPLFKNTSVGPLYSGCRLTLLRPEKDGE ATGVDAICTHRPDPTGPGLDREQLYLELSQLTHSITELGPYTLDRDSLYVNGFTHRSSVPTTSTGV VSEEPFTLNFTINNLRYMADMGQPGSLKFNITDNVMQHLLSPLFQRSSLGARYTGCRVIALRSVK NGAETRVDLLCTYLQPLSGPGLPIKQVFHELSQQTHGITRLGPYSLDKDSLYLNGYNEPGPDEPPT TPKPATTFLPPLSEATTAMGYHLKTLTLNFTISNLQYSPDMGKGSATFNSTEGVLQHLLRPLFQKSS MGPFYLGCQLISLRPEKDGAATGVDTTCTYHPDPVGPGLDIQQLYWELSQLTHGVTQLGFYVLD RDSLFINGYAPQNLSIRGEYQINFHIVNWNLSNPDPTSSEYITLLRDIQDKVTTLYKGSQLHDTFRF CLVTNLTMDSVLVTVKALFSSNLDPSLVEQVFLDKTLNASFHWLGSTYQLVDIHVTEMESSVYQP TSSSSTQHFYLNFTITNLPYSQDKAQPGTTNYQRNKRNIEDALNQLFRNSSIKSYFSDCQVSTFRS VPNRHHTGVDSLCNFSPLARRVDRVAIYEEFLRMTRNGTQLQNFTLDRSSVLVDGYSPNRNEPLT GNSDLPFWAVILIGLAGLLGVITCLICGVLVTTRRRKKEGEYNVQQQCPGYYQSHLDLEDLQ |
| 128 | ECT_TM RhMuc 16 | MALPVTALLLPLALLLHAARPGSRKFNTTERVLQGLLRPLFKNTSVGPLYSGCRLTLLRPEKDGE ATGVDVICTHRPDPTGPGLDREQLYLELSQLTHSITELGPYTLDNDSLFVNGFTHRSSVPTTSTGM VSEEPFTLNFTINNLRYMADMGQPGSLKFNITDNVMQHLLSPLFQRSSVGARYTGCRVIALRSVK NGAKTQVDILCTYLQPLSGPALPIKQVFHELSQQTHGITRLGPYFLDKDSLYLNGYNEPGPDEPPT TPKPATTFLPPLSEATTAMGYHLKTLTLNFTISNLQYSPDMGNGSAAFNSTERVLKHLLRALFQKS SMGPFYLGCQLISLRPEKNGAATGVDTTCTYHTDPVGPGLDIQQLYWELSQLTHGVTQLGFYVL DRDSLFINGYAPQNLSIRGKYQIHFHIVNWNFSNPDPTSSEYIALLRDIQDKVTTLYKGSQLHDTFR YCLVTNLTMDSMLVTVKALFSSDLDPSLVEQVFLDKTLNASSHWLGSTYQLVDIHVTEMEPSVY QPTKPTSSSSTQHFYLNFTITNLPYSQDISQPSTTNYQRNKRNIEDALNQLFRNSSIKSYFSDCQVS TFRSVPNSHHTGVDSLCNFSPLARRVDRVAIYEEFLRMTRNGSQLQNFTLDRSSVLVDGYSPNRN EPLTGNSDLPFWAIILICLAVLLGLITCLICGFLATTRWRKKEEYSFRQQCPGYYRSHLDLEDLQ |

## Claims

1. A bispecific antibody, comprising
a first antigen-binding region and a second antigen-binding region, wherein the first antigen-binding region specifically binds to a tumor-associated antigen, and/or the second antigen-binding region specifically binds to CD3,
wherein the second antigen-binding region is an scFv of an anti-CD3 antibody, and the scFv comprises a VH and a VL optionally linked via a linker comprising, for example, an amino acid sequence (G4S)n, wherein n = 1, 2, 3, 4, or 5, preferably n = 3 or 4, more preferably n = 3.

2. The bispecific antibody according to claim 1, wherein the VH contained in the scFv comprises 3 complementarity determining regions from a heavy chain variable region (HCDRs): HCDR1, HCDR2 and HCDR3, and the VL contained in the scFv comprises 3 complementarity determining regions from a light chain variable region (LCDRs): LCDR1, LCDR2 and LCDR3, wherein
(i) the HCDR1, the HCDR2 and the HCDR3 are selected from three complementarity determining regions HCDR1, HCDR2 and HCDR3 contained in a VH set forth in SEQ ID NO: 3, respectively, and the LCDR1, the LCDR2 and the LCDR3 are selected from three complementarity determining regions LCDR1, LCDR2 and LCDR3 contained in a VL set forth in SEQ ID NO: 4, respectively; or
(ii) the HCDR1 consists of an amino acid sequence set forth in SEQ ID NO: 5, the HCDR2 consists of an amino acid sequence set forth in SEQ ID NO: 6, the HCDR3 consists of an amino acid sequence set forth in SEQ ID NO: 7, the LCDR1 consists of an amino acid sequence set forth in SEQ ID NO: 8, the LCDR2 consists of an amino acid sequence set forth in SEQ ID NO: 9, and the LCDR3 consists of an amino acid sequence set forth in SEQ ID NO: 10.

3. The bispecific antibody according to claim 2, wherein
the VH comprises or consists of an amino acid sequence set forth in SEQ ID NO: 3, or an amino acid sequence having at least 90%, 91%, 92%, 93%, 94%, 95%, 96%, 97%, 98% or 99% identity thereto,
and/or the VL comprises or consists of an amino acid sequence set forth in SEQ ID NO: 4, or an amino acid sequence having at least 90%, 91%, 92%, 93%, 94%, 95%, 96%, 97%, 98% or 99% identity thereto.

4. The bispecific antibody according to any one of claims 1-3, wherein the first antigen-binding region is a Fab specifically binding to a first antigen.

5. The bispecific antibody according to claim 4, wherein the Fab comprises a CH1, wherein the CH1 is a CH1 from IgG1, IgG2, IgG3, or IgG4, preferably a CH1 from IgG1.

6. The bispecific antibody according to claim 5, wherein the CH1 (i) comprises or consists of an amino acid sequence having at least 85%, 90%, 91%, 92%, 93%, 94%, 95%, 96%, 97%, 98%, or 99% identity to an amino acid sequence set forth in SEQ ID NO: 44; or
(ii) comprises or consists of the amino acid sequence set forth in SEQ ID NO: 44.

7. The bispecific antibody according to any one of claims 1-6, wherein the bispecific antibody is an IgG-like bispecific antibody comprising an Fc dimer, wherein two Fc regions constituting the Fc dimer are identical or different.

8. The bispecific antibody according to claim 7, wherein one or both of the Fc regions comprise a mutation that reduces binding to an Fcy receptor, e.g., comprises one or more of an L234A/L235A mutation, a D265A mutation and a P329A mutation, for example, comprises an L234A/L235A mutation, a D265A mutation and a P329A mutation.

9. The bispecific antibody according to claim 8, wherein one or both of the Fc regions comprise or consist of an amino acid sequence set forth in SEQ ID NO: 46 or 49, or an amino acid sequence having at least 90%, 91%, 92%, 93%, 94%, 95%, 96%, 97%, 98%, 99% or more identity thereto.

10. The bispecific antibody according to any one of claims 7-9, wherein the two Fc regions are different, and preferably, a corresponding knob mutation and a corresponding hole mutation are introduced into a first monomeric Fc region and a second monomeric Fc region, respectively.

11. The bispecific antibody according to claim 10, wherein
d) one Fc-region polypeptide comprises a knob mutation T366W, and the other Fc-region polypeptide comprises hole mutations T366S, L368A and Y407V, or
e) one Fc-region polypeptide comprises knob mutations T366W and Y349C, and the other Fc-region polypeptide comprises hole mutations T366S, L368A, Y407V and S354C, or
f) one Fc-region polypeptide comprises knob mutations T366W and S354C, and the other Fc-region polypeptide comprises hole mutations T366S, L368A, Y407V and Y349C;
optionally, the Fc region further comprises a mutation that reduces binding to the Fcy receptor, e.g., one or more of an L234A/L235A mutation, a D265A mutation or a P329A mutation, for example, an L234A/L235A mutation, a D265A mutation and a P329A mutation.

12. The bispecific antibody according to any one of claims 7-11, wherein one or both of the Fc regions comprise a hinge region, e.g., EPKSS or EPKSC.

13. The bispecific antibody according to claim 12, wherein
(i) the first Fc region comprises a knob mutation, and
a) comprises or consists of an amino acid sequence set forth in SEQ ID NO: 43 or 89; or
b) comprises or consists of an amino acid sequence having at least 90% identity, e.g., 95%, 96%, 97%, 99% or more identity, to SEQ ID NO: 43 or 89 and comprising an L234A/L235A mutation, a D265A mutation, a P329A mutation, and a knob mutation (e.g., S354C and T366W); and/or
(ii) the second Fc region comprises a hole mutation, and
a) comprises or consists of an amino acid sequence set forth in SEQ ID NO: 30, 42 or 59; or
b) comprises or consists of an amino acid sequence having at least 90% identity, e.g., 95%, 96%, 97%, 99% or more identity, to SEQ ID NO: 30, 42 or 59 and comprising an L234A/L235A mutation, a D265A mutation, a P329A mutation, and a hole mutation (e.g., Y349C, T366S, L368A, and Y407V).

14. The bispecific antibody according to any one of claims 1-13, wherein the bispecific antibody is an IgG-like bispecific antibody comprising one Fab fragment specifically binding to the first antigen, one scFv specifically binding to a second antigen, and an Fc heterodimer, wherein
the Fab fragment comprises VH-CH1 and VL-CL, and the scFv comprises VH-linker-VL or VL-linker-VH;
the Fc region comprising the hole mutation constitutes a first heavy chain;
the C-terminus of the CH1 of the Fab fragment is fused to the N-terminus of the scFv fragment, and the C-terminus of the scFv fragment is fused to a CH2 or the hinge region of the Fc region comprising the knob mutation, thereby constituting a second heavy chain;
and
the VL-CL of the Fab fragment constitutes a light chain.

15. The bispecific antibody according to any one of claims 1-13, wherein the bispecific antibody is an IgG-like bispecific antibody comprising two Fab fragments specifically binding to the first antigen, one scFv specifically binding to a second antigen, and an Fc heterodimer, wherein
the Fab fragment comprises VH-CH1 and VL-CL, and the scFv comprises VH-linker-VL or VL-linker-VH;
the C-terminus of the CH1 of the first Fab fragment is fused to a CH2 or the hinge region of the Fc region comprising the hole mutation, thereby constituting a first heavy chain;
the C-terminus of the CH1 of the second Fab fragment is fused to the N-terminus of the scFv fragment, and the C-terminus of the scFv fragment is fused to a CH2 or the hinge region of the Fc region comprising the knob mutation, thereby constituting a second heavy chain;
and
the VL-CL of the first and second Fab fragments constitute two light chains, respectively;
wherein the first Fab and second Fab fragments are identical or different.

16. The bispecific antibody according to any one of claims 1-13, wherein the bispecific antibody is an IgG-like bispecific antibody comprising one Fab fragment specifically binding to the first antigen, one scFv specifically binding to a second antigen, and an Fc heterodimer, wherein
the Fab fragment comprises VH-CH1 and VL-CL, and the scFv comprises VH-linker-VL or VL-linker-VH;
the C-terminus of the CH1 of the Fab fragment is fused to a CH2 or the hinge region of the Fc region comprising the hole mutation, thereby constituting a first heavy chain;
the C-terminus of the scFv fragment is fused to a CH2 or the hinge region of the Fc region comprising the knob mutation, thereby constituting a second heavy chain;
and
the VL-CL of the Fab fragment constitutes a light chain.

17. The bispecific antibody according to any one of claims 1-13, wherein the bispecific antibody is an IgG-like bispecific antibody comprising two Fab fragments specifically binding to the first antigen, one scFv specifically binding to a second antigen, and an Fc heterodimer, wherein
the Fab fragment comprises VH-CH1 and VL-CL, and the scFv comprises VH-linker-VL or VL-linker-VH;
the C-terminus of the CH1 of the first Fab fragment is fused to the N-terminus of the VH of the second Fab fragment, and the C-terminus of the CH1 of the second Fab fragment is fused to a CH2 or the hinge region of the Fc region comprising the hole mutation, thereby constituting a first heavy chain;
the C-terminus of the scFv fragment is fused to a CH2 or the hinge region of the Fc region comprising the knob mutation, thereby constituting a second heavy chain; and
the VL-CL of the first and second Fab fragments constitute two light chains;
wherein the first Fab and second Fab fragments are identical or different.

18. The bispecific antibody according to any one of claims 1-18, wherein the first antigen is a tumor-associated antigen, for example, selected from GPRC5D or MUC16 (e.g., human GPRC5D or human MUC16).

19. The bispecific antibody according to claim 18, wherein the first antigen is human GPRC5D, and the first antigen-binding region comprises the 3 CDRs of the heavy chain variable region VH: HCDR1, HCDR2 and HCDR3, and the 3 CDRs of the light chain variable region VL: LCDR1, LCDR2 and LCDR3, wherein
(i) the HCDR1, the HCDR2 and the HCDR3 are selected from three complementarity determining regions HCDR1, HCDR2 and HCDR3 contained in a VH set forth in any one of SEQ ID NOs: 62, 38 and 40, respectively; and the LCDR1, the LCDR2 and the LCDR3 are three complementarity determining regions LCDR1, LCDR2 and LCDR3 contained in a VL set forth in SEQ ID NO: 63, respectively;
(ii) the HCDR1, the HCDR2 and the HCDR3 are selected from three complementarity determining regions HCDR1, HCDR2 and HCDR3 contained in a VH set forth in SEQ ID NO: 72, respectively; and the LCDR1, the LCDR2 and the LCDR3 are three complementarity determining regions LCDR1, LCDR2 and LCDR3 contained in a VL set forth in SEQ ID NO: 73, respectively; or
(iii) the HCDR1, the HCDR2 and the HCDR3 are selected from three complementarity determining regions HCDR1, HCDR2 and HCDR3 contained in a VH set forth in SEQ ID NO: 82, respectively; and the LCDR1, the LCDR2 and the LCDR3 are three complementarity determining regions LCDR1, LCDR2 and LCDR3 contained in a VL set forth in SEQ ID NO: 83, respectively.

20. The bispecific antibody according to claim 19, wherein the first antigen-binding region comprises
(i) an HCDR1 set forth in SEQ ID NO: 64, an HCDR2 set forth in SEQ ID NO: 65, 39, or 41, an HCDR3 set forth in SEQ ID NO: 66, an LCDR1 set forth in SEQ ID NO: 67, an LCDR2 set forth in SEQ ID NO: 68, and an LCDR3 set forth in SEQ ID NO: 69;
(ii) an HCDR1 set forth in SEQ ID NO: 74, an HCDR2 set forth in SEQ ID NO: 75, an HCDR3 set forth in SEQ ID NO: 76, an LCDR1 set forth in SEQ ID NO: 77, an LCDR2 set forth in SEQ ID NO: 78, and an LCDR3 set forth in SEQ ID NO: 79; or
(iii) an HCDR1 set forth in SEQ ID NO: 84, an HCDR2 set forth in SEQ ID NO: 85, an HCDR3 set forth in SEQ ID NO: 86, an LCDR1 set forth in SEQ ID NO: 87, an LCDR2 set forth in SEQ ID NO: 78, and an LCDR3 set forth in SEQ ID NO: 88.

21. The bispecific antibody according to claim 19 or 20, wherein the first antigen-binding region comprises a heavy chain variable region VH, wherein the heavy chain variable region
(i) comprises or consists of an amino acid sequence having at least 90%, 91%, 92%, 93%, 94%, 95%, 96%, 97%, 98%, or 99% identity to an amino acid sequence of SEQ ID NO: 62, 72, 82, 16, 23, 25, 27, 29, 38, or 40; or
(ii) comprises or consists of an amino acid sequence of SEQ ID NO: 62, 72, 82, 16, 23, 25, 27, 29, 38 or 40.

22. The bispecific antibody according to any one of claims 19-21, wherein the first antigen-binding region comprises a light chain variable region VL, wherein the light chain variable region
(i) comprises or consists of an amino acid sequence having at least 90%, 91%, 92%, 93%, 94%, 95%, 96%, 97%, 98%, or 99% identity to an amino acid sequence of SEQ ID NO: 63, 73, 83, 17, 19, or 21; or
(ii) comprises or consists of an amino acid sequence of SEQ ID NO: 63, 73, 83, 17, 19 or 21.

23. The bispecific antibody according to any one of claims 19-22, wherein the first antigen-binding region comprises a heavy chain variable region VH and a light chain variable region VL, wherein
(i) the heavy chain variable region comprises or consists of an amino acid sequence set forth in SEQ ID NO: 62, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 63, or an amino acid sequence having at least 90%, 91%, 92%, 93%, 94%, 95%, 96%, 97%, 98% or 99% identity thereto;
(ii) the heavy chain variable region comprises or consists of an amino acid sequence set forth in SEQ ID NO: 72, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 73, or an amino acid sequence having at least 90%, 91%, 92%, 93%, 94%, 95%, 96%, 97%, 98% or 99% identity thereto;
(iii) the heavy chain variable region comprises or consists of an amino acid sequence set forth in SEQ ID NO: 82, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 83, or an amino acid sequence having at least 90%, 91%, 92%, 93%, 94%, 95%, 96%, 97%, 98% or 99% identity thereto; or
(iv) the heavy chain variable region comprises or consists of an amino acid sequence set forth in SEQ ID NO: 16, 23, 25, 27, 29, 38 or 40, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 17, 19 or 21, or an amino acid sequence having at least 90%, 91%, 92%, 93%, 94%, 95%, 96%, 97%, 98% or 99% identity thereto.

24. The bispecific antibody according to any one of claims 19-23, wherein the first antigen-binding region comprises a heavy chain variable region VH and a light chain variable region VL, wherein the VH and the VL comprise or consist of amino acid sequences set forth below, respectively:
(vii). SEQ ID NO: 62 and SEQ ID NO: 63;
(viii). SEQ ID NO: 72 and SEQ ID NO: 73;
(ix). SEQ ID NO: 82 and SEQ ID NO: 83;
(x). SEQ ID NO: 16, 23, 25, 27, 29, 38, or 40 and SEQ ID NO: 21;
(xi). SEQ ID NO: 16 or 25 and SEQ ID NO: 17; or
(xii). SEQ ID NO: 16 or 27 and SEQ ID NO: 19.

25. The bispecific antibody according to claim 18, wherein the first antigen is human MUC16, and the first antigen-binding region comprises the 3 CDRs of the heavy chain variable region VH: HCDR1, HCDR2 and HCDR3, and the 3 CDRs of the light chain variable region VL: LCDR1, LCDR2 and LCDR3, wherein
the HCDR1, the HCDR2 and the HCDR3 are selected from three complementarity determining regions HCDR1, HCDR2 and HCDR3 contained in a VH set forth in SEQ ID NO: 92 or 113, respectively; and the LCDR1, the LCDR2 and the LCDR3 are three complementarity determining regions LCDR1, LCDR2 and LCDR contained in a VL set forth in SEQ ID NO: 96, 100, 102 or 114, respectively.

26. The bispecific antibody according to claim 25, wherein the first antigen-binding region comprises
an HCDR1 set forth in SEQ ID NO: 93, an HCDR2 set forth in SEQ ID NO: 94, an HCDR3 set forth in SEQ ID NO: 95, an LCDR1 set forth in SEQ ID NO: 97, 101, or 103, an LCDR2 set forth in SEQ ID NO: 98, and an LCDR3 set forth in SEQ ID NO: 99.

27. The bispecific antibody according to claim 25 or 26, wherein the first antigen-binding region comprises a heavy chain variable region VH, wherein the heavy chain variable region
comprises or consists of an amino acid sequence having at least 90%, 91%, 92%, 93%, 94%, 95%, 96%, 97%, 98% or 99% identity to an amino acid sequence of SEQ ID NO: 92 or 113, or comprises or consists of an amino acid sequence of SEQ ID NO: 92 or 113.

28. The bispecific antibody according to any one of claims 25-27, wherein the first antigen-binding region comprises a light chain variable region VL, wherein the light chain variable region comprises or consists of an amino acid sequence having at least 90%, 91%, 92%, 93%, 94%, 95%, 96%, 97%, 98%, or 99% identity to an amino acid sequence of SEQ ID NO: 96, 100, 102, or 114, or comprises or consists of an amino acid sequence of SEQ ID NO: 96, 100, 102, or 114.

29. The bispecific antibody according to any one of claims 25-28, wherein the first antigen-binding region comprises a heavy chain variable region VH and a light chain variable region VL, wherein
the heavy chain variable region comprises or consists of an amino acid sequence set forth in SEQ ID NO: 92 or 113, or an amino acid sequence having at least 90%, 91%, 92%, 93%, 94%, 95%, 96%, 97%, 98% or 99% identity thereto, and the light chain variable region comprises or consists of an amino acid sequence set forth in SEQ ID NO: 96, 100, 102 or 114, or an amino acid sequence having at least 90%, 91%, 92%, 93%, 94%, 95%, 96%, 97%, 98% or 99% identity thereto.

30. The bispecific antibody according to any one of claims 25-29, wherein
the first antigen-binding region comprises a heavy chain variable region VH and a light chain variable region VL, wherein the VH comprises or consists of the amino acid sequence set forth in SEQ ID NO: 92, and the VL comprises or consists of the amino acid sequence set forth in SEQ ID NO: 96, 100 or 102; or
the first antigen-binding region comprises a heavy chain variable region VH and a light chain variable region VL, wherein the VH comprises or consists of the amino acid sequence set forth in SEQ ID NO: 113, and the VL comprises or consists of the amino acid sequence set forth in SEQ ID NO: 114.

31. A nucleic acid molecule, encoding any one of the chains in the bispecific antibody according to any one of claims 1-30, or consisting of a nucleic acid sequence.

32. An expression vector, comprising the nucleic acid molecule according to claim 31, wherein preferably, the expression vector is pCDNA, e.g., pCDNA3.1.

33. A host cell, comprising the nucleic acid molecule according to claim 31 or the expression vector according to claim 32, wherein preferably, the host cell is prokaryotic or eukaryotic, e.g., a 293 cell or a CHO cell, such as a 293F cell or a 293T cell or a CHO-S cell.

34. A method for preparing the bispecific antibody according to any one of claims 1-30, wherein the method comprises culturing the host cell of the nucleic acid molecule according to claim 31 or the expression vector according to claim 32 under a condition suitable for expressing the chains of the antibody, and optionally recovering the antibody from the host cell (or the host cell medium).

35. An immunoconjugate, comprising the bispecific antibody according to any one of claims 1-30.

36. A pharmaceutical composition or a medicament or a formulation, comprising the bispecific antibody according to any one of claims 1-30 or the immunoconjugate according to claim 35, and optionally a pharmaceutical supplementary material.

37. A pharmaceutical combination product, comprising the bispecific antibody according to any one of claims 1-30 or the immunoconjugate according to claim 35, and one or more additional therapeutic agents (e.g., a chemotherapeutic agent, a cytokine, a cytotoxic agent, an additional antibody, a small molecule drug, or an immunomodulatory agent).

38. A method for preventing or treating a cancer in a subject, comprising administering to the subject an effective amount of the bispecific antibody according to any one of claims 1-30, or the immunoconjugate according to claim 35, or the pharmaceutical composition or the formulation according to claim 36, or the pharmaceutical combination product according to claim 37.

39. The method according to claim 38, wherein tumor cells of the cancer have an elevated protein level and/or nucleic acid level (e.g., elevated expression) of a first antigen.

40. The method according to claim 38, wherein the first antigen is selected from GPRC5D or MUC16.

41. The method according to any one of claims 38-40, wherein the cancer is a solid tumor or a hematological tumor, such as ovarian carcinoma (e.g., serous ovarian carcinoma, such as ovarian serous cystadenocarcinoma, ovarian serous adenocarcinoma, ovarian mucinous adenocarcinoma, endometrioid adenocarcinoma, or ovarian clear cell adenocarcinoma), myeloma, colon cancer, rectal cancer, or colorectal cancer.

42. The method according to any one of claims 38-41, wherein the method further comprises administering in combination with an additional therapy such as a therapeutic modality (e.g., surgical therapy or radiotherapy) and/or an additional therapeutic agent (e.g., a chemotherapeutic agent, a cytokine, a cytotoxic agent, an additional antibody, a small molecule drug, or an immunomodulatory agent).

43. A method for detecting the presence of a first antigen in a biological sample, comprising
(i) contacting the biological sample with the bispecific antibody according to any one of claims 1-30 under a condition that allows the bispecific antibody to bind to the first antigen, and
(ii) detecting whether a complex is formed by the antibody or the bispecific antibody and the first antigen,
wherein the formation of the complex indicates the presence of the first antigen.
